# EUROPEAN PATENT APPLICATION

(11) **EP 4 644 393 A1**
(43) Date of publication of application: **05.11.2025**
(21) Application number: 23910637.0
(22) Date of filing: 26.12.2023
(51) Int. Cl.: C07D 487/04, A61K 31/519, A61P 35/00

(54) **WRN INHIBITOR**

(30) Priority: 30.12.2022 CN 202211693494; 25.04.2023 CN 202310452664; 29.05.2023 CN 202310613550; 20.06.2023 CN 202310734540; 02.08.2023 CN 202310965757; 25.08.2023 CN 202311081481; 08.12.2023 CN 202311684693
(71) Applicant: Suzhou Puhe Biopharma Co., Ltd., Suzhou, Jiangsu 215124 (CN)
(72) Inventor: LIU, Bin, Suzhou, Jiangsu 215124 (CN); GAO, Feng, Suzhou, Jiangsu 215124 (CN); GUO, Yongqi, Suzhou, Jiangsu 215124 (CN); WU, Yongyong, Suzhou, Jiangsu 215124 (CN); JING, Liandong, Suzhou, Jiangsu 215124 (CN); LI, Zhizhong, Suzhou, Jiangsu 215124 (CN); WU, Zhuo, Suzhou, Jiangsu 215124 (CN)
(74) Representative: Cabinet Becker et Associés
(86) International application number: PCT/CN2023/142038
(87) International publication number: WO 2024/140714

(57) **Abstract**

Provided is a compound of formula (I) serving as a WRN inhibitor, as well as a compound or a pharmaceutically acceptable salt, isotopic variant, tautomer, or stereoisomer thereof. Further provided are a pharmaceutical composition comprising said compound and a use thereof in the treatment of a cancer.

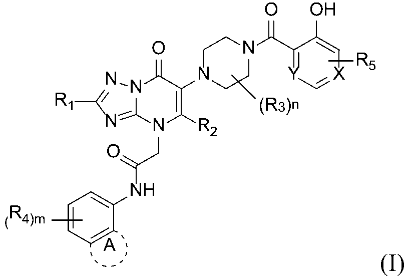

## Description

**This application claims the priorities of:**
Chinese Application Number: 202211693494.7 filed on December 30, 2022;
Chinese Application Number: 202310452664.0 filed on April 25, 2023;
Chinese Application Number: 202310613550.X filed on May 29, 2023;
Chinese Application Number: 202310734540.1 filed on June 20, 2023;
Chinese Application Number: 202310965757.3 filed on August 02, 2023;
Chinese Application Number: 202311081481.9 filed on August 25, 2023; and
Chinese Application Number: 202311684693.6 filed on December 08, 2023,
which are incorporated herein by reference in their entirety.

### TECHNICAL FIELD

The present disclosure belongs to the field of medicines, and in particular to WRN inhibitors.

### BACKGROUND

Abnormal DNA mismatch repairs (MMRs) may lead to high mutation (deletions or insertions) in the DNA nucleotide repeat region, which is called microsatellite instability (MSI). Microsatellite Instability-High (MSI-H) may induce the occurrence of tumors, including colorectal cancer, gastric cancer, endometrial cancer, ovarian cancer, and so on (Nature, 2019, 568, 551-556), among which colorectal cancer (15%) and gastric cancer (22%) have the highest mutation rates. Although the PD-1/PD-L1 immunotherapy produces very good therapeutic effects in multiple tumors at present, e.g., pembrolizumab has greatly improved the median progression-free survival (PFS) in patients with MSI-H advanced colorectal cancer as compared with chemotherapy and approved by the FDA as a first-line therapy (N. Engl. J. Med. 2020, 383, 2207-2218), there are still many MSI-H tumor patients incapable of benefiting therefrom. In addition, the ASCO conference reported in 2022 that in the Phase 2 clinical CheckMate 142 (NCT02060188) trial, in the treatment of patients having metastatic colorectal cancer with dual immunotherapy PD -1+CTLA-4 (nivolumab+ipilimumab), more than a half of the tumor patients would relapse after 4 years of follow-up, whether they are in the first-line treatment or has underwent a second-line treatment. Thus, there is an urgent need to develop new treatment means.

Synthetic lethality means that in tumor cells, the inactivation of either gene has no significant effect on the survival of tumor cells, but the co-inactivation of both genes may lead to the death of tumor cells (Nat. Rev. Drug Discov. 2020, 19(1): 23-38, Cancer Discov. 2021, 11(7):1626-1635). Synthetic lethal targeted drugs may generally produce a good therapeutic safety window, while also increasing the development accessibility of certain targets with high mutation rates and difficulty in druggability. Currently, the most successful cases of the synthetic lethality are PARP1/2 inhibitors such as Olaparib, Rucaparib, and Niraparib. These drugs have achieved excellent therapeutic effects in the treatment of BRCA1/2 mutant ovarian cancer, breast cancer, etc., and have been approved for marketing (Nat. Rev. Drug Discov. 2020, 19 (10):711-736; and Nat. Rev. Clin. Oncol. 2020, 17(3):136-137). In 2019, Adam J. Bass published articles in Nature in succession, proving that Werner helicase (WRN) is a synthetic lethal target for MSI-H tumors (Nature, 2019, 568, 551-556; and Nature, 2019, 586, 292-298). Either the knockout of the whole WRN or the K557M mutation of the WRN's helicase (with loss of helicase function) induces the occurrence of cycle arrest and apoptosis in MSI-H tumor cells. Not only that, Mathew J. Garnett et al. also found that even if the tumor cells become resistant in the MSI-H patients who have underwent chemotherapy and immunotherapy, it is still possible to further inhibit the tumor growth by the inhibition of WRN (Cancer Discov. 2021, 11, 1923-1937).

Despite the progress in WRN research, there is still no WRN inhibitor to treat MSI-H related cancers in the clinic. Novel WRN inhibitors provided in the present disclosure are expected to meet the clinical requirements.

### SUMMARY

In an aspect, the present disclosure provides a compound of Formula (I), or a pharmaceutically acceptable salt, isotopic variant, tautomer, or stereoisomer thereof, or a mixture thereof: 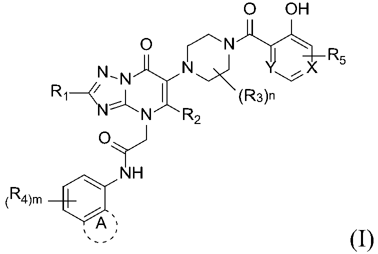 wherein,
X and Y are each independently selected from CH and N, and at least one of X and Y is N atom;
Ring A is absent or selected from C₃₋₁₀ cycloalkyl, 5-10 membered heteroaryl, 5-10 membered heterocyclyl, and C₆₋₁₀ aryl;
R₁ is selected from 5-12 membered heteroaryl and 5-12 membered heterocyclyl, and the R₁ is optionally substituted with 1, 2, 3, 4 or 5 Rₓ, provided that R₁ is not pyridyl or 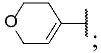
Rₓ is selected from H, D, halogen, NH₂, CN, OH, C₁₋₆ alkyl, C₁₋₆ alkoxy, C₁₋₆ haloalkyl, -C(O)Rₐ, -C(O)ORₐ, -OC(O)Rₐ, -C(O)NH-Rₐ, -NHC(O)-Rₐ, -(CH₂)ₚ-ORₐ, -(CH₂)ₚ-C(O)Rₐ, - P(O)-(Rₐ)₂, and -S(O)₂-Rₐ, wherein Rₐ is selected from H, C₁₋₆ alkyl, C₁₋₆ haloalkyl, and C₃₋₆ cycloalkyl, and p is selected from 0, 1, 2, 3, and 4; or two Rₓ on the same atom are taken together to form oxo or thio;
R₂ is selected from H, D, halogen, C₁₋₆ alkyl, C₁₋₆ haloalkyl, C₁₋₆ alkoxy, C₁₋₆ alkylthio, and C₃₋₆ cycloalkyl;
R₃ is selected from H, D, halogen, NH₂, CN, OH, C₁₋₆ alkyl, C₁₋₆ haloalkyl, C₁₋₆ alkoxy, and C₃₋₆ cycloalkyl, or R₃ on two different carbon atoms are connected to form a bridged ring, or two R₃ on the same carbon atom are connected to form C₃₋₁₀ cycloalkyl, 5-10 membered heteroaryl, C₃₋₁₀ cycloalkyl or 3-10 membered heterocyclyl;
R₄ is selected from H, D, halogen, NH₂, CN, OH, SF₅, SCF₃, C₁₋₆ alkyl, C₁₋₆ haloalkyl, C₁₋₆ alkoxy, C₁₋₆ haloalkoxy, C₁₋₆ alkylthio, C₃₋₁₀ cycloalkyl, 3-10 membered heterocyclyl, 5-10 membered heteroaryl, and C₆₋₁₀ aryl;
R₅ is selected from H, D, halogen, NH₂, CN, OH, C₁₋₆ alkyl, C₁₋₆ deuterated alkyl, C₁₋₆ haloalkyl, C₁₋₆ alkoxy, C₁₋₆ alkylthio, and C₃₋₆ cycloalkyl;
m is selected from 0, 1, 2, 3, 4, and 5;
n is selected from 0, 1, 2, 3, 4, 5, 6, 7, and 8;
R₁ - R₅ may be optionally deuterated, up to fully deuterated.

In another aspect, the present disclosure provides a pharmaceutical composition comprising the compound of the present disclosure, and optionally a pharmaceutically acceptable excipient.

In another aspect, the present disclosure provides a pharmaceutical composition comprising the compound of the present disclosure and a pharmaceutically acceptable excipient, and further comprising an additional therapeutic agent.

In another aspect, the present disclosure provides use of the compound of the present disclosure in the manufacture of a medicament for treating and/or preventing a WRN-mediated disease.

In another aspect, the present disclosure provides a method for treating and/or preventing a WRN-mediated disease in a subject, comprising administering to the subject the compound or composition of the present disclosure.

In another aspect, the present disclosure provides the compound or composition of the present disclosure for use in treating and/or preventing of a WRN-mediated disease.

In a specific embodiment, the diseases treated by the present disclosure include cancers selected from the group consisting of acoustic neuroma, adenocarcinoma, adrenal cancer, anal cancer, angiosarcoma (e.g., lymphangiosarcoma, lymphangioendothelioma, hemangioma), appendix cancer, benign monoclonal gamma disease, bile duct cancer, bladder cancer, brain cancer (e.g., meningioma, glioma, e.g., astrocytoma, oligodendroglioma, medulloblastoma), bronchial cancer, carcinoid tumor cervical cancer (e.g., cervical adenocarcinoma), choriocarcinoma, chordoma, craniopharyngioma, colorectal cancer (e.g., colon cancer, rectal cancer, large intestine adenocarcinoma), epithelial cancer, ependymoma, endothelial sarcoma (e.g., Kaposi's sarcoma, multiple idiopathic hemorrhagic sarcoma), endometrial cancer (e.g., uterine cancer, uterine sarcoma), esophageal cancer (e.g., esophageal adenocarcinoma, Barrett's adenocarcinoma), Ewing's sarcoma, eye cancer (e.g., intraocular melanoma, retinoblastoma), hypereosinophilia, gallbladder cancer, stomach cancer (e.g., gastric adenocarcinoma), gastrointestinal stromal tumor (GIST), head and neck cancer (e.g., head and neck squamous cell carcinoma, oral cancer (e.g., oral squamous cell carcinoma, laryngeal cancer (e.g., laryngeal cancer, pharyngeal cancer, nasopharyngeal cancer, oropharyngeal cancer))), hematopoietic system cancer (e.g., leukemia, such as acute lymphoblastic leukemia (ALL) (e.g., B-cell ALL, T-cell ALL), acute myeloid leukemia (AML) (e.g., B-cell AML, T-cell AML), chronic myeloid leukemia (CML) (e.g., B-cell CML, T-cell CML), chronic lymphocytic leukemia (CLL) (e.g., B-cell CLL, T-cell CLL), follicular lymphoma, chronic lymphocytic leukemia/small lymphocytic lymphoma (CLL/SLL), marginal zone B-cell lymphoma (e.g., mucosa-associated lymphoid tissue (MALT) lymphoma, lymph node marginal zone B-cell lymphoma, splenic marginal zone B-cell lymphoma), primary mediastinal B-cell lymphoma, Burkitt's lymphoma, lymphoplasmacytic lymphoma, hairy cell leukemia (HCL), immunoblastic large cell lymphoma, precursor B lymphoblastic lymphoma, and primary central nervous system (CNS) lymphomas; and T-cell non-Hodgkin's lymphomas, such as precursor T-lymphoblastic lymphoma/leukemia, peripheral T-cell lymphomas (e.g., cutaneous T-cell lymphomas (e.g., mycosis fungoides, Sezary syndrome), angioimmunoblastic T-cell lymphoma, extranodal natural killer T-cell lymphoma, enteropathy-associated T-cell lymphoma, subcutaneous panniculitis-like t-cell lymphoma, anaplastic large cell lymphoma); a mixture of one or more of the above leukemias/lymphomas; multiple myeloma (MM)), heavy chain disease (e.g., α-chain diseases, γ-chain diseases, µ-chain diseases), hemangioblastoma, inflammatory myofibroblast tumor immune cell amyloidosis, renal cancer (e.g., Wilms tumor or renal cell carcinoma), liver cancer (e.g., hepatocellular carcinoma, malignant liver cell carcinoma), lung cancer (e.g., bronchogenic carcinoma, small cell lung cancer (SCLC), non-small cell lung cancer (NSCLC), lung adenocarcinoma), leiomyosarcoma (LMS), mastocytosis (e.g., systemic mastocytosis), myelodysplastic syndrome (MDS), mesothelioma, myeloproliferative diseases (MPD) (e.g., polycythemia vera (PV), essential thrombocythemia (ET), idiopathic extramedullary metaplasia (AMM), chronic idiopathic myelofibrosis, chronic myeloid leukemia (CML), chronic neutrophilic leukemia (CNL), hypereosinophilic syndrome (HES), neuroblastoma, neurofibromas (e.g., neurofibromatosis type 1 or type 2, neurinomastosis), neuroendocrine cancer (e.g., gastroenteropancreatic neuroendocrine tumor (GEP-NET), carcinoid tumor), osteosarcoma, ovarian cancer (e.g., cystadenocarcinoma, ovarian embryonal carcinoma, ovarian adenocarcinoma), papillary adenocarcinoma, and penile cancer.

Other objects and advantages of the present disclosure will be apparent to those skilled in the art from the following detailed description, examples and claims.

### Definition

### Chemical Definition

The definitions of specific functional groups and chemical terms are described in more detail below.

When a numerical range is listed, each value and subrange within the stated range is intended to be included. For example, "C₁₋₆ alkyl" includes C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

"C₁₋₆ alkyl" refers to a linear or branched saturated hydrocarbon group having 1 to 6 carbon atoms. In some embodiments, C₁₋₄ alkyl and C₁₋₂ alkyl are alternative. Examples of C₁₋₆ alkyl include: methyl (C₁), ethyl (C₂), n-propyl (C₃), isopropyl (C₃), n-butyl (C₄), tert-butyl (C₄), sec-butyl (C₄), isobutyl (C₄), n-pentyl (C₅), 3-pentyl (C₅), pentyl (C₅), neopentyl (C₅), 3-methyl-2-butyl (C₅), tert-pentyl (C₅) and n-hexyl (C₆). The term "C₁₋₆ alkyl" also includes a heteroalkyl group in which one or more (e.g., 1, 2, 3 or 4) carbon atoms are replaced by heteroatom(s) (e.g., oxygen, sulfur, nitrogen, boron, silicon, phosphorus). The alkyl group may be optionally substituted with one or more substituents, e.g., 1 to 5 substituents, 1 to 3 substituents, or 1 substituent. Conventional alkyl abbreviations include: Me (-CH₃), Et (-CH₂CH₃), iPr (-CH(CH₃)₂), nPr (-CH₂CH₂CH₃), n-Bu (-CH₂CH₂CH₂CH₃), or i-Bu (-CH₂CH(CH₃)₂).

"C₂₋₆ alkenyl" refers to a linear or branched hydrocarbon group having 2 to 6 carbon atoms and at least one carbon-carbon double bond. In some embodiments, C₂₋₄ alkenyl is alternative. Examples of C₂₋₆ alkenyl include: vinyl (C₂), 1-propenyl (C₃), 2-propenyl (C₃), 1-butenyl (C₄), 2-butenyl (C₄), butadienyl (C₄), pentenyl (C₅), pentadienyl (C₅), hexenyl (C₆), and the like. The term "C₂₋₆ alkenyl" also includes a heteroalkenyl group in which one or more (e.g., 1, 2, 3 or 4) carbon atoms are replaced by heteroatom(s) (e.g., oxygen, sulfur, nitrogen, boron, silicon, phosphorus). The alkenyl group may be optionally substituted with one or more substituents, e.g., by 1 to 5 substituents, 1 to 3 substituents or 1 substituent.

"C₂₋₆ alkynyl " refers to a linear or branched hydrocarbon group having 2 to 6 carbon atoms, at least one carbon-carbon triple bond, and optionally one or more carbon-carbon double bonds. In some embodiments, C₂₋₄ alkynyl is alternative. Examples of C₂₋₆ alkynyl include, but are not limited to, ethynyl (C₂), 1-propynyl (C₃), 2-propynyl (C₃), 1-butynyl (C₄), 2-butynyl (C₄), pentynyl (C₅), hexynyl (C₆), and the like. The term "C₂₋₆ alkynyl" also includes a heteroalkynyl group in which one or more (e.g., 1, 2, 3 or 4) carbon atoms are replaced by heteroatom(s) (e.g., oxygen, sulfur, nitrogen, boron, silicon, phosphorus). The alkynyl groups may be optionally substituted with one or more substituents, e.g., by 1 to 5 substituents, 1 to 3 substituents, or 1 substituent.

"C₁₋₆ alkylene" refers to a divalent group formed by removing another hydrogen from a C₁₋₆ alkyl, and may be substituted or unsubstituted. In some embodiments, C₁₋₄ alkylene, C ₂₋₄ alkylene, and C₁₋₃ alkylene are alternative. The unsubstituted alkylene includes, but is not limited to, methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), and the like. Exemplary substituted alkylene groups, e.g., alkylene groups substituted with one or more alkyl (methyl) groups, include, but are not limited to, substituted methylene groups (-CH(CH₃)-, -C(CH₃)₂-), substituted ethylene (-CH(CH₃)CH₂-, - CH₂CH(CH₃)-, -C(CH₃)₂CH₂-, -CH₂C(CH₃)₂-), substituted propylene groups (-CH(CH₃)CH₂CH₂-, -CH₂CH(CH₃)CH₂-, -CH₂CH₂CH(CH₃)-, -C(CH₃)₂CH₂CH₂-, - CH₂C(CH₃)₂CH₂-), and the like.

"C alkenylene" refers to a divalent group formed by removing another hydrogen of a C₂₋₆alkenyl group, and may be substituted or unsubstituted. In some embodiments, C₂₋₄ alkenylene is particularly alternative. Exemplary unsubstituted alkenylene groups include, but are not limited to, vinylene (-CH=CH-) and propenylene (e.g., -CH=CHCH₂-, -CH₂-CH=CH-). Exemplary substituted alkenylene groups, e.g., alkenylene groups substituted with one or more alkyl(methyl) groups, include, but are not limited to, substituted ethylene groups (-C(CH₃)=CH-, -CH=C(CH₃)-), substituted propenylene groups (-C(CH₃)=CHCH₂-, - CH=C(CH₃)CH₂-, -CH=CHCH(CH₃)-, -CH=CHC(CH₃)₂-, -CH(CH₃)-CH=CH-, -C(CH₃)₂-CH=CH-, -CH₂-C(CH₃)=CH-, -CH₂-CH=C(CH₃)-), and the like.

"C₂₋₆ alkynylene" refers to a divalent group formed by removing another hydrogen of a C₂₋₆ alkynyl group, and may be substituted or unsubstituted. In some embodiments, C₂₋₄ alkynylene is particularly alternative. Exemplary alkynylene groups include, but are not limited to, ethynylene (-C=C-), substituted or unsubstituted propynylene (-C≡CCH₂-), and the like.

"Halo" or "halogen" refers to fluorine (F), chlorine (Cl), bromine (Br) and iodine (I).

Therefore, "C₁₋₆ haloalkyl" refers to the above-mentioned "C₁₋₆ alkyl " substituted with one or more halogen groups. In some embodiments, C₁₋₄ haloalkyl is particularly alternative, more alternatively C₁₋₂ haloalkyl. Exemplary haloalkyl groups include, but are not limited to: -CF₃, -CH₂F, -CHF₂, -CHFCH₂F, -CH₂CHF₂, -CF₂CF₃, -CCl₃, -CH₂Cl, -CHCl₂, 2,2,2-trifluoro-1,1-dimethyl-ethyl, and the like. The haloalkyl group may be substituted at any available attachment point, e.g., 1 to 5 substituents, 1 to 3 substituents, or 1 substituent.

"C₁₋₆ alkoxy" refers to a -OR group in which R is the C₁₋₆ alkyl group as defined above. C₁₋₄ alkoxy is alternative.

"C₁₋₆ haloalkoxy" refers to the "C₁₋₆ alkoxy" group which is substituted with one or more halogen groups. In some embodiments, C₁₋₄ haloalkoxyalkyl is particularly alternative, and C₁₋₂ haloalkoxyalkyl is more alternative.

"C₃₋₁₀ cycloalkyl" refers to a non-aromatic cyclic hydrocarbon group having 3 to 10 ring carbon atoms and zero heteroatoms. In some embodiments, C₄₋₁₀ cycloalkyl, C₅₋₁₀ cycloalkyl, C₄₋₇ cycloalkyl, C₃₋₇ cycloalkyl, C₃₋₆ cycloalkyl, C₃₋₅ cycloalkyl and C₃₋₄ cycloalkyl are particularly alternative, and C₅₋₆ cycloalkyl is more alternative. The cycloalkyl also includes a ring system in which the above cycloalkyl ring is fused to one or more aryl or heteroaryl groups, wherein the point of attachment is on the cycloalkyl ring, and in such a case, the number of carbons continues to represent the number of carbons in the cycloalkyl system. Exemplary cycloalkyl groups include, but are not limited to, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇), cycloheptadienyl (C₇), cycloheptatrienyl (C₇), etc. The cycloalkyl group may be optionally substituted with one or more substituents, e.g., 1 to 5 substituents, 1 to 3 substituents, or 1 substituent.

"3-12 membered heterocyclyl " refers to a group of 3-12 membered non-aromatic ring system having ring carbon atoms and 1 to 5 ring heteroatoms, in which each heteroatom is independently selected from nitrogen, oxygen, sulfur, boron, phosphorus, and silicon. In the heterocyclyl groups containing one or more nitrogen atoms, the point of attachment may be a carbon or nitrogen atom as long as the valence permits. In some embodiments, a 3-10 membered heterocyclyl is alternative, which is a 3-10 membered non-aromatic ring system having ring carbon atoms and 1 to 3 ring heteroatoms; in some embodiments, a 4-10 membered heterocyclyl is alternative, which is a 4-10 membered non-aromatic ring system having ring carbon atoms and 1 to 4 ring heteroatoms; in some embodiments, a 5-10 membered heterocyclyl is alternative, which is a 5-10 membered non-aromatic ring system having ring carbon atoms and 1 to 5 ring heteroatoms; in some embodiments, a 5-8 membered heterocyclyl is alternative, which is a 5-8 membered non-aromatic ring system having ring carbon atoms and 1 to 5 ring heteroatoms; in some embodiments, a 3-7 membered heterocyclyl is alternative, which is a 3-7 membered non-aromatic ring system having ring carbon atoms and 1 to 4 ring heteroatoms; a 3-6 membered heterocyclyl is alternative, which is a 3-6 membered non-aromatic ring system having ring carbon atoms and 1 to 3 ring heteroatoms; a 4-7 membered heterocyclyl is alternative, which is a 4-7 membered non-aromatic ring system having ring carbon atoms and 1 to 3 ring heteroatoms; a 4-6 membered heterocyclyl is alternative, which is a 4-6 membered non-aromatic ring system having ring carbon atoms and 1 to 3 ring heteroatoms; a 5-6 membered heterocyclyl is more alternative, which is a 5-6 membered non-aromatic ring system having ring carbon atoms and 1 to 3 ring heteroatoms; and a 3-5 membered heterocyclyl is more alternative, which is a 3-5 membered non-aromatic ring system having ring carbon atoms and 1 to 3 ring heteroatoms. The heterocyclyl also includes a bicyclic heterocyclyl, which refers to a ring system in which the above heterocyclyl ring is fused to one or more cycloalkyl groups, wherein the point of attachment is on the cycloalkyl ring, or a ring system in which the above heterocyclyl ring is fused to one or more aryl or heteroaryl groups, wherein the point of attachment is on the heterocyclyl ring; and in such a case, the number of ring members continues to represent the number of ring members in the heterocyclyl ring system. Exemplary 3 membered heterocyclyl groups containing one heteroatom include, but are not limited to, aziridinyl, oxiranyl, and thiorenyl. Exemplary 4 membered heterocyclyl groups containing one heteroatom include, but are not limited to, azetidinyl, oxetanyl, and thietanyl. Exemplary 5 membered heterocyclyl groups containing one heteroatom include, but are not limited to, tetrahydrofuranyl, dihydrofuranyl, tetrahydrothienyl, dihydrothienyl, pyrrolidinyl, dihydropyrrolyl, and pyrrolyl-2,5-dione. Exemplary 5 membered heterocyclic groups containing two heteroatoms include, but are not limited to, dioxolanyl, oxasulfuranyl, disulfuranyl, and oxazolidin-2-one. Exemplary 5 membered heterocyclic groups containing three heteroatoms include, but are not limited to, triazolinyl, oxadiazolinyl, and thiadiazolinyl. Exemplary 6 membered heterocyclic groups containing one heteroatom include, but are not limited to, piperidinyl, tetrahydropyranyl, dihydropyridinyl, and thianyl. Exemplary 6 membered heterocyclic groups containing two heteroatoms include, but are not limited to, piperazinyl, morpholinyl, dithianyl, and dioxanyl. Exemplary 6 membered heterocyclic groups containing three heteroatoms include, but are not limited to, hexahydrotriazinyl. Exemplary 7 membered heterocyclic groups containing one heteroatom include, but are not limited to, azepanyl, oxepanyl, and thianyl. Exemplary 5 membered heterocyclyl groups fused to a C₆ aryl ring (also referred to herein as 5,6-bicyclic heterocyclyl groups) include, but are not limited to, indolinyl, isoindolinyl, dihydrobenzofuranyl, dihydrobenzothienyl, benzoxazolinonyl, and the like. Exemplary 6 membered heterocyclyl groups fused to a C₆ aryl ring (also referred to herein as 6,6-bicyclic heterocyclyl groups) include, but are not limited to, tetrahydroquinolinyl, tetrahydroisoquinolinyl, tetrahydrobenzopyranyl, tetrahydropyranopyridinyl, and the like. The heterocyclyl group may be optionally substituted with one or more substituents, e.g., 1 to 5 substituents, 1 to 3 substituents, or 1 substituent.

" C₆₋₁₀ aryl" refers to a monocyclic or polycyclic (e.g., bicyclic) 4n+2 aromatic ring system (e.g., having 6 or 10 π electrons shared in a cyclic arrangement) having 6-10 ring carbon atoms and zero heteroatoms. In some embodiments, the aryl has six ring carbon atoms ("C₆ aryl"; e.g., phenyl). In some embodiments, the aryl has ten ring carbon atoms ("C₁₀ aryl"; e.g., naphthyl, e.g., 1- naphthyl and 2- naphthyl). The aryl also includes a ring system in which the above aryl ring is fused to one or more cycloalkyl or heterocyclic groups, and the point of attachment is on the aryl ring, and in such case, the number of carbon atoms continues to represent the number of carbon atoms in the aryl ring system. The aryl group may be optionally substituted with one or more substituents, e.g., 1 to 5 substituents, 1 to 3 substituents, or 1 substituent.

"5-14 membered heteroaryl" refers to a group of a 5-14 membered monocyclic or bicyclic 4n+2 aromatic ring system (e.g., having 6, 10, or 14 π electrons shared in a cyclic arrangement) having ring carbon atoms and 1-4 ring heteroatoms, wherein each heteroatom is independently selected from nitrogen, oxygen, and sulfur. In the heteroaryl groups containing one or more nitrogen atoms, the point of attachment may be a carbon or nitrogen atom as long as the valence permits. The heteroaryl bicyclic ring systems may include one or more heteroatoms in either or both of the rings. The heteroaryl also includes a bicyclic heteroaryl group, which refers to a ring system in which the above-mentioned heteroaryl ring is fused to one or more cycloalkyl or heterocyclic groups, and the point of attachment is on the heteroaryl ring, , and in such case, the number of carbon atoms continues to represent the number of carbon atoms in the heteroaryl ring system. In some embodiments, 5-12 membered heteroaryl group is alternative, which is a 5-12 membered monocyclic or bicyclic 4n+2 aromatic ring systems having ring carbon atoms and 1-4 ring heteroatoms. In some embodiments, 5-10 membered heteroaryl is alternative, which is 6-10 membered monocyclic or bicyclic 4n+2 aromatic ring systems having ring carbon atoms and 1-4 ring heteroatoms. In some embodiments, 5-9 membered heteroaryl is alternative, which is 5-9 membered monocyclic or bicyclic 4n+2 aromatic ring systems having ring carbon atoms and 1-4 ring heteroatoms. In other embodiments, 5-6 membered heteroaryl is particularly alternative, which is a 5-6 membered monocyclic or bicyclic 4n+2 aromatic ring systems having ring carbon atoms and 1-4 ring heteroatoms. Exemplary 5 membered heteroaryls containing one heteroatom include, but are not limited to, pyrrolyl, furanyl, and thienyl. Exemplary 5 membered heteroaryls containing two heteroatoms include, but are not limited to, imidazolyl, pyrazolyl, oxazolyl, isoxazolyl, thiazolyl, and isothiazolyl. Exemplary 5 membered heteroaryl groups containing three heteroatoms include, but are not limited to, triazolyl, oxadiazolyl (e.g., 1,2,4- oxadiazolyl) and thiadiazolyl. Exemplary 5 membered heteroaryl groups containing four heteroatoms include, but are not limited to, tetrazolyl. Exemplary 6 membered heteroaryl groups containing one heteroatom include, but are not limited to, pyridinyl. Exemplary 6 membered heteroaryl groups containing two heteroatoms include, but are not limited to, pyridazinyl, pyrimidinyl and pyrazinyl. Exemplary 6 membered heteroaryl groups containing three or four heteroatoms include, but are not limited to, triazinyl and tetrazinyl, respectively. Exemplary 7 membered heteroaryl groups containing one heteroatom include, but are not limited to, azacycloheptatrienyl, oxacycloheptatrienyl and thiacycloheptatrienyl. Exemplary 5,6- bicyclic heteroaryl groups include, but are not limited to, indolyl, isoindolyl, indazolyl, benzotriazolyl, benzothienyl, isobenzothienyl, benzofuranyl, benzisofuranyl, benzimidazolyl, benzoxazolyl, benzisoxazolyl, benzoxadiazolyl, benzothiazolyl, benzisothiazolyl, benzothiadiazolyl, indazinyl, and purinyl. Exemplary 6,6- bicyclic heteroaryl groups include, but are not limited to, naphthyridinyl, pteridinyl, quinolyl, isoquinolyl, cinnolinyl, quinoxalinyl, phthalazinyl, and quinazolinyl. The heteroaryl group may be optionally substituted with one or more substituents, e.g., with 1 to 5 substituents, 1 to 3 substituents, or 1 substituent.

"Cycloalkylene", "heterocyclylene", "arylene" or "heteroarylene" is a divalent group formed by removing another hydrogen from the above-defined "cycloalkyl", "heterocyclyl", "aryl" or "heteroaryl", and may be substituted or unsubstituted. For example, "C₅₋₇ cycloalkylene" refers to a divalent group formed by removing another hydrogen from the C₅₋₇ cycloalkyl, "5-8 membered heterocyclylene" refers to a divalent group formed by removing another hydrogen from the 5-8 membered heterocyclyl, "C₆₋₁₀ arylene" refers to a divalent group formed by removing another hydrogen from the C₆₋₁₀ aryl, and "5-6 membered heteroarylene" refers to a divalent group formed by removing another hydrogen from the 5-6 membered heteroaryl.

The alkyl, alkenyl, alkynyl, cycloalkyl, heterocyclyl, aryl and heteroaryl groups and the like as defined herein are optionally substituted groups.

Exemplary substituents on carbon atoms include, but are not limited to, halogen, -CN, -NO₂, -N₃, -SO₂H, -SO3H, -OH, -OR^{aa}, -ON(R^{bb})², -N(R^{bb})₂, -N(R^{bb})₃⁺X⁻, -N(OR^{cc})R^{bb}, -SH,⁻SR^{aa}, -SSR^{cc}, -C(=O)R^{aa}, -CO₂H, -CHO, -C(OR^{cc})₂, -CO₂R^{aa}, -OC(=O)R^{aa}, -OCO₂R^{aa}, - C(=O)N(R^{bb})², -OC(=O)N(R^{bb})₂, -NR^{bb}C(=O)R^{aa}, -NR^{bb}CO₂R^{aa}, -NR^{bb}C(=O)N(R^{bb})₂, - C(=NR^{bb})R^{aa}, -C(=NR^{bb})OR^{aa}, -OC(=NR^{bb})R^{aa}, -OC(=NR^{bb})OR^{aa}, -C(=NR^{bb})N(R^{bb})₂, - OC(=NR^{bb})N(R^{bb})₂, -NR^{bb}C(=NR^{bb})N(R^{bb})₂, -C(=O)NR^{bb}SO₂R^{aa}, -NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃, -C(=S)N(R^{bb})₂, - C(=O)SR^{aa}, -C(=S)SR^{aa}, -SC(=S)SR^{aa}, -SC(=O)SR^{aa}, -OC(=O)SR^{aa}, -SC(=O)OR^{aa}, - SC(=O)R^{aa}, -P(=O)₂R^{aa}, -OP(=O)₂^{aa}, -P(=O)(R^{aa})₂, -OP(=O)(R^{aa})₂, -OP(=O)(OR^{cc})₂, -P(=O)₂ N(R^{bb})₂, -OP(=O)₂N(R^{bb})₂, -P(=O)(NR^{bb})₂, -OP(=O)(NR^{bb})₂, -NR^{bb}P(=O)(OR^{cc})₂, - NR^{bb}P(=O)(NR^{bb})₂, -P(R^{cc})₂, -P(R^{cc})₃, -OP(R^{cc})₂, -OP(R^{cc})₃, -B(R^{aa})₂, -B(OR^{cc})₂, -BR^{aa}(OR^{cc}), alkyl, haloalkyl, alkenyl, alkynyl, cycloalkyl, heterocyclyl, aryl and heteroaryl, wherein each alkyl, alkenyl, alkynyl, cycloalkyl, heterocyclyl, aryl and heteroaryl is independently substituted with 0, 1, 2, 3, 4 or 5 R^{dd} groups;
or two geminal hydrogen groups on the carbon atom are replaced by groups =O, =S, =NN(R^{bb})₂, ⁼ NNR^{bb}C(=O)R^{aa}, =NNR^{bb}C(=O)OR^{aa}, =NNR^{bb}S(=O)₂R^{aa}, =NR^{bb} or =NOR^{cc};
each R^{aa} is independently selected from alkyl, haloalkyl, alkenyl, alkynyl, cycloalkyl, heterocyclyl, aryl, and heteroaryl, or two R^{aa} groups combine to form a heterocyclyl or heteroaryl ring, wherein each alkyl, alkenyl, alkynyl, cycloalkyl, heterocyclyl, aryl, or heteroaryl is independently substituted with 0, 1, 2, 3, 4, or R⁵ R^{dd} groups;
each R^{bb} is independently selected from: hydrogen, -OH, -OR^{aa}, -N(R^{cc})₂, -CN, - C(=O)R^{aa}, -C(=O)N(R^{cc})₂, -CO₂R^{aa}, -SO₂R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, - P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl and heteroaryl, or two R^{bb} groups combine to form a heterocyclyl or heteroaryl ring, wherein each alkyl, alkenyl, alkynyl, cycloalkyl, heterocyclyl, aryl or heteroaryl is independently substituted with 0, 1, 2, 3, 4 or 5 R^{dd} groups;
each R^{cc} is independently selected from hydrogen, alkyl, haloalkyl, alkenyl, alkynyl, cycloalkyl, heterocyclyl, aryl and heteroaryl, or two R^{cc} groups combine to form a heterocyclyl or heteroaryl ring, wherein each alkyl, alkenyl, alkynyl, cycloalkyl, heterocyclyl, aryl or heteroaryl is independently substituted with 0, 1, 2, 3, 4 or 5 R^{dd} groups;
each R^{dd} is independently selected from halogen, -CN, -NO₂, -N₃, -SO₂H, -SO₃H, - OH, -OR^{ee}, -ON(R^{ff})₂, -N(R^{ff})₂, -N(R^{ff})₃⁺X⁻, -N(OR^{ee})R^{ff}, -SH, -SR^{ee}, -SSR^{ee}, -C(=O)R^{ee}, - CO₂H, -CO₂R^{cc}, -OC(=O)R^{ee}, -OCO₂R^{ee}, -C(=O)N(R^{ff})₂, -OC(=O)N(R^{ff})₂, -NR^{ff}C(=O)R^{ee}, - NR^{ff}CO₂R^{ee}, -NR^{ff}C(=O)N(R^{ff})₂, -C(=NR^{ff})OR^{ee}, -OC(=NR^{ff})R^{ee}, -OC(=NR^{ff})OR^{ee}, - C(=NR^{ff})N(R^{ff})₂, -OC(=NR^{ff})N(R^{ff})₂, -NR^{ff}C(=NR^{ff})N(R^{ff})₂, -NR^{ff}SO₂R^{ee}, -SO₂N(R^{ff})₂, - SO₂R^{cc}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, - C(=S)SR^{ee}, -SC(=S)SR^{ee}, -P(=O)₂R^{ee}, -P(=O)(R^{ee})₂, -OP(=O)(R^{cc})₂, -OP(=O)(OR^{cc})₂, alkyl, haloalkyl, alkenyl, alkynyl, cycloalkyl, heterocyclyl, aryl, and heteroaryl, wherein each alkyl, alkenyl, alkynyl, cycloalkyl, heterocyclyl, aryl or heteroaryl is independently substituted with 0, 1, 2, 3, 4 or 5 R^{gg} groups, or two geminal R^{dd} substituents may combine to form =O or =S;
each R^{ee} is independently selected from alkyl, haloalkyl, alkenyl, alkynyl, cycloalkyl, aryl, heterocyclyl, and heteroaryl, wherein each alkyl, alkenyl, alkynyl, cycloalkyl, heterocyclyl, aryl, or heteroaryl is independently substituted with 0, 1, 2, 3, 4, or 5 R^{gg} groups;
each R^{ff} is independently selected from hydrogen, alkyl, haloalkyl, alkenyl, alkynyl, cycloalkyl, heterocyclyl, aryl and heteroaryl, or two R^{ff} groups combine to form a heterocyclyl or heteroaryl ring, wherein each alkyl, alkenyl, alkynyl, cycloalkyl, heterocyclyl, aryl or heteroaryl is independently substituted with 0, 1, 2, 3, 4 or 5 R^{gg} groups; and
each R^{gg} is independently: halogen, -CN, -NO₂, -N₃, -SO₂H, -SO₃H, -OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺ X⁻, -NH₂(C₁₋₆ alkyl)⁺X⁻, -NH₃⁺ X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl)-C(=O)NH₂, -C(=O)N(C₁₋₆ alkyl)₂, -OC(=O)NH(C₁₋₆ alkyl), -NHC(=O)(C₁₋₆ alkyl), -N(C₁₋₆ alkyl)C(=O)(C₁₋₆ alkyl), -NHCO₂(C₁₋₆ alkyl), -NHC(=O)N(C₁₋₆ alkyl)₂, -NHC(=O)NH(C₁₋₆ alkyl), -NHC(=O)NH₂, -C(=NH)O(C₁₋₆ alkyl), -OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, - OC(NH)NH(C₁₋₆ alkyl), -OC(NH)NH₂, -NHC(NH)N(C₁₋₆ alkyl)₂, -NHC(=NH)NH₂, - NHSO₂(C₁₋₆ alkyl), -SO₂N(C₁₋₆ alkyl)₂, -SO₂NH(C₁₋₆ alkyl), -SO₂NH₂, -SO₂C₁₋₆ alkyl, - SO₂OC₁₋₆ alkyl, -OSO₂C₁₋₆ alkyl, -SOC₁₋₆ alkyl, -Si(C₁₋₆ alkyl)₃, -OSi(C₁₋₆ alkyl)₃, - C(=S)N(C₁₋₆ alkyl)₂, C(=S)NH(C₁₋₆ alkyl), C(=S)NH₂, -C(=O)S(C₁₋₆ alkyl)-C₁₋₆ alkyl), - C(=S)S C₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ haloalkyl, C₂-C₆ alkenyl, C₂-C₆ alkynyl, C₃-C₇ cycloalkyl, C₆-C₁₀ aryl, C₃-C₇ heterocyclyl, C₅-C₁₀ heteroaryl; or two geminal R^{gg} substituents may be combined to form =O or =S; wherein X⁻ is a counter ion.

Exemplary substituents on the nitrogen atom include, but are not limited to, hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{bb})R^{aa},-C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, - C(=O)SR^{cc}, -C(=S)SR^{cc}, -P(=O)₂R^{aa}, -P(=O)(R^{aa}), -P(=O)₂N(R^{cc})₂, -P(=O)(NR^{cc})₂, alkyl, haloalkyl, alkenyl, alkynyl, cycloalkyl, heterocyclyl, aryl and heteroaryl, or two R^{cc} groups attached to the nitrogen atom combine to form a heterocyclyl or heteroaryl ring, wherein each alkyl, alkenyl, alkynyl, cycloalkyl, heterocyclyl, aryl or heteroaryl is independently substituted with 0, 1, 2, 3, 4 or 5 R^{dd} groups , and wherein R^{aa}, R^{bb}, R^{cc} and R^{dd} are as described above.

### Other definitions

The term "pharmaceutically acceptable salt" as used herein refers to those carboxylates, amino acid addition salts of the compounds of the present disclosure, which are suitable for use in contact with patient tissues within the scope of reliable medical judgment, do not produce undue toxicity, irritation, allergic response or the like, are commensurate to a reasonable benefit/risk ratio, are effective for their intended use, and include zwitterionic forms of the compounds of the present disclosure (if possible).

"Subjects" for administration include, but are not limited to, humans (i.e., males or females of any age group, e.g., pediatric subjects (e.g., infants, children, adolescents) or adult subjects (e.g., young adults, middle-aged adults, or old adults)) and/or non-human animals, e.g., mammals, e.g., primates (e.g., cynomolgus monkeys, rhesus monkeys), cattle, pigs, horses, sheep, goats, rodents, cats, and/or dogs. In some embodiments, the subject is a human. In some embodiments, the subject is a non-human animal. The terms "human," "patient," and "subject" are used interchangeably herein.

"Disease," "disorder," and "condition" are used interchangeably herein.

Generally, the "effective amount" of a compound refers to an amount sufficient to elicit a target biological response. As appreciated by those of ordinary skill in the art, the effective amount of the compound of the present disclosure may vary depending on factors such as the biological target, the pharmacokinetics of the compound, the disease being treated, the mode of administration, and the age, health, and symptoms of the subject. The effective amount includes a therapeutically effective amount and a prophylactically effective amount.

"Combination" and related terms refer to the simultaneous or sequential administration of the compound of the present disclosure and an additional therapeutic agent. For example, the compound of the present disclosure may be administered simultaneously or sequentially with the additional therapeutic agent in separate unit dosage forms, or may be administered simultaneously with the additional therapeutic agent in a single unit dosage form.

### DETAILED DESCRIPTION

As used herein, "the compound of the present disclosure" refers to a compound of Formula (I) (including its sub-formulae, such as Formulae (II), (II-1), (II-2), (III), (IV-2), (IV-2), (IV-3), (IV-4), (IV -5), (IV-6), (IV-7), (IV-8), (IV-9), (IV-10), (IV-3A), (IV-6A), (V), (VI), (VI-1), (VI-2), etc.), pharmaceutically acceptable salts, enantiomers, diastereomers, solvates, hydrates or isotopic variants thereof, or a mixture thereof.

In an embodiment, the present disclosure relates to a compound of Formula (I), or a pharmaceutically acceptable salt, isotopic variant, tautomer or stereoisomer thereof, or a mixture thereof: 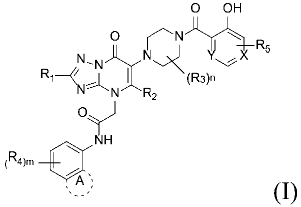 wherein,
X and Y are each independently selected from CH and N, and at least one of X and Y is N atom;
Ring A is absent or selected from C₃₋₁₀ cycloalkyl, 5-10 membered heteroaryl, 5-10 membered heterocyclyl and C₆₋₁₀ aryl;
R₁ is selected from 5-12 membered heteroaryl and 5-12 membered heterocyclyl, and the R₁ is optionally substituted with 1, 2, 3, 4 or 5 Rₓ, provided that R₁ is not pyridyl or 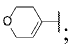 ;
Rₓ is selected from H, D, halogen, NH₂, CN, OH, C₁₋₆ alkyl, C₁₋₆ alkoxy, C₁₋₆ haloalkyl, -C(O)Rₐ, -C(O)ORₐ, -OC(O)Rₐ, -C(O)NH-Rₐ, -NHC(O)-Rₐ, -(CH₂)ₚ-ORₐ, -(CH₂)ₚ-C(O)Rₐ, - P(O)-(Rₐ)₂ and -S(O)₂-Rₐ, wherein Rₐ is selected from H, C₁₋₆ alkyl, C₁₋₆ haloalkyl and C₃₋₆ cycloalkyl, and p is selected from 0, 1, 2, 3 and 4; or two Rₓ on the same atom are taken together to form oxo or thio;
R₂ is selected from H, D, halogen, C₁₋₆ alkyl, C₁₋₆ haloalkyl, C₁₋₆ alkoxy, C₁₋₆ alkylthio and C₃₋₆ cycloalkyl;
R₃ is selected from H, D, halogen, NH₂, CN, OH, C₁₋₆ alkyl, C₁₋₆ haloalkyl, C₁₋₆ alkoxy and C₃₋₆ cycloalkyl, or R₃ on two different carbon atoms are connected to form a bridged ring, or two R₃ on the same carbon atom are connected to form C₃₋₁₀ cycloalkyl, 5-10 membered heteroaryl, C₃₋₁₀ cycloalkyl or 3-10 membered heterocyclyl;
R₄ is selected from H, D, halogen, NH₂, CN, OH, SF₅, SCF₃, C₁₋₆ alkyl, C₁₋₆ haloalkyl, C₁₋₆ alkoxy, C₁₋₆ haloalkoxy, C₁₋₆ alkylthio, C₃₋₁₀ cycloalkyl, 3-10 membered heterocyclyl, 5-10 membered heteroaryl and C₆₋₁₀ aryl;
R₅ is selected from H, D, halogen, NH₂, CN, OH, C₁₋₆ alkyl, C₁₋₆ deuterated alkyl, C₁₋₆ haloalkyl, C₁₋₆ alkoxy, C₁₋₆ alkylthio and C₃₋₆ cycloalkyl;
m is selected from 0, 1, 2, 3, 4 and 5;
n is selected from 0, 1, 2, 3, 4, 5, 6, 7 and 8;
R₁-R₅ may be optionally deuterated, up to fully deuterated.
X and Y

In an embodiment, X is CH; and in another embodiment, X is N.

In an embodiment, Y is CH; and in another embodiment, Y is N.

In an embodiment, at least one of X and Y is N atom.

### Ring A

In one embodiment, the Ring A is absent; in another embodiment, the Ring A is C₃₋₁₀ cycloalkyl; in another embodiment, the Ring A is 5-10 membered heteroaryl, such as 5-8 membered heteroaryl; in another embodiment, the Ring A is 5-10 membered heterocyclyl, such as 5-7 membered heterocyclyl; and in another embodiment, the Ring A is C₆₋₁₀ aryl.

In a specific embodiment, the Ring A is a 5-6 membered heteroaryl; in another embodiment, the Ring A and the benzene ring to which it is attached are taken together to form 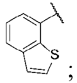 in another embodiment, the Ring A and the benzene ring to which it is attached are taken together to form 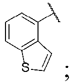 and in another embodiment, the Ring A and the benzene ring to which it is attached are taken together to form

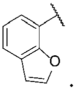

### Ring B

In one embodiment, the Ring B is absent; in another embodiment, the Ring B is C₃₋₁₀ cycloalkyl; in another embodiment, the Ring B is 5-10 membered heteroaryl, such as 5-8 membered heteroaryl; in another embodiment, the Ring B is 5-10 membered heterocyclyl, such as 5-7 membered heterocyclyl; and in another embodiment, the Ring B is C₆₋₁₀ aryl.

In an embodiment, the Ring B is optionally substituted with 1, 2, 3, 4 or 5 substituents selected from halogen, C₁₋₆ alkyl and C₁₋₆ haloalkyl.

### R₁

In an embodiment, R₁ is a 5-12 membered heteroaryl, such as a 5-10 membered heteroaryl; and in another embodiment, R₁ is a 5-12 membered heterocyclyl, provided that R₁ is not pyridyl or

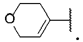

In an embodiment, R₁ is a 5-12 membered bicyclic heteroaryl, such as a 5-10 membered bicyclic heteroaryl; and in another embodiment, R₁ is a 5-12 membered bicyclic heterocyclyl, such as a 5-10 membered bicyclic heterocyclyl.

In an embodiment, R₁ is optionally substituted with 1, 2, 3, 4 or 5 Rₓ.

In a specific embodiment, R₁ is 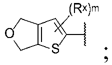 in another specific embodiment, R₁ is 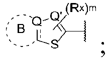 in another specific embodiment, R₁ is 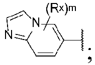 in another specific embodiment, R₁ is 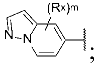 in another specific embodiment, R₁ is 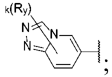 in another specific embodiment, R₁ is 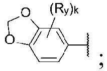 in another specific embodiment, R₁ is 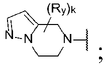 in another specific embodiment, R₁ is 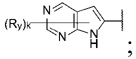 in another specific embodiment, R₁ is 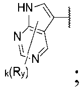 in another specific embodiment, R₁ is 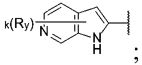 in another specific embodiment, R₁ is 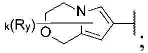 and in another specific embodiment, R₁ is

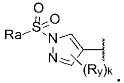

In an alternative embodiment, R₁ is 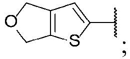 in another alternative embodiment, R₁ is 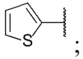 in another alternative embodiment, R₁ is 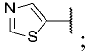 in another alternative embodiment, R₁ is 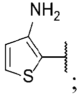 in another alternative embodiment, R₁ is 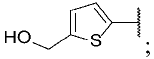 in another alternative embodiment, R₁ is 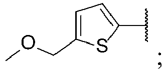 in another alternative embodiment, R₁ is 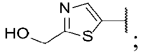 in another alternative embodiment, R₁ is 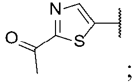 in another alternative embodiment, R₁ is 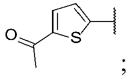 in another alternative embodiment, R₁ is 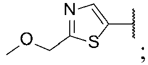 in another alternative embodiment, R₁ is 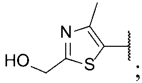 in another alternative embodiment, R₁ is 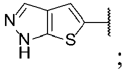 in another alternative embodiment, R₁ is 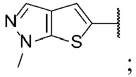 in another alternative embodiment, R₁ is 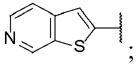 in another alternative embodiment, R₁ is 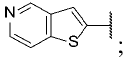 in another alternative embodiment, R₁ is 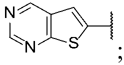 in another alternative embodiment, R₁ is 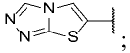 in another alternative embodiment, R₁ is 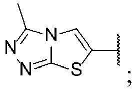 in another alternative embodiment, R₁ is 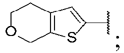 in another alternative embodiment, R₁ is 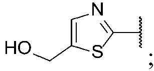 in another alternative embodiment, R₁ is 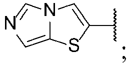 in another alternative embodiment, R₁ is 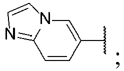 in another alternative embodiment, R₁ is 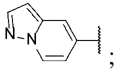 in another alternative embodiment, R₁ is 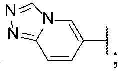 in another alternative embodiment, R₁ is 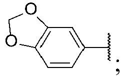 in another alternative embodiment, R₁ is 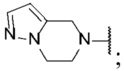 in another alternative embodiments, R₁ is 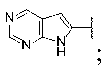 in another alternative embodiment, R₁ is 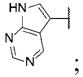 in another alternative embodiment, R₁ is 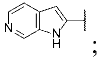 in another alternative embodiment, R₁ is 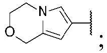 and in another alternative embodiment, R₁ is

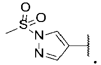

In an embodiment, R₁ may be optionally deuterated, up to fully deuterated.

### R₂

In an embodiment, R₂ is H; in another embodiment, R₂ is D; in another embodiment, R₂ is halogen; in another embodiment, R₂ is C₁₋₆ alkyl, such as C₁₋₄ alkyl; in another embodiment, R₂ is C₁₋₆ haloalkyl; in another embodiment, R₂ is C₁₋₆ alkoxy; in another embodiment, R₂ is C₁₋₆ alkylthio; and in another embodiment, R₂ is C₃₋₆ cycloalkyl.

In a specific embodiment, R₂ is H; in another specific embodiment, R₂ is CH₃; in another specific embodiment, R₂ is CH₂CH₃; in another specific embodiment, R₂ is trifluoroethyl; in another specific embodiment, R₂ is methoxy; and in another specific embodiment, R₂ is cyclopropyl.

In an embodiment, R₂ may be optionally deuterated, up to fully deuterated.

### R₃

In an embodiment, R₃ is H; in another embodiment, R₃ is D; in another embodiment, R₃ is halogen; in another embodiment, R₃ is NH₂; in another embodiment, R₃ is CN; in another embodiment, R₃ is OH; in another embodiment, R₃ is C₁₋₆ alkyl; in another embodiment, R₃ is C₁₋₄ alkyl; in another embodiment, R₃ is C₁₋₆ haloalkyl; in another embodiment, R₃ is C₁₋₆ alkoxy; in another embodiment, R₃ is C₃₋₆ cycloalkyl; in another embodiment, two R₃ are connected to the carbon atom on which they are attached to form a 3-6 membered spiro ring or bridged ring; in another embodiment, R₃ on two different carbon atoms are connected to form a bridged ring; in another embodiment, two R₃ on the same carbon atom are connected to form a C₃₋₁₀ cycloalkyl, such as C₃₋₇ cycloalkyl, e.g., C₃₋₅ cycloalkyl; in another embodiment, two R₃ on the same carbon atom are connected to form a C₃₋₁₀ cycloalkyl, such as a C₃₋₇ cycloalkyl, e.g., a C₃₋₅ cycloalkyl; in another embodiment, two R₃ on the same carbon atom are connected to form a 5-10 membered heteroaryl; in another embodiment, two R₃ on the same carbon atom are connected to form a C₃₋₁₀ cycloalkyl, such as cyclopropyl, cyclobutyl; and in another embodiment, two R₃ on the same carbon atom are connected to form a 3-10 membered heterocyclyl.

In a specific embodiment, R₃ is H; in another specific embodiment, R₃ is D; in another embodiment, R₃ is halogen, such as F, Cl or Br; in another embodiment, R₃ is CN; in another embodiment, R₃ is methyl; in another embodiment, R₃ is ethyl; in another embodiment, R₃ is trifluoromethyl; in another embodiment, R₃ is cyclopropyl; and in another embodiment, two R₃ on the same carbon atom are connected to form a cyclopropyl or cyclobutyl group.

In an embodiment, R₃ may be optionally deuterated, up to fully deuterated.

### R₄

In an embodiment, R₄ is H; in another embodiment, R₄ is D; in another embodiment, R₄ is halogen, such as F, Cl or Br; in another embodiment, R₄ is NH₂; in another embodiment, R₄ is CN; in another embodiment, R₄ is OH; in another embodiment, R₄ is SF₅; in another embodiment, R₄ is SCF₃; in another embodiment, R₄ is C₁₋₆ alkyl; in another embodiment, R₄ is C₁₋₆ haloalkyl, such as methyl and ethyl; in another embodiment, R₄ is C₁₋₄ alkyl; in another embodiment, R₄ is C₁₋₆ haloalkyl, such as trifluoromethyl, difluoromethyl; in another embodiment, R₄ is C₁₋₆ alkoxy; in another embodiment, R₄ is C₁₋₆ haloalkoxy, such as OCF₃; in another embodiment, R₄ is C₁₋₆ alkylthio, such as methylthio and ethylthio; in another embodiment, R₄ is a C₃₋₆ cycloalkyl group; in another embodiment, R₄ is a 3-10 membered heterocyclyl group; in another embodiment, R₄ is a 4-10 membered heterocyclyl group; in another embodiment, R₄ is a 5-10 membered heteroaryl group, such as pyridinyl; and in another embodiment, R₄ is a C₆₋₁₀ aryl group.

In an embodiment, R₄ may be optionally deuterated, up to fully deuterated.

### R₄ₐ, R_{4b} and R_{4d}

In an embodiment, R₄ₐ is H; in another embodiment, R₄ₐ is halogen, such as F; in another embodiment, R₄ₐ is CN; in another embodiment, R₄ₐ is C₁₋₆ alkyl; in another embodiment, R₄ₐ is C₁₋₆ alkoxy; and in another embodiment, R₄ₐ is C₁₋₆ haloalkyl.

In an embodiment, R_{4b} is halogen, such as Cl, Br; in another embodiment, R_{4b} is CN; in another embodiment, R_{4b} is C₁₋₆ alkyl, alternatively C₁₋₄ alkyl, such as CH₂CH₃; in another embodiment, R_{4b} is C₁₋₆ alkoxy; and in another embodiment, R_{4b} is C₁₋₆ haloalkyl, alternatively C₁₋₄ haloalkyl, such as CF₃.

In an embodiment, R_{4d} is H; in another embodiment, R_{4d} is CN; in another embodiment, R_{4d} is halogen, such as F, Cl; in another embodiment, R_{4d} is C₁₋₆ alkyl; in another embodiment, R_{4d} is C₁₋₄ alkyl, alternatively C₁₋₂ alkyl, such as CH₃; in another embodiment, R_{4d} is C₁₋₆ alkoxy; and in another embodiment, R_{4d} is C₁₋₆ haloalkyl.

### R₅

In an embodiment, R₅ is H; in another embodiment, R₅ is D; in another embodiment, R₅ is halogen; in another embodiment, R₅ is NH₂; in another embodiment, R₅ is CN; in another embodiment, R₅ is OH; in another embodiment, Rs is C₁₋₆ alkyl, alternatively C₁₋₄ alkyl, such as methyl, ethyl; in another embodiment, R₅ is C₁₋₆ deuterated alkyl, such as CD₃; in another embodiment, R₅ is C₁₋₆ haloalkyl, such as trifluoromethyl; in another embodiment, R₅ is C₁₋₆ alkoxy, such as methoxy, ethoxy; in another embodiment, R₅ is C₁₋₆ alkylthio, such as methylthio; and in another embodiment, R₅ is C₃₋₆ cycloalkyl, such as cyclopropyl.

In an embodiment, R₅ may be optionally deuterated, up to fully deuterated.

### -̅ -̅ -̅

In an embodiment, -̅ -̅ -̅ represents a single bond; in another embodiment, -̅ -̅ -̅ represents a double bond; and in another embodiment, Q is N and the Ring B is present, -̅ -̅ -̅ represents a single bond.

### Rₓ

In an embodiment, Rₓ is H; in another embodiment, Rₓ is D; in another embodiment, Rₓ is halogen; in another embodiment, Rₓ is NH₂; in another embodiment, Rₓ is CN; in another embodiment, Rₓ is OH; in another embodiment, Rₓ is C₁₋₆ alkyl; in another embodiment, Rₓ is C₁₋₄ alkyl, e.g.; in another embodiment, Rₓ is C₁₋₆ alkoxy; in another embodiment, Rₓ is C₁₋₆ haloalkyl; in another embodiment, Rₓ is -C(O)Rₐ; in another embodiment, Rₓ is -C(O)ORₐ; in another embodiment, Rₓ is -OC(O)Rₐ; in another embodiment, Rₓ is -C(O)NH-Rₐ; in another embodiment, Rₓ is -NHC(O)-Rₐ; in another embodiment, Rₓ is -(CH₂)ₚ-ORₐ; in another embodiment, Rₓ is -(CH₂)ₚ-C(O)Rₐ; in another embodiment, Rₓ is -P(O)-(Rₐ)₂; in another embodiment, Rₓ is -S(O)₂-Rₐ; in another embodiment, two Rₓ on the same atom are taken together to form oxo; and in another embodiment, two Rₓ on the same atom are taken together to form thio.

In a specific embodiment, Rₓ is H; in another specific embodiment, Rₓ is NH₂; in another specific embodiment, Rₓ is CH₂OH; in another specific embodiment, Rₓ is CH₂OCH₃; in another specific embodiment, Rₓ is C(O)CH₃; in another specific embodiment, Rₓ is -S(O)₂-CH₃; and in another specific embodiment, two Rₓ on the same carbon atom are taken together to form an oxo group.

### R_{y}

In an embodiment, R_{y} is H; in another embodiment, R_{y} is D; in another embodiment, R_{y} is halogen; in another embodiment, R_{y} is C₁₋₆ alkyl; and in another embodiment, R_{y} is C₁₋₆ haloalkyl.

### Q and Q'

In an embodiment, Q is CH; and in another embodiment, Q is N.

In an embodiment, Q' is CH; and in another embodiment, Q' is N.

In an embodiment, at most one of Q and Q' is N.

### Rₐ

In an embodiment, Rₐ is H; in another embodiment, Rₐ is C₁₋₆ alkyl, such as CH₃; in another embodiment, Rₐ is C₁₋₆ haloalkyl; and in another embodiment, Rₐ is C₃₋₆ cycloalkyl.

### m, n, k, and p

In an embodiment, m is selected from 0, 1, 2, 3, 4 and 5.

In an embodiment, n is selected from 0, 1, 2, 3, 4, 5, 6, 7 and 8.

In an embodiment, k is selected from 0, 1, 2, 3, 4 and 5.

In an embodiment, p is selected from 0, 1, 2, 3 and 4.

Any technical solution or any combination thereof in any specific embodiments as described above may be combined with any technical solution or any combination thereof in other specific embodiments. For example, any technical solution or any combination thereof of Ring A may be combined with any technical solution or any combination thereof of Ring B, X, Y, L, R₁, R₂, R₃, R₄, R₄ₐ, R_{4b}, R_{4d}, R₅, Rₓ, R_{y}, Q, Q', Rₐ, m, n, k and p, etc., or any combination thereof. The present disclosure is intended to include all combinations of these technical solutions, which are not listed one by one due to space limitations.

In a more specific embodiment, the present disclosure provides the compound of Formula (I), or a pharmaceutically acceptable salt, isotopic variant, tautomer or stereoisomer thereof, or a mixture thereof, wherein:
X and Y are each independently selected from CH and N, and at least one of X and Y is N atom;
Ring A is absent or selected from a 5-6 membered heteroaryl group, wherein the 5-6 membered heteroaryl group and the benzene ring to which it is attached are taken together to form a heteroaryl group of 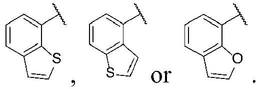 Alternatively, the Ring A is absent;
R₁ is a 5-10 membered heteroaryl group, and the R₁ is optionally substituted with 1, 2 or 3 Rₓ;
alternatively, Rₓ is H, NH₂, CH₃, CH₂OH, CH₂OCH₃ or C(O)CH₃ or -S(O)₂-CH₃, or two Rₓ on the same carbon atom are taken together to form an oxo group;
alternatively, R₁ is selected from 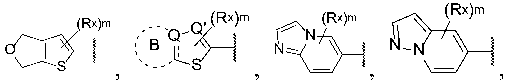 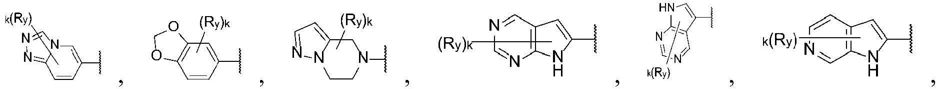 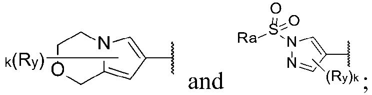
Ring B is absent or selected from C₃₋₁₀ cycloalkyl, 5-10 membered heterocyclyl, 5-10 membered heteroaryl and C₆₋₁₀ aryl, and the Ring B is optionally substituted with 1, 2, 3, 4 or 5 substituents selected from halogen, C₁₋₆ alkyl and C₁₋₆ haloalkyl;
Q and Q' are independently selected from CH and N, and at most one of Q and Q' is N;
R_{y} is selected from H, D, halogen, C₁₋₆ alkyl and C₁₋₆ haloalkyl;
R₂ is selected from C₁₋₄ alkyl, such as CH₃ or CH₂CH₃;
R₃ is selected from H, D and C₁₋₄ alkyl, such as H, D or CH₃, or R₃ on two different carbon atoms are connected to form a bridged ring, or two R₃ on the same carbon atom are connected to form a C₃₋₅ cycloalkyl, such as cyclopropyl;
R₄ is selected from H, F, Cl, Br, CH₃, CH₂CH₃, SCF₃, OCF₃, CF₃ and pyridinyl;
R₅ is selected from H, CH₃ and CD₃, alternatively H or CH₃;
Rₐ is selected from C₁₋₆ alkyl;
m is selected from 0, 1, 2 and 3;
k is selected from 0, 1, 2, 3, 4 and 5.

In a more specific embodiment, the present disclosure provides the compound of Formula (I), or a pharmaceutically acceptable salt, isotopic variant, tautomer or stereoisomer thereof, or a mixture thereof, wherein R₁ is selected from: 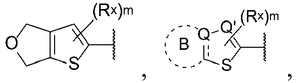 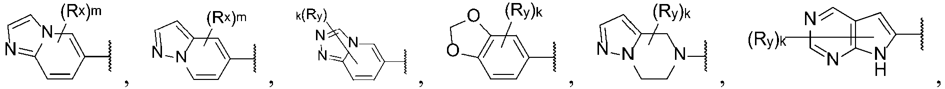 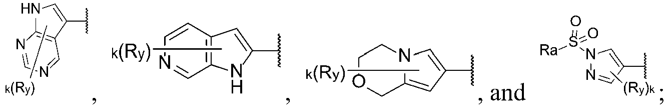 wherein,
-̅ -̅ -̅ represents a single bond or a double bond. When Q is N and the Ring B is present, -̅ -̅ -̅ represents a single bond;
Q and Q' are independently selected from CH and N, and at most one of Q and Q' is N;
Ring B is absent or selected from C₃₋₁₀ cycloalkyl, 5-10 membered heterocyclyl, 5-10 membered heteroaryl and C₆₋₁₀ aryl, and the Ring B is optionally substituted with 1, 2, 3, 4 or 5 substituents selected from halogen, C₁₋₆ alkyl and C₁₋₆ haloalkyl;
Rₓ is selected from H, NH₂, halogen, C₁₋₆ alkyl, C₁₋₆ haloalkyl, -C (O)Rₐ, -C(O)ORₐ, - OC(O)Rₐ, -C(O)NH-Rₐ, -NHC(O)-Rₐ, -(CH₂)ₚ-ORₐ and -(CH₂)ₚ-C(O)Rₐ, wherein Rₐ is selected from H, C₁₋₆ alkyl and C₁₋₆ haloalkyl, and p is selected from 1, 2 and 3;
R_{y} is selected from H, D, halogen, C₁₋₆ alkyl and C₁₋₆ haloalkyl;
Rₐ is selected from C₁₋₆ alkyl;
m is selected from 0, 1, 2, 3, 4 and 5;
k is selected from 0, 1, 2, 3, 4 and 5;
alternatively,
-̅ -̅ -̅ represents a single bond or a double bond. When Q is N and the Ring B is present, -̅ -̅ -̅ represents a single bond;
Q and Q' are independently selected from CH and N, and at most one of Q and Q' is N;
Ring B is absent or selected from 5-7 membered heterocyclyl or 5-6 membered heteroaryl, and the Ring B is optionally substituted with 1, 2 or 3 substituents selected from halogen and C₁₋₄ alkyl;
Rₓ is selected from H, NH₂, C₁₋₄ alkyl, -C(O)Rₐ and -(CH₂)ₚ-ORₐ, wherein Rₐ is selected from H and C₁₋₄ alkyl, and p is selected from 1 and 2; Rₓ is alternatively H, NH₂, CH₃, CH₂OH, CH₂OCH₃ or C(O)CH₃;
R_{y} is selected from H, D, halogen, C₁₋₆ alkyl and C₁₋₆ haloalkyl;
Rₐ is selected from CH₃;
m is selected from 0, 1, 2 and 3;
k is selected from 0, 1, 2 and 3;
more alternatively,
R₁ is selected from:

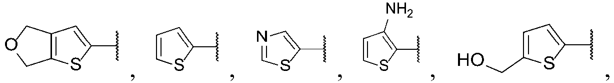

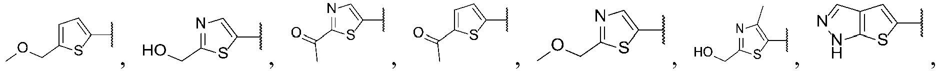

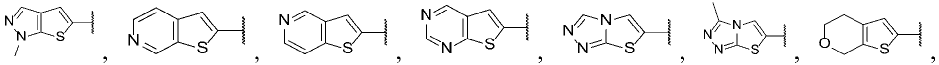

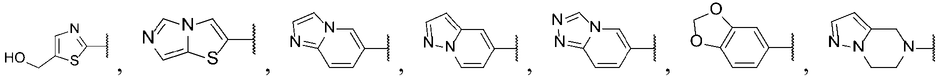

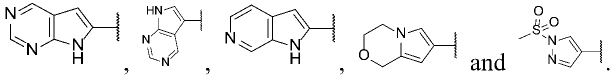

In a more specific embodiment, the present disclosure provides the compound of Formula (I), or a pharmaceutically acceptable salt, isotopic variant, tautomer or stereoisomer thereof, or a mixture thereof, which is selected from the structures of: 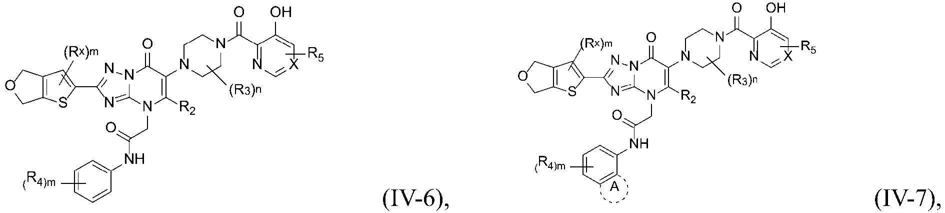 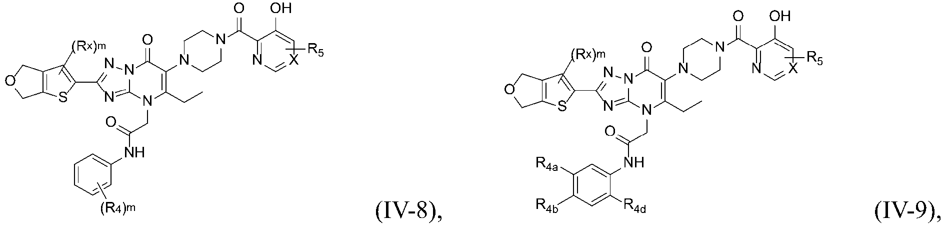 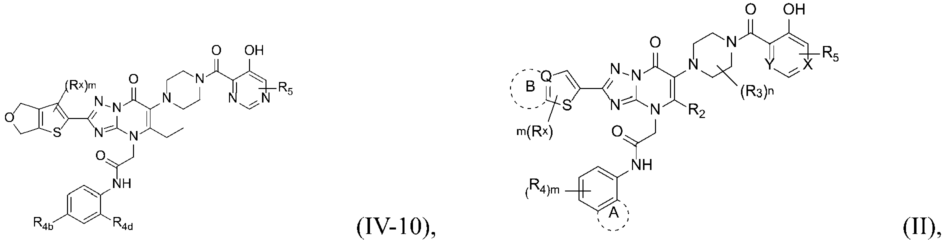 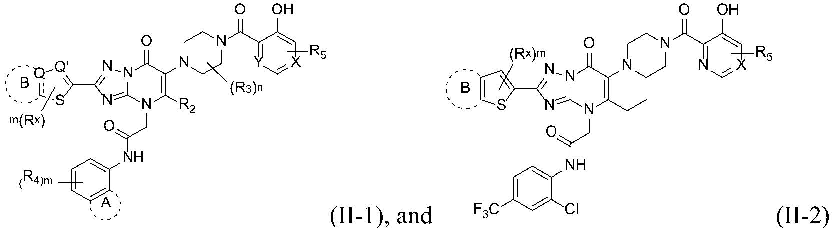 wherein,
the variables are as defined herein.

In a more specific embodiment, the present disclosure provides a compound of Formula (IV-6) or Formula (IV-7), or a pharmaceutically acceptable salt, isotopic variant, tautomer or stereoisomer thereof, or a mixture thereof: 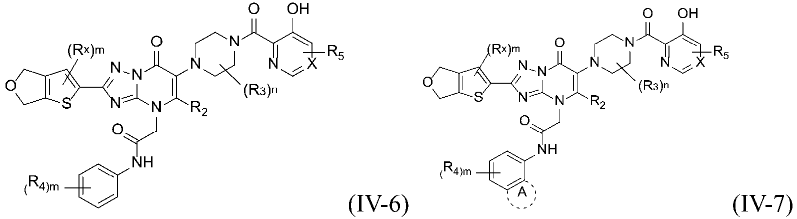 wherein,
Ring A is absent or selected from C₃₋₁₀ cycloalkyl, 5-10 membered heteroaryl, 5-10 membered heterocyclyl and C₆₋₁₀ aryl;
X is selected from CH and N;
Rₓ is selected from H, D, C₁₋₆ alkyl, C₁₋₆ alkoxy and C₁₋₆ haloalkyl;
R₂ is selected from H, D, C₁₋₆ alkyl, C₁₋₆ alkoxy and C₁₋₆ haloalkyl;
R₃ is selected from H, D, C₁₋₆ alkyl, C₁₋₆ alkoxy and C₁₋₆ haloalkyl, or R₃ on two different carbon atoms are connected to form a bridged ring, or two R₃ on the same carbon atom are connected to form a C₃₋₁₀ cycloalkyl, a 5-10 membered heteroaryl, a C₃₋₁₀ cycloalkyl or a 3-10 membered heterocyclyl;
R₄ is selected from H, D, halogen, CN, SCF₃, C₁₋₆ alkyl, C₁₋₆ alkoxy, C₁₋₆ haloalkyl, C₁₋₆ haloalkoxy, C₃₋₆ cycloalkyl, 4-10 membered heterocyclyl, 5-10 membered heteroaryl and C₆₋₁₀ aryl;
R₅ is selected from H, D, C₁₋₆ alkyl, C₁₋₆ deuterated alkyl, C₁₋₆ alkoxy and C₁₋₆ haloalkyl;
m is selected from 0, 1, 2, 3, 4 and 5;
n is selected from 0, 1, 2, 3, 4, 5, 6, 7 and 8.

In a more specific embodiment, the present disclosure provides the compound of Formula (IV-6) or (IV-7), or a pharmaceutically acceptable salt, isotopic variant, tautomer or stereoisomer thereof, or a mixture thereof, wherein:
Ring A is absent or selected from 5-10 membered heteroaryl, 5-10 membered heterocyclyl and C₆₋₁₀ aryl;
X is selected from CH and N;
Rₓ is selected from H, C₁₋₆ alkyl, C₁₋₆ alkoxy and C₁₋₆ haloalkyl;
R₂ is selected from C₁₋₆ alkyl and C₁₋₆ haloalkyl;
R₃ is selected from H, D, C₁₋₆ alkyl and C₁₋₆ haloalkyl, or R₃ on two different carbon atoms are connected to form a bridged ring, or two R₃ on the same carbon atom are connected to form a C₃₋₁₀ cycloalkyl or a 3-10 membered heterocyclic group;
R₄ is selected from H, halogen, CN, SCF₃, C₁₋₆ alkyl, C₁₋₆ alkoxy, C₁₋₆ haloalkyl and C₁₋₆ haloalkoxy;
R₅ is selected from H, C₁₋₆ alkyl, C₁₋₆ alkoxy and C₁₋₆ haloalkyl;
m is selected from 0, 1, 2, 3, 4 and 5;
n is selected from 0, 1, 2, 3, 4, 5, 6, 7 and 8.

In a more specific embodiment, the present disclosure provides the compound of Formula (IV-6) or (IV-7), or a pharmaceutically acceptable salt, isotopic variant, tautomer or stereoisomer thereof, or a mixture thereof, wherein:
Ring A is absent;
X is selected from CH and N;
Rₓ is selected from H and C₁₋₆ alkyl;
R₂ is selected from C₁₋₆ alkyl;
R₃ is selected from H, D and C₁₋₆ alkyl, or two R₃ on different carbon atoms are connected to form a bridged ring, or two R₃ on the same carbon atom are connected to form a C₃₋₇ cycloalkyl;
R₄ is selected from H, halogen, SCF₃, C₁₋₆ alkyl, C₁₋₆ alkoxy, C₁₋₆ haloalkyl and C₁₋₆ haloalkoxy;
R₅ is selected from H and C₁₋₆ alkyl;
m is selected from 0, 1, 2, 3 and 4;
n is selected from 0, 1, 2, 3, 4, 5, 6, 7 and 8.

In a more specific embodiment, the present disclosure provides the compound of Formula (IV-6) or (IV-7), or a pharmaceutically acceptable salt, isotopic variant, tautomer or stereoisomer thereof, or a mixture thereof, wherein:
Ring A is selected from a 5-6 membered heteroaryl group, and the 5-6 membered heteroaryl group and the benzene ring to which it is attached are taken together to form the heteroaryl group of 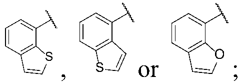 X is selected from CH and N;
Rₓ is selected from H and C₁₋₄ alkyl, alternatively H;
R₂ is selected from C₁₋₄ alkyl, such as CH₃ or CH₂CH₃;
R₃ is selected from H, D and C₁₋₄ alkyl, such as H, D or CH₃, or R₃ on two different carbon atoms are connected to form a bridged ring, or two R₃ on the same carbon atom are connected to form a C₃₋₅ cycloalkyl, such as cyclopropyl;
R₄ is selected from H, halogen, SCF₃, C₁₋₄ alkyl and C₁₋₄ haloalkyl, such as H, F, Cl, Br, CH₃, CH₂CH₃, CF₃, SCF₃, OCF₃ or OCH₃;
R₅ is selected from H and C₁₋₄ alkyl, e.g., H or CH₃;
m is selected from 0, 1, 2 and 3;
n is selected from 0, 1, 2, 3, 4, 5, 6, 7 and 8.

In a more specific embodiment, the present disclosure provides a compound of Formula (IV-8) or (IV-9), or a pharmaceutically acceptable salt, isotopic variant, tautomer or stereoisomer thereof, or a mixture thereof: 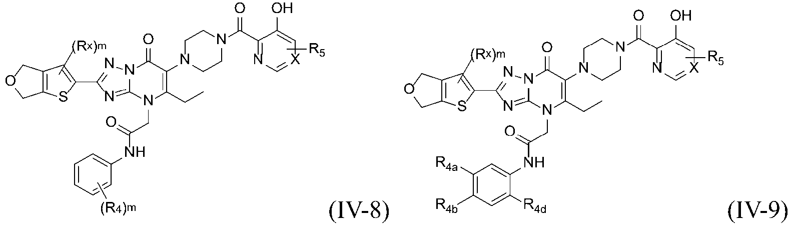 wherein,
X is selected from N and CH;
R₄ is selected from H, CN, halogen, C₁₋₆ alkyl, C₁₋₆ alkoxy and C₁₋₆ haloalkyl;
R₄ₐ is selected from H, halogen, CN, C₁₋₆ alkyl, C₁₋₆ alkoxy and C₁₋₆ haloalkyl;
R_{4b} is selected from halogen, CN, C₁₋₆ alkyl, C₁₋₆ alkoxy and C₁₋₆ haloalkyl;
R_{4d} is selected from H, CN, halogen, C₁₋₆ alkyl, C₁₋₆ alkoxy and C₁₋₆ haloalkyl;
R₅ is selected from H, C₁₋₆ alkyl, C₁₋₆ alkoxy and C₁₋₆ haloalkyl, alternatively C₁₋₆ alkyl or C₁₋₆ haloalkyl;
Rₓ is selected from H, C₁₋₆ alkyl, C₁₋₆ alkoxy and C₁₋₆ haloalkyl;
m is selected from 0, 1, 2, 3, 4 and 5.

In a more specific embodiment, the present disclosure provides the compound of Formula (IV-8) or (IV-9), or a pharmaceutically acceptable salt, isotopic variant, tautomer or stereoisomer thereof, or a mixture thereof, wherein:
X is selected from N and CH;
R₄ is selected from H, halogen, C₁₋₄ alkyl and C₁₋₄ haloalkyl;
R₄ₐ is selected from H and halogen;
R_{4b} is selected from halogen, C₁₋₄ alkyl and C₁₋₄ haloalkyl;
R_{4d} is selected from H, halogen and C₁₋₄ alkyl;
R₅ is selected from H, C₁₋₄ alkyl and C₁₋₄ haloalkyl, alternatively C₁₋₄ alkyl or C₁₋₄ haloalkyl;
Rₓ is selected from H, C₁₋₄ alkyl and C₁₋₄ haloalkyl;
m is selected from 0, 1, 2, 3 and 4;
alternatively,
X is selected from N and CH;
R₄ is selected from H, halogen, C₁₋₂ alkyl and C₁₋₂ haloalkyl, such as H, Cl, Br, CH₃, CH₂CH₃ or CF₃;
R₄ₐ is selected from H and halogen, alternatively H or F;
R_{4b} is selected from halogen, C₁₋₂ alkyl and C₁₋₂ haloalkyl, alternatively Cl, Br, CF₃ or CH₂CH₃;
R_{4d} is selected from H, halogen and C₁₋₂ alkyl, alternatively H, F, Cl or CH₃;
R₅ is selected from H and C₁₋₂ alkyl, alternatively CH₃;
Rₓ is selected from H and C₁₋₂ alkyl, alternatively H;
m is selected from 0, 1, 2 and 3.

In a more specific embodiment, the present disclosure provides a compound of Formula (IV-10), or a pharmaceutically acceptable salt, isotopic variant, tautomer, stereoisomer, solvate or hydrate thereof, or a mixture thereof: 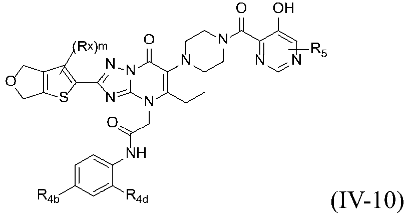 wherein,
R_{4b} is selected from halogen and C₁₋₆ haloalkyl;
R_{4d} is selected from halogen and C₁₋₆ alkyl;
R₅ is selected from C₁₋₆ alkyl and C₁₋₆ haloalkyl;
Rₓ is selected from H, C₁₋₆ alkyl and C₁₋₆ haloalkyl;
m is 0 or 1;
alternatively,
R_{4b} is selected from halogen and C₁₋₄ haloalkyl;
R_{4d} is selected from halogen and C₁₋₄ alkyl;
R₅ is selected from C₁₋₄ alkyl and C₁₋₄ haloalkyl;
Rₓ is selected from H, C₁₋₄ alkyl and C₁₋₄ haloalkyl;
m is 0 or 1;
more alternatively,
R_{4b} is selected from halogen and C₁₋₂ haloalkyl, alternatively C 1, Br or CF₃;
R_{4d} is selected from halogen and C₁₋₂ alkyl, alternatively F, Cl or CH₃;
R₅ is C₁₋₂ alkyl, alternatively CH₃;
Rₓ is H or C₁₋₂ alkyl, alternatively H;
m is 0 or 1.

In a more specific embodiment, the present disclosure provides a compound of Formula (II) or (II-1), or a pharmaceutically acceptable salt, isotopic variant, tautomer, stereoisomer, solvate or hydrate thereof, or a mixture thereof: 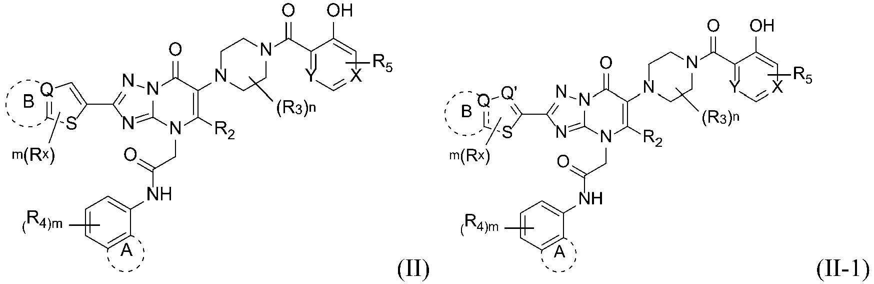 wherein,
-̅ -̅ -̅ represents a single bond or a double bond. When Q is N and the Ring B is present, -̅ -̅ -̅ represents a single bond;
Q and Q' are independently selected from CH and N, and at most one of Q and Q' is N;
X and Y are each independently selected from CH and N, and X and Y contain at least one N atom;
Ring A is absent or selected from C₃₋₁₀ cycloalkyl, 5-10 membered heteroaryl, 5-10 membered heterocyclyl and C₆₋₁₀ aryl, and the Ring A is alternatively absent;
Ring B is absent or selected from C₃₋₁₀ cycloalkyl, 5-10 membered heteroaryl, 5-10 membered heterocyclyl and C₆₋₁₀ aryl, and the Ring B is optionally substituted with 1, 2, 3, 4 or 5 substituents selected from C₁₋₆ alkyl and C₁₋₆ haloalkyl;
Rₓ is selected from H, NH₂, CN, OH, C₁₋₆ alkyl, C₁₋₆ alkoxy, C₁₋₆ haloalkyl, -C(O)Rₐ, -OC(O)Rₐ, -C(O)ORₐ, -C(O)NH-Rₐ, -NHC(O)Rₐ and -(CH₂)ₚ-ORₐ, wherein Rₐ is selected from H, C₁₋₆ alkyl and C₁₋₆ haloalkyl, and p is selected from 0, 1, 2, 3 and 4;
R₂ is selected from H, D, C₁₋₆ alkyl, C₁₋₆ alkoxy and C₁₋₆ haloalkyl;
R₃ is selected from H, D, C₁₋₆ alkyl, C₁₋₆ alkoxy, and C₁₋₆ haloalkyl;
R₄ is selected from H, D, halogen, CN, C₁₋₆ alkyl, C₁₋₆ alkoxy and C₁₋₆ haloalkyl;
R₅ is selected from H, D, halogen, CN, C₁₋₆ alkyl, C₁₋₆ alkoxy and C₁₋₆ haloalkyl;
m is selected from 0, 1, 2, 3 and 4;
n is selected from 0, 1 and 2.

In a more specific embodiment, the present disclosure provides the compound of Formula (II) or (II-1), or a pharmaceutically acceptable salt, isotopic variant, tautomer, stereoisomer, solvate or hydrate thereof, or a mixture thereof, wherein:
-̅ -̅ -̅ represents a single bond or a double bond. When Q is N and the Ring B is present, -̅ -̅ -̅ represents a single bond;
Q and Q' are independently selected from CH and N, and at most one of Q and Q' is N;
X and Y are each independently selected from CH and N, and X and Y contain at least one N atom;
Ring A is absent or selected from a 5-10 membered heteroaryl group and a C₆₋₁₀ aryl group, and the Ring A is alternatively absent;
Ring B is absent or selected from 5-10 membered heteroaryl, 5-10 membered heterocyclyl and C₆₋₁₀ aryl, and the Ring B is optionally substituted with 1, 2, 3, 4 or 5 substituents selected from C₁₋₆ alkyl and C₁₋₆ haloalkyl;
Rₓ is selected from H, NH₂, CN, C₁₋₆ alkyl, C₁₋₆ haloalkyl, -C(O)Rₐ, -OC(O)Rₐ, - C(O)ORₐ and -(CH₂)ₚ-ORₐ, wherein Rₐ is selected from H, C₁₋₆ alkyl and C₁₋₆ haloalkyl, and p is selected from 0, 1, 2, 3 and 4;
R₂ is selected from C₁₋₆ alkyl and C₁₋₆ haloalkyl;
R₃ is selected from H, C₁₋₆ alkyl and C₁₋₆ haloalkyl;
R₄ is selected from H, halogen, C₁₋₆ alkyl and C₁₋₆ haloalkyl;
R₅ is selected from H, C₁₋₆ alkyl and C₁₋₆ haloalkyl;
m is selected from 0, 1, 2, 3 and 4;
n is selected from 0, 1 and 2.

In a more specific embodiment, the present disclosure provides the compound of Formula (II) or (II-1), or a pharmaceutically acceptable salt, isotopic variant, tautomer, stereoisomer, solvate or hydrate thereof, or a mixture thereof, wherein:
-̅ -̅ -̅ represents a single bond or a double bond. When Q is N and the Ring B is present, -̅ -̅ -̅ represents a single bond;
Q and Q' are independently selected from CH and N, and at most one of Q and Q' is N;
X and Y are each independently selected from CH and N, and X and Y contain at least one N atom;
Ring A is absent or selected from a 5-8 membered heteroaryl group, and the Ring A is alternatively absent;
Ring B is absent or selected from 5-8 membered heteroaryl or 5-8 membered heterocyclyl, and the Ring B is optionally substituted with 1, 2 or 3 substituents selected from C₁₋₆ alkyl;
Rₓ is selected from H, NH₂, C₁₋₆ alkyl, -C(O)Rₐ and -(CH₂)ₚ-ORₐ, wherein Rₐ is selected from H and C₁₋₆ alkyl, and p is selected from 0, 1, 2 and 3;
R₂ is selected from C₁₋₆ alkyl;
R₃ is selected from H and C₁₋₆ alkyl;
R₄ is selected from H, halogen, C₁₋₆ alkyl and C₁₋₆ haloalkyl;
R₅ is selected from H and C₁₋₆ alkyl;
m is selected from 0, 1, 2 and 3;
n is selected from 0, 1 and 2.

In a more specific embodiment, the present disclosure provides the compound of Formula (II) or (II-1), or a pharmaceutically acceptable salt, isotopic variant, tautomer, stereoisomer, solvate or hydrate thereof, or a mixture thereof, wherein:
-̅ -̅ -̅ represents a single bond or a double bond. When Q is N and the Ring B is present, -̅ -̅ -̅ represents a single bond;
Q and Q' are independently selected from CH and N, and at most one of Q and Q' is N;
X and Y are each independently selected from CH and N, and X and Y contain at least one N atom;
Ring A is absent or selected from a 5-6 membered heteroaryl group, e.g., 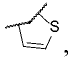 and the Ring A is alternatively absent;
Ring B is absent or selected from 5-6 membered heterocyclyl and 5-6 membered heteroaryl, and the Ring B is optionally substituted with 1 or 2 substituents selected from C₁₋₄ alkyl; alternatively, the Ring B is absent or selected from 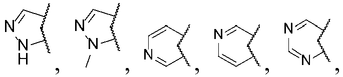 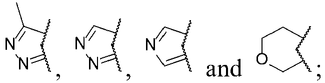
Rₓ is selected from H, NH₂, C₁₋₄ alkyl, -C(O)Rₐ and -(CH₂)ₚ-ORₐ, wherein Rₐ is selected from H and C₁₋₄ alkyl, and p is selected from 1 and 2;
Rₓ is alternatively H, NH₂, CH₃, CH₂OH, CH₂OCH₃ or C(O)CH₃;
R₂ is selected from C₁₋₄ alkyl, such as CH₃ or CH₂CH₃;
R₃ is selected from H and C₁₋₄ alkyl, alternatively H;
R₄ is selected from H, halogen, C₁₋₄ alkyl and C₁₋₄ haloalkyl, such as H, F, Cl, CH₃ or CF₃;
R₅ is selected from H and C₁₋₄ alkyl, such as H or CH₃;
m is selected from 0, 1, 2 and 3;
n is selected from 0, 1 and 2.

In a more specific embodiment, the present disclosure provides a compound of Formula (II-2) or (II-3), or a pharmaceutically acceptable salt, isotopic variant, tautomer, stereoisomer, solvate or hydrate thereof, or a mixture thereof: 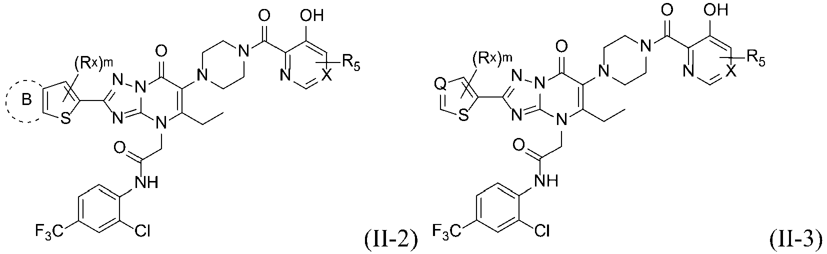 wherein,
X is selected from CH and N;
Q is selected from CH and N;
Ring B is absent or selected from 5-10 membered heteroaryl and C₆₋₁₀ aryl, wherein the 5-10 membered heteroaryl or C₆₋₁₀ aryl is optionally substituted with 1, 2, 3, 4 or 5 substituents selected from C₁₋₆ alkyl and C₁₋₆ haloalkyl;
Rₓ is selected from H, NH₂, C₁₋₆ alkyl, C₁₋₆ haloalkyl, -C(O)Rₐ, -C(O)ORₐ, -OC(O)Rₐ and -(CH₂)ₚ-ORₐ, wherein Rₐ is selected from H, C₁₋₆ alkyl and C₁₋₆ haloalkyl, and p is selected from 0, 1, 2, 3 and 4;
R₅ is selected from H, C₁₋₆ alkyl and C₁₋₆ haloalkyl;
m is selected from 0, 1, 2, 3 and 4;
alternatively,
X is selected from CH and N;
Q is selected from CH and N;
Ring B is absent or selected from 5-8 membered heteroaryl, wherein the 5-8 membered heteroaryl is optionally substituted with 1, 2 or 3 substituents selected from C₁₋₆ alkyl;
Rₓ is selected from H, NH₂, C₁₋₆ alkyl, -C(O)Rₐ and -(CH₂)ₚ-ORₐ, wherein Rₐ is selected from H and C₁₋₆ alkyl, and p is selected from 0, 1, 2 and 3;
R₅ is selected from H and C₁₋₆ alkyl;
m is selected from 0, 1, 2 and 3;
more alternatively,
X is selected from CH and N;
Q is selected from CH and N;
Ring B is absent or selected from 5-6 membered heteroaryl, wherein the 5-6 membered heteroaryl is optionally substituted with 1 or 2 substituents selected from C₁₋₄ alkyl; alternatively, the Ring B is absent or selected from 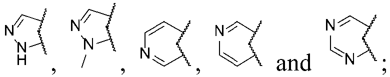
Rₓ is selected from H, NH₂, C₁₋₄ alkyl, -C(O)Rₐ and -(CH₂)ₚ-ORₐ, wherein Rₐ is selected from H and C₁₋₄ alkyl, and p is selected from 1 and 2;
Rₓ is alternatively H, NH₂, CH₃, CH₂OH, CH₂OCH₃ or C(O)CH₃;
R₅ is selected from H and C₁₋₄ alkyl, such as H or CH₃;
m is selected from 0, 1 and 2.

In a more specific embodiment, the present disclosure provides a compound of Formula (VI-1) or (VI-2), or a pharmaceutically acceptable salt, isotopic variant, tautomer, stereoisomer, solvate or hydrate thereof, or a mixture thereof: 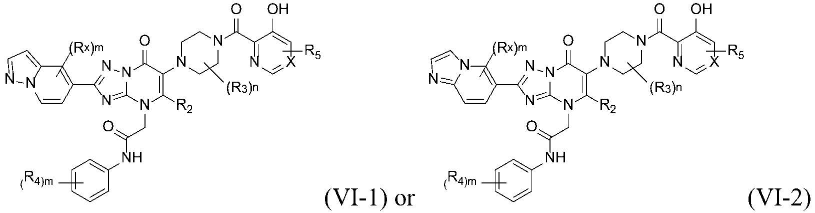
X is selected from CH and N;
Rₓ is selected from H, C₁₋₆ alkyl and C₁₋₆ haloalkyl;
R₂ is selected from H, C₁₋₆ alkyl, C₁₋₆ alkoxy and C₁₋₆ haloalkyl;
R₃ is selected from H, C₁₋₆ alkyl, C₁₋₆ alkoxy and C₁₋₆ haloalkyl;
R₄ is selected from H, CN, halogen, C₁₋₆ alkyl, C₁₋₆ alkoxy and C₁₋₆ haloalkyl;
R₅ is selected from H, C₁₋₆ alkyl, C₁₋₆ alkoxy and C₁₋₆ haloalkyl;
m is selected from 0, 1, 2, 3 and 4;
n is selected from 0, 1, 2 and 3;
alternatively,
X is selected from CH and N;
Rₓ is selected from H and C₁₋₆ alkyl;
R₂ is selected from H and C₁₋₆ alkyl;
R₃ is selected from H and C₁₋₆ alkyl;
R₄ is selected from H, halogen, C₁₋₆ alkyl and C₁₋₆ haloalkyl;
R₅ is selected from H and C₁₋₆ alkyl;
m is selected from 0, 1, 2 and 3;
n is selected from 0, 1 and 2;
more alternatively,
X is selected from CH and N;
Rₓ is selected from H and C₁₋₄ alkyl, alternatively H;
R₂ is selected from H and C₁₋₄ alkyl, alternatively CH₂CH₃;
R₃ is selected from H and C₁₋₄ alkyl, alternatively H;
R₄ is selected from H, halogen, C₁₋₄ alkyl and C₁₋₄ haloalkyl, such as H, F, Cl, CH₃ or CF₃;
R₅ is selected from H and C₁₋₄ alkyl, alternatively CH₃;
m is selected from 0, 1, 2 and 3;
n is selected from 0, 1 and 2.

In a more specific embodiment, the present disclosure provides a compound of Formula (VI-3) or (VI-4), or a pharmaceutically acceptable salt, isotopic variant, tautomer, stereoisomer, solvate or hydrate thereof, or a mixture thereof, wherein the compound has the structure of (VI-3) or (VI-4): 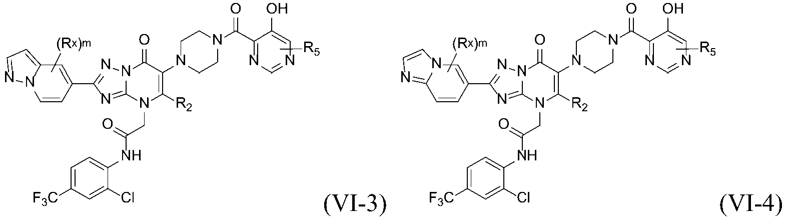 wherein,
Rₓ is selected from H, C₁₋₆ alkyl and C₁₋₆ haloalkyl;
R₂ is selected from H, C₁₋₆ alkyl and C₁₋₆ haloalkyl;
R₅ is selected from H, C₁₋₆ alkyl and C₁₋₆ haloalkyl;
m is selected from 0, 1, 2 and 3;
alternatively,
Rₓ is selected from H and C₁₋₄ alkyl, alternatively H;
R₂ is selected from H and C₁₋₄ alkyl, alternatively CH₂CH₃;
R₅ is selected from H and C₁₋₄ alkyl, alternatively CH₃;
m is selected from 0, 1, 2 and 3.

In an embodiment, the present disclosure provides a compound of Formula (I), or a pharmaceutically acceptable salt, isotopic variant, tautomer or stereoisomer thereof: 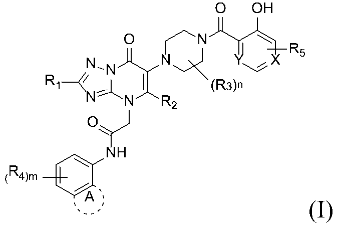 wherein,
X and Y are each independently selected from CH and N, and at least one of X and Y is N atom;
R₁ is selected from 5-12 membered heteroaryl and 5-12 membered heterocyclic group; and the R₁ may be substituted with 1, 2 or 3 Rₓ;
provided that, when R₁ is selected from a 5-12 membered monocyclic heterocyclic group, R₁ is 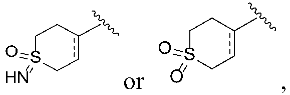 and -̅ -̅ -̅ -̅ -̅ -̅ represents a single bond or a double bond;
provided that, when R₁ is selected from 5-12 membered heteroaryl, R₁ is not pyridinyl;
R₂ is selected from H, C₁₋₆ alkyl, C₁₋₆ haloalkyl, C₁₋₆ alkoxy, C₁₋₆ alkylthio and C₃₋₆ cycloalkyl;
R₃ is independently selected from H, halogen, CN, C₁₋₆ alkyl, C₁₋₆ haloalkyl and C₃₋₆ cycloalkyl; or two R₃ are connected to the carbon atom(s) to which they are attached to form a 3-6 membered spiro ring or a bridged ring;
R₄ is independently selected from H, halogen, CN, SF₅, C₁₋₆ alkyl, C₁₋₆ haloalkyl, C₁₋₆ alkylthio, C₃₋₆ cycloalkyl and 4-10 membered heterocyclyl;
Ring A is present or absent, and selected from a 5-6 membered heteroaryl group and a 5-7 membered heterocyclic group;
R₅ is selected from H, C₁₋₆ alkyl, C₁₋₆ haloalkyl, C₁₋₆ alkoxy, C₁₋₆ alkylthio and C₃₋₆ cycloalkyl;
Rₓ is selected from H, C₁₋₆ alkyl, C₁₋₆ haloalkyl, C₁₋₆ alkoxy, CN, NH₂, -C(O)Rₐ, -C (O)ORₐ, -(CH₂)ₚ-ORₐ, -P(O)-(Rₐ)₂ and -S(O)₂-Rₐ;
Rₐ is selected from H, C₁₋₆ alkyl and C₃₋₆ cycloalkyl;
m is selected from 0, 1, 2 and 3;
n is selected from 0, 1 and 2;
p is selected from 0, 1 and 2.

In an embodiment, the present disclosure provides the compound of Formula (I), or a pharmaceutically acceptable salt, isotopic variant, tautomer or stereoisomer thereof, or a mixture thereof, wherein:
X and Y are each independently selected from CH and N, and at least one of X and Y is N atom;
Ring B is present or absent, and selected from a 5-6 membered heteroaryl and a 5-6 membered heterocyclic group;
X₁ and X₂ are each independently selected from CH and N, and -̅ -̅ -̅ -̅ -̅ -̅ represents a single bond or a double bond;
Q is selected from CH and N;
R₂ is selected from H, C₁₋₆ alkyl, C₁₋₆ haloalkyl, C₁₋₆ alkoxy, C₁₋₆ alkylthio and C₃₋₆ cycloalkyl;
R₃ is independently selected from H, halogen, CN, methyl, ethyl, trifluoromethyl and cyclopropyl;
R₄ is independently selected from H, halogen, CN, SF₅, C₁₋₆ alkyl, C₁₋₆ haloalkyl, C₁₋₆ alkylthio, C₃₋₆ cycloalkyl and 4-10 membered heterocyclyl;
Ring A is present or absent, and selected from a 5-6 membered heteroaryl group and a 5-7 membered heterocyclic group;
R₅ is selected from H, methyl, ethyl, trifluoromethyl, methoxy, ethoxy, methylthio and cyclopropyl;
Rₓ is selected from H, C₁₋₆ alkyl, C₁₋₆ haloalkyl, C₁₋₆ alkoxy, CN, NH₂, -C(O)Rₐ, - C(O)ORₐ, -(CH₂)ₚ-ORₐ, -P(O)-(Rₐ)₂ and -S(O)₂-Rₐ;
Rₐ is selected from H and C₁₋₆ alkyl;
m is selected from 0, 1, 2 and 3;
n is selected from 0, 1 and 2;
p is selected from 0, 1 and 2.

In an embodiment, the present disclosure provides a compound of Formula (IV-1), Formula (IV-2), Formula (IV-3), Formula (IV-4), Formula (IV-5), Formula (IV-6), Formula (VI-1) or Formula (VI-2), or a pharmaceutically acceptable salt, isotopic variant, tautomer or stereoisomer thereof, or a mixture thereof, which have a general structure of: 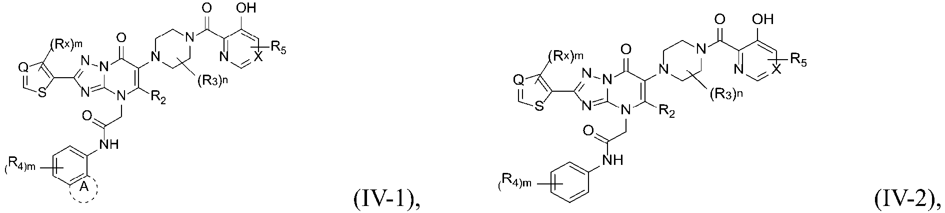 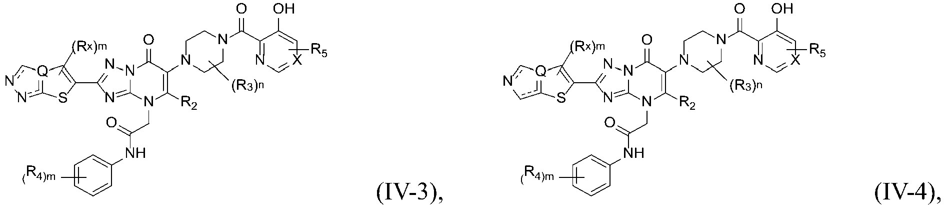 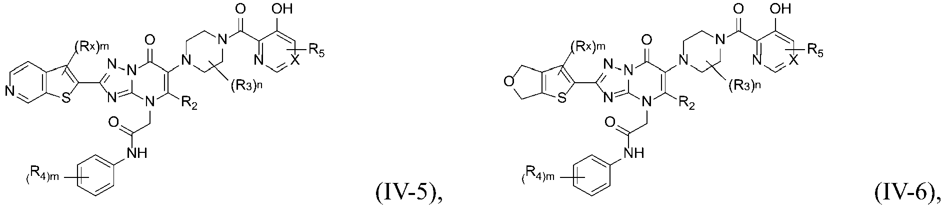 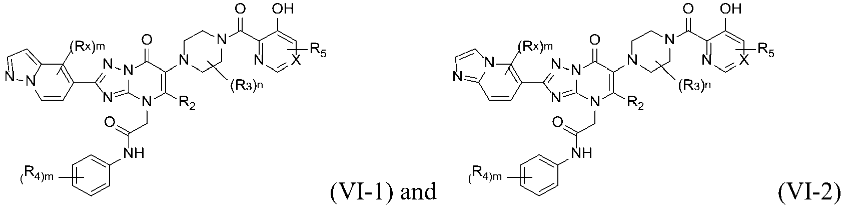 wherein,
X is selected from CH and N;
-̅ -̅ -̅ -̅ -̅ -̅ represents a single bond or a double bond;
Q is selected from CH and N;
R₂ is selected from H, C₁₋₆ alkyl, C₁₋₆ haloalkyl, C₁₋₆ alkoxy, C₁₋₆ alkylthio and C₃₋₆ cycloalkyl;
R₃ is independently selected from H, halogen, CN, C₁₋₆ alkyl, C₁₋₆ haloalkyl and C₃₋₆ cycloalkyl; or two R₃ are connected to the carbon atom(s) to which they are attached to form a 3-6 membered spiro ring or a bridged ring;
R₄ is independently selected from H, halogen, CN, SF₅, C₁₋₆ alkyl, C₁₋₆ haloalkyl, C₁₋₆ alkylthio, C₃₋₆ cycloalkyl and 4-10 membered heterocyclyl;
Ring A is present or absent, and selected from a 5-6 membered heteroaryl group and a 5-7 membered heterocyclic group;
R₅ is selected from H, C₁₋₆ alkyl, C₁₋₆ haloalkyl, C₁₋₆ alkoxy, C₁₋₆ alkylthio and C₃₋₆ cycloalkyl;
Rₓ is selected from H, C₁₋₆ alkyl, C₁₋₆ haloalkyl, C₁₋₆ alkoxy, CN, NH₂, -C(O)Rₐ, - C(O)ORₐ, -(CH₂)ₚ-ORₐ, -P(O)-(Rₐ)₂ and -S(O)₂-Rₐ;
Rₐ is selected from H, C₁₋₆ alkyl and C₃₋₆ cycloalkyl;
m is selected from 0, 1, 2 and 3;
n is selected from 0, 1 and 2;
p is selected from 0, 1 and 2.

In another embodiment, the present disclosure relates to a compound of Formula (II), Formula (III) or Formula (VI), or a pharmaceutically acceptable salt, isotopic variant, tautomer or stereoisomer thereof: 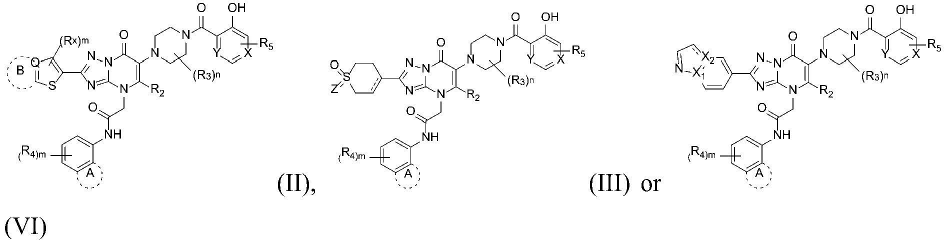 wherein,
X and Y are each independently selected from CH and N, and at least one of X and Y is N atom;
Ring B is present or absent, and selected from a 5-6 membered heteroaryl and a 5-6 membered heterocyclic group;
Z is selected from O and NH, and -̅ -̅ -̅ -̅ -̅ -̅ represents a single bond or a double bond;
X₁ and X₂ are each independently selected from CH and N, and -̅ -̅ -̅ -̅ -̅ -̅ represents a single bond or a double bond;
Q is selected from CH and N;
R₂ is selected from H, C₁₋₆ alkyl, C₁₋₆ haloalkyl, C₁₋₆ alkoxy, C₁₋₆ alkylthio and C₃₋₆ cycloalkyl;
R₃ is independently selected from H, halogen, CN, C₁₋₆ alkyl, C₁₋₆ haloalkyl and C₃₋₆ cycloalkyl; or two R₃ are connected to the carbon atom(s) to which they are attached to form a 3-6 membered spiro ring or a bridged ring;
R₄ is independently selected from H, halogen, CN, SF₅, C₁₋₆ alkyl, C₁₋₆ haloalkyl, C₁₋₆ alkylthio, C₃₋₆ cycloalkyl and 4-10 membered heterocyclyl;
Ring A is present or absent, and selected from a 5-6 membered heteroaryl group and a 5-7 membered heterocyclic group;
R₅ is selected from H, C₁₋₆ alkyl, C₁₋₆ haloalkyl, C₁₋₆ alkoxy, C₁₋₆ alkylthio and C₃₋₆ cycloalkyl;
Rₓ is selected from H, C₁₋₆ alkyl, C₁₋₆ haloalkyl, C₁₋₆ alkoxy, CN, NH₂, -C(O)Rₐ, - C(O)ORₐ, -(CH₂)ₚ-ORₐ, -P(O)-(Rₐ)₂ and -S(O)₂-Rₐ;
Rₐ is selected from H, C₁₋₆ alkyl and C₃₋₆ cycloalkyl;
m is selected from 0, 1, 2 and 3;
n is selected from 0, 1 and 2;
p is selected from 0, 1 and 2.

In another more specific embodiment, the present disclosure relates to a compound of Formula (II), Formula (III) or Formula (VI), or a pharmaceutically acceptable salt, isotopic variant, tautomer or stereoisomer thereof:
wherein,
X and Y are each independently selected from CH and N, and at least one of X and Y is N atom;
Ring B is present or absent, and selected from a 5-6 membered heteroaryl group and a 5-6 membered heterocyclic group;
Z is selected from O and NH, and -̅ -̅ -̅ -̅ -̅ -̅ represents a single bond or a double bond;
X₁ and X₂ are each independently selected from CH and N, and -̅ -̅ -̅ -̅ -̅ -̅ represents a single bond or a double bond;
Q is selected from CH and N;
R₂ is selected from H, C₁₋₆ alkyl, C₁₋₆ haloalkyl, C₁₋₆ alkoxy, C₁₋₆ alkylthio and C₃₋₆ cycloalkyl;
R₃ is independently selected from H, halogen, CN, methyl, ethyl, trifluoromethyl, and cyclopropyl;
R₄ is independently selected from H, halogen, CN, SF₅, C₁₋₆ alkyl, C₁₋₆ haloalkyl, C₁₋₆ alkylthio, C₃₋₆ cycloalkyl and 4-10 membered heterocyclyl;
Ring A is present or absent, and selected from a 5-6 membered heteroaryl group and a 5-7 membered heterocyclic group;
R₅ is selected from H, methyl, ethyl, trifluoromethyl, methoxy, ethoxy, methylthio and cyclopropyl;
Rₓ is selected from H, C₁₋₆ alkyl, C₁₋₆ haloalkyl, C₁₋₆ alkoxy, CN, NH₂, -C(O)Rₐ, - C(O)ORₐ, -(CH₂)ₚ-ORₐ, -P(O)-(Rₐ)₂ and -S(O)₂-Rₐ;
Rₐ is selected from H and C₁₋₆ alkyl;
m is selected from 0, 1, 2 and 3;
n is selected from 0, 1 and 2;
p is selected from 0, 1 and 2.

In another embodiment, the present disclosure is directed to a compound of Formula (IV-1), Formula (IV-2), Formula (IV-3), Formula (IV-4), Formula (IV-5), Formula (IV-6) or Formula (V-1), or a pharmaceutically acceptable salt, isotopic variant, tautomer or stereoisomer thereof: 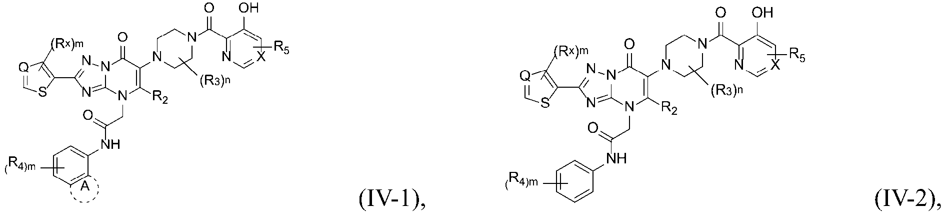 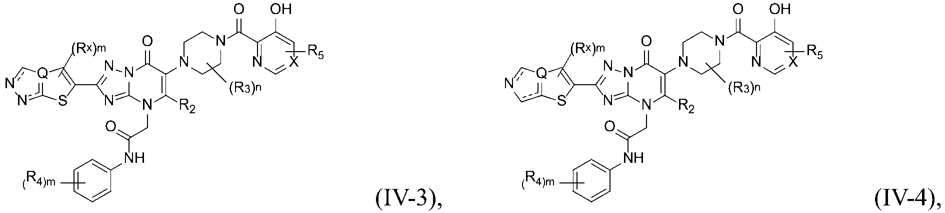 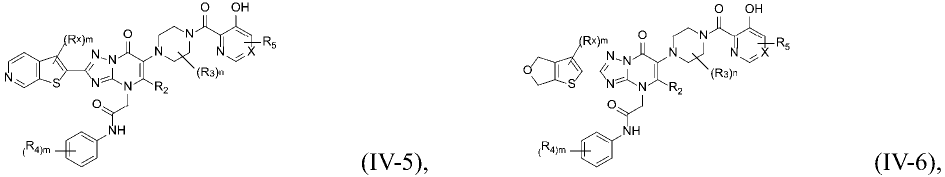 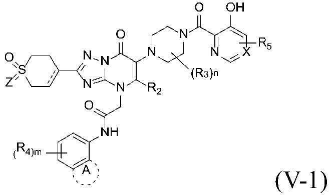 wherein,
X is selected from CH and N;
Z is selected from O and NH, and -̅ -̅ -̅ -̅ -̅ -̅ represents a single bond or a double bond;
Q is selected from CH and N;
R₂ is selected from H, C₁₋₆ alkyl, C₁₋₆ haloalkyl, C₁₋₆ alkoxy, C₁₋₆ alkylthio and C₃₋₆ cycloalkyl;
R₃ is independently selected from H, halogen, CN, C₁₋₆ alkyl, C₁₋₆ haloalkyl and C₃₋₆ cycloalkyl; or two R₃ are connected to the carbon atom(s) to which they are attached to form a 3-6 membered spiro ring or a bridged ring;
R₄ is independently selected from H, halogen, CN, SF₅, C₁₋₆ alkyl, C₁₋₆ haloalkyl, C₁₋₆ alkylthio, C₃₋₆ cycloalkyl and 4-10 membered heterocyclyl;
Ring A is present or absent, and selected from a 5-6 membered heteroaryl group and a 5-7 membered heterocyclic group;
R₅ is selected from H, C₁₋₆ alkyl, C₁₋₆ haloalkyl, C₁₋₆ alkoxy, C₁₋₆ alkylthio and C₃₋₆ cycloalkyl;
Rₓ is selected from H, C₁₋₆ alkyl, C₁₋₆ haloalkyl, C₁₋₆ alkoxy, CN, NH₂, -C(O)Rₐ, - C(O)ORₐ, -(CH₂)ₚ-ORₐ, -P(O)-(Rₐ)₂ and -S(O)₂-Rₐ;
Rₐ is selected from H, C₁₋₆ alkyl and C₃₋₆ cycloalkyl;
m is selected from 0, 1, 2 and 3;
n is selected from 0, 1 and 2;
p is selected from 0, 1 and 2.

In another more specific embodiment, the present disclosure relates to the compound of Formula (IV-1), Formula (IV-2), Formula (IV-3), Formula (IV-4), Formula (IV-5), Formula (IV-6) or Formula (V-1), or a pharmaceutically acceptable salt, isotopic variant, tautomer, or stereoisomer thereof:
wherein,
X is selected from CH and N;
Z is selected from O and NH, and -̅ -̅ -̅ -̅ -̅ -̅ represents a single bond or a double bond;
Q is selected from CH and N;
R₂ is selected from H, methyl, ethyl, trifluoroethyl, methoxy and cyclopropyl;
R₃ is independently selected from H, F, CN, methyl, ethyl, trifluoromethyl and cycloalkyl; or two R₃ are connected to the carbon atom to which they are attached to form a cycloalkyl or cyclobutyl group;
R₄ is independently selected from H, F, Cl, Br, CN, SF₅, methyl, ethyl, trifluoromethyl, difluoromethyl, methylthio, ethylthio and cyclopropyl;
Ring A is present or absent, and selected from a 5-6 membered heteroaryl group and a 5-7 membered heterocyclic group;
R₅ is selected from H, methyl, ethyl, trifluoromethyl, methoxy, methylthio and cyclopropyl;
Rₓ is selected from H, C₁₋₆ alkyl, C₁₋₆ haloalkyl, C₁₋₆ alkoxy, CN, NH₂, -C(O)Rₐ, - C(O)ORₐ, -(CH₂)ₚ-ORₐ, -P(O)-(Rₐ)₂ and -S(O)₂-Rₐ;
Rₐ is selected from H, C₁₋₆ alkyl and C₃₋₆ cycloalkyl;
m is selected from 0, 1, 2 and 3;
n is selected from 0, 1 and 2;
p is selected from 0, 1 and 2.

In another more specific embodiment, the present disclosure is directed to a compound of Formula (IV-3A) or Formula (IV-6A), or a pharmaceutically acceptable salt, isotopic variant, tautomer, or stereoisomer thereof: 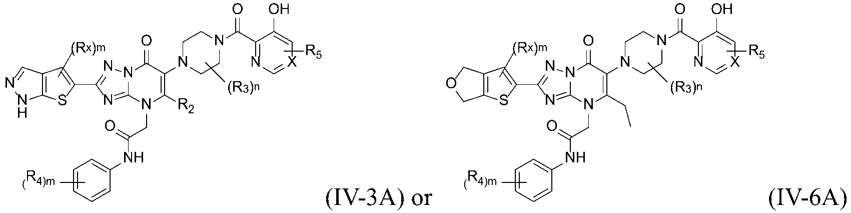 wherein,
X is selected from CH and N;
R₂ is selected from H, C₁₋₆ alkyl, C₁₋₆ haloalkyl, C₁₋₆ alkoxy, C₁₋₆ alkylthio and C₃₋₆ cycloalkyl;
R₃ is independently selected from H, halogen, CN, C₁₋₆ alkyl, C₁₋₆ haloalkyl and C₃₋₆ cycloalkyl; or two R₃ are connected to the carbon atom(s) to which they are attached to form a 3-6 membered spiro ring or a bridged ring;
R₄ is independently selected from H, halogen, CN, SF₅, C₁₋₆ alkyl, C₁₋₆ haloalkyl, C₁₋₆ alkylthio, C₃₋₆ cycloalkyl and 4-10 membered heterocyclyl;
R₅ is selected from H, C₁₋₆ alkyl, C₁₋₆ haloalkyl, C₁₋₆ alkoxy, C₁₋₆ alkylthio and C₃₋₆ cycloalkyl;
Rₓ is selected from H, C₁₋₆ alkyl, C₁₋₆ haloalkyl, C₁₋₆ alkoxy, CN, NH₂, -C(O)Rₐ, - C(O)ORₐ, -(CH₂)ₚ-ORₐ, -P(O)-(Rₐ)₂ and -S(O)₂-Rₐ;
Rₐ is selected from H, C₁₋₆ alkyl and C₃₋₆ cycloalkyl;
m is selected from 0, 1, 2 and 3;
n is selected from 0, 1 and 2;
p is selected from 0, 1 and 2.

In another more specific embodiment, the present disclosure relates to the compound of Formula (IV-3A) or Formula (IV-6A), or a pharmaceutically acceptable salt, isotopic variant, tautomer, stereoisomer, solvate or hydrate thereof, or a mixture thereof:
wherein,
X is selected from CH and N;
R₂ is selected from H, methyl, ethyl, trifluoroethyl, methoxy and cyclopropyl;
R₃ is independently selected from H, F, CN, methyl, ethyl, trifluoromethyl and cycloalkyl; or two R₃ are connected to the carbon atom to which they are attached to form a cycloalkyl or cyclobutyl group;
R₄ is independently selected from H, F, Cl, Br, CN, SF₅, methyl, ethyl, trifluoromethyl, difluoromethyl, methylthio, ethylthio and cyclopropyl;
R₅ is selected from H, methyl, ethyl, trifluoromethyl, methoxy, methylthio and cyclopropyl;
Rₓ is selected from H, C₁₋₆ alkyl, C₁₋₆ haloalkyl, C₁₋₆ alkoxy, CN, NH₂, -C(O)Rₐ, - C(O)ORₐ, -(CH₂)ₚ-ORₐ, -P(O)-(Rₐ)₂ and -S(O)₂-Rₐ;
Rₐ is selected from H, C₁₋₆ alkyl and C₃₋₆ cycloalkyl;
m is selected from 0, 1, 2 and 3;
n is selected from 0, 1 and 2;
p is selected from 0, 1 and 2.

In another specific embodiment, the present disclosure relates to a compound of Formula (VI-1) or Formula (VI-2), or a pharmaceutically acceptable salt, isotopic variant, tautomer, or stereoisomer thereof: 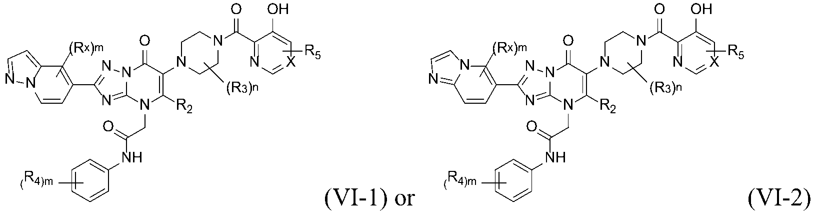
X is selected from CH and N;
R₂ is selected from H, C₁₋₆ alkyl, C₁₋₆ haloalkyl, C₁₋₆ alkoxy, C₁₋₆ alkylthio and C₃₋₆ cycloalkyl;
R₃ is independently selected from H, halogen, CN, C₁₋₆ alkyl, C₁₋₆ haloalkyl and C₃₋₆ cycloalkyl; or two R₃ are connected to the carbon atom(s) to which they are attached to form a 3-6 membered spiro ring or a bridged ring;
R₄ is independently selected from H, halogen, CN, SF₅, C₁₋₆ alkyl, C₁₋₆ haloalkyl, C₁₋₆ alkylthio, C₃₋₆ cycloalkyl and 4-10 membered heterocyclyl;
R₅ is selected from H, C₁₋₆ alkyl, C₁₋₆ haloalkyl, C₁₋₆ alkoxy, C₁₋₆ alkylthio and C₃₋₆ cycloalkyl;
Rₓ is selected from H, C₁₋₆ alkyl, C₁₋₆ haloalkyl, C₁₋₆ alkoxy, CN, NH₂, -C(O)Rₐ, - C(O)ORₐ, -(CH₂)ₚ-ORₐ, -P(O)-(Rₐ)₂ and -S(O)₂-Rₐ;
Rₐ is selected from H, C₁₋₆ alkyl and C₃₋₆ cycloalkyl;
m is selected from 0, 1, 2 and 3;
n is selected from 0, 1 and 2;
p is selected from 0, 1 and 2.

In another embodiment, the present disclosure relates to a compound or its tautomer, stereoisomer or pharmaceutically acceptable salt, wherein the compound is selected from: 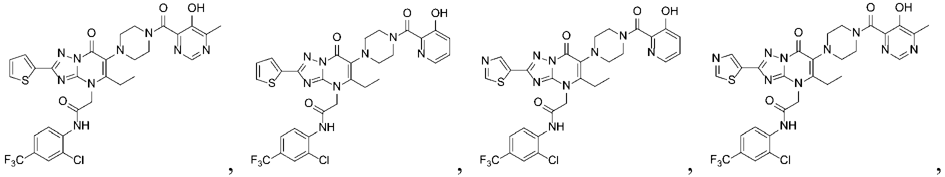 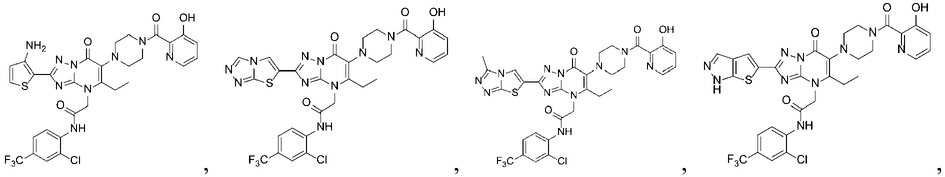 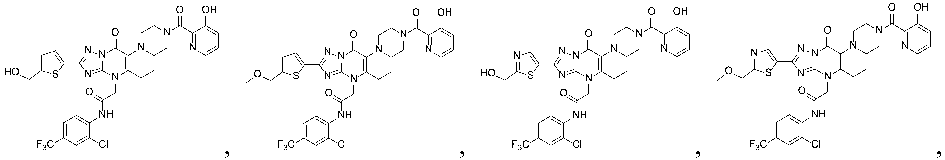 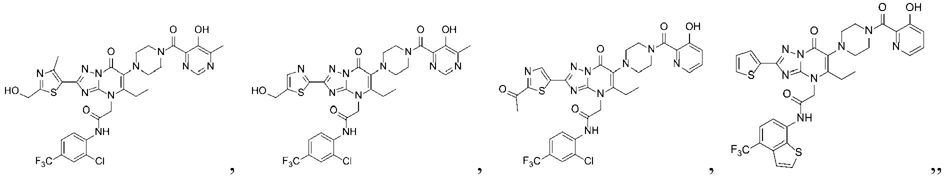 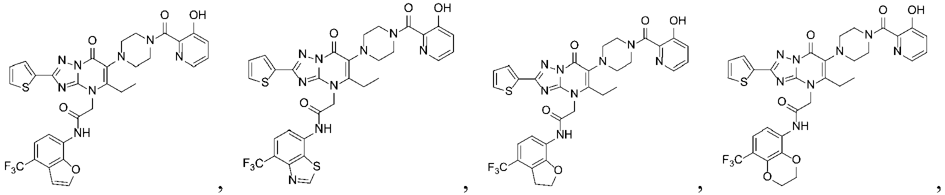 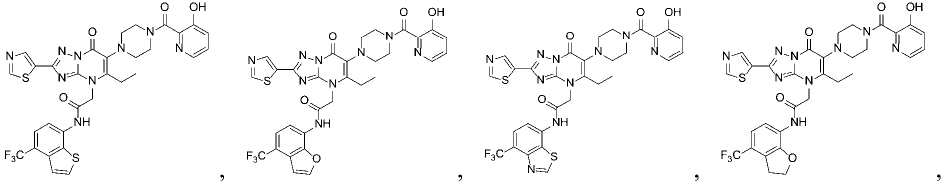 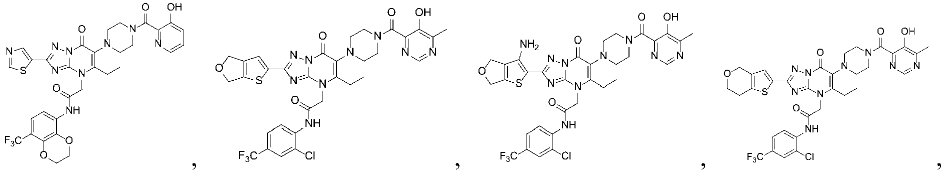 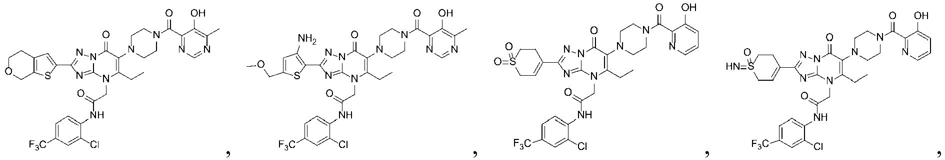 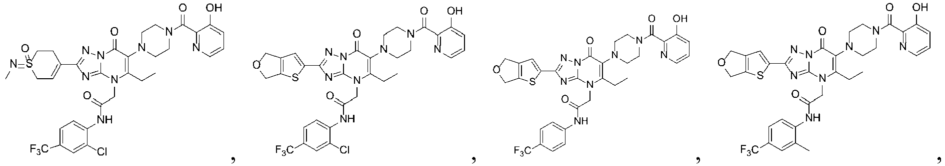 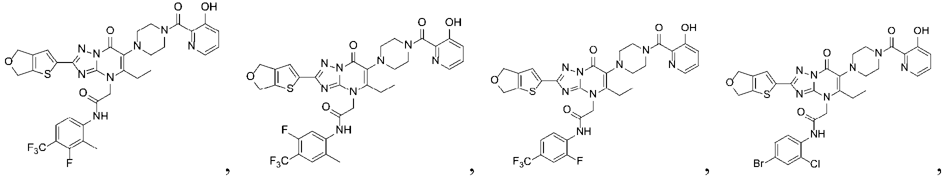 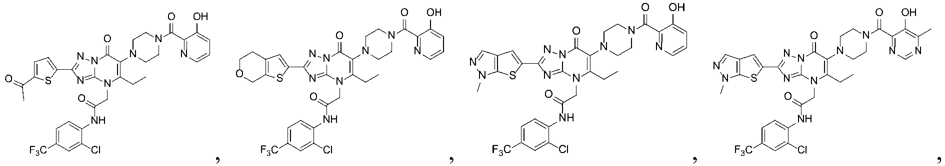 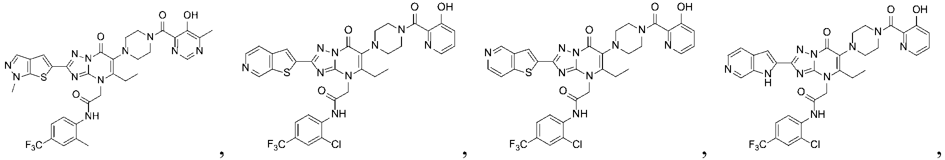 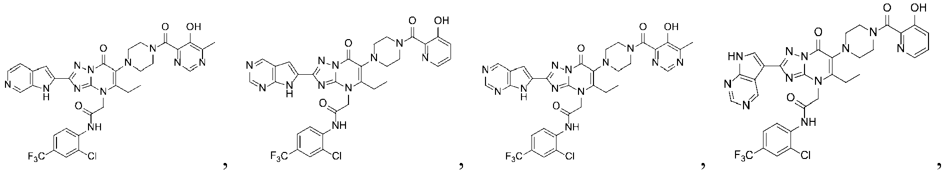 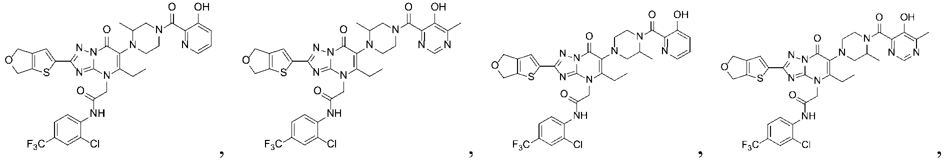 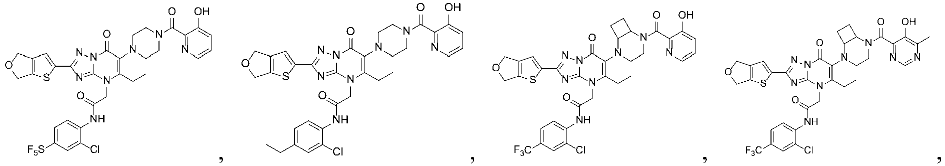 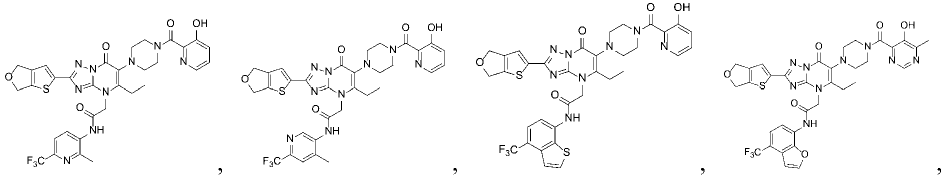 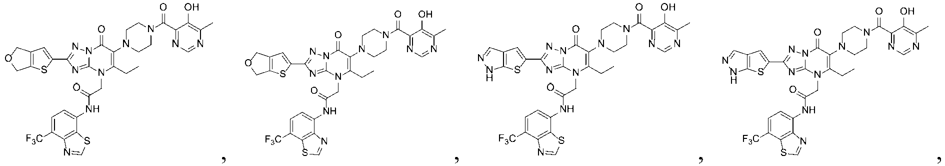 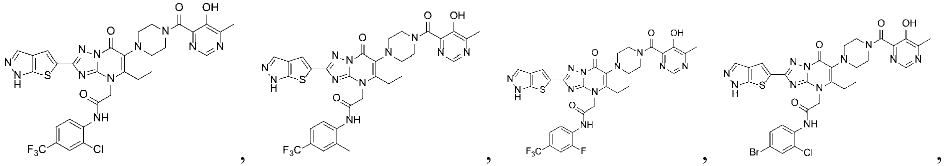 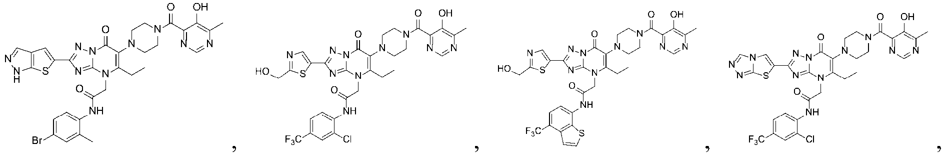 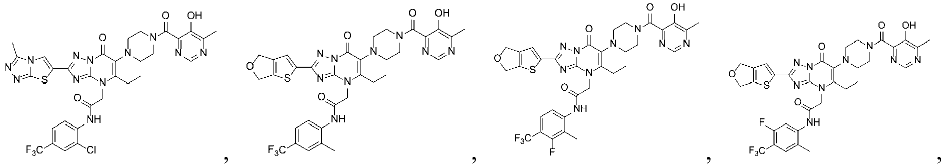 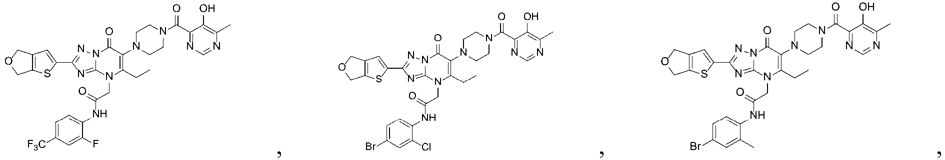 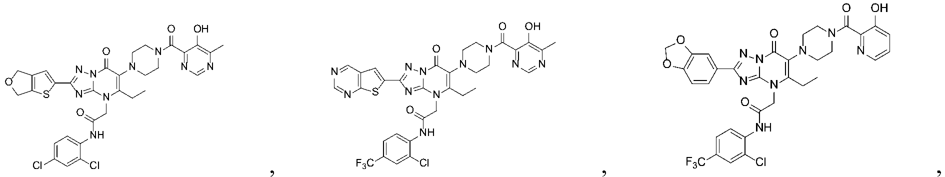 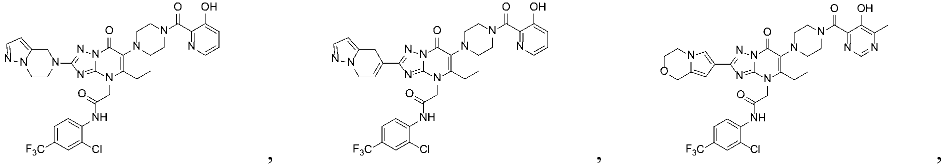 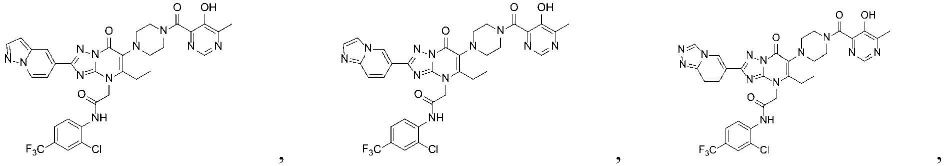 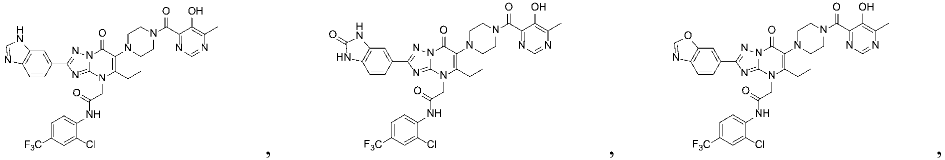 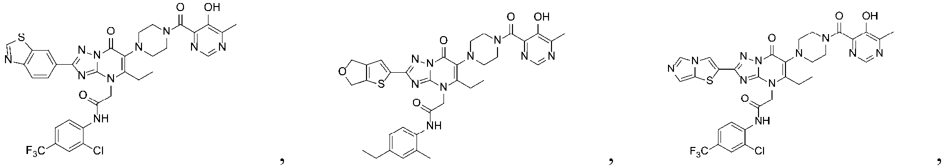 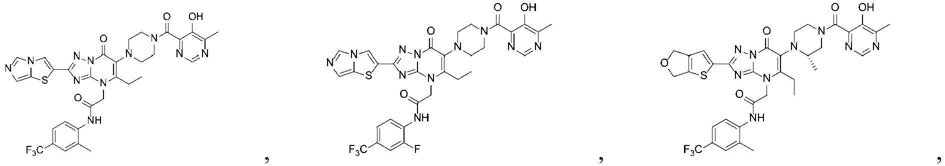 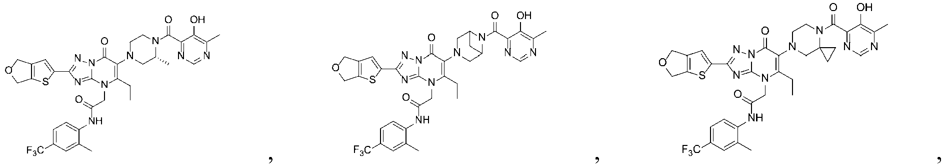 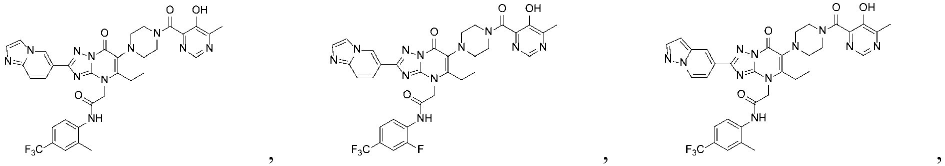 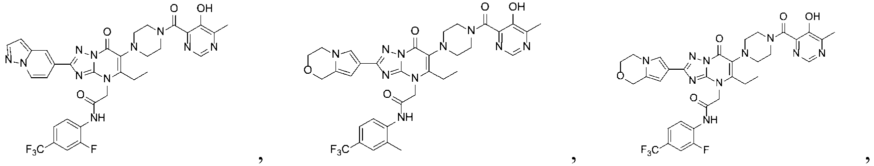 and

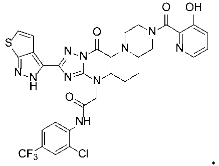

In another embodiment, the present disclosure relates to a compound, or a pharmaceutically acceptable salt, isotopic variant, tautomer, stereoisomer thereof, wherein the compound is selected from: 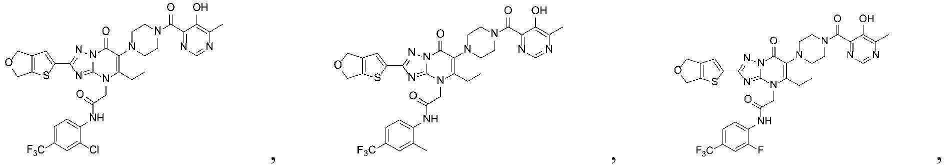 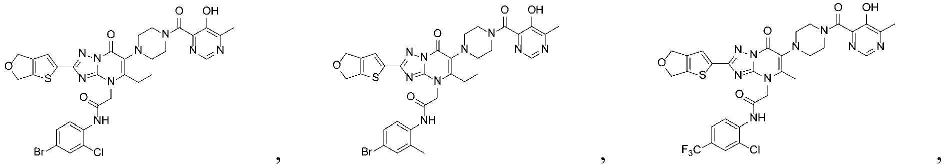 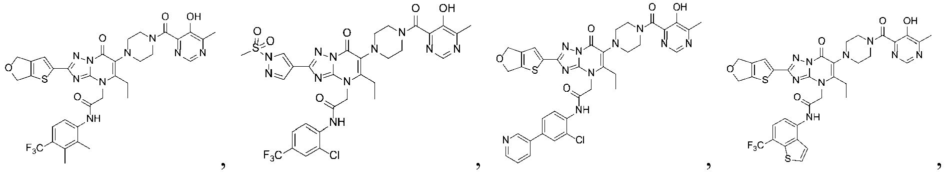 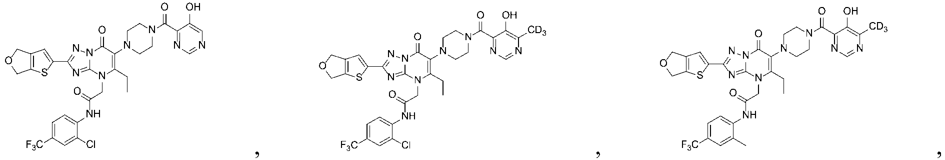 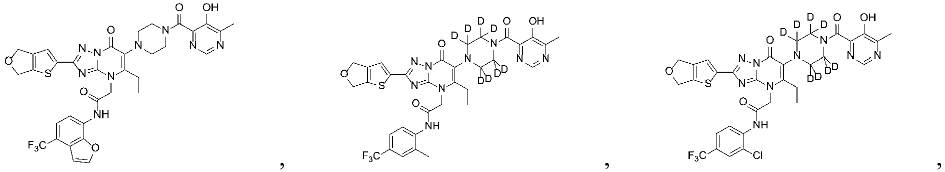 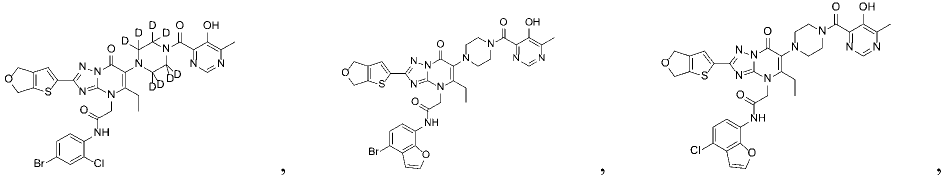 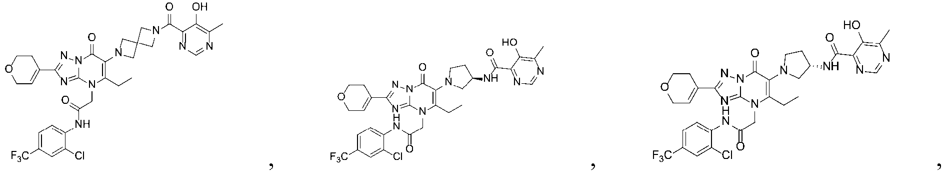 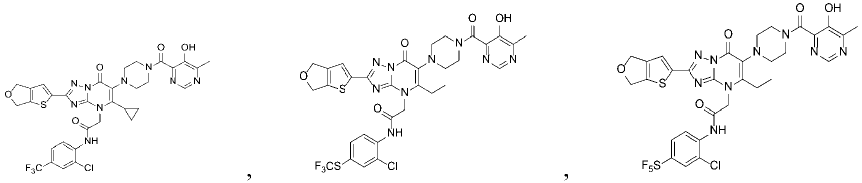 and

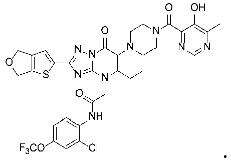

The compounds of the present disclosure may include one or more asymmetric centers, and therefore may exist in a variety of stereoisomeric forms, e.g., enantiomers and /or diastereoisomeric forms. For example, the compounds of the present disclosure may be individual enantiomers, diastereomers or geometric isomers (e.g., cis and trans isomers), or a mixture of stereoisomers including racemic mixtures and mixtures enriched in one or more stereoisomers. The isomers may be separated from mixtures via methods known to those skilled in the art, including chiral high pressure liquid chromatography (HPLC) and the formation and crystallization of chiral salts; or alternatively the isomers may be prepared by asymmetric synthesis.

The compounds of the present disclosure may also exist as tautomers. For those existing in different tautomeric forms, the compound is not limited to any specific tautomer, but intended to encompass all tautomeric forms.

The present disclosure also includes isotopically labeled compounds (isotopic variants), which are equivalent to those described in Formula (A), but have one or more atoms replaced by atom(s) having an atomic mass or mass number different from that commonly found in nature. Examples of the isotopes that may be introduced into the compounds of the present disclosure include isotopes of hydrogen, carbon, nitrogen, oxygen, phosphorus, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F and ³⁶Cl, respectively. All the compounds of the present disclosure containing the above-mentioned isotopes and/or other isotopes of other atoms, their prodrugs and pharmaceutically acceptable salts of the compounds or prodrugs fall within the scope of the present disclosure. Certain isotopically labeled compounds of the present disclosure, such as those having radioactive isotopes (e.g., ³H and ¹⁴C) introduced, may be used in drug and/or substrate tissue distribution assays. Tritium (i.e., ³H) and carbon-14 (i.e., ¹⁴C) isotopes are particularly alternative because they are easy to prepare and detect. Furthermore, substitution with heavier isotopes, such as deuterium (i.e., ²H) may be alternative in some cases due to the higher metabolic stability, which may provide therapeutic benefits, such as increased half-life in vivo or reduced dosage requirements. Isotopically labeled compounds of Formula (A) of the present disclosure and their prodrugs may generally be prepared by replacing non-isotopically labeled reagents with readily available isotopically labeled reagents when carrying out the processes disclosed in the following schemes and/or the Examples and Preparations.

### Pharmaceutical Compositions And Kits

On the other hand, the present disclosure provides a pharmaceutical composition comprising the compound of the present disclosure (also referred to as an "active ingredient") and a pharmaceutically acceptable excipient. In some embodiments, the pharmaceutical composition comprises an effective amount of a compound of the present disclosure. In some embodiments, the pharmaceutical composition comprises a therapeutically effective amount of the compound of the present disclosure. In some embodiments, the pharmaceutical composition comprises a prophylactically effective amount of the compound of the present disclosure.

The pharmaceutically acceptable excipient used in the present disclosure refers to nontoxic carriers, adjuvants or vehicles that do not destroy the pharmacological activity of the compound with which they are formulated together. Pharmaceutically acceptable carriers, adjuvants or vehicles that may be used in the composition of the present disclosure include, but are not limited to, ion exchangers, aluminum oxide, aluminum stearate, lecithin, serum proteins (e.g., human serum albumin), buffer substances (e.g., phosphates), glycine, sorbic acid, potassium sorbate, partial glyceride mixtures of saturated vegetable fatty acids, water, salts or electrolytes (e.g., protamine sulfate), disodium hydrogen phosphate, potassium hydrogen phosphate, sodium chloride, zinc salts, silica gel, magnesium trisilicate, polyvinyl pyrrolidone, cellulose-based substances, polyethylene glycol, sodium carboxymethyl cellulose, polyacrylates, waxes, polyethylene-polyoxypropylene-block polymers, polyethylene glycol and lanolin.

The present disclosure also includes a kit (e.g., a pharmaceutical package). The kit as provided may include the compound of the present disclosure, an additional therapeutic agent, and a first and a second containers (e.g., vial, ampoule, bottle, syringe, and/or dispersible package or other suitable containers) for containing the compound of the present disclosure and the additional therapeutic agent. In some embodiments, the kit as provided may also optionally include a third container containing a pharmaceutical excipient for diluting or suspending the compound of the present disclosure and/or the additional therapeutic agents. In some embodiments, the compound of the present disclosure provided in the first container and the additional other therapeutic agents provided in the second container are combined to form a unit dosage form.

### Administration

The pharmaceutical composition provided by the present disclosure may be administered by many ways, including but not limited to: oral administration, parenteral administration, inhalation administration, topical administration, rectal administration, nasal administration, buccal administration, vaginal administration, administration by implant, or other modes of administration. For example, the parenteral administrations as used herein include subcutaneous administration, intradermal administration, intravenous administration, intramuscular administration, intraarticular administration, intraarterial administration, intrasynovial cavity administration, intrasternal administration, intrathecal administration, intralesional administration, and intracranial injection or infusion technology.

Generally, an effective amount of the compound as provided herein is administered. The amount of the compound to be actually administered may be determined by a physician according to relevant circumstances, including the condition being treated, the route of administration selected, the compound actually administered, the age, weight and response of individual patients, the severity of the patient's symptoms, and the like.

When used to prevent the conditions described in the present disclosure, the compounds as provided herein are administered to a subject at risk of developing the condition, typically based on the physician's advice and under the physician's supervision, and the dosage level is as described above. The subjects at risk of developing a specific condition typically include those with a family history of the condition, or those identified by genetic testing or screening as being particularly susceptible to developing the condition.

The pharmaceutical composition as provided herein may also be administered over a long period of time ("chronic administration"). The chronic administration refers to the administration of a compound or a pharmaceutical composition thereof over a long period of time, e.g., 3 months, 6 months, 1 year, 2 years, 3 years, 5 years, etc., or an indefinitely continuous administration, e.g., for the rest of the subject's life. In some embodiments, the chronic administration is intended to provide a constant level of the compound in the blood over a long period of time, e.g., within the therapeutic window.

The pharmaceutical composition of the present disclosure may be further delivered by various administration methods. For example, in some embodiments, the pharmaceutical composition may be administered by bolus injection, e.g., in order to increase the concentration of the compound in the blood to an effective level. The bolus dose depends on the target systemic level of the active component through the body. For example, an intramuscular or subcutaneous bolus dose allows for slow release of the active component, while a direct intravenous bolus injection (e.g., by intravenous (IV) drip) may lead to more rapid delivery, so that the concentration of the active component in the blood increases to an effective level rapidly. In other embodiments, the pharmaceutical composition may be administered by ways of continuous infusion, e.g., by intravenous (IV) drip to provide a steady-state concentration of the active component in the subject's body. In addition, in other embodiments, the pharmaceutical composition may be first administered by bolus injection, followed by continuous infusion.

Oral compositions may be in the form of bulk liquid solutions or suspensions or bulk powders. However, more generally, in order to facilitate accurate dosing, the composition is provided in a unit dosage form. The term "unit dosage form" refers to physically discrete units suitable for use as unit dose for human patients and other mammals, and each unit contains a predetermined amount of active substances suitable for producing the desired therapeutic effect and a suitable pharmaceutical excipient. Typical unit dosage forms include pre-filled, premeasured ampoules or syringes of liquid compositions, or pills, tablets, capsules or the like in the case of solid compositions. In such compositions, the compound is usually a relatively small component (about 0.1 to about 50% by weight, or alternatively about 1 to about 40% by weight), and the rest is various carriers or excipients and processing aids useful for forming the desired administration form.

For oral dosage, a representative regimen is one to five oral doses per day, especially two to four oral doses, typically three oral doses. By using these dosage modes, it provides about 0.01 to about 20 mg/kg of the compound of the present disclosure per dose, alternatively about 0.1 to about 10 mg/kg per dose, especially about 1 to about 5 mg/kg per dose.

In order to provide a similar or less blood level in relation to that provided by using an injectable dose, a transdermal dose is generally selected in an amount of about 0.01 to about 20% by weight, alternatively about 0.1 to about 20% by weight, alternatively about 0.1 to about 10% by weight, and more alternatively about 0.5 to about 15% by weight.

From about 1 to about 120 hours, especially from 24 to 96 hours, the injected dosage level is in the range of about 0.1 mg/kg/hour to at least 10 mg/kg/hour. In order to afford a sufficient steady state level, a pre-load bolus injection of about 0.1 mg/kg to about 10 mg/kg or more may also be administered. For a 40 to 80 kg human patient, the maximum total dose should not exceed about 2 g/day.

Liquid forms suitable for oral administration may include a suitable aqueous or nonaqueous carrier, as well as buffers, as suspending and dispersing agents, colorants, flavoring agents, etc. Solid forms may include, e.g., any components of: binders, such as microcrystalline cellulose, tragacanth, or gelatin; excipients, such as starch or lactose, disintegrants, such as alginic acid, Primogel, or corn starch; lubricants, such as magnesium stearate; glidants, such as colloidal silicon dioxide; sweeteners, such as sucrose or saccharin; or flavoring agents, such as peppermint, methyl salicylate, or orange flavouring; or compounds with similar properties.

Injectable compositions are typically based on sterile saline or phosphate buffered saline for injection, or other injectable excipients known in the art. As previously mentioned, in such compositions, the active compound is typically a relatively small component, often in an amount of about 0.05 to 10% by weight, and the rest is injectable excipients or the like.

Typically, transdermal compositions are formulated as topical ointments or creams containing active ingredients. When formulated as an ointment, the active ingredient is typically combined with paraffin or a water-miscible ointment base. Alternatively, the active ingredient may be formulated as a cream, e.g., with an oil-in-water cream base. Such transdermal formulations are well known in the art and typically include additional components for enhancing the stable skin penetration of the active ingredient or formulation. All such known transdermal formulations and components are included within the scope as provided in the present disclosure.

The compounds of the present disclosure may also be administered by transdermal means. Thus, the transdermal administration may be achieved using reservoir or porous membrane types or patches of multiple solid bases.

The above components of the composition for oral, injection or topical administration are representative only. Other materials and processing techniques are described in Remington's Pharmaceutical Sciences, 17th edition, 1985, Mack Publishing Company, Easton, Pennsylvania, Part 8, which is incorporated herein by reference.

The compounds of the present disclosure may also be administered in a manner of sustained release, or from a sustained release delivery system. The description of representative sustained release materials may be found in the Remington's Pharmaceutical Sciences.

The present disclosure also relates to a pharmaceutically acceptable formulation of the compound of the present disclosure. In an embodiment, the formulation comprises water. In another embodiment, the formulation comprises a cyclodextrin derivative. The most common cyclodextrins are α-, β- and γ-cyclodextrins consisting of 6, 7 and 8 α-1,4-linked glucose units, respectively, which optionally include on the linked sugar moiety one or more substituents including, but not limited to, methylated, hydroxyalkylated, acylated and sulfoalkyl ether substitutions. In some embodiments, the cyclodextrin is a sulfoalkyl ether β-cyclodextrin, e.g., sulfobutyl ether β-cyclodextrin, also known as Captisol. See, e.g., US5,376,645. In some embodiments, the formulation includes hexapropyl-β-cyclodextrin (e.g., in water, 10-50%).

### EXAMPLES

The reagents used in the present disclosure are commercially available reagents purchased directly, or synthesized by common methods well known in the art.

Notes of commonly used abbreviations:
PE = petroleum ether; EA = ethyl acetate; MeOH = methanol; DCM = dichloromethane; DCE = dichloroethane; CH₃CN = acetonitrile; 1,4-dioxane = 1,4-dioxane; DMSO = dimethyl sulfoxide; HFIP = hexafluoroisopropanol; DMF = N,N-dimethylformamide; DME = ethylene glycol dimethyl ether; Hex = n-hexane; IPA = isopropanol; NMP = N-methylpyrrolidone; NMO = N-methylmorpholine-N-oxide; TEA = triethylamine; DIEA = diisopropylethylamine; CuI = cuprous iodide; CuCN = cuprous cyanide; triphosgene = triphosgene; p-TsOH = p-toluenesulfonic acid; T₃P = 1-propylphosphoric acid cyclic anhydride; T_{S}N₃= p-toluenesulfonyl azide; PPA = polyphosphoric acid; SEM-Cl = 2-(Trimethylsilyl)ethoxymethyl chloride; DMC = diethyl carbonate; NBS = N-bromosuccinimide; TBS-Cl = tert-butyldimethylchlorosilane; LDA = lithium diisopropylamide; HMPA = hexamethylphosphoric triamide; HATU = 2-(7-azabenzotriazole)-N,N,N',N'-tetramethyluronium hexafluorophosphate.

MSI-H = MicroSatellite Instability-High; MSI-L = MicroSatellite Instability-Low; MSS = Microsatellite Stable.

### Example 1:

### Preparation of Key Intermediates

### Preparation of Intermediate a1

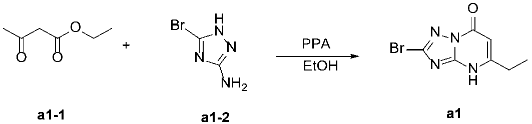

**Steps:** The raw material ethyl acetoacetate **a1-1** (9.0 g, 69.2 mmol) and 5-bromo-1-H-3-amino-1,2,4-triazole **a1-2** (11.3 g, 69.2 mmol) were dissolved in 90 mL of ethanol. Polyphosphoric acid PPA (8.0 g, 69.2 mmol) was added slowly. After the completion of addition, the mixture was heated to 80°C for 12 hours. The reaction was cooled to room temperature, and the solvent was evaporated under reduced pressure. The reaction solution was poured into 100 mL of ice water. The solution was adjusted to about pH ~8 with aqueous saturated sodium bicarbonate solution. The mixture was extracted with dichloromethane, and the extract was dried over anhydrous sodium sulfate. The filtrate was concentrated to afford **a1** (7.5 g) as white solid (yield: 45%). LCMS ESI-MS m/z: 243 [M+H]⁺.

### Preparation of Intermediates a5, a7-a13

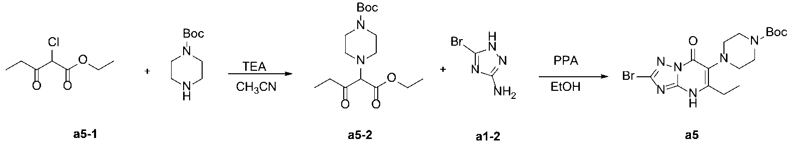

**Step 1:** The raw material **a5-1** (24.0 g, 134 mmol) and tert-butyl piperazine-1-carboxylate (25.0 g, 134 mmol) were dissolved in 240 mL of acetonitrile. TEA (40.8 g, 403 mmol) was slowly added. After the completion of addition, the mixture was heated to 60°C for 16 hours. The reaction was cooled to room temperature, and the solvent was evaporated under reduced pressure. The reaction solution was poured into 100 mL of ice water, extracted with dichloromethane, and dried over anhydrous sodium sulfate. The filtrate was concentrated, and the crude product was separated by column chromatography (PE/EA, 9/1) to afford **a5-2** (24 g) as yellow oil (yield: 54%). LCMS ESI-MS m/z: 329 [M+H]⁺.

**Step 2:** The intermediate **a5-2** (22.2 g, 67.6 mmol) from the previous step and 5-bromo-1-H-3-amino1,2,4-triazole **a1-2** (11.0 g, 67.6 mmol) were dissolved in 200 mL of ethanol. Polyphosphoric acid PPA (7.8 g, 67.6 mmol) was slowly added. After the completion of addition, the mixture was heated to 80°C for 12 hours. The reaction was cooled to room temperature, and the solvent was evaporated under reduced pressure. The reaction solution was poured into 100 mL of ice water. The solution was adjusted to about pH 8 with aqueous saturated sodium bicarbonate solution. The mixture was extracted with dichloromethane, and dried over anhydrous sodium sulfate. The filtrate was concentrated, and the crude product was separated by column chromatography (PE/EA, 3/1) to afford **a5** (4.4 g) as yellow solid (yield: 15%). LCMS ESI-MS m/z: 427 [M+H]⁺.

Referring to the synthetic route of intermediate **a1** or **a5,** the following target intermediates were synthesized by using similar raw materials/analogues.

| **Intermediates** | **Intermediate Structure** | **LC-MS ESI-MS m/z:[M+H]⁺** |
|---|---|---|
| **a5** | 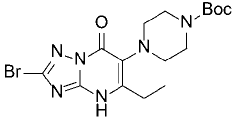 | 427 |
| **a7** | 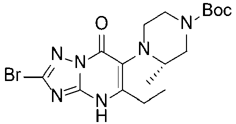 | 441 |
| **a8** | 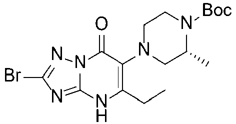 | 441 |
| **a9** | 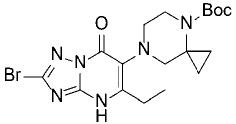 | 453 |
| **a10** | 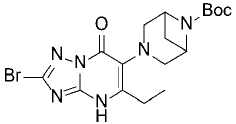 | 439 |
| **a11** | 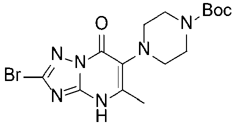 | 413 |
| **a12** | 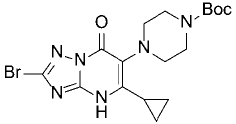 | 439 |
| **a13** | 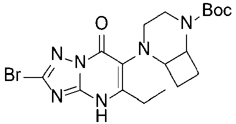 | 453 |

### Preparation of Intermediates a2, a6, a14-a32

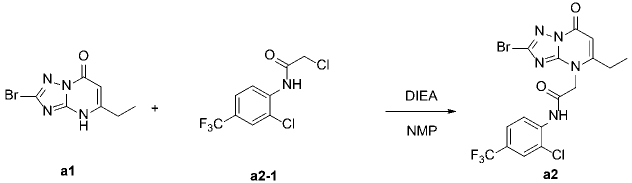

**Steps:** The intermediate **a1** (7.5 g, 30.9 mmol) and the raw material **a2-1** (10.1 g, 37.0 mmol) were dissolved in 75 mL of NMP. DIEA (12.0 g, 92.6 mmol) was slowly added. After the completion of addition, the mixture was heated to 50°C for 16 hours. The reaction was cooled to room temperature, and the reaction was quenched. The reaction solution was poured into 100 mL of ice water, extracted with dichloromethane, and dried over anhydrous sodium sulfate. The filtrate was concentrated and the crude product was separated by HPLC preparative chromatography (chromatographic column: XSelect Prep OBD C18 Column, 30*150 mm, 5µm; mobile phase A: water (10 mmol/L NH₄HCO₃), mobile phase B: acetonitrile; flow rate: 90 mL/min; retention time: 3 min) to afford **a2** (5.2 g) as yellow solid (yield: 35%). LCMS ESI-MS m/z: 478 [M+H]⁺.

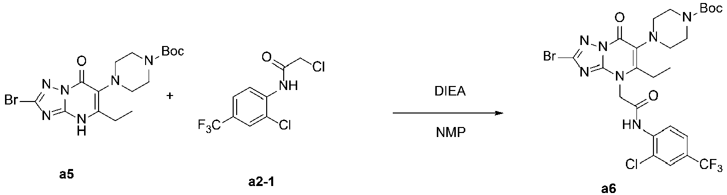

Steps: The intermediate **a5** (1.1 g, 2.57 mmol) and the raw material **a2-1** (1.1 g, 3.86 mmol) were dissolved in 11 mL of NMP. DIEA (1.0 g, 7.72 mmol) was slowly added. After the completion of addition, the mixture was heated to 70°C for 12 hours. The reaction was cooled to room temperature, and the reaction was quenched. The reaction solution was poured into 50 mL of ice water, extracted with dichloromethane, and dried over anhydrous sodium sulfate. The filtrate was concentrated and the crude product was separated by flash reverse column chromatography (chromatographic column: C18; CH₃CN/H₂O = 5/1) to afford a6 (996 mg) as yellow solid (yield: 58%). LCMS ESI-MS m/z: 662 [M+H]⁺.

Referring to the synthetic route of intermediate **a2** or **a6,** the following target intermediates were synthesized by using similar raw materials/analogues.

**[a2-1** analogues were synthesized by reacting a similar heteroaryl-NH₂ as raw material with chloroacetyl chloride.]

| **Intermediates** | **Intermediate Structures** | **LC-MS ESI-MS m/z:[M+H]⁺** |
|---|---|---|
| **a6** | 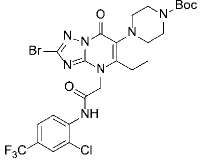 | 662 |
| **a14** | 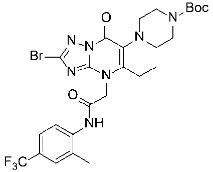 | 642 |
| **a15** | 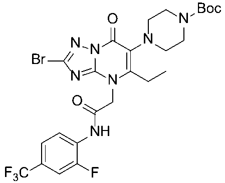 | 646 |
| **a16** | 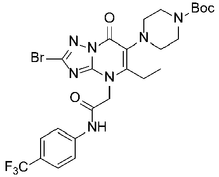 | 628 |
| **a17** | 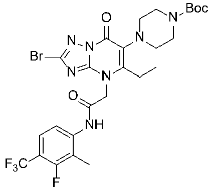 | 660 |
| **a18** | 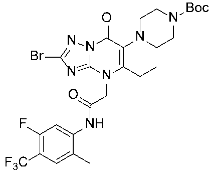 | 660 |
| **a19** | 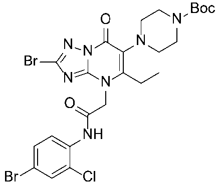 | 673 |
| **a20** | 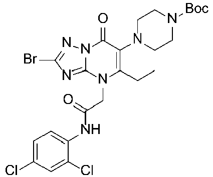 | 629 |
| **a21** | 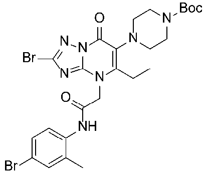 | 653 |
| **a22** | 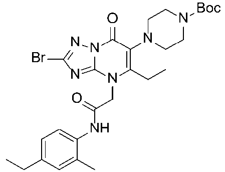 | 602 |
| **a23** | 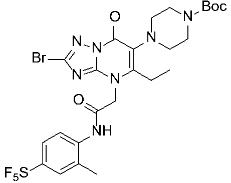 | 602 |
| **a24** | 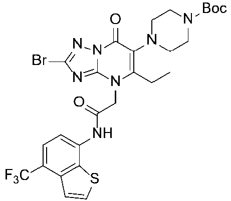 | 684 |
| **a25** | 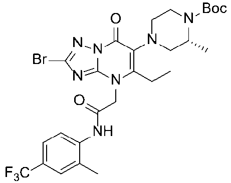 | 656 |
| **a26** | 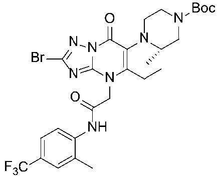 | 656 |
| **a27** | 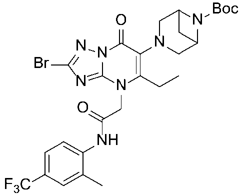 | 654 |
| **a28** | 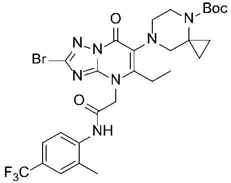 | 668 |
| **a29** | 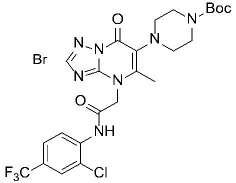 | 648 |
| **a30** | 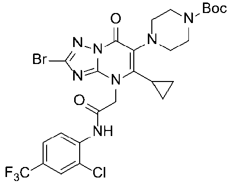 | 674 |
| **a31** | 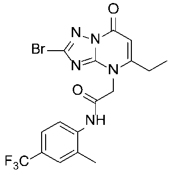 | 458 |
| **a32** | 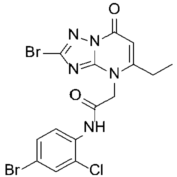 | 488/489 |

### Preparation of Intermediate a3

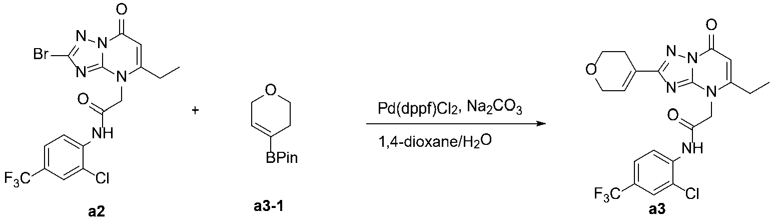

**Steps:** Under nitrogen protection, the intermediate **a2** (5.0 g, 10.4 mmol), the raw material **a3-1** (2.9 g, 13.6 mmol) and sodium carbonate (3.3 g, 31.3 mmol) were dissolved in 50 mL of a mixed solution of 1,4-dioxane and water (v/v, 4/1). Catalyst Pd(dppf)Cl₂ (900 mg, 1.0 mmol) was added. The mixture was heated to 100°C for 2 hours. The reaction was quenched, and filtered. 100 mL of water was added to the reaction solution. The mixture was extracted with ethyl acetate, and dried over anhydrous sodium sulfate. The filtrate was concentrated and the crude product was separated by HPLC preparative chromatography (chromatographic column: WelFlash C18-I, 20-40 µm, 330 g; mobile phase A water: (10 mmol/L NH₄HCO₃), mobile phase B: acetonitrile; flow rate: 80 mL/min; retention time: 12 min) to afford a3 (2.6 g) as yellow solid (yield: 52%). LCMS ESI-MS m/z: 482 [M+H]⁺.

### Preparation of Intermediate a4

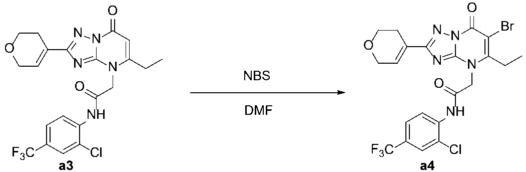

Steps: The intermediate **a3** (2.4 g, 5.0 mmol) was dissolved in 25 mL of DMF, and NBS (1.8 g, 10.0 mmol) was added. The mixture was reacted at room temperature for 2 hours. The reaction was quenched, and filtered. 100 mL of water was added to the reaction solution. The mixture was extracted with ethyl acetate, and dried over anhydrous sodium sulfate. The filtrate was concentrated, and the crude product was separated by HPLC preparative chromatography (chromatographic column: WelFlash C18-I, 20-40 µm, 330 g; mobile phase A water: (10 mmol/L NH₄HCO₃), mobile phase B: acetonitrile; flow rate: 90 mL/min; retention time: 11 min) to afford **a4** (1.6 g) as white solid (yield: 57%). LCMS ESI-MS m/z: 560 [M+H]⁺.

### Preparation of Intermediate b1

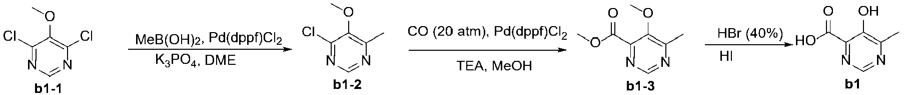

**Step 1:** Under nitrogen protection, the raw material 4,6-dichloro-5-methoxypyrimidine **b1-1** (20.0 g, 111 mmol), the raw material methylboric acid (7.0 g, 117 mmol) and potassium phosphate (59.2 g, 279 mmol) were dissolved in 120 mL of DME, and the catalyst Pd(dppf)Cl₂ (4.6 g, 5.6 mmol) was added. The mixture was heated to 85°C for 12 hours. The reaction was quenched, and filtered. 100 mL of water was added to the reaction solution. The mixture was extracted with ethyl acetate, and dried over anhydrous sodium sulfate. The filtrate was concentrated, and the crude product was separated by column chromatography (PE/EA, 10/1) to afford **b 1-2** (6.0 g) as white solid (yield: 3 4%). LCMS ESI-MS m/z: 159 [M+H]⁺.

**Step 2:** Under the carbon monoxide atmosphere, the intermediate **b1-2** (6.0 g, 37.0 mmol) from the previous step and TEA (7.66 g, 75.0 mmol) were dissolved in 90 mL of methanol, and the catalyst Pd(dppf)Cl₂ (1.85 g, 2.3 mmol) was added. The mixture was heated to added. The mixture was heated to 100°C under CO (20 atm) for 12 hours. The reaction was quenched, and filtered. The solvent was evaporated under reduced pressure, and the crude product was separated by HPLC preparative chromatography (chromatographic column: WelFlash C18-I, 20-40 um, 120 g; mobile phase A: water (10 mmol/L NH₄HCO₃) mobile phase B: acetonitrile; flow rate: 60 mL/min) to afford **b1-3** (5.0 g) as white solid (yield: 73%). LCMS ESI-MS m/z: 183 [M+H]⁺.

**Step 3:** The intermediate **b1-3** (3.0 g, 16.5 mmol) from the previous step was dissolved in 15 mL of aqueous HBr solution (40%). The mixture was heated to 40°C for 10 hours, and the reaction was quenched. HI (15 mL) was added to the reaction solution, and the reaction was continued at 40°C for 6 hours. The solvent was evaporated under reduced pressure. The crude product was adjusted to pH 8 with aqueous NaOH solution (1N), and then to pH 3 with concentrated hydrochloric acid. The solvent was evaporated under reduced pressure. The crude product was separated by flash reverse column chromatography (chromatographic column: C18; CH₃CN/H₂O, 1/1) to give **b1** (1.5 g) as yellow solid (yield: 59%). LCMS ESI-MS m/z: 155 [M+H]⁺.

### Preparation of Intermediate b2

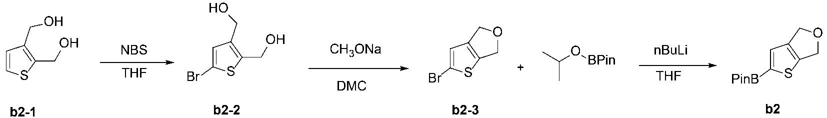

**Step 1:** In an ice bath and under nitrogen protection, the raw material **b2-1** (500 mg, 3.81 mmol) was dissolved in 10 mL of tetrahydrofuran, and NBS (679 mg, 3.81 mmol) was added. The mixture was reacted at room temperature for 0.5 hours. The reaction was quenched, and filtered. The reaction solution was adjusted to about pH 8 with aqueous saturated sodium bicarbonate. The mixture was extracted with ethyl acetate, and dried over anhydrous sodium sulfate. The filtrate was concentrated, and the crude product was separated by column chromatography (PE/EA, 10/1) to afford **b2-2** (540 mg) as yellow oil (yield: 63%). LCMS ESI-MS m/z: 224 [M+H]⁺.

**Step 2:** Under nitrogen protection, the intermediate **b2-2** (510 mg, 2.28 mmol) from the previous step was dissolved in 3 mL of diethyl carbonate (DMC). CH₃ONa (185 mg, 3.42 mmol) was added. The mixed solution was heated to 125°C for 0.5 hour, and cooled to room temperature. 20 mL of ice water was added to the reaction solution. The mixture was extracted with methyl tert-butyl ether, and dried over anhydrous sodium sulfate. The filtrate was concentrated, and the crude product was separated by column chromatography (PE/EA, 1/1) to afford **b2-3** (300 mg) as yellow oil (yield: 64%). LCMS ESI-MS m/z: 205 [M+H]⁺.

**Step 3:** At -78°C and under nitrogen protection, the intermediate b2-3 (300 mg, 1.46 mmol) from the previous step was dissolved in 6 mL of anhydrous tetrahydrofuran, and ⁿBuLi (2.5M, 1.47 mL) was added dropwise. After the addition was completed, the reaction was stirred for additional 1 hour. Isopropyl alcohol pinacol borate (327 mg, 1.75 mmol) was added to the reaction solution, and the reaction was continued at -78°C for 1 hour. The mixture was quenched with saturated aqueous ammonium chloride solution. The mixture was extracted with ethyl acetate, dried over anhydrous sodium sulfate, and concentrated to afford **b2** (300 mg) as yellow oil (yield: 81%). LCMS ESI-MS m/z: 253 [M+H]⁺.

### Preparation of Intermediate b3

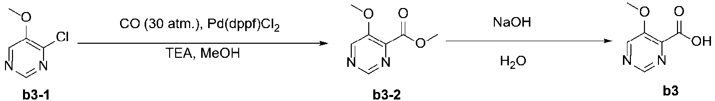

**Step 1:** Under carbon monoxide atmosphere (30 atm), the raw material 4-chloro-5-methoxypyrimidine **b3-1** (5.0 g, 34.6 mmol) and TEA (7.0 g, 69.2 mmol) were dissolved in 100 mL of methanol, and the catalyst Pd(dppf)Cl₂ (1.5 g, 2.07 mmol) was added. The mixture was heated to 100°C for 12 hours under constant protection of carbon monoxide, and the reaction was quenched and filtered. The solvent was evaporated under reduced pressure, and the crude product was separated by column chromatography (PE /EA, 1/1) to afford **b3-2** (4.0 **g)** as yellow oil (yield:69%). LCMS ESI-MS m/z: 275 [M+H]⁺.

**Step 2:** The intermediate **b3-2** (1.0 g, 5.94 mmol) from the previous step was dissolved in 20 mL of aqueous solution and NaOH (0.5 g, 11.9 mmol) was added. The mixture was warmed to 40°C for 10 hours and the reaction was quenched. The reaction solution was adjusted to about pH 5 by adding diluted hydrochloric acid. The solvent was evaporated under reduced pressure. The crude product was separated by flash reverse column chromatography (chromatographic column: C18; CH₃CN / H₂O, 1/10) to give **b3** (400 mg) as white solid (yield: 44%). LCMS ESI-MS m/z: 155 [M+H]⁺.

### Preparation of Intermediate b4

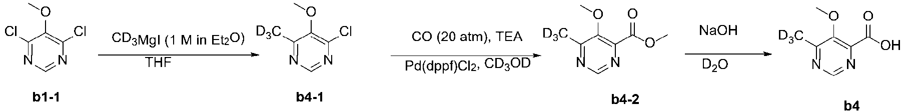

**Step 1:** In an ice bath and under nitrogen protection, the raw material 4,6-dichloro-5-methoxypyrimidine **b1-1** (11.0 g, 61.5 mmol) was dissolved in 2.20 mL of anhydrous tetrahydrofuran, and a solution of CD₃MgI (1 M, 62 mL) in ether was added dropwise. After the addition was completed, the mixture was reacted at room temperature for 12 hours, and the reaction was quenched. 100 mL of water was added to the reaction solution, and the organic solvent was evaporated under reduced pressure. The mixture was extracted with ethyl acetate, dried over anhydrous sodium sulfate, and concentrated. The crude product was separated by column chromatography (PE/EA, 1/1) to obtain **b4-1** (4.0 g) as yellow oil (yield: 40%). LCMS ESI-MS m/z: 161 [M+H]⁺.

**Step** 2: Under carbon monoxide atmosphere, the intermediate **b4-1** (5.0 g, 30.9 mmol) from the previous step and TEA (9.3 g, 92.8 mmol) were dissolved in 50 mL of deuterated methanol CD₃OD, and a catalyst Pd(dppf)Cl₂ (0.7 g, 0.93 mmol) was added. The mixture was heated to 100°C under CO (30 atm) for 12 hours, and the reaction was quenched and filtered. 300 mL of water was added to the reaction solution. The mixture was extracted with ethyl acetate, and dried over anhydrous sodium sulfate. The filtrate was concentrated, and the crude product was separated by column chromatography (PE/EA, 1/1) to obtain **b4-2** (2.0 g) as yellow oil (yield:35%). LCMS ESI-MS m/z: 186 [M+H]⁺.

**Step 3:** The intermediate **b4-2** (1.0 g, 5.4 mmol) from the previous step was dissolved in 20 mL of heavy water (D₂O). NAOH (0.4 g, 10.8 mmol) was added to the reaction solution, and the mixture was reacted at room temperature for 6 hours. The solvent was evaporated under reduced pressure. The crude product was adjusted to pH 5 with diluted hydrochloric acid (2 M), and the solvent was evaporated under reduced pressure. The crude product was separated by flash reverse column chromatography (chromatographic column: C18; CH₃CN / H₂O, 1/20) to give **b4** (920 mg) as white solid (yield: 99 %). LCMS ESI-MS m/z: 172 [M+H]⁺.

### Preparation of Intermediates c1-c4

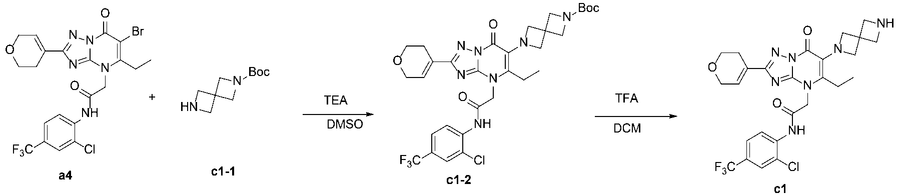

**Step 1:** Under nitrogen protection, the intermediate **a4** (210 mg, 0.3 mmol) and TEA (114 mg, 1.1 mmol) were dissolved in 2 mL of DMSO, and the raw material **c1-1** (371 mg, 1.9 mmol) was added. The mixture was heated to 85°C for 12 hours and the reaction was quenched. 100 mL of water was added to the reaction solution. The mixture was extracted with ethyl acetate, and dried over anhydrous sodium sulfate. The filtrate was concentrated, and the crude product was separated by HPLC chromatography (chromatographic column: WelFlash C18-I, 20-40 µm, 180 g; mobile phase A: water (10 mmol/L NH₄HCO₃), mobile phase B: acetonitrile; flow rate: 70 mL/min; retention time: 12 min) to afford **c1-2** (120 mg) as white solid (yield: 47 %). LCMS ESI-MS m/z: 678 [M+H]⁺.

**Step 2:** The intermediate **c1-1** (120 mg, 0.2 mmol) from the previous step and trifluoroacetic acid (0.5 mL) were dissolved in 2 mL of dichloromethane and the mixture was reacted at room temperature for 2 hours. Then, the reaction was quenched. The solvent was evaporated under reduced pressure, and the crude product was separated by HPLC preparative chromatography (chromatographic column: WelFlash C18-I, 20-40 um, 40 g; mobile phase A: water (10 mmol/L NH₄HCO₃) mobile phase B: acetonitrile; flow rate: 50 mL/min; retention time: 9 min) to give **c1** (80 mg) as white solid (yield: 78%). LCMS ESI-MS m/z: 578 [M+H]⁺.

Referring to the synthetic route of intermediate **c1**, the following target intermediates were synthesized using similar raw materials/analogues.

| **Intermediates** | **Intermediate Structures** | **LC-MS ESI-MS m/z:[M+H]⁺** |
|---|---|---|
| **c2** | 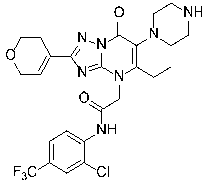 | 566 |
| **c3** | 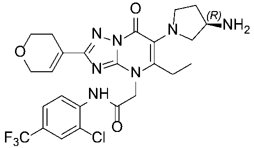 | 566 |
| **c4** | 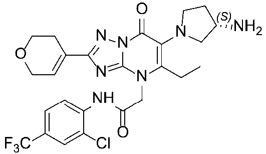 | 566 |

### Preparation of Intermediates c5-c6, c12-c17

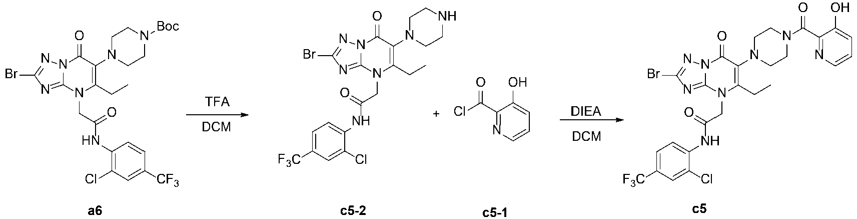

**Step 1:** The intermediate **a6** (996 mg, 1.5 mmol) was dissolved in 10 mL of dichloromethane, and 0.5 mL of trifluoroacetic acid was added dropwise. The mixture was reacted at room temperature for 1 hour, and the reaction was quenched. 20 mL of water was added to the reaction solution, and the pH was adjusted to about 9 with aqueous saturated sodium bicarbonate. The mixture was extracted with dichloromethane, dried over anhydrous sodium sulfate, and concentrated to afford **c5-2** (694 mg) as yellow solid (yield:82 %). LCMS ESI-MS m/z: 562 [M+H]⁺.

**Step 2:** The intermediate **c5-2** (494 mg, 0.88 mmol) from the previous step and DIEA (567 mg, 4.4 mmol) were dissolved in 5 mL of dichloromethane, and the raw material 3-hydroxy-2-pyridinecarbonyl chloride **c5-1** (277 mg, 1.76 mmol) was added. The mixture was reacted at room temperature for 2 hours, and the reaction was quenched. The solvent was evaporated under reduced pressure, and the crude product was separated by flash column chromatography (chromatographic column: WelFlash C18-I, 20-40 um, 130 g; mobile phase A: water, mobile phase B: acetonitrile; flow rate: 60 mL/min; retention time: 14 min) to afford **c5** (360 mg) as yellow solid (yield: 60%). LCMS ESI-MS m/z: 683 [M+H]⁺.

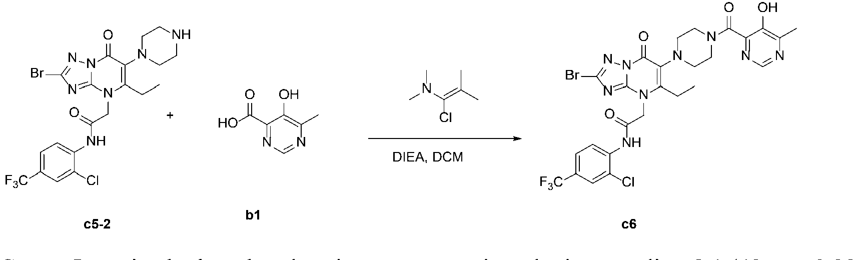

**Steps:** In an ice bath and under nitrogen protection, the intermediate **b1** (49 mg, 0.32 mmol) and 1-chloro-N,N,2-trimethylpropyl-1-enyl-1-amine (43 mg, 0.32 mmol) were dissolved in 2 mL of dichloromethane, and stirred for 1 hour in the ice bath. DIEA (138 mg, 1.06 mmol) and the intermediate **c5-2** (120 mg, 0.21 mmol) were added. The mixture was reacted at room temperature for 1 hour, The reaction was quenched. 15 mL of water was added to the reaction solution. The mixture was extracted with dichloromethane, and the extract was dried over anhydrous sodium sulfate. The filtrate was concentrated, and the crude product was separated by flash reverse column chromatography (chromatographic column: C18; CH₃CN/H₂O, 4/5) to afford **c6** (45 mg) as light yellow solid (yield: 30%). LCMS ESI-MS m/z: 698 [M+H]⁺.

Referring to the synthetic route of intermediate **c5** or **c6,** the following target intermediate were synthesized using similar raw materials/analogues .

| **Intermediates** | **Intermediate Structures** | **LC-MS ESI-MS m/z:[M+H]⁺** |
|---|---|---|
| **c6** | 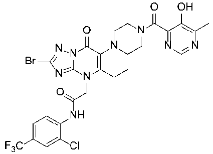 | 698 |
| **c12** | 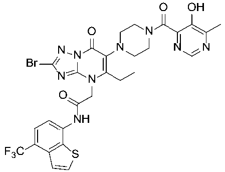 | 720 |
| **c13** | 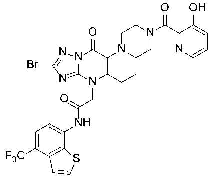 | 705 |
| **c14** | 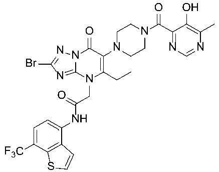 | 720 |
| **c15** | 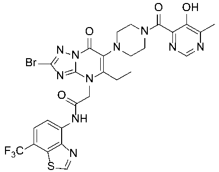 | 721 |
| **c16** | 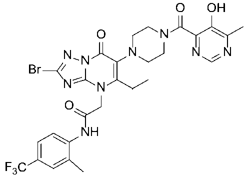 | 678 |
| **c17** | 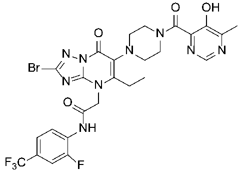 | 682 |

### Preparation of Intermediates c7-c11

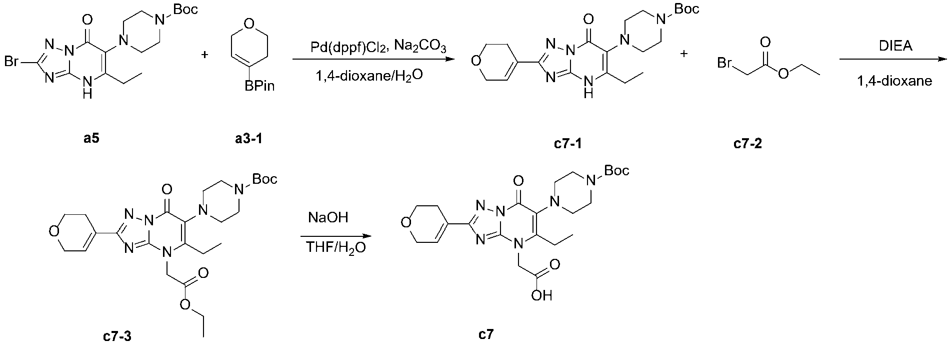

**Step 1:** Under nitrogen protection, the intermediate **a5** (2.5 g, 5.85 mmol), the raw material **a3-1** (1.2 g, 5.85 mmol) and sodium carbonate (1.9 g, 17.5 mmol) were dissolved in 50 mL of a mixed solution of 1,4-dioxane and water (v/v, 1/1). Catalyst Pd(dppf)Cl₂ (400 mg, 0.59 mmol) was added. The mixture was heated to 100°C for 12 hours. The reaction was quenched and filtered. 100 mL of water was added to the reaction solution. The mixture was extracted with ethyl acetate, and dried over anhydrous sodium sulfate. The filtrate was concentrated, and the crude product was separated by HPLC preparative chromatography (chromatographic column: WelFlash C18-I, 20-40 µm, 330 g; mobile phase A water: (10 mmol/L NH₄HCO₃), mobile phase B: acetonitrile; flow rate: 40 mL/min; retention time: 16 min) to afford **c 7-1** (1.5 g) as yellow oil (yield: 58%). LCMS ESI-MS m/z: 431 [M+H]⁺.

**Step 2:** The intermediate **c7-1** (1.5 g, 3.48 mmol) from the previous step and the raw material ethyl bromoacetate **c7-2** (0.8 g, 4.88 mmol) were dissolved in 25 mL of 1,4-dioxane. DIEA (1.4 g, 10.5 mmol) was slowly added. The mixture was heated to 80°C for 4 hours. The reaction was cooled to room temperature, and the reaction was quenched. The reaction solution was poured into 100 mL of ice water, extracted with dichloromethane, and dried over anhydrous sodium sulfate. The filtrate was concentrated, and the crude product was separated by HPLC preparative chromatography (chromatographic column: XSelect Prep OBD C18 Column, 30*150 mm, 5µm; mobile phase A: water (10 mmol/L NH₄HCO₃), mobile phase B: acetonitrile; flow rate: 40 mL/min; retention time: 16 min) to afford c7-3 (1.4 g) as yellow solid (yield: 78%). LCMS ESI-MS m/z: 517 [M+H]⁺.

**Step 3:** The intermediate **c7-3** (1.2 g, 2.32 mmol) from the previous step was dissolved in 18 mL of a mixed solution of tetrahydrofuran and water (v/v, 2/1). NAOH aqueous solution (3.5 mL, 1 M) was slowly added dropwise. The mixture was reacted at room temperature for 1 hour, and the reaction was quenched. The reaction solution was adjusted to about pH 4 with dilute hydrochloric acid. extracted with dichloromethane, and dried over anhydrous sodium sulfate. The filtrate was concentrated, and the crude product was separated by HPLC preparative chromatography (chromatographic column: XSelect Prep OBD C18 Column, 30*150 mm, 5µm; mobile phase A: water, mobile phase B: acetonitrile; flow rate: 40 mL/min; retention time: 16 min) to afford c7 (900 mg) as yellow solid (yield: 92%). LCMS ESI-MS m/z: 489 [M+H]⁺

Referring to the synthetic route of intermediate **c7,** the following target intermediate were synthesized using similar raw materials/analogues .

| **Intermediates** | **Intermediate Structures** | **LC-MS ESI-MS m/z:[M+H]⁺** |
|---|---|---|
| **c8** | 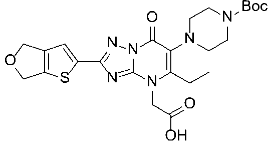 | 531 |
| **c9** | 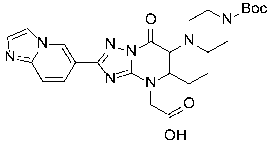 | 523 |
| **c10** | 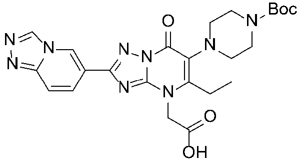 | 524 |
| **c11** | 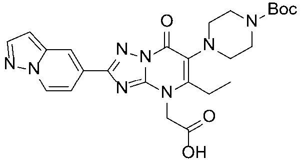 | 523 |

### Preparation of Intermediate c18

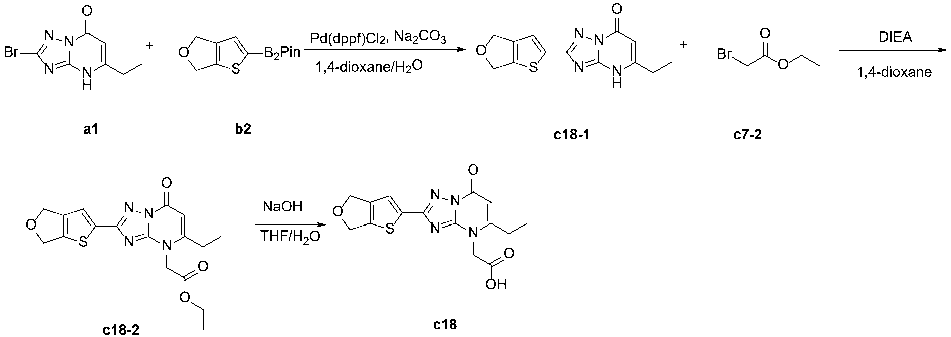

**Step 1:** Under nitrogen protection, the intermediate **a1** (1.42 g, 5.85 mmol), the raw material **b2** (1.47 g, 5.85 mmol) and sodium carbonate (1.9 g, 17.5 mmol) were dissolved in 30 mL of a mixed solution of 1,4-dioxane and water (v/v, 1/1). Catalyst Pd(dppf)Cl₂ (400 mg, 0.59 mmol) was added. The mixture was heated to 100°C for 2 hours. The reaction was quenched, and filtered. 80 mL of water was added to the reaction solution. The mixture was extracted with ethyl acetate, and dried over anhydrous sodium sulfate. The filtrate was concentrated, and the crude product was separated by column chromatography (PE /EA, 1/1) to afford **c 18-1** (1.0 g) as yellow solid (yield: 60 %). LCMS ESI-MS m/z: 289 [M+H]⁺.

**Step 2:** The intermediate **c18-1** (1.0 g, 3.47 mmol) from the previous step and the raw material ethyl bromoacetate **c7-2** (0.8 g, 4.88 mmol) were dissolved in 25 mL of 1,4-dioxane. DIEA (1.4 g, 10.5 mmol) was slowly added. The mixture was heated to 80°C for 4 hours. The reaction was cooled to room temperature, and the reaction was quenched. The reaction solution was poured into 100 mL of ice water, extracted with dichloromethane, and dried over anhydrous sodium sulfate. The filtrate was concentrated, and the crude product was separated by flash reverse column chromatography (chromatographic column: C18; CH₃CN/H₂O, 3/1) to obtain **c18-2** (1.1 g) as light yellow solid (yield: 85%). LCMS ESI-MS m/z: 375 [M+H]⁺.

**Step 3:** The intermediate **c18-2** (1.1 g, 2.94 mmol) from the previous step was dissolved in 30 mL of a mixed solution of tetrahydrofuran and water (v/v, 2/1). Aqueous NAOH solution (5.0 mL, 1 M) was slowly added dropwise. The mixture was reacted at room temperature for 1 hour, and the reaction was quenched. The reaction solution was adjusted to about pH 4 with dilute hydrochloric acid, extracted with dichloromethane, and dried over anhydrous sodium sulfate. The filtrate was concentrated, and the crude product was separated by HPLC preparative chromatography (chromatographic column: XSelect Prep OBD C18 Column, 30*150 mm, 5µm; mobile phase A: water, mobile phase B: acetonitrile; flow rate: 60 mL/min) to obtain **c18** (730 mg) as yellow solid (yield: 72%). LCMS ESI-MS m/z: 347 [M+H]⁺.

### Preparation of Intermediates d1-d7

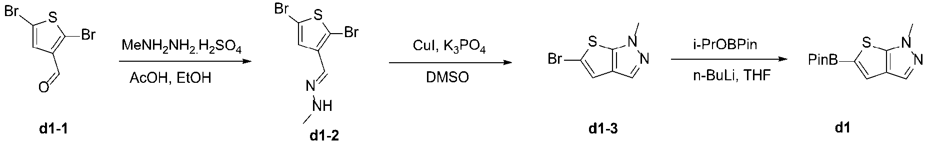

**Step 1:** Under nitrogen protection, the raw material **d1-1** (2.0 g, 7.40 mmol) and methylhydrazine sulfate (1.1 g, 7.40 mmol) were dissolved in 40 mL of ethanol. Acetic acid (100 mg, 0.14 mmol) was added. The mixture was heated to 80°C for 2 hours, and the reaction was quenched. The solvent was evaporated under reduced pressure. The mixture was adjusted to about pH 8 by adding aqueous saturated sodium bicarbonate. The mixture was extracted with ethyl acetate, dried over anhydrous sodium sulfate, and concentrated to obtain **d1-2** (2.5 g) as yellow oil (yield: 89%). LCMS ESI-MS m/z: 297 [M+H]⁺.

**Step** 2: Under nitrogen protection, the intermediate **d1-2** (2.5 g, 8.38 mmol) from the previous step and potassium phosphate (1.78 g, 8.39 mmol) were dissolved in 38 mL of DMSO, and the catalyst CuI (160 mg, 0.83 mmol) was added. The mixture was heated to 100°C for 3 hours. The reaction was quenched, and filtered. 100 mL of water was added to the reaction solution. The mixture was extracted with ethyl acetate, and dried over anhydrous sodium sulfate. The filtrate was concentrated, and the crude product was separated by HPLC preparative chromatography (chromatographic column: WelFlash C18-I, 20-40 µm, 330 g; mobile phase A water: (0.1% TFA), mobile phase B: acetonitrile; flow rate: 40 mL/min; retention time: 12 min) to afford **d1-3** (130 mg) as white solid (yield: 7%). LCMS ESI-MS m/z: 217 [M+H]⁺.

**Step 3:** Under nitrogen protection at -78°C, the intermediate **d1-3** (130 mg, 0.59 mmol) from the previous step was dissolved in 2.5 mL of anhydrous tetrahydrofuran, and n-BuLi (0.28 mL, 2.5 M) was slowly added. After the addition, the mixture was stirred at -78°C for 1 hour. 2-Isopropoxyboronic acid pinacol ester (134 mg, 1.23 mmol) was added to the reaction solution. The mixture was stirred for 30 minutes and the reaction was quenched. 10 mL of saturated aqueous ammonium chloride solution and ethyl acetate were added to the reaction solution, and the mixture was dried over anhydrous sodium sulfate and concentrated to obtain **d1** (110 mg) as yellow oil (yield:70%). LCMS ESI-MS m/z: 265 [M+H]⁺.

Referring to the synthetic route of intermediate **d1**, the following target intermediate were synthesized using similar raw materials/analogues .

| **Intermediates** | **Intermediate Structures** | **LC-MS ESI-MS m/z:[M+H]⁺** |
|---|---|---|
| **d2** | 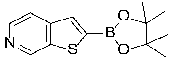 | 262 |
| **d3** | 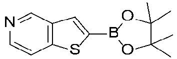 | 262 |
| **d4** | 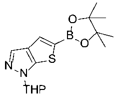 | 335 |
| **d5** | 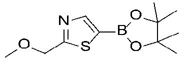 | 256 |
| **d6** | 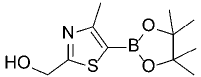 | 256 |
| **d7** | 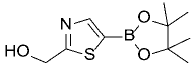 | 242 |

### Preparation of Intermediates d8 and d15

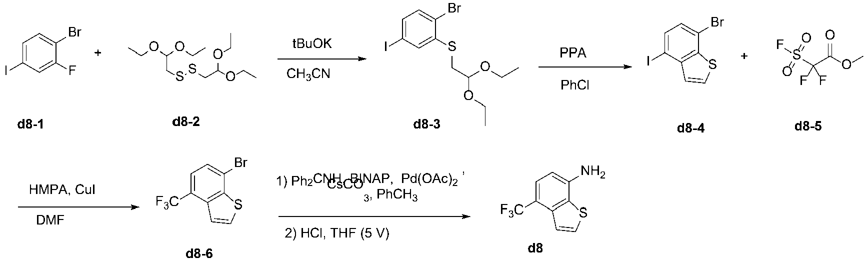

**Step 1:** Under nitrogen protection, the raw material 1-bromo-2-fluoro-4-iodobenzene **d8-1** (1.95 g, 6.5 mmol) and the raw material **d8-2** (2.32 g, 7.8 mmol) were dissolved in 40 mL of acetonitrile. Potassium tert-butoxide (2.2 g, 19.4 mmol) was added, and the mixture was heated to 50°C for 6 hours. The reaction was quenched, and filtered. 100 mL of water was added to the reaction solution. The mixture was extracted with ethyl acetate, and dried over anhydrous sodium sulfate. The filtrate was concentrated, and the crude product was separated by column chromatography (PE/EA, 20/1) to afford **d8-3** (2.22 g) as yellow oil (yield:79%).

**Step 2:** The intermediate **d8-3** (2.22 g, 5.15 mmol) from the previous step was dissolved in 133 mL of chlorobenzene. Polyphosphoric acid PPA (2.37 g, 20.6 mmol) was added, and the mixture was heated to 130°C for 5 hours. The reaction was quenched, and the mixture was cooled to room temperature. 100 mL of ice water was added to the reaction solution. The mixture was extracted with ethyl acetate, and dried over anhydrous sodium sulfate. The filtrate was concentrated, and the crude product was separated by flash reverse column chromatography (chromatographic column: C18; CH₃CN/H₂O, 9/10) to obtain **d8-4** (1.05 g) as yellow oil (yield:60%).

**Step 3:** Under nitrogen protection, the intermediate **d8-4** (1.05 g, 3.1 mmol) from the previous step and the raw material **d8-5** (1.95 g, 9.3 mmol) were dissolved in 21 mL of DMF, and the catalyst CuI (290 mg, 1.55 mmol) and HMPA (2.39 g, 13.3 mmol) were added. The mixture was heated to 100°C for 2 hours. The reaction was quenched, and filtered. 60 mL of water was added to the reaction solution. The mixture was extracted with methyl tert-butyl ether, and dried over anhydrous sodium sulfate. The filtrate was concentrated, and the crude product was separated by column chromatography (PE /EA, 100/1) to afford **d8-6** (0.8 g) as yellow solid (yield:92%).

**Step 4:** Under nitrogen protection, the intermediate **d8-6** (0.8 g, 2.85 mmol) from the previous step and the raw material diphenyl ketone imine (1.03 g, 5.69 mmol) were dissolved in 16 mL of toluene. Catalyst Pd(OAc)₂ (60 mg, 0.28 mmol), the ligand BINAP (0.35 g, 0.57 mmol) and cesium carbonate (1.85 g, 5.69 mmol) were added. The mixture was heated to 110°C for 2 hours. The reaction was quenched, and filtered. 60 mL of water was added to the reaction solution. The mixture was extracted with ethyl acetate, dried over anhydrous sodium sulfate, and concentrated to afford a crude mixture. The mixture was dissolved in 4 mL of tetrahydrofuran. 2M of hydrogen chloride tetrahydrofuran solution (16 mL) was added. The mixture was reacted at room temperature for 1 hour, and the reaction was quenched. The reaction solution was adjusted to about pH 8 by adding aqueous saturated sodium bicarbonate. The mixture was extracted with ethyl acetate, and dried over anhydrous sodium sulfate. The filtrate was concentrated, and the crude product was separated by flash reverse column chromatography (chromatographic column: C18; CH₃CN/H₂O, 10/7) to afford **d8** (0.5 g) as yellow solid (yield: 81%). LCMS ESI-MS m/z: 218 [M+H]⁺.

Referring to the synthetic route of intermediate **d8,** the following target intermediate were synthesized using similar raw materials/analogues .

| **Intermediates** | **Intermediate Structures** | **LC-MS ESI-MS m/z:[M+H]⁺** |
|---|---|---|
| **d15** | 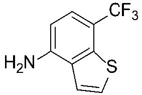 | 218 |

### Preparation of Intermediate d9

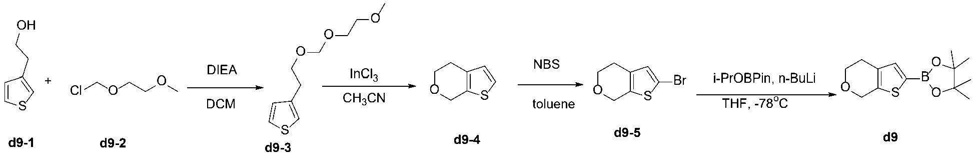

**Step 1:** Under nitrogen protection, the raw material **d9-1** (3.5 g, 27.3 mmol) and the raw material **d9-2** (4.1 g, 32.8 mmol) were dissolved in 70 mL of dichloromethane. DIEA (10.6 g, 81.9 mmol) was added, and the mixture was reacted at room temperature for 3 hours and the reaction was quenched. 100 mL of water was added to the reaction solution. The mixture was extracted with dichloromethane, dried over anhydrous sodium sulfate, and concentrated to obtain **d9-3** (3.3 g) as yellow oil.

**Step 2:** The intermediate **d9-3** (1.7 g, 7.86 mmol) from the previous step was dissolved in 17 mL of acetonitrile, and InCl₃ (0.35 g, 1.57 mmol) was added. The mixture was heated to 80°C for 5 hours, the reaction was quenched, and the mixture was cooled to room temperature. 100 mL of ice water was added to the reaction solution. The mixture was extracted with ethyl acetate, dried over anhydrous sodium sulfate, and concentrated to obtain **d9-4** (1.5 g) as yellow oil.

¹ H NMR (400 MHz, DMSO-d ₆) *δ* 7.35 (d, *J =* 4.9 Hz, 1H), 6.87 (d, *J =* 5.0 Hz, 1H), 4.73 (s, 2H), 3.84 (t, J = 5.6 Hz, 2H), 2.66 (d, J = 11.3 Hz, 2H).

**Step 3:** Under nitrogen protection, the intermediate **d9-4** (1.5 g, 7.56 mmol) from the previous step was dissolved in 30 mL of toluene, and NBS (1.3 g, 7.56 mmol) was added. The mixture was reacted at room temperature for 12 hours. The reaction was quenched, and filtered. 60 mL of water was added to the reaction solution. The mixture was extracted with methyl tert-butyl ether, and dried over anhydrous sodium sulfate. The filtrate was concentrated, and the crude product was separated by column chromatography (PE /EA, 100/1) to obtaind**9-5** (500 mg) as yellow oil (yield:17%).

¹ H NMR (300 MHz, DMSO-d ₆) *δ* 6.99 (s, 1H), 4.62 (s, 2H), 3.83 (t, *J=* 5.6 Hz, 2H), 2.61 (tt, *J* = 5.7, 1.9 Hz, 2H).

**Step 4:-78°C,** under nitrogen protection, the intermediate **d9-5** (0.5 g, 2.28 mmol) was dissolved in 10 mL of anhydrous tetrahydrofuran, and a hexane solution of n-BuLi (1.4 mL, 2 M) was added dropwise. After the addition, the mixture was stirred for 30 minutes. Isopropoxyboronic acid pinacol ester iPrOBpin (0.51 g, 2.74 mmol) was added to the mixture, and the mixture was reacted at room temperature for 1 hour and the reaction was quenched. 60 mL of saturated NH₄Cl aqueous solution was added to the reaction solution. The mixture was extracted with ethyl acetate, dried over anhydrous sodium sulfate, and concentrated to afford d9 (550 mg) as yellow solid (yield:91%). LCMS ESI-MS m/z: 267 [M+H]⁺.

### Preparation of Intermediates d10-d11

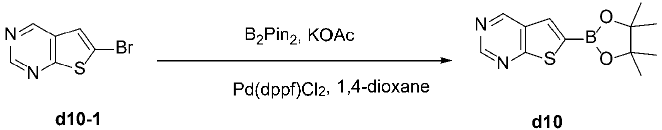

**Steps:** Under nitrogen protection, the raw material **d10-1** (50 mg, 0.23 mmol) and pinacol diborate B₂Pin₂ (89 mg, 0.35 mmol) were dissolved in 1 mL of 1,4-dioxane, and the catalyst Pd(dppf)Cl₂ (17 mg, 0.02 mmol) and KOAc (57 mg, 0.58 mmol) were added. The mixture was heated to 80°C for 2 hours. The reaction was quenched, and filtered. 5 mL of water was added to the reaction solution. The mixture was extracted with ethyl acetate, dried over anhydrous sodium sulfate, and concentrated to afford **d10** (20 mg) as yellow solid (yield:33%). LCMS ESI-MS m/z: 263 [M+H]⁺.

Referring to the synthetic route of intermediate **d10,** the following target intermediate were synthesized using similar raw materials/analogues .

| **Intermediates** | **Intermediate Structures** | **LC-MS ESI-MS m/z:[M+H]⁺** |
|---|---|---|
| **d11** | 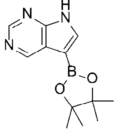 | 246 |

### Preparation of intermediate d12

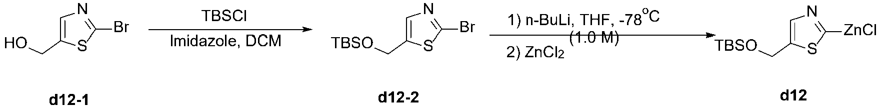

**Step 1:** Under nitrogen protection, the raw material **d12-1** (2.5 g, 12.88 mmol) and imidazole (1.8 g, 25.77 mmol) were dissolved in 40 mL of dichloromethane. TBS-Cl (2.1 g, 14.2 mmol) was added, and the mixture was reacted at room temperature for 1 hour. The reaction was quenched. 100 mL of water was added to the reaction solution. The mixture was extracted with dichloromethane, and the extract was dried over anhydrous sodium sulfate. The filtrate was concentrated, and the crude product was separated by column chromatography (PE/EA, 10/1) to obtain **d12-2** (3.0 g) as yellow oil (yield:76%). LCMS ESI-MS m/z: 308 [M+H]⁺.

**Step 2:** At -78°C and under nitrogen protection, the intermediate **d12-2** (1.0 g, 3.24 mmol) was dissolved in 23 mL of anhydrous tetrahydrofuran, and n-BuLi (1.3 mL, 2.5 M) in hexane solution was added dropwise, and the mixture was stirred for 30 minutes. The pre-prepared tetrahydrofuran solution of ZnCl ₂ (10.7 mL, 1M) was added to the reaction solution, and the mixture was reacted for 1 hour, and then the reaction was quenched. The residue was directly used for the next reaction.

### Preparation of Intermediate d13

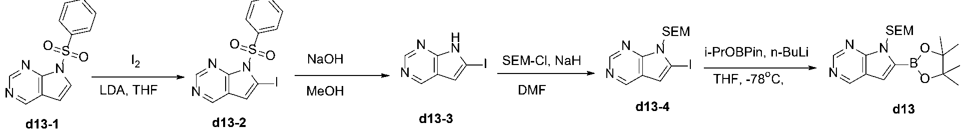

**Step 1:** At -78°C and under nitrogen protection, the raw material **d13-1** (1.1 g, 4.24 mmol) was dissolved in 10 mL of anhydrous tetrahydrofuran, and a hexane solution of LDA (4.24 mL, 2 M) was added dropwise. After the addition, a tetrahydrofuran solution of I₂ (1.62 g, 6.36 mmol, 1 mL) prepared in advance was added. The mixture was reacted at -78°C for 1 hour and the reaction was quenched. 50 mL of saturated sodium thiosulfate Na₂S₂O₃ aqueous solution was added to the reaction solution. The mixture was extracted with dichloromethane, and the extract was dried over anhydrous sodium sulfate. The filtrate was concentrated, and the crude product was separated by flash reverse column chromatography (chromatographic column: C18; CH₃CN/H₂O, 7/10) to afford **d13-2** (310 mg) as yellow solid (yield: 18%). LCMS ESI-MS m/z: 386 [M+H]⁺.

**Step 2:** The intermediate **d13-2** (386 mg, 1.0 mmol) was dissolved in 4 mL of methanol, and aqueous NaOH solution (3.9 mL, 2.5 N) was added. The mixture was reacted at room temperature for 1 hour. 30 mL of ice water was added to the reaction solution. The mixture was extracted with ethyl acetate, dried over anhydrous sodium sulfate, and concentrated to afford **d13-3** (200 mg) as yellow solid (yield:81%). LCMS ESI-MS m/z: 246 [M+H]⁺.

**Step 3:** In an ice bath, the intermediate **d13-3** (0.2 g, 0.81 mmol) was dissolved in 4 mL of DMF, and NaH (30 mg, 1.22 mmol, 60%) was slowly added. After stirring for 1 hour, SEM-Cl (0.2 g, 1.22 mmol) was added to the reaction solution, and the mixture was heated to room temperature for 1 hour and the reaction was quenched. 50 mL of ice water was added to the reaction solution. The mixture was extracted with dichloromethane, and the extract was dried over anhydrous sodium sulfate. The filtrate was concentrated, and the crude product was separated by flash reverse column chromatography (chromatographic column: C18; CH₃CN/H₂O, 1/1) to afford **d13-4** (200 mg) as yellow solid (yield: 65%). LCMS ESI-MS m/z: 376 [M+H]⁺.

**Step 4:** At -78°C and under nitrogen protection, the intermediate **d13-4** (170 mg, 0.45 mmol) was dissolved in 4 mL of anhydrous tetrahydrofuran, and a solution of n-BuLi in hexane (0.22 mL, 2.5 M) was added dropwise. After the addition, the mixture was stirred for 30 minutes. Isopropoxyboronic acid pinacol ester iPrOBpin (101 mg, 0.54 mmol) was added to the mixture, and the mixture was reacted at room temperature for 1 hour. The reaction was quenched. 60 mL of saturated NH₄Cl aqueous solution was added to the reaction solution. The mixture was extracted with dichloromethane, dried over anhydrous sodium sulfate, and concentrated to afford **d13** (80 mg) as yellow solid (yield:48%). LCMS ESI-MS m/z: 376 [M+H]⁺.

### Preparation of Intermediate d14

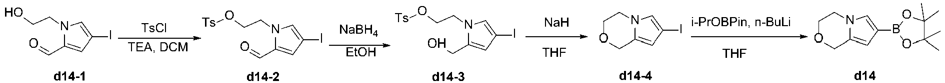

**Step 1:** Raw material **d14-1** (2.4 g, 9.1 mmol) and TEA (2.75 g, 27.2 mmol) were dissolved in 24 mL of anhydrous dichloromethane, and p-toluenesulfonyl chloride TsCl (3.1 g, 54.3 mmol) was added dropwise. After the addition was completed, the mixture was reacted at room temperature for 12 hours and the reaction was quenched. 50 mL of ice water was added to the reaction solution. The mixture was extracted with dichloromethane, and the extract was dried over anhydrous sodium sulfate. The filtrate was concentrated, and the crude product was separated by flash column chromatography (PE/EA, 3/1) to afford **d14-2** (3.1 g) as light pink solid (yield: 8.1%). LCMS ESI-MS m/z: 420 [M+H]⁺.

**Step 2:** In an ice bath and under nitrogen protection, the intermediate **d14-2** (3.1 g, 7.39 mmol) was dissolved in 31 mL of ethanol, and NaBH₄ (0.28 g, 7.39 mmol) was added. The mixture was reacted at room temperature for 1 hour. 30 mL of ice water was added to the reaction solution, and the pH was adjusted to about 6 with trifluoroacetic acid. The mixture was extracted with dichloromethane, dried over anhydrous sodium sulfate, and concentrated to afford **d14-3** (1.3 g) as yellow solid (yield:4.1 %). LCMS ESI-MS m/z: 422 [M+H]⁺.

**Step 3:** The intermediate **d14-3** (1.3 g, 3.09 mmol) was dissolved in 12 mL of anhydrous tetrahydrofuran, and NaH (123 mg, 3.09 mmol, 60%) was slowly added. The mixture was reacted at room temperature for 12 hours and the reaction was quenched. 40 mL of ice water was added to the reaction solution. The mixture was extracted with dichloromethane, and the extract was dried over anhydrous sodium sulfate. The filtrate was concentrated, and the crude product was separated by flash column chromatography (PE / EA, 50/1) to afford **d14-4** (440 mg) as yellow solid (yield: 57 %). LCMS ESI-MS m/z: 250 [M+H]⁺.

**Step 4:** At -78°C and under nitrogen protection, the intermediate **d14-4** (170 mg, 0.68 mmol) was dissolved in 2 mL of anhydrous tetrahydrofuran, and a hexane solution of n-BuLi (0.27 mL, 2.5 M) was added dropwise. After the addition, the mixture was stirred for 30 minutes. Isopropoxyboronic acid pinacol ester iPrOBpin (152 mg, 0.82 mmol) was added to the mixture, and the mixture was reacted at room temperature for 1 hour. The reaction was quenched. 10 mL of saturated NH₄Cl aqueous solution was added to the reaction solution. The mixture was extracted with dichloromethane, dried over anhydrous sodium sulfate, and concentrated to afford **d14** (120 mg) as yellow solid (yield:7.1 %). LCMS ESI-MS m/z: 376 [M+H]⁺.

### Preparation of Intermediates d16-d17

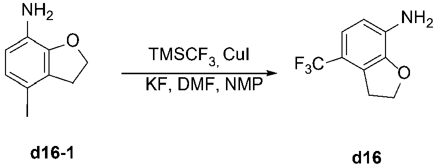

**Steps:** Under nitrogen protection, TMSCF₃ (490 mg, 3.44 mmol) and KF (200 mg, 3.44 mmol) were dissolved in 12 mL of a mixed solution of DMF and NMP (v/v, 1/1). Catalyst CuI (657 mg, 3.44 mmol) was added. The mixture was stirred at room temperature for 3 hours. The raw material **d16-1** (600 mg, 2.29 mmol) was added to the reaction solution. The mixture was heated to 70°C for 12 hours. The reaction was quenched, and filtered. 60 mL of water was added to the reaction solution. The mixture was extracted with dichloromethane, and the extract was dried over anhydrous sodium sulfate. The filtrate was concentrated, and the crude product was separated by flash reverse column chromatography (chromatographic column: C18; CH₃CN/H₂O, 1/1) to give **d16** (150 mg) as brown oil (yield: 32%). LCMS ESI-MS m/z: 204 [M+H]⁺.

Referring to the synthetic route of intermediate **d16,** the following target intermediate were synthesized using similar raw materials/analogues .

| **Intermediates** | **Intermediate Structures** | **LC-MS ESI-MS m/z:[M+H]⁺** |
|---|---|---|
| **d17** | 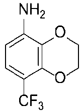 | 220 |

### Preparation of Intermediates d18-d19

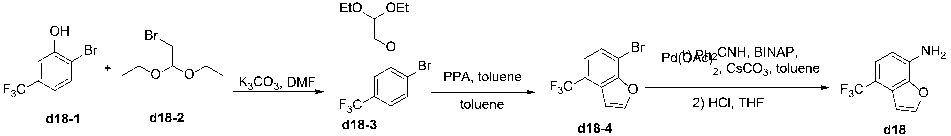

**Step 1:** Under nitrogen protection, the raw material 2-bromo-5-trifluoromethylphenol **d18-1** (5.0 g, 20.75 mmol) and potassium carbonate (8.6 g, 62.2 mmol) were dissolved in 100 mL DMF. The raw material **d18-2** (12.3 g, 62.2 mmol) was added dropwise, and the mixture was heated to 80°C for 12 hours. The reaction was quenched, and filtered. 250 mL of water was added to the reaction solution. The mixture was extracted with ethyl acetate, and dried over anhydrous sodium sulfate. The filtrate was concentrated, and the crude product was separated by column chromatography (PE/EA, 20/1) to obtain **d18-3** (6.0 g) as yellow oil (yield:81%).

**Step 2:** The intermediate **d18-3** (6.0 g, 16.8 mmol) and polyphosphoric acid (30 g, 261 mmol) were dissolved in 72 mL of toluene, and the mixture was heated to 120°C for 5 hours and the reaction was quenched. 100 mL of water was added to the reaction solution, and the pH was adjusted to about 8 with ammonia water. The mixture was extracted with methyl tert-butyl ether, and dried over anhydrous sodium sulfate. The filtrate was concentrated, and separated by column chromatography (PE/EA, 20/1) to obtain **d18-4** (1.0 g) as yellow oil (yield:23%).

**Step 3:** Under nitrogen protection, the intermediate **d18-4** (1.0 g, 3.77 mmol) and the raw material diphenyl ketone imine (1.4 g, 7.55 mmol) were dissolved in 20 mL of toluene. The catalyst Pd(OAc)₂ (80 mg, 0.38 mmol), the ligand BINAP (0.5 g, 0.8 mmol) and cesium carbonate (2.5 g, 7.55 mmol) were added. The mixture was heated to 110°C for 2 hours. The reaction was quenched, and filtered. 100 mL of water was added to the reaction solution. The mixture was extracted with ethyl acetate, dried over anhydrous sodium sulfate, and concentrated to afford a crude mixture. The mixture was dissolved in 5 mL of tetrahydrofuran, and a 2M solution of hydrogen chloride in tetrahydrofuran (20 mL) was added. The mixture was reacted at room temperature for 1 hour and the reaction was quenched. The reaction solution was adjusted to about pH 8 by adding aqueous saturated sodium bicarbonate. The mixture was extracted with ethyl acetate, dried over anhydrous sodium sulfate, and concentrated. The crude product was separated by flash reverse column chromatography (chromatographic column: C18; CH₃CN/H₂O, 1/1) to give **d18** (150 mg) as yellow solid (yield: 20 %). LCMS ESI-MS m/z: 202 [M+H]⁺.

Referring to the synthetic route of intermediate **d18,** the following target intermediate were synthesized using similar raw materials/analogues .

| **Intermediates** | **Intermediate Structures** | **LC-MS ESI-MS m/z:[M+H]⁺** |
|---|---|---|
| **d19** | 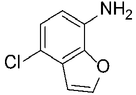 | 168 |

### Preparation of Intermediate d20

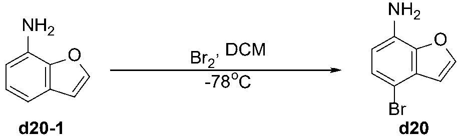

**Steps:** At -78°C and under nitrogen protection, the raw material 7-aminobenzofuran **d20-1** (600 mg, 4.13 mmol) was dissolved in 14 mL of anhydrous dichloromethane, and the pre-prepared liquid BR₂ solution (0.7 g, 4.13 mmol, DCM 11 mL) was added. The mixture was reacted at -78°C for 1 hour and the reaction was quenched. 20 mL of saturated sodium thiosulfate aqueous solution was added to the reaction solution. The mixture was extracted with dichloromethane, and the extract was dried over anhydrous sodium sulfate. The filtrate was concentrated, and the crude product was separated by flash reverse column chromatography (chromatographic column: C18; CH₃CN/H₂O, 1/1) to give **d20** (200 mg) as yellow oil(yield: 23 %). LCMS ESI-MS m/z: 212 [M+H]⁺.

### Preparation of Intermediate d21

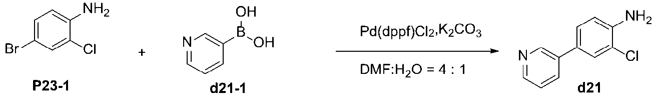

**Steps:** Under nitrogen protection, 2-chloro-4-bromoaniline **P23-1** (1.1 g, 5.32 mmol) and the raw material pyridine-3-boronic acid **d21-1** (1.0 g, 7.99 mmol) were dissolved in 20 mL of a mixed solution of DMF and water (v/v, 4/1). Catalyst Pd(dppf)Cl₂ (400 mg, 0.53 mmol) and potassium carbonate (1.5 g, 10.65 mmol) were added. The mixture was heated to 110°C for 1 hour. The reaction was quenched, and filtered. 50 mL of water was added to the reaction solution. The mixture was extracted with ethyl acetate, and dried over anhydrous sodium sulfate. The filtrate was concentrated to afford a crude mixture. The crude product is separated by flash reverse column chromatography (chromatographic column: C18; CH₃CN/H₂O, 1/1) to give **d21** (900 mg) as yellow solid (yield: 83%). LCMS ESI-MS m/z: 205 [M+H]⁺.

### Preparation of Intermediate d22

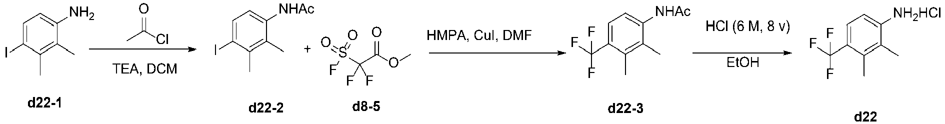

**Step 1:** In an ice bath and under nitrogen protection, the raw material 4-iodo-2,3-dimethyl-aniline **d22-1** (5.1 g, 20.6 mmol) and TEA (4.1 g, 40.5 mmol) was dissolved in 10 mL of dichloromethane, and acetyl chloride (1.9 g, 24.7 mmol) was added. The mixture was reacted in an ice bath for 2 hours and the reaction was quenched. 50 mL of water was added to the reaction solution. The mixture was extracted with dichloromethane, and the extract was dried over anhydrous sodium sulfate. The filtrate was concentrated, and the crude product was separated by flash reverse column chromatography (chromatographic column: C18; CH₃CN/H₂O, 10/7) to give white solid **d22-2** (5.5 g) (yield: 92%). LCMS ESI-MS m/z: 290 [M+H]⁺.

**Step 2:** Under nitrogen protection, the intermediate **d22-2** (1.5 g, 5.18 mmol) and the raw material methyl 2,2-difluoro-2-fluorosulfonylacetate **d8-5** (4.9 g, 25.9 mmol) were dissolved in 15 mL of DMF, and the catalyst CuI (1.4 g, 7.78 mmol) and HMPA (4.6 g, 25.9 mmol) were added. The mixture was heated to 80°C for 12 hours. The reaction was quenched, and filtered. 60 mL of water was added to the reaction solution. The mixture was extracted with ethyl acetate, and dried over anhydrous sodium sulfate. The filtrate was concentrated, and the crude product was separated by flash reverse column chromatography (chromatographic column: C18; CH₃CN/H₂O, 10/7) to give yellow solid **d22-3** (950 mg) (yield: 79 %). LCMS ESI-MS m/z: 232 [M+H]⁺.

**Step 3:** The intermediate **d22-3** (950 mg, 4.10 mmol) was dissolved in 15 mL of ethanol. Diluted hydrochloric acid (7.6 mL, 6M) was added, and the mixture was heated to 80°C for 2 hours and the reaction was quenched. The solvent was evaporated under reduced pressure to afford **d22** (750 mg) as yellow solid (yield:96%). LCMS ESI-MS m/z: 190 [M+H]⁺.

### Preparation of Intermediate d23

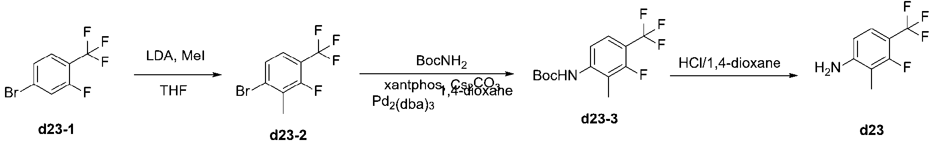

**Step 1:** At -78°C and under nitrogen protection, the raw material 4-bromo-2-fluoro-trifluoromethylbenzene **d23-1** (2.1 g, 8.64 mmol) was dissolved in 21 mL of anhydrous tetrahydrofuran, and a hexane solution of LDA (6.5 mL, 2 M) was added dropwise. The mixture was stirred at this temperature for 1 hour. MeI (1.4 g, 9.51 mmol) was added to the reaction solution. The mixture was warmed to room temperature for 2 hours, and the reaction was quenched. 50 mL of saturated aqueous ammonium chloride solution was added to the reaction solution. The mixture was extracted with dichloromethane, dried over anhydrous sodium sulfate, and concentrated to obtain **d23-2** (1.5 g) as yellow oil (yield:68%). LCMS ESI-MS m/z: 257 [M+H]⁺.

**Step 2:** Under nitrogen protection, the intermediate **d23-2** (550 mg, 2.14 mmol), Cs₂CO₃ (1.4 g, 4.28 mmol) and the raw material BocNH₂ (275.8 mg, 2.35 mmol) were dissolved in 9 mL of 1,4-dioxane. Catalyst Pd₂(dba)₃ (58.8 mg, 0.064 mmol) and ligand XantPhos (49.5 mg, 0.086 mmol) were added. The mixture was heated to 80°C for 1 hour. The reaction was quenched, and filtered. 60 mL of water was added to the reaction solution. The mixture was extracted with ethyl acetate, and dried over anhydrous sodium sulfate. The filtrate was concentrated, and the crude product was separated by flash reverse column chromatography (chromatographic column: C18; CH₃CN/H₂O, 5/4) to give **d23-3** (700 mg) as yellow solid (yield: 95 %). LCMS ESI-MS m/z: 294 [M+H]⁺.

**Step 3:** The intermediate **d23-3** (700 mg, 4.10 mmol) was dissolved in 15 mL of HCl 1,4-dioxane solution (2M), and the mixture was reacted at room temperature for 1 hour, and the reaction was quenched. The solvent was evaporated under reduced pressure. The reaction solution was adjusted to about pH 8 by adding a saturated NaHCO₃ aqueous solution. The mixture was extracted with ethyl acetate, and dried over anhydrous sodium sulfate. The filtrate was concentrated, and the crude product was separated by flash reverse column chromatography (chromatographic column: C18; CH₃CN/H₂O, 10/7) to give **d23** (300 mg) as yellow solid (yield: 65 %). LCMS ESI-MS m/z: 194 [M+H]⁺.

### Preparation of Intermediate d24

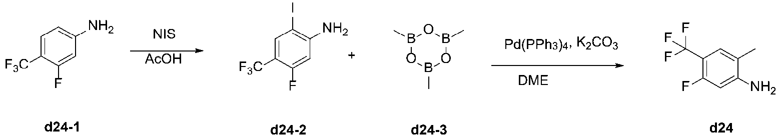

**Step 1:** Under nitrogen protection, the raw material 3-fluoro-4-trifluoromethylaniline **d24-1** (2.0 g, 11.2 mmol) was dissolved in 21 mL of acetic acid. N-iodosuccinimide NIS (2.5 g, 11.2 mmol) was slowly added, and the mixture was reacted at room temperature for 3 hours. 50 mL of saturated ammonium chloride aqueous solution was added to the reaction solution. The mixture was extracted with dichloromethane, and the extract was dried over anhydrous sodium sulfate. The filtrate was concentrated, and the crude product was separated by flash reverse column chromatography (chromatographic column: C18; CH₃CN/H₂O, 5/4) to give **d24-2** (1.5 g) as yellow solid (yield: 44%). LCMS ESI-MS m/z: 306 [M+H]⁺.

**Step 2:** Under nitrogen protection, the intermediate **d24-2** (1.5 g, 4.92 mmol), K₂CO₃ (2.0 g, 14.8 mmol) and the raw material trimethylcyclotriboroxane **d24-3** (930 mg, 7.38 mmol) were dissolved in 15 mL of DME. Catalyst Pd(PPh₃)₄ (0.28 g, 0.25 mmol) was added. The mixture was heated to 100°C for 1 hour. The reaction was quenched, and filtered. 60 mL of water was added to the reaction solution. The mixture was extracted with ethyl acetate, and dried over anhydrous sodium sulfate. The filtrate was concentrated, and the crude product was separated by flash reverse column chromatography (chromatographic column: C18; CH₃CN/H₂O, 5/3) to give **d24** (300 mg) as yellow oil (yield: 32 %). LCMS ESI-MS m/z: 194 [M+H]⁺.

### Example 2:

### Preparation of Target Molecules P1-P3

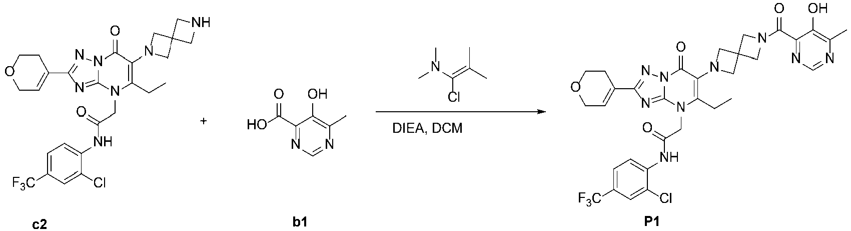

**Step:** Under nitrogen protection, the intermediate **b1** (16 mg, 0.1 mmol) and 1-chloro-N,N,2-trimethylpropyl-1-en-1-amine (13.9 mg, 0.1 mmol) were dissolved in 1 mL of dichloromethane and the mixture was stirred at room temperature for 1 hour. Intermediate **c2** (51 mg, 0.1 mmol) and DIEA (56 mg, 0.4 mmol) were added to the reaction solution, and the mixture continued to react at room temperature for 1 hour, and the reaction was quenched. 10 mL of water was added to the reaction solution. The mixture was extracted with dichloromethane, and the extract was dried over anhydrous sodium sulfate. The filtrate was concentrated, and the crude product was separated by HPLC chromatography (chromatographic column: XSelect Prep OBD C18 Column, 30*150 mm, 5µm; mobile phase A: water (10 mmol/L NH₄HCO₃), mobile phase B: acetonitrile; flow rate: 60 mL/min; retention time: 3 min) to give **P1** (7.3 mg) as white solid (yield: 11%). LCMS ESI-MS m/z: 714 [M+H]⁺.

Referring to the synthetic route of compound **P1,** similar raw materials/intermediates were used to synthesize the following target molecules.

| **Target molecules** | **Molecular structure** | **¹ H NMR** | **LC-MS ESI-MS m/z:[M+H]⁺** |
|---|---|---|---|
| **P2** | 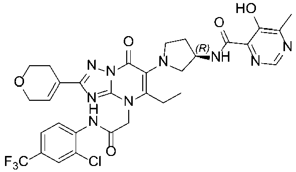 | ¹ H NMR (300 MHz, DMSO- *d* ₆) *δ* 12.43 (s, 1H), 10.40 (s, 1H), 9.57 (s, 1H), 8.69 (s, 1H), 8.08 (d, *J* = 8.5 Hz, 1H), 7.97 (s, 1H), 7.72 (d, *J* = 9.2 Hz, 1H), 6.85 (s, 1H), 5.34 (s, 2H), 4.68 (s, 1H), 4.27 (s, 2H), 3.82 (s, 2H), 3.49 (s, 1H), 3.17 (s, 3H), 2.97 (d, *J* = 8.0 Hz, 2H), 2.54 (s, 2H), 2.46 (s, 3H), 2.33 - 2.06 (m, 2H), 1.21 (t, *J* = 7.4 Hz, 3H). | 703 |
| **P3** | 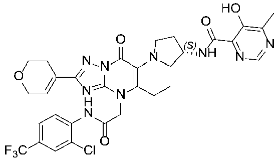 | ¹ H NMR (300 MHz, DMSO- *d* ₆) *δ* 12.42 (s, 1H), 10.40 (s, 1H), 9.68 (s, 1H), 8.65 (s, 1H), 8.08 (d, *J* = 8.6 Hz, 1H), 7.97 (d, *J* = 1.9 Hz, 1H), 7.73 (d, *J* = 8.8 Hz, 1H), 6.85 (s, 1H), 5.34 (s, 2H), 4.68 (s, 1H), 4.27 (s, 2H), 3.82 (t, *J* = 5.5 Hz, 2H), 3.54 - 3.44 (m, 1H), 3.17 (s, 3H), 2.98 (d, *J* = 8.0 Hz, 2H), 2.54 (s, 2H), 2.45 (s, 3H), 2.30 (d, *J* = 12.6 Hz, 1H), 2.11 (d, *J* = 14.8 Hz, 1H), 1.21 (t, *J* = 7.6 Hz, 3H). | 703 |

### Example 3:

### Preparation of Target Molecules P4-P21, P33-P51, A3

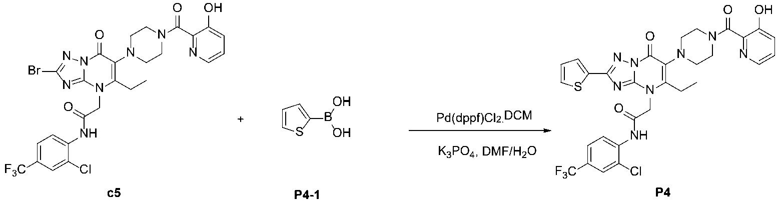

**Steps:** Under nitrogen protection, the intermediate **c5** (55 mg, 0.08 mmol), the raw material thiophene 2-carboxylic acid **P4-1** (11 mg, 0.08 mmol) and potassium phosphate (51 mg, 0.24 mmol) were dissolved in 1 mL of a mixed solution of DMF and water (v/v, 4/1). Catalyst Pd(dppf)Cl₂.CH₂Cl₂ (6.6 mg, 0.01 mmol) was added. The mixture was heated to 90°C for 1 hour, and the reaction was quenched. 5 mL of water was added to the reaction solution. The mixture was extracted with ethyl acetate, and dried over anhydrous sodium sulfate. The filtrate was concentrated, and the crude product was separated by HPLC chromatography (chromatographic column: Xbridge Prep phenyl OBD Colum, 30*150 nm, 5 µm; mobile phase A: water (10 mmol/L NH₄HCO₃), mobile phase B: acetonitrile; flow rate: 60 mL/min, retention time: 8.7 min;) to afford **P4** (10.1 mg) white solid (yield: 18%). LCMS ESI-MS m/z: 687 [M+H]⁺.

¹ H NMR (300 MHz, DMSO- *d₆) δ* 10.42 (s, 2H), 8.06 (d, *J* = 8.2 Hz, 2H), 7.97 (d, *J* = 2.0 Hz, 1H), 7.74 (dd, *J* = 13.0, 6.5 Hz, 3H), 7.30 (s, 2H), 7.21 (t, *J* = 4.3 Hz, 1H), 5.37 (s, 2H), 4.57 (d, *J* = 12.1 Hz, 1H), 3.48 (s, 3H), 3.42 (d, *J* = 13.3 Hz, 1H), 3.26 (d, *J* = 13.2 Hz, 3H), 2.99 (d, *J* = 12.7 Hz, 1H), 2.82 (d, *J* = 11.3 Hz, 1H), 2.62 (d, *J* = 28.8 Hz, 1H), 1.21 (t, *J* = 7.7 Hz, 4H).

Referring to the synthetic route of compound **P4,** similar raw materials/intermediates (e.g., intermediates **c6, c12-c13, c16-c17, b2, d1-d7, d 9 -d 11, d13-d14,** etc.), to synthesize the following target molecules:

| **Target molec ules** | **Molecular structure** | **¹ H NMR** | **LC-MS ESI-MS m/z:[M +H]⁺** |
|---|---|---|---|
| **P5** | 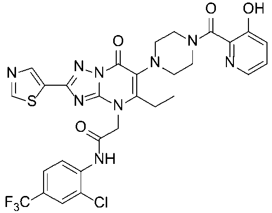 | ¹ H NMR (400 MHz, DMSO- *d ₆) δ* 9.27 (s, 1H), 8.50 (s, 1H), 8.36 (s, 1H), 8.05-7.98 (d, *J* = 8.7 Hz, 3H), 7.72 (dd, *J* = 8.8, 2.2 Hz, 1H), 7.28 (dd, *J* = 12.5, 7.8 Hz, 2H), 5.38 (s, 2H), 4.56 (d, *J* = 12.5 Hz, 1H), 3.52 - 2.91 (m, 8H), 2.82 (d, *J* = 11.2 Hz, 1H), 2.65 (d, *J* = 10.7 Hz, 1H), 1.25 - 1.16 (m, 3H). | 689 |
| **P6** | 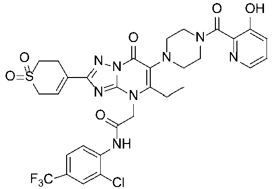 | ¹ H NMR (300 MHz, DMSO- *d ₆) δ* 10.38 (s, 2H), 8.10 - 7.96 (m, 3H), 7.71 (d, *J* = 8.4 Hz, 1H), 7.29 (d, *J* = 2.6 Hz, 2H), 6.76 (s, 1H), 5.32 (s, 2H), 4.55 (d, *J* = 12.3 Hz, 1H), 3.99 (s, 2H), 3.51 (s, 2H), 3.37 (s, 3H), 3.22 (s, 1H), 3.10 (s, 2H), 2.99 (s, 3H), 2.80 (d, *J* = 10.7 Hz, 1H), 2.63 (d, *J* = 10.5 Hz, 1H), 1.20 (q, *J* = 7.4 Hz, 4H). | 735 |
| **P7** | 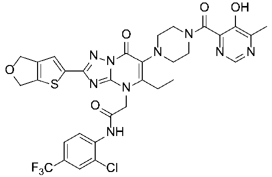 | ¹ H NMR (400 MHz, DMSO- *d ₆) δ* 10.37 (d, *J* = 8.6 Hz, 1H), 8.52 (s,1H) 8.05 (d, *J* = 8.6 Hz, 1H), 7.97 (d, *J* = 2.1 Hz, 1H), 7.71 (d,*J* = 8.7 Hz, 1H), 7.55 (s, 1H), 5.35 (s, 2H), 5.01 (d, *J* = 3.7 Hz, 2H), 4.88 (s, 2H), 4.69 (s, 1H), 4.52 (d, *J* = 12.8 Hz, 1H), 3.50 (d, *J* = 9.6 Hz, 4H), 3.01 (s, 3H), 2.82 (d, *J* = 11.5 Hz, 1H), 2.68 (d, *J* = 16.3 Hz, 1H), 2.39 (d, *J* = 16.3 Hz, 2H), 2.01 (s. 1H), 1.22 (d, *J* = 12.0 Hz, 3H). | 744 |
| **P8** | 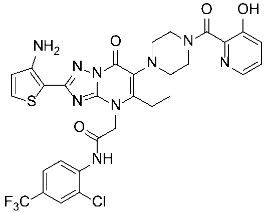 | ¹ H NMR (400 MHz, DMSO- *d ₆) δ* 10.34 (s, 2H), *δ* 8.05 (d, *J* = 8.6 Hz, 2H), 7.97 (s, 1H), 7.43 (d, *J* = 5.3 Hz, 1H), 7.45 (s,1H), 7.30 (s, 2H), 6.66 (d, *J* = 5.4 Hz, 1H), 6.26 (s, 2H), 5.37 (s, 2H), 4.58 (s, 1H), 3.51 (s, 3H), 3.23 (s,1H), 3.01 (s, 3H), 2.80 (s, 1H), 2.64 (d, *J* = 10.9 Hz, 1H), 1.20 (t, *J* = 7.8 Hz, 3H). | 702 |
| **P9** | 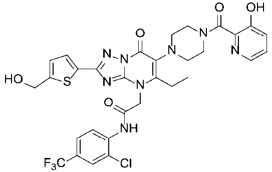 | ¹ H NMR (400 MHz, DMSO- *d* ₆) *δ* 10.37 (s, 2H), 8.13 - 8.00 (m, 2H), 7.97 (d, *J* = 2.1 Hz, 1H), 7.71 (dd, *J* = 8.7, 2.2 Hz, 1H), 7.60 (d, *J* = 3.6 Hz, 1H), 7.34 - 7.21 (m, 2H), 7.02 (d, *J* = 3.7 Hz, 1H), 5.65 (s, 1H), 5.35 (s, 2H), 4.67 (s, 2H), 4.56 (d, *J* = 12.3 Hz, 1H), 3.45 (dd, *J* = 30.9, 11.8 Hz, 3H), 3.25 (d, *J* = 12.5 Hz, 1H), 2.97 (dd, *J* = 19.0, 9.5 Hz, 3H), 2.81 (d, *J* = 11.2 Hz, 1H), 2.64 (d, *J* = 11.0 Hz, 1H), 1.20 (t, *J* = 7.4 Hz, 3H). | 717 |
| **P10** | 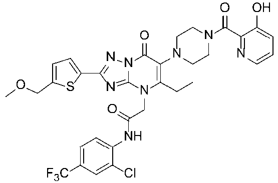 | ¹ H NMR (400 MHz, DMSO- *d* ₆) *δ* 10.41 (s, 2H), 8.11 - 8.02 (m, 2H), 7.98 (s, 1H), 7.72 (d, *J* = 8.7 Hz, 1H), 7.63 (d, *J* = 3.6 Hz, 1H), 7.29 (d, *J* = 3.1 Hz, 2H), 7.12 (d, *J* = 3.6 Hz, 1H), 5.35 (s, 2H), 4.59 (d, *J* = 27.6 Hz, 3H), 3.56 - 3.36 (m, 3H), 3.30 (d, *J* = 11.2 Hz, 4H), 3.01 - 2.97 (m, 3H), 2.82 (d, *J* = 11.2 Hz, 1H), 2.64 (d, *J* = 10.9 Hz, 1H), 1.20 (t, *J* = 7.5 Hz, 3H). | 731 |
| **P11** | 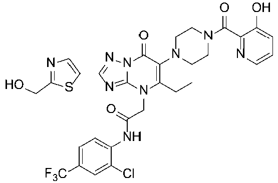 | ¹ H NMR (300 MHz, DMSO- *d* ₆) *δ* 10.42 (s, 2H), 8.29 (s, 1H), 8.02 (d, *J* = 25.9 Hz, 3H), 7.72 (s, 1H), 7.30 (s, 2H), 6.27 (s, 1H), 5.36 (s, 2H), 4.78 (s, 2H), 4.56 (d, *J* = 11.8 Hz, 1H), 3.59 - 3.40 (m,4H), 3.01 (s, 3H), 2.83 (s,1H), 2.65 (s, 1H), 1.20 (s, 3H). | 718 |
| **P12** | 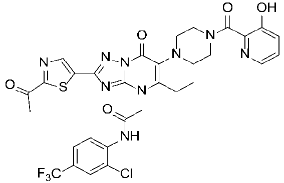 | ¹ H NMR (300 MHz, DMSO- *d* ₆) *δ* 10.43 (s, 1H), 8.67 (s, 1H), 8.07 - 7.98 (m, 2H), 7.97 (d, *J* = 2.1 Hz, 1H), 7.70 (dd, *J* = 8.9, 2.2 Hz, 1H), 7.29 (d, *J* = 2.9 Hz, 2H), 5.37 (s, 2H), 4.56 (d, *J* = 12.5 Hz, 1H), 3.48 (d, *J* = 16.3 Hz, 3H), 3.27 - 3.14 (m, 1H), 3.01 (t, *J* = 10.1 Hz, 4H), 2.82 (d, *J* = 11.1 Hz, 1H), 2.67 (s, 4H), 1.21 (t, *J* = 7.8 Hz, 3H). | 730 |
| **P13 ^{#}** | 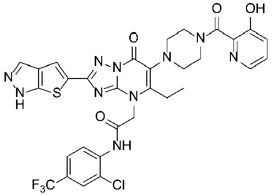 | ¹ H NMR (400 MHz, DMSO- *d ₆) δ* 8.14 (s, 1H), 8.07 (d, *J* = 8.6 Hz, 2H), 7.96 (s, 1H), 7.78 - 7.67 (m, 2H), 7.29 (d, *J* = 3.0 Hz, 2H), 5.35 (s, 2H), 4.56 (d, *J* = 12.3 Hz, 1H), 3.49 (d, *J* = 11.2 Hz, 4H), 3.25 (d, *J* = 12.8 Hz, 1H), 2.99 (d, *J* = 19.5 Hz, 2H), 2.82 (d, *J* = 11.0 Hz, 1H), 2.64 (d, *J* = 11.5 Hz, 1H), 1.21 (dd, *J* = 13.5, 5.7 Hz, 3H). | 727 |
| **P14 ^{#}** | 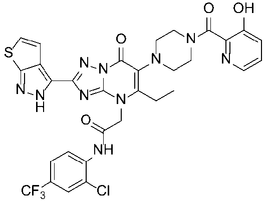 | ¹ H NMR (400 MHz, DMSO- *d ₆) δ* 10.48 (s, 1H), 8.06 (d, *J* = 8.1 Hz, 2H), 7.97 (d, *J* = 2.2 Hz, 1H), 7.71 (dd, *J* = 8.7, 2.2 Hz, 1H), 7.41 (d, *J* = 5.5 Hz, 1H), 7.29 (d, *J* = 5.6 Hz, 3H), 5.43 (s, 2H), 4.57 (d, *J* = 12.4 Hz, 1H), 3.63 - 3.42 (m, 4H), 3.17 - 2.94 (m, 3H), 2.84 (d, *J* = 11.0 Hz, 1H), 2.67 (d, *J* = 11.2 Hz, 1H), 1.22 (t, *J* = 7.4 Hz, 5H). | 727 |
| **P15** | 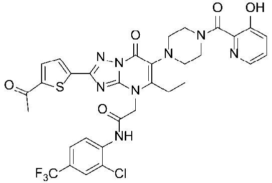 | ¹ H NMR (300 MHz, DMSO- *d ₆) δ* 10.42 (s, 2H), 8.09 - 7.92 (m, 4H), 7.83 (d, *J* = 4.0 Hz, 1H), 7.71 (d, *J* = 8.7 Hz, 1H), 7.29 (d, *J* = 3.0 Hz, 2H), 5.37 (s, 2H), 4.57 (d, *J* = 12.3 Hz, 1H), 3.46 (d, *J* = 12.6 Hz, 4H), 3.46 (s, 1H)₃.02 (s, 3H), 2.82 (d, *J* = 10.7 Hz, 1H), 2.58 (s, 3H), 1.21 (t, *J* = 7.4 Hz, 3H). | 729 |
| **P16** | 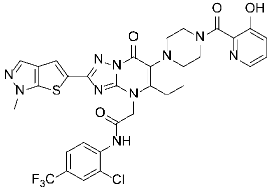 | ¹ H NMR (300 MHz, DMSO- *d ₆) δ* 8.20 - 7.92 (m, 3H), 7.83 (s, 1H), 7.71 (s, 2H), 7.30 (s, 2H), 5.35 (s, 2H), 4.57 (d, *J* = 12.8 Hz, 1H), 3.99 (s, 3H), 3.01 (s, 3H), 2.81 (s, 1H), 1.22 (d, *J* = 8.2 Hz, 3H). | 741 |
| **P17** | 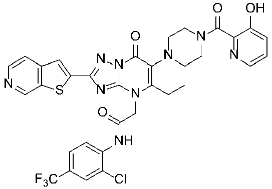 | ¹ H NMR (300 MHz, DMSO- *d ₆) δ* 10.47 (s, 2H), 9.33 (s, 1H), 8.53 (d, *J* = 5.6 Hz, 1H), 8.20 (s, 1H), 8.09 (s, 1H), 8.04 - 7.86 (m, 2H), 7.72 (d, *J* = 8.9 Hz, 1H), 7.30 (s, 2H), 5.41 (s, 2H), 4.58 (d, *J* = 11.9 Hz, 1H), 3.03 (s, 3H), 2.85 (d, *J* = 10.8 Hz, 1H), 2.67 (d, *J* = 10.8 Hz, 1H), 1.21 (d, *J* = 8.5 Hz, 3H). | 738 |
| **P18** | 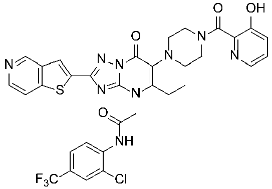 | ¹ H NMR (400 MHz, DMSO- d ₆) *δ* 10.49 (s, 2H), 9.22 (s, 1H), 8.49 (d, *J* = 5.6 Hz, 1H), 8.26 (s, 1H), 8.13 (d, *J* = 5.6 Hz, 1H), 8.07 (t, *J* = 6.9 Hz, 2H), 7.97 (s, 1H), 7.71 (d, *J* = 8.7 Hz, 1H), 7.29 (d, *J* = 3.9 Hz, 2H), 5.40 (s, 2H), 4.62 - 4.54 (m, 1H), 3.50 (d, *J* = 9.7 Hz, 3H), 3.27 (d, *J* = 11.9 Hz, 1H), 3.06 - 3.00 (m, 3H), 2.84 (d, *J* = 11.2 Hz, 1H), 2.66 (d, *J* = 10.8 Hz, 1H), 1.22 (t, *J* = 7.4 Hz, 3H). | 738 |
| **P19** | 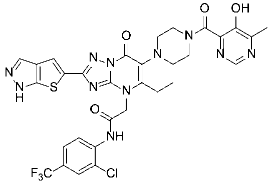 | ¹ H NMR (300 MHz, DMSO- *d* ₆) *δ* 8.54 (s, 1H), 8.21 - 7.90 (m, 3H), 7.72 (d, *J* = 8.7 Hz, 2H), 5.37 (s, 2H), 4.54 (d, *J* = 12.6 Hz, 1H), 3.52 (d, *J* = 10.4 Hz, 4H), 3.02 (d, *J* = 9.0 Hz, 3H), 2.84 (d, *J* = 11.3 Hz, 1H), 2.70 - 2.62 (m, 1H), 2.44 (s, 3H), 1.29 - 1.14 (m, 3H). | 742 |
| **P20** | 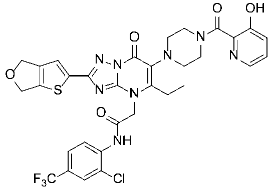 | ¹ H NMR (300 MHz, DMSO- *d ₆) δ* 10.36 (s, 1H), 8.11 - 7.94 (m, 3H), 7.72 (d, *J* = 8.7 Hz, 1H), 7.56 (s, 1H), 7.29 (s, 2H), 5.35 (s, 2H), 5.02 (s, 2H), 4.89 (s, 2H), 4.56 (d, *J* = 12.3 Hz, 1H), 3.44 (s, 3H), 3.23 (s, 1H), 3.00 (s, 3H), 2.82 (d, *J* = 10.8 Hz, 1H), 2.64 (d, J = 10.4 Hz, 1H), 1.21 (d, *J* = 8.4 Hz, 3H). | 729 |
| **P21** | 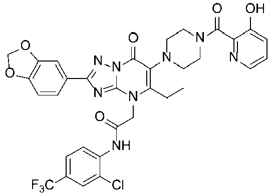 | ¹ H NMR (300 MHz, DMSO- *d* ₆) *δ* 10.45 (s, 2H), 8.06 (dd, *J* = 6.7, 2.8 Hz, 2H), 7.97 (d, *J* = 2.1 Hz, 1H), 7.75 - 7.62 (m, 2H), 7.55 (d, *J* = 1.6 Hz, 1H), 7.29 (d, *J* = 3.0 Hz, 2H), 7.06 (d, *J* = 8.1 Hz, 1H), 6.12 (s, 2H), 5.37 (s, 2H), 4.57 (d, *J* = 12.4 Hz, 1H), 3.59 - 3.40 (m, 3H), 3.24 (s, 1H), 2.99 (q, *J* = 13.0, 10.5 Hz, 3H), 2.82 (d, *J* = 11.2 Hz, 1H), 2.64 (d, *J* = 11.0 Hz, 1H), 1.22 (d, *J* = 8.4 Hz, 3H). | 725 |
| **P33** | 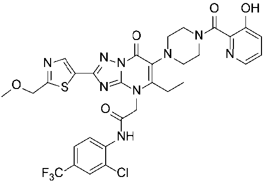 | ¹ H NMR (300 MHz, DMSO- *d ₆) δ* 8.34 (s, 1H), 8.15 - 7.94 (m, 3H), 7.72 (d, *J* = 8.8 Hz, 1H), 7.29 (d, *J* = 2.9 Hz, 2H), 5.37 (s, 2H), 4.77 (s, 2H), 4.57 (d, *J* = 12.6 Hz, 1H), 3.45 (s, 6H), 3.01 (s, 3H), 2.82 (d, *J* = 11.3 Hz, 1H), 2.65 (d, *J* = 10.6 Hz, 1H), 1.21 (t, *J* = 7.5 Hz, 4H). | 732 |
| **P34** | 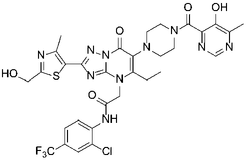 | ¹ H NMR (300 MHz, DMSO- *d* ₆) *δ* 8.54 (s, 1H), 8.11 - 7.92 (m, 2H), 7.72 (d, *J* = 8.7 Hz, 1H), 5.36 (s, 2H), 4.71 (s, 2H), 4.53 (d, *J* = 12.4 Hz, 1H), 3.03 (d, *J* = 10.1 Hz, 3H), 2.83 (d, *J* = 11.0 Hz, 1H), 2.70 (s, 4H), 2.44 (s, 3H), 1.22 (d, *J* = 7.3 Hz, 3H). | 747 |
| **P35** | 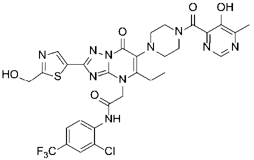 | ¹ H NMR (300 MHz, DMSO- *d ₆) δ* 8.49 (s, 1H), 8.29 (s, 1H), 8.13 - 7.88 (m, 3H), 7.83 - 7.66 (m, 1H), 5.37 (s, 2H), 4.78 (s, 2H), 4.51 (s, 1H), 3.56 (s, 4H), 3.02 (s, 3H), 2.83 (d, *J* = 10.9 Hz, 1H), 2.68 (s, 1H), 2.48 - 2.27 (m, 3H), 1.22 (d, *J* = 7.7 Hz, 3H). | 733 |
| **P36** | 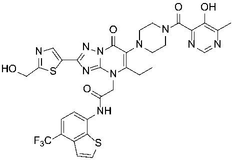 | ¹ H NMR (300 MHz, DMSO- *d ₆) δ* 8.49 (s, 1H), 8.29 (s, 1H), 8.13 - 7.88 (m, 3H), 7.81 - 7.64 (m, 1H), 5.37 (s, 2H), 4.78 (s, 2H), 4.52 (s, 1H), 3.83 (s, 4H), 3.02 (s, 3H), 2.83 (d, *J* = 10.6 Hz, 1H), 2.68 (s, 1H), 2.37 (d, *J* = 32.3 Hz, 3H), 1.22 (d, J = 7.7 Hz, 3H). | 755 |
| **P37** | 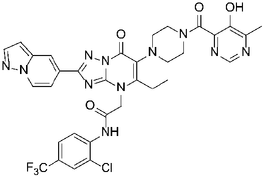 | ¹ H NMR (300 MHz, DMSO- d ₆) *δ* 10.42 (s, 1H), 8.80 (d, *J* = 7.2 Hz, 1H), 8.56 (s, 1H), 8.43 (s, 1H), 8.21 - 7.88 (m, 3H), 7.72 (d,*J* = 8.7 Hz, 1H), 7.50 (d, *J* = 7.2 Hz, 1H), 6.82 (s, 1H), 5.42 (s, 2H), 4.55 (d, *J* = 12.4 Hz, 1H), 3.82 - 3.47 (m, 4H), 3.03 (d, *J* = 9.5 Hz, 3H), 2.85 (d, *J* = 11.0 Hz, 1H), 2.68 (d, *J* = 11.1 Hz, 1H), 2.45 (s, 3H), 1.24 (d, *J* = 6.5 Hz, 3H). | 736 |
| **P38** | 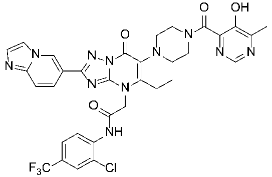 | ¹ H NMR (300 MHz, DMSO *-d ₆) δ* 9.35 (s, 1H), 8.55 (s, 1H), 8.16 - 8.05 (m, 2H), 7.98 (s, 1H), 7.86 (d, *J* = 9.5 Hz, 1H), 7.76 - 7.62 (m, 3H), 5.41 (s, 2H), 4.54 (d, *J* = 12.7 Hz, 1H), 3.48 (s, 4H), 3.02 (d, *J* = 9.5 Hz, 3H), 2.85 (d, *J* = 10.8 Hz, 1H), 2.68 (d, *J* = 11.1 Hz, 1H), 2.44 (s, 3H), 1.22 (t, *J* = 7.2 Hz, 3H). | 736 |
| **P39** | 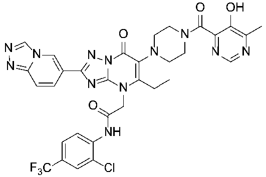 | ¹ H NMR (300 MHz, DMSO- *d ₆) δ* 9.35 (d, *J* = 11.5 Hz, 2H), 8.54 (s, 1H), 8.09 (d, *J* = 8.5 Hz, 1H), 7.95 (d, *J* = 17.1 Hz, 3H), 7.72 (d, J = 8.6 Hz, 1H), 5.41 (s, 2H), 4.54 (d, *J* = 12.5 Hz, 1H), 3.45(s, 5H), 3.02 (d, *J* = 9.8 Hz, 3H), 2.85 (d, *J* = 10.8 Hz, 1H), 2.68 (d, *J* = 11.1 Hz, 1H), 2.44 (s, 2H), 1.21 (d, *J* = 8.0 Hz, 3H). | 737 |
| **P40** | 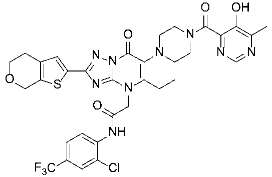 | ¹ H NMR (300 MHz, DMSO- *d ₆) δ* 8.46 (s, 1H), 8.05 (d, *J* = 8.6 Hz, 1H), 7.96 (d, *J* = 2.1 Hz, 1H), 7.71 (dd, *J* = 8.7, 2.1 Hz, 1H), 7.52 (s, 1H), 5.34 (s, 2H), 4.77 (s, 2H), 4.64 - 4.40 (m, 1H), 3.87 (t, *J* = 5.5 Hz, 2H), 3.54 - 3.46 (m, 3H), 3.26 (d, *J* = 12.0 Hz, 3H), 2.99 (d, *J* = 10.1 Hz, 3H), 2.81 (d, *J* = 10.9 Hz, 1H), 2.71 (d, *J* = 5.8 Hz, 3H), 2.41 (s, 3H), 1.20 (q, *J* = 7.3, 6.2 Hz, 3H). | 758 |
| **P41** | 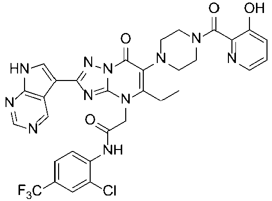 | ¹ H NMR (400 MHz, Methanol- *d* ₄) *δ* 9.66 (s, 1H), 8.84 - 8.79 (m, 1H), 8.15 (d, *J* = 8.5 Hz, 1H), 8.04 (s, 2H), 7.81 (s, 1H), 7.59 (d, *J* = 8.7 Hz, 1H), 7.31 (s, 2H), 5.47 (s, 2H), 4.75 (s, 2H), 4.58 (s, 1H), 3.77 (d, *J* = 11.2 Hz, 2H), 3.45 (s, 1H), 3.15 (s, 3H), 2.97 (d, *J* = 11.5 Hz, 1H), 2.83 - 2.75 (m, 1H), 1.34 (t, *J* = 7.5 Hz, 3H). | 722 |
| **P42** | 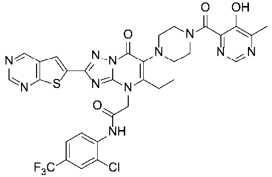 | ¹ H NMR (300 MHz, DMSO- *d* ₆) *δ* 9.40 (s, 1H), 9.15 (s, 1H), 8.48 (s, 1H), 8.22 (s, 1H), 8.07 (d, *J* = 8.6 Hz, 1H), 7.97 (d, *J* = 2.1 Hz, 1H), 7.71 (d, *J* = 8.5 Hz, 1H), 5.41 (s, 2H), 4.54 (d, *J* = 12.4 Hz, 1H), 3.51 (s, 4H), 3.03 (s, 3H), 2.85 (d, *J* = 11.0 Hz, 1H), 2.75 - 2.63 (m, 1H), 2.43 (s, 2H), 2.14 (s, 1H), 1.23 (s, 3H). | 754 |
| **P43** | 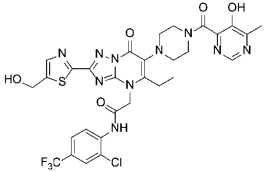 | ¹ H NMR (300 MHz, DMSO- *d* ₆) *δ* 10.41 (s, 1H), 8.57 (s, 1H), 8.08 (d, *J* = 8.6 Hz, 1H), 7.98 (d, *J* = 2.1 Hz, 1H), 7.88 (d, *J* = 1.0 Hz, 1H), 7.72 (dd, *J* = 9.1, 2.1 Hz, 1H), 5.74 (s, 1H), 5.40 (s, 2H), 4.76 (s, 2H), 4.55 (d, *J* = 12.7 Hz, 1H), 3.52 (d, *J* = 11.1 Hz, 3H), 3.27 (s, 1H), 3.03 (d, *J* = 9.2 Hz, 3H), 2.85 (d, *J* = 11.3 Hz, 1H), 2.68 (d, *J* = 10.7 Hz, 1H), 2.45 (s, 3H), 1.21 (t, *J* = 7.4 Hz, 3H). | 733 |
| **P44** | 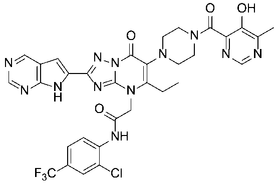 | ¹ H NMR (300 MHz, DMSO- *d ₆) δ* 9.08 (s, 1H), 8.82 (s, 1H), 8.51 (s, 1H), 8.09 (d, *J* = 8.6 Hz, 1H), 7.98 (d, *J* = 2.2 Hz, 1H), 7.76 - 7.69 (m, 1H), 7.23 (s, 1H), 5.42 (s, 2H), 4.55 (d, *J* = 12.5 Hz, 1H), 3.53 (d, *J* = 10.2 Hz, 4H), 3.04 (d, *J* = 8.2 Hz, 4H), 2.86 (d, *J* = 11.1 Hz, 1H), 2.69 (d, *J* = 10.6 Hz, 1H), 2.44 (s, 3H), 1.23 (t, *J* = 7.3 Hz, 3H). | 737 |
| **P45** | 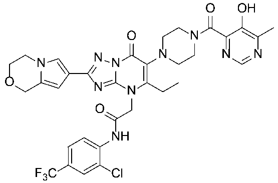 | ¹ H NMR (300 MHz, DMSO- *d ₆) δ* 8.51 (s, 1H), 8.07 (d, J = 8.6 Hz, 1H), 7.97 (d, *J* = 2.1 Hz, 1H), 7.72 (dd, *J* = 8.8, 2.2 Hz, 1H), 7.34 (d, *J* = 1.7 Hz, 1H), 6.28 (s, 1H), 5.33 (s, 2H), 4.74 (s, 2H), 4.52 (d, *J* = 12.5 Hz, 1H), 3.98 (s, 4H), 3.57 (s, 3H), 3.05 - 2.90 (m, 4H), 2.81 (d, *J* = 11.0 Hz, 1H), 2.64 (d, *J* = 10.2 Hz, 1H), 2.43 (s, 3H), 1.24 - 1.17 (m, 3H). | 741 |
| **P46** | 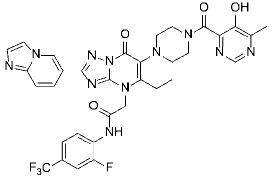 | ¹ H NMR (300 MHz, DMSO- *d* ₆) *δ* 10.77 (s, 1H), 9.33 (s, 1H), 8.51 (s, 1H), 8.25 (t, *J* = 8.1 Hz, 1H), 8.12 (s, 1H), 7.83 (t, *J* = 10.2 Hz, 2H), 7.72 - 7.53 (m, 3H), 5.39 (s, 2H), 4.54 (d, *J* = 12.2 Hz, 1H), 3.52 (s, 4H), 3.00 (s, 3H), 2.85 (d, *J* = 11.4 Hz, 1H), 2.71 (d, *J* = 12.5 Hz, 1H), 2.43 (s, 3H), 1.22 (d, *J* = 8.3 Hz, 3H). | 720 |
| **P47** | 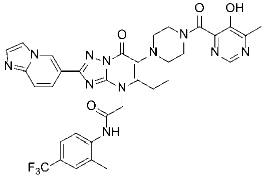 | ¹ H NMR (400 MHz, DMSO- *d ₆) δ* 10.08 (s, 1H), 9.36 (s, 1H), 8.57 (s, 1H), 8.15 (s, 1H), 7.87 (dd, *J* = 9.4, 1.7 Hz, 1H), 7.74 (dd, *J* = 17.9, 8.9 Hz, 2H), 7.65 (dd, *J* = 5.9, 1.6 Hz, 2H), 7.57 - 7.50 (m, 1H), 5.34 (s, 2H), 4.55 (d, *J* = 12.3 Hz, 1H), 3.59 - 3.49 (m, 3H), 3.26 (d, *J* = 10.2 Hz, 1H), 3.09 - 2.96 (m, 3H), 2.86 (d, *J* = 11.1 Hz, 1H), 2.70 - 2.64 (m, 1H), 2.45 (s, 3H), 2.39 (s, 3H), 1.23 (t, *J* = 7.4 Hz, 3H). | 716 |
| **P48** | 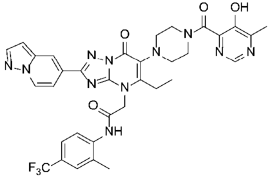 | ¹ H NMR (300 MHz, DMSO- *d* ₆) *δ* 10.10 (s, 1H), 8.81 (d, *J* = 7.3 Hz, 1H), 8.54 (s, 1H), 8.43 (s, 1H), 8.09 (d, *J* = 2.3 Hz, 1H), 7.74 (d, *J* = 8.4 Hz, 1H), 7.64 (s, 1H), 7.58 - 7.46 (m, 2H), 6.84 (d, *J* = 2.3 Hz, 1H), 5.35 (s, 2H), 4.55 (d, *J* = 12.7 Hz, 1H), 3.53 (s, 3H), 3.28 (s, 1H), 3.13 - 2.93 (m, 3H), 2.85 (d, *J* = 11.2 Hz, 1H), 2.68 (d, *J* = 11.6 Hz, 1H), 2.44 (s, 3H), 2.39 (s, 3H), 1.28 - 1.18 (m, 3H). | 716 |
| **P49** | 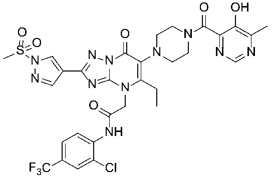 | ¹ H NMR (300 MHz, DMSO- *d ₆) δ* 10.44 (s, 1H), 8.68 (s, 1H), 8.46 (s, 1H), 8.07 (d, *J* = 8.6 Hz, 1H), 7.97 (d, *J* = 2.1 Hz, 1H), 7.71 (dd, *J* = 8.8, 2.1 Hz, 1H), 5.38 (s, 2H), 4.53 (d, *J* = 12.6 Hz, 1H), 3.67 (s, 3H), 3.27 (d, *J* = 14.8 Hz, 5H), 3.05 - 2.97 (m, 3H), 2.83 (d, J = 11.0 Hz, 1H), 2.67 (s, 1H), 2.42 (s, 2H), 2.12 (s, 1H), 1.21 (dd, *J* = 10.8, 4.6 Hz, 3H). | 764 |
| **P50 ^{#}** | 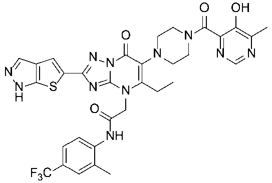 | ¹ H NMR (300 MHz, DMSO- *d* ₆) *δ* 13.58 (s, 1H), 10.10 (s, 1H), 8.55 (s, 1H), 8.15 (s, 1H), 7.79 - 7.50 (m, 4H), 5.30 (s, 2H), 4.54 (d, *J* = 12.3 Hz, 1H), 3.52 (q, *J* = 10.2, 9.7 Hz, 4H), 3.03 (q, *J* = 7.3 Hz, 3H), 2.84 (d, *J* = 10.9 Hz, 1H), 2.72 - 2.61 (m, 1H), 2.42 (d, *J* = 15.1 Hz, 6H), 1.22 (t, *J* = 7.3 Hz,3H). | 722 |
| **P51 ^{#}** | 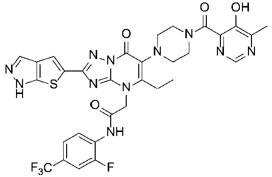 | ¹ H NMR (400 MHz, DMSO- *d* ₆) *δ* 13.58 (s, 1H), 10.77 (s, 1H), 8.43 (d, *J* = 23.6 Hz, 1H), 8.23 (t, *J* = 8.1 Hz, 1H), 8.12 (s, 1H), 7.85 - 7.68 (m, 2H), 7.56 (d, *J* = 8.7 Hz, 1H), 5.35 (s, 2H), 4.70 (s, 1H), 4.53 (d, *J* = 12.6 Hz, 1H), 3.66 - 3.46 (m, 4H), 3.00 (s, 3H), 2.83 (d, *J* = 11.0 Hz, 1H), 2.67 (p, *J* = 2.0 Hz, 1H), 2.42 (s, 2H), 2.12 (s, 1H), 1.22 (d, *J* = 14.7 Hz, 3H). | 726 |
| **A3** | 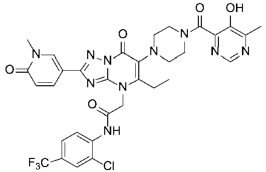 | ¹ H NMR (400 MHz, DMSO- *d* ₆) *δ* 10.39 (s, 1H), 10.22 (s, 1H), 8.58 (s, 1H), 8.47 (s, 1H), 8.07 (d, *J* = 7.6 Hz, 1H), 8.02 (d, *J* = 11.6 Hz, 1H), 7.97 (s, 1H), 7.72 (d, *J* = 8.0 Hz, 1H), 6.53 (d, *J* = 9.6 Hz, 1H), 5.36 (s, 2H), 3.53 (s, 3H), 3.50 - 3.46 (m, 1H), 3.04 - 2.94 (m, 3H), 3.30 - 3.15 (m, 3H), 2.86 - 2.79 (m, 1H), 2.68 - 2.62 (m, 1H), 2.44 (s, 3H), 1.23 (s, 3H). | 727 |

# = After the reaction was completed, the compound was dissolved in 4 M of diluted hydrochloric acid and the mixture was stirred at room temperature for 2 hours. After removing the protecting group, the final product was obtained.

### Example 4:

### Preparation of Target Molecule P22

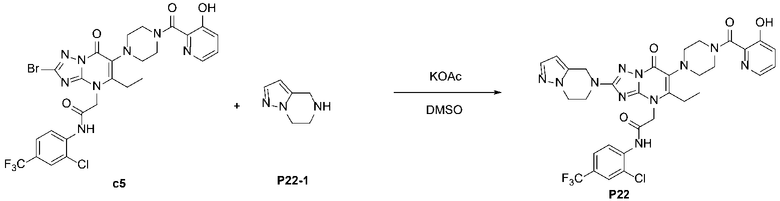

**Steps:** Under nitrogen protection, the intermediate **c5** (50 mg, 0.07 mmol), the raw material **P22-1** (45 mg, 0.37 mmol) and potassium acetate (72 mg, 0.73 mmol) were dissolved in 1 mL of DMSO. The mixture was heated to 120°C for 12 hours, and the reaction was quenched. 5 mL of water was added to the reaction solution. The mixture was extracted with ethyl acetate, and dried over anhydrous sodium sulfate. The filtrate was concentrated, and the crude product was separated by HPLC chromatography (chromatographic column: Column: XBridge Prep Shield RP18 OBD Column, 30*150 mm, 5µm; mobile phase A: water (10 mmol/L NH₄HCO₃), mobile phase B: acetonitrile; flow rate: 60 mL/min, retention time: 10.9 min) to afford **P22** (13.2 mg) as white solid (yield: 24%). LCMS ESI-MS m/z: 726 [M+H]⁺.

¹ H NMR (300 MHz, DMSO- *d* ₆) *δ* 10.21 (s, 2H), 8.11 - 7.92 (m, 3H), 7.72 (dd, *J* = 8.7, 2.2 Hz, 1H), 7.42 (d, *J* = 1.9 Hz, 1H), 7.29 (d, *J* = 3.0 Hz, 2H), 6.16 (d, *J* = 1.9 Hz, 1H), 5.24 (s, 2H), 4.72 (s, 2H), 4.54 (d, *J* = 12.5 Hz, 1H), 4.17 (t, *J* = 5.4 Hz, 2H), 3.98 (t, *J* = 5.4 Hz, 2H), 3.57 - 3.41 (m, 3H), 3.23 (d, *J* = 12.3 Hz, 1H), 2.96 (d, *J* = 11.0 Hz, 3H), 2.82 - 2.71 (m, 1H), 2.59 (d, *J* = 11.2 Hz, 1H), 1.16 (t, *J* = 7.3 Hz, 3H).

### Example 5:

### Preparation of Target Molecules P23-P32, P56-P62

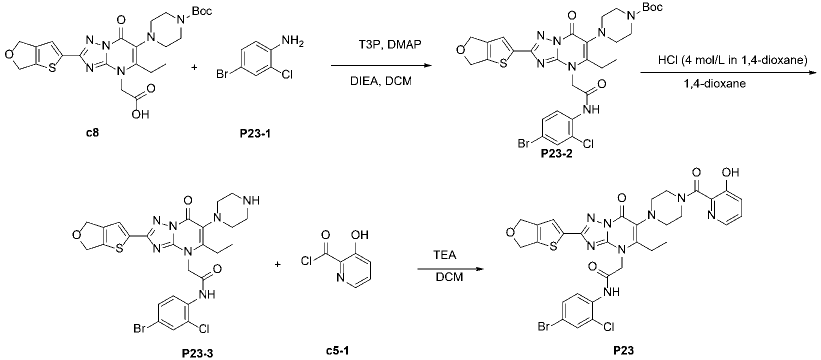

**Step 1:** Under nitrogen protection, the intermediate **c8** (110 mg, 0.21 mmol), the raw material 4-bromo-2-aniline **P23-1** (86 mg, 0.42 mmol) and DMAP (76 mg, 0.63 mmol) were dissolved in 3 mL of dichloromethane. DIEA (161 mg, 1.3 mmol) and T₃P (528 mg, 0.84 mmol) were added, and the mixture was reacted at room temperature for 1 hour. The reaction was quenched. 15 mL of water was added to the reaction solution, and the mixture was extracted with dichloromethane, and the extract was dried over anhydrous sodium sulfate. The filtrate was concentrated, and the crude product was separated by flash reverse column chromatography (chromatographic column: C18; CH₃CN/H₂O, 1/1) to afford **P23-2** (80 mg) as brown solid (yield:54%). LCMS ESI-MS m/z: 718 [M+H]⁺.

**Step 2:** The intermediate **P23-2** from the previous step (80 mg, 0.11 mmol) was dissolved in 2 mL of 1,4-dioxane, and 0.5 mL of 4 M dilute hydrochloric acid was added. The mixture was reacted at room temperature for 1 hour, and the reaction was quenched. 1.5 mL of water was added to the reaction solution, and the pH was adjusted to about pH 9 with saturated sodium bicarbonate. The mixture was extracted with dichloromethane, dried over anhydrous sodium sulfate, and concentrated to afford **P23-3** (50 mg) as brown solid (yield:73%). LCMS ESI-MS m/z: 618 [M+H]⁺.

**Step 3:** In an ice bath and under nitrogen protection, the intermediate **P23-3** (50 mg, 0.08 mmol), the raw material **c5-1** (38 mg, 0.24 mmol) and TEA (41 mg, 0.40 mmol) were dissolved in 1 mL of dichloromethane. The mixture was reacted for 1 hour under ice bath and the reaction was quenched. 5 mL of water was added to the reaction solution. The mixture was extracted with dichloromethane, and the extract was dried over anhydrous sodium sulfate. The filtrate was concentrated, and the crude product was separated by HPLC (chromatographic column: Xselect CSH Prep C18 OBD Colum, 19*250 nm, 5 µm; mobile phase A: water (0.1% TFA), mobile phase B: acetonitrile; flow rate: 25 mL/min; retention time: 11.5 min) to afford **P23** (10.3 mg) as white solid (yield: 17%). LCMS ESI-MS m/z: 739 [M+H]⁺.

¹ H NMR (400 MHz, DMSO- *d* ₆) *δ* 10.23 (s, 2H), 8.06 (dd, *J* = 3.8, 2.2 Hz, 1H), 7.81 (d, *J* = 2.3 Hz, 1H), 7.67 (d, *J* = 8.7 Hz, 1H), 7.58 - 7.49 (m, 2H), 7.35 - 7.23 (m, 2H), 5.27 (s, 2H), 5.02 (q, *J* = 6.5, 4.9 Hz, 2H), 4.89 (t, *J* = 3.5 Hz, 2H), 4.55 (d, *J* = 12.3 Hz, 1H), 3.54 - 3.41 (m, 2H), 3.39 (s, 1H), 3.21 (d, *J* = 11.8 Hz, 1H), 3.06 - 2.91 (m, 3H), 2.81 (d, *J* = 11.2 Hz, 1H), 2.63 (d, *J* = 10.9 Hz, 1H), 1.25 - 1.16 (m, 3H).

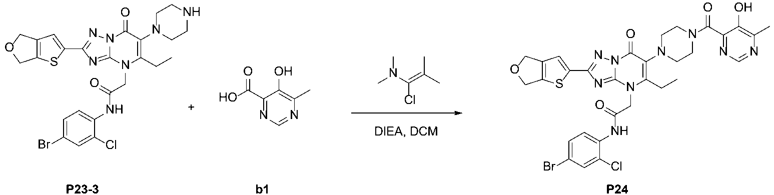

**Step:** Under nitrogen protection, the intermediate **b1** (37 mg, 0.24 mmol) and 1-chloro-N,N,2-trimethylpropyl-1-enyl-1-amine (65 mg, 0.48 mmol) were dissolved in 2 mL of dichloromethane and the mixture was stirred at room temperature for 1 hour. DIEA (104 mg, 0.8 mmol) and the intermediate **P23-3** (100 mg, 0.16 mmol) were added to the reaction solution. The mixture was reacted at room temperature for 1 hour, and the reaction was quenched. 15 mL of water was added to the reaction solution. The mixture was extracted with dichloromethane, and the extract was dried over anhydrous sodium sulfate. The filtrate was concentrated, and the crude product was separated by flash reverse column chromatography (chromatographic column: C18; CH₃CN/H₂O, 5/3) to afford **P24** (29 mg) as white solid (yield: 23%). LCMS ESI-MS m/z: 754 [M+H]⁺.

Referring to the synthetic route of compound **P23** or **P24,** similar raw materials/intermediates (e.g., intermediates **d8, d15, d18-d24,** etc.) were used to synthesize the following target molecules:

| **Targ et mole cules** | **Molecular structure** | **¹ H NMR/ ¹⁹ F NMR** | **LC-MS ESI-MS m/z:[M +H]⁺** |
|---|---|---|---|
| **P24** | 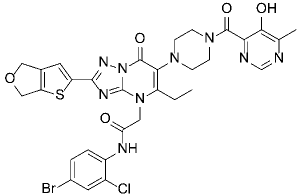 | ¹ H NMR (400 MHz, DMSO- *d* ₆) *δ* 10.22 (s, 1H), 8.55 (s, 1H), 7.81 (d, *J* = 2.2 Hz, 1H), 7.67 (d, *J* = 8.7 Hz, 1H), 7.58 - 7.51 (m, 2H), 5.27 (s, 2H), 5.02 (d, *J* = 3.4 Hz, 2H), 4.89 (d, *J* = 3.3 Hz, 2H), 4.53 (d, *J* = 12.5 Hz, 1H), 3.49 (t, *J* = 11.5 Hz, 3H), 3.25 (s, 1H), 3.00 (q, *J* = 10.2, 8.8 Hz, 3H), 2.82 (d, *J* = 11.2 Hz, 1H), 2.65 (d, *J* = 11.6 Hz, 1H), 2.44 (s, 3H), 1.19 (t, *J* = 7.4 Hz, 3H). | 754 |
| **P25** | 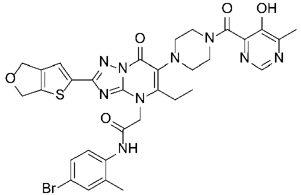 | ¹ H NMR (400 MHz, DMSO- *d* ₆) *δ* 9.92 (s, 1H), 8.50 (s, 1H), 7.56 (s, 1H), 7.46 (s, 1H), 7.35 (s, 2H), 5.21 (s, 2H), 5.02 (d, *J* = 3.6 Hz, 2H), 4.89 (d, *J* = 3.6 Hz, 2H), 4.52 (d, *J* = 11.4 Hz, 1H), 3.50 (d, *J* = 11.2 Hz, 3H), 3.25 (s, 1H), 2.99 (dd, *J* = 20.6, 10.7 Hz, 3H), 2.82 (d, *J* = 11.2 Hz, 1H), 2.64 (d, *J* = 10.8 Hz, 1H), 2.43 (s, 3H), 2.25 (s, 3H), 1.20 (t, *J* = 7.4 Hz, 3H). | 734 |
| **P26** | 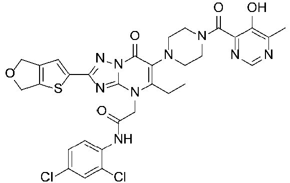 | ¹ H NMR (300 MHz, DMSO- *d* ₆) *δ* 10.25 (s, 1H), 8.55 (s, 1H), 7.77 - 7.69 (m, 2H), 7.56 (s, 1H), 7.42 (dd, *J* = 8.8, 2.5 Hz, 1H), 5.27 (s, 2H), 5.02 (d, *J* = 3.3 Hz, 2H), 4.89 (t, *J* = 3.4 Hz, 2H), 4.59 - 4.48 (m, 1H), 3.61 - 3.43 (m, 4H), 3.26 (s, 1H), 3.00 (d, *J* = 7.8 Hz, 3H), 2.82 (d, *J* = 10.8 Hz, 1H), 2.65 (d, *J* = 10.6 Hz, 1H), 2.44 (s, 3H), 1.21 (q, *J* = 7.6, 5.6 Hz, 3H). | 710 |
| **P27** | 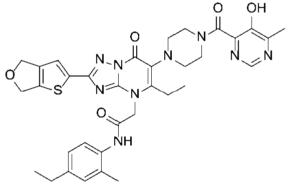 | ¹ H NMR (300 MHz, DMSO- *d* ₆) *δ* 9.80 (s, 1H), 8.45 (s, 1H), 7.57 (s, 1H), 7.22 (d, *J* = 8.1 Hz, 1H), 7.06 (d, *J* = 2.0 Hz, 1H), 6.99 (dd, *J* = 8.1, 2.0 Hz, 1H), 5.19 (s, 2H), 5.03 (d, *J* = 3.4 Hz, 2H), 4.90 (t, *J* = 3.4 Hz, 2H), 4.53 (d, *J* = 12.5 Hz, 1H), 3.50 (d, *J* = 10.7 Hz, 6H), 3.02 (d, *J* = 7.2 Hz, 2H), 2.95 (s, 1H), 2.82 (d, *J* = 11.0 Hz, 1H), 2.64 (d, *J* = 10.3 Hz, 1H), 2.56 (d, *J* = 7.6 Hz, 1H), 2.41 (s, 3H), 2.21 (s, 3H), 1.21 (t, *J* = 7.3 Hz, 3H), 1.14 (t, *J* = 7.6 Hz, 3H). | 684 |
| **P28** | 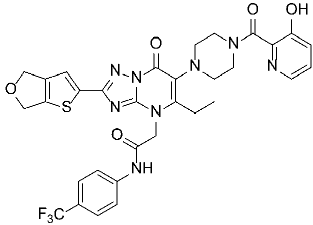 | ¹ H NMR (400 MHz, DMSO- *d* ₆) *δ* 10.93 (d, *J* = 6.7 Hz, 1H), 10.43 (s, 1H), 8.07 (dd, *J* = 3.8, 2.1 Hz, 1H), 7.79 (d, *J* = 8.5 Hz, 2H), 7.71 (d, *J* = 8.6 Hz, 2H), 7.53 (s, 1H), 7.33 - 7.27 (m, 2H), 5.23 (s, 2H), 5.01 (t, *J* = 3.4 Hz, 2H), 4.87 (t, *J* = 3.3 Hz, 2H), 4.56 (d, *J* = 12.5 Hz, 1H), 3.48 (dt, *J* = 17.2, 7.9 Hz, 3H), 3.23 (s, 1H), 2.98 (dt, *J* = 13.5, 8.6 Hz, 3H), 2.82 (d, *J* = 11.2 Hz, 1H), 2.64 (d, *J* = 10.8 Hz, 1H), 1.18 (t, *J* = 7.4 Hz, 3H). | 695 |
| **P29** | 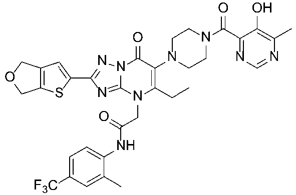 | ¹ H NMR (400 MHz, DMSO- *d* ₆) *δ* 10.07 (s, 1H), 8.55 (d, *J* = 6.7 Hz, 1H), 7.82 - 7.68 (m, 1H), 7.63 (s, 1H), 7.56 (s, 1H), 7.53 (d, *J* = 8.6 Hz, 1H), 5.25 (d, *J* = 16.7 Hz, 2H), 5.02 (d, *J* = 3.7 Hz, 2H), 4.89 (t, *J* = 3.5 Hz, 2H), 4.53 (d, *J* = 12.6 Hz, 1H), 3.51 (d, *J* = 11.7 Hz, 3H), 3.26 (s, 1H), 3.08 - 2.96 (m, 3H), 2.83 (d, *J* = 11.2 Hz, 1H), 2.66 (d, *J* = 11.5 Hz, 1H), 2.44 (s, 3H), 2.37 (s, 3H), 1.21 (t, *J* = 8.7 Hz, 3H). | 724 |
| **P30** | 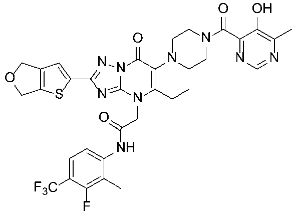 | ¹ H NMR (300 MHz, DMSO- *d* ₆) *δ* 10.29 (s, 1H), 8.53 (s, 1H), 7.57 (t, *J* = 6.4 Hz, 3H), 5.29 (s, 2H), 5.02 (s, 2H), 4.89 (s, 2H), 4.53 (d, *J* = 12.5 Hz, 1H), 3.50 (d, *J* = 9.7 Hz, 3H), 3.26 (s, 1H), 3.03 (s, 3H), 2.83 (d, *J* = 11.1 Hz, 1H), 2.65 (d, *J* = 10.7 Hz, 1H), 2.43 (s, 3H), 2.26 (d, *J* = 2.5 Hz, 3H), 1.21 (d, *J* = 8.1 Hz, 3H). | 742 |
| **P31** | 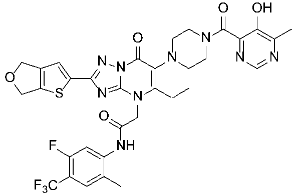 | ¹ H NMR (300 MHz, DMSO- *d* ₆) *δ* 10.12 (s, 1H), 8.55 (s, 1H), 7.72 (dd, *J* = 31.0, 10.5 Hz, 2H), 7.55 (s, 1H), 5.32 (s, 2H), 5.02 (s, 2H), 4.88 (s, 2H), 4.53 (d, *J* = 12.4 Hz, 1H), 3.51 (d, *J* = 10.7 Hz, 3H), 3.26 (s, 1H), 3.00 (s, 3H), 2.83 (d, *J* = 11.3 Hz, 1H), 2.65 (d, *J* = 10.1 Hz, 1H), 2.44 (s, 3H), 2.37 (s, 3H), 1.23 - 1.17 (m, 3H). | 742 |
| **P32** | 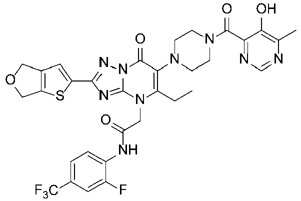 | ¹ H NMR (300 MHz, DMSO-*d* ₆) *δ* 10.57 (s, 1H), 8.56 (s, 1H), 8.23 (t, *J* = 8.1 Hz, 1H), 7.80 (d, *J* = 10.9 Hz, 1H), 7.56 (d, *J* = 11.2 Hz, 2H), 5.33 (s, 2H), 5.01 (s, 2H), 4.88 (s, 2H), 4.53 (d, *J* = 12.4 Hz, 1H), 3.51 (d, *J* = 11.5 Hz, 3H), 3.26 (s, 1H), 2.99 (s, 3H), 2.83 (d, *J* = 11.2 Hz, 1H), 2.66 (d, *J* = 10.8 Hz, 1H), 2.44 (s, 3H), 1.19 (d, *J* = 7.2 Hz, 3H). | 728 |
| **P56** | 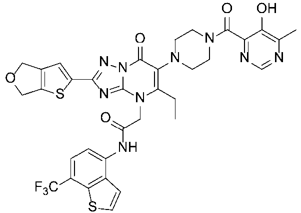 | ¹ H NMR (300 MHz, DMSO- *d* ₆) *δ* 10.78 (s, 1H), 8.51 (s, 1H), 8.08 - 7.95 (m, 3H), 7.78 (d, *J* = 8.4 Hz, 1H), 7.55 (s, 1H), 5.40 (s, 2H), 5.01 (s, 2H), 4.87 (s, 2H), 4.53 (d, *J* = 12.6 Hz, 1H), 3.50 (s, 4H), 3.04 (d, *J* = 8.6 Hz, 3H), 2.91 - 2.78 (m, 2H), 2.67 (d, *J* = 9.6 Hz, 1H), 2.43 (s, 2H), 1.23 (d, *J* = 8.1 Hz, 3H). | 766 |
| **P57** | 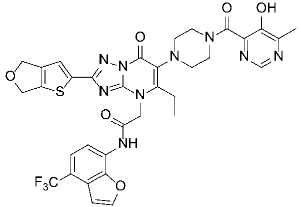 | ¹ H NMR (300 MHz, DMSO- *d* ₆) *δ* 11.12 (s, 1H), 8.54 (s, 1H), 8.36 (d, *J* = 2.2 Hz, 1H), 8.06 (d, *J* = 8.3 Hz, 1H), 7.65 - 7.52 (m, 2H), 7.17 - 7.12 (m, 1H), 5.40 (s, 2H), 5.01 (s, 2H), 4.87 (s, 2H), 4.54 (d, *J* = 12.6 Hz, 1H), 3.52 (d, *J* = 11.3 Hz, 3H), 3.01 (d, *J* = 9.8 Hz, 3H), 2.84 (d, *J* = 11.1 Hz, 1H), 2.75 - 2.56 (m, 2H), 2.44 (s, 2H), 1.20 (t, *J* = 7.4 Hz, 3H). ¹⁹ F NMR (282 MHz, DMSO- *d* ₆) *δ* -59.46. | 750 |
| **P58** | 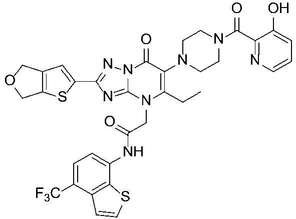 | ¹ H NMR: (300 MHz, DMSO- *d ₆) δ* 10.60 (s, 2H), 8.09 (dd, *J* = 15.2, 4.2 Hz, 2H), 7.80 (q, *J* = 8.1 Hz, 2H), 7.57 (d, *J* = 5.8 Hz, 2H), 7.30 (d, *J* = 3.0 Hz, 2H), 5.36 (s, 2H), 5.02 (s, 2H), 4.88 (s, 2H), 4.57 (d, *J* = 12.6 Hz, 1H), 3.50 (d, *J* = 11.6 Hz, 3H), 3.24 (s, 1H), 3.01 (d, *J* = 27.5 Hz, 3H), 2.83 (d, *J* = 10.8 Hz, 1H), 2.66 (d, *J* = 12.2 Hz, 1H), 1.22 (d, *J* = 8.0 Hz, 3H). | 751 |
| **P59** | 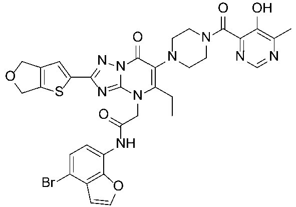 | ¹ H NMR (400 MHz, DMSO- *d ₆) δ* 10.86 (s, 1H), 8.53 (s, 1H), 8.24 (q, *J* = 2.6 Hz, 1H), 7.75 (dt, *J* = 8.7, 2.7 Hz, 1H), 7.55 (d, *J* = 2.5 Hz, 1H), 7.43 (dt, *J* = 8.4, 2.8 Hz, 1H), 6.99 (q, *J* = 2.6 Hz, 1H), 5.34 (s, 2H), 5.01 (s, 2H), 4.87 (s, 2H), 4.71 - 4.51 (m, 1H), 3.51 (d, *J* = 11.7 Hz, 3H), 3.00 (s, 3H), 3.25 (s, 1H), 2.83 (d, *J* = 11.4 Hz, 1H), 2.66 (d, *J* = 11.2 Hz, 1H), 2.49 - 2.37 (m, 3H), 1.21 (q, *J* = 7.8, 7.0 Hz, 3H). | 760 |
| **P60** | 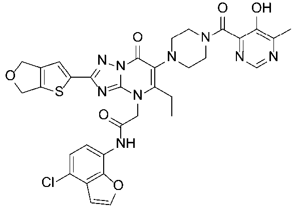 | ¹ H NMR (300 MHz, DMSO- *d* ₆) *δ* 10.86 (s, 1H), 8.51 (s, 1H), 8.23 (d, *J* = 2.3 Hz, 1H), 7.79 (d,*J* = 8.5 Hz, 1H), 7.55 (s, 1H), 7.29 (d, *J* = 8.5 Hz, 1H), 7.07 (d, *J* = 2.2 Hz, 1H), 5.34 (s, 2H), 5.01 (d, *J* = 3.4 Hz, 2H), 4.53 (d, *J* = 12.5 Hz, 1H), 3.51 (d, *J* = 11.0 Hz, 4H), 3.30 (s, 2H), 3.00 (d, *J* = 8.7 Hz, 3H), 2.83 (d, *J* = 11.2 Hz, 1H), 2.66 (d, *J* = 11.0 Hz, 1H), 2.43 (s, 3H), 1.28 - 1.14 (m, 3H). | 716 |
| **P61** | 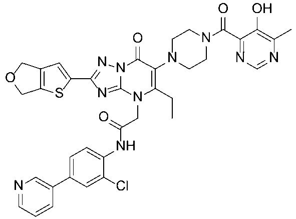 | ¹ H NMR (300 MHz, DMSO- *d* ₆) *δ* 10.27 (s, 1H), 8.92 (d, *J* = 2.3 Hz, 1H), 8.68 - 8.43 (m, 2H), 8.11 (d, *J* = 8.1 Hz, 1H), 8.03 - 7.83 (m, 2H), 7.72 (d, *J* = 8.5 Hz, 1H), 7.64 - 7.42 (m, 2H), 5.32 (s, 2H), 5.02 (s, 2H), 4.99 - 4.81 (m, 2H), 4.53 (d, *J* = 12.5 Hz, 1H), 3.65 (m, 4H), 3.02 (s, 3H), 2.83 (d, *J* = 10.9 Hz, 1H), 2.66 (d, *J =* 10.3 Hz, 1H), 2.43 (s, 3H), 1.29 - 1.16 (m, 3H). | 753 |
| **P62** | 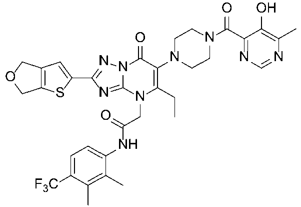 | ¹ H NMR (300 MHz, DMSO- *d* ₆) *δ* 10.22 (s, 1H), 8.47 (s, 1H), 7.63 - 7.47 (m, 2H), 7.38 (d, *J* = 8.5 Hz, 1H), 5.24 (s, 2H), 5.02 (d, *J* = 3.4 Hz, 2H), 4.90 (t, *J* = 3.4 Hz, 2H), 4.61 - 4.48 (m, 1H), 3.49 (d, *J* = 10.9 Hz, 4H), 3.03 (d, *J* = 8.3 Hz, 3H), 2.82 (d, *J* = 11.2 Hz, 1H), 2.64 (d, *J* = 10.7 Hz, 1H), 2.42 (s, 3H), 2.34 (s, 3H), 2.23 (s, 3H), 1.22 (d, *J* = 8.0 Hz, 3H). | 738 |

### Example 6:

### Preparation of Target Molecules P52-P55

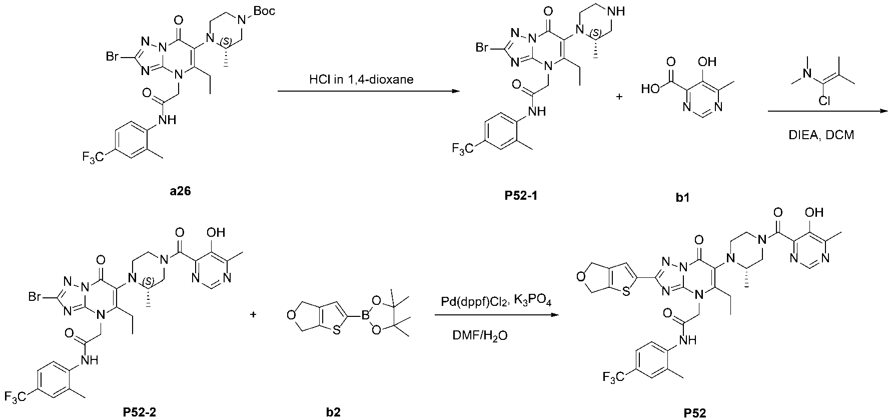

**Step 1: The intermediate a26** (388 mg, 0.59 mmol) was dissolved in 4 mL of 1, 4 - dioxane solution of HCl (4M). The mixture was reacted at room temperature for 1 hour, and then the reaction was quenched. The solvent was evaporated under reduced pressure to afford a brown solid **P52-1** (333 mg). LCMS ESI-MS m/z: 556 [M+H]⁺.

**Step 2:** Under nitrogen protection, the intermediate **b1** (62 mg, 0.40 mmol) and 1-chloro-N,N,2-trimethylpropyl-1-enyl-1-amine (108 mg, 0.81 mmol) were dissolved in 3 mL of dichloromethane. The mixture was stirred at room temperature for 1 hour. DIEA (348 mg, 2.70 mmol) and the intermediate **P52-1** (150 mg, 0.07 mmol) were added to the reaction solution. The mixture was reacted at room temperature for 1 hour, and the reaction was quenched. 15 mL of water was added to the reaction solution. The mixture was extracted with dichloromethane, and the extract was dried over anhydrous sodium sulfate. The filtrate was concentrated, and the crude product was separated by flash reverse column chromatography (chromatographic column: C18; CH₃CN/H₂O, 4/5) to obtain **P52-2** (144 mg) as white solid (yield: 77%). LCMS ESI-MS m/z: 692 [M+H]⁺.

**Step 3:** Under nitrogen protection, the intermediate **P52-2** (144 mg, 0.20 mmol), the intermediate **b2** (61 mg, 0.24 mmol) and K₃PO₄ (441 mg, 2.08 mmol) were dissolved in a mixed solution of 3 mL of DMF and water (v/v, 5/1). Catalyst Pd(dppf)Cl ₂ (91.3 mg, 0.12 mmol) was added. The mixture was warmed to 80°C for 1 hour, and the reaction was quenched. 20 mL of water was added to the reaction solution. The mixture was extracted with dichloromethane, and the extract was dried over anhydrous sodium sulfate. The filtrate was concentrated, and the crude product was separated by HPLC chromatography (chromatographic column: YMC-Actus Triart C18 ExRS30*150 mm, 5µm; mobile phase A: water (10 mmol/L NH₄HCO₃ +0.05% NH₃.H₂O), mobile phase B: acetonitrile; flow rate: 60 mL/min; retention time: 7.0 min;) to afford **P52** (31 mg) as white solid (yield: 20 %). LCMS ESI-MS m/z: 738 [M+H]⁺.

¹ H NMR (300 MHz, DMSO- d ₆) *δ* 10.07 (s, 1H), 8.55 (s, 1H), 7.70 (d, *J* = 8.4 Hz, 1H), 7.65 - 7.48 (m, 3H), 5.28 (q, *J* = 17.6 Hz, 2H), 5.02 (d, *J* = 3.6 Hz, 2H), 4.90 (d, *J* = 3.9 Hz, 2H), 4.52 (s, 1H), 3.64 (s, 2H), 3.48 (s, 3H), 2.98 - 2.79 (m, 3H), 2.44 (d, *J* = 3.5 Hz, 3H), 2.37 (s, 3H), 1.31 - 1.13 (m, 3H), 0.82 (dd, *J* = 54.2, 6.2 Hz, 3H).

Referring to the synthetic route of compound **P52,** similar raw materials/intermediates (e.g., intermediates **a25, a27-a28,** etc.) were used to synthesize the following target molecules:

| **Target molecules** | **Molecular structure** | **¹ H NMR** | **LC-MS ESI-MS m/z:[M+H]⁺** |
|---|---|---|---|
| **P53** | 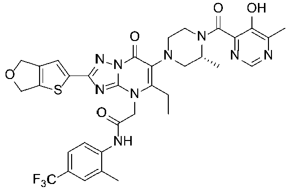 | ¹ H NMR (300 MHz, DMSO- *d* ₆) *δ* 10.07 (s, 1H), 8.53 (d, *J* = 5.8 Hz, 1H), 7.72 (d, *J* = 8.4 Hz, 1H), 7.67 - 7.60 (m, 1H), 7.56 (s, 2H), 5.28 (s, 2H), 5.03 (s, 2H), 4.89 (s, 2H), 4.85 - 4.31 (m, 1H), 3.71 (d, *J* = 9.6 Hz, 2H), 3.54 (t, *J* = 13.1 Hz, 2H), 3.20 (s, 1H), 2.99 - 2.90 (m, 1H), 2.83 (d, *J* = 10.9 Hz, 1H), 2.68 (s,2H), 2.44 (d, *J* = 3.2 Hz, 3H), 2.37 (s, 3H), 1.42 (t, *J* = 7.6 Hz, 3H), 1.28 - 1.17 (m, 3H). | 738 |
| **P54** | 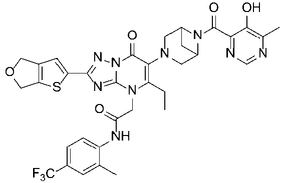 | ¹ H NMR (300 MHz, DMSO- *d ₆) δ* 12.81 (s, 1H), 10.07 (d, *J* = 13.7 Hz, 1H), 8.62 (d, *J* = 16.6 Hz, 1H), 7.63 (s, 2H), 7.53 (d, *J* = 9.5 Hz, 2H), 5.43 - 5.18 (s, 3H), 5.02 (s, 2H), 4.88 (s, 2H), 4.64 (d, *J* = 17.3 Hz, 1H), 3.86 (dd, *J* = 20.6, 10.0 Hz, 1H), 3.34 - 3.50 (s, 2H), 3.23 (d, *J* = 9.8 Hz, 2H), 2.98 (s, 2H), 2.78 (d, *J* = 6.5 Hz, 2H), 2.48 (s, 4H), 2.27 (m, 1H), 1.25 (t, *J* = 7.3 Hz, 3H). | 736 |
| **P55** | 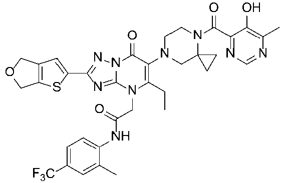 | ¹ H NMR: (300 MHz, DMSO- *d ₆) δ* 10.09 (s, 1H), 8.50 (d, *J* = 14.4 Hz, 1H), 7.72 (d, *J* = 8.4 Hz, 1H), 7.63 (s, 1H), 7.58 - 7.49 (m, 2H), 5.28 (s, 2H), 5.03 (s, 2H), 4.90 (s, 2H), 4.54 (d, *J* = 12.2 Hz, 1H), 4.30 (d, *J* = 11.3 Hz, 1H), 3.66 (dt, *J* = 25.4, 12.2 Hz, 2H), 3.08 (s, 2H), 2.83 (d, *J* = 10.8 Hz, 1H), 2.43 (s, 3H), 2.38 (s, 3H), 1.23 (d, *J* = 5.6 Hz, 3H), 0.85 (d, *J* = 6.8 Hz, 1H), 0.69 (d, *J* = 9.2 Hz, 2H), 0.54 (s, 1H). | 750 |

### Example 7:

### Preparation of Target Molecules P63-P64

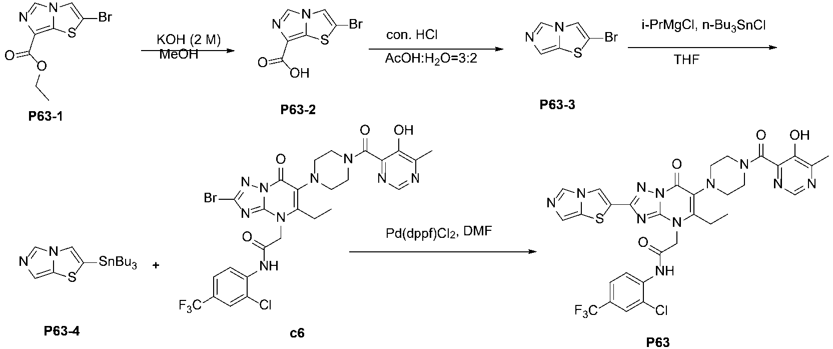

**Step 1:** Raw material **P63-1** (2.4 g, 8.72 mmol) was dissolved in 24 mL of methanol. The mixture was stirred for 5 minutes, and then KOH (96 mL, 2M) aqueous solution was added. The mixture was reacted at room temperature for 12 hours, and the reaction was quenched. The solvent was evaporated under reduced pressure, and the reaction solution was adjusted to about pH 5 by adding diluted hydrochloric acid. A solid was precipitated, which was washed with water and dried to afford **P63-2** (1.4 g) as white solid (yield:65 %). LCMS ESI-MS m/z: 247 [M+H]⁺.

**Step 2:** Compound **P63-2** (1.4 g, 5.66 mmol) was dissolved in a mixed solution of 2.5 mL of acetic acid and water (v/v, 3/2). The mixture was stirred for 5 minutes, and then concentrated hydrochloric acid (1.0 mL) was added. The mixture was warmed to 105°C for 12 hours and the reaction was quenched. The solvent was evaporated under reduced pressure, and the reaction solution was adjusted to about pH 8 by adding a aqueous saturated sodium bicarbonate. The reaction solution was extracted with ethyl acetate, dried over anhydrous sodium sulfate, and concentrated. The crude product was separated by flash reverse column chromatography (chromatographic column: C18; CH₃CN/H₂O = 10/7) to afford **P63-3** (0.6 g) as white solid (yield:52 %). LCMS ESI-MS m/z: 203 [M+H]⁺.

**Step 3:** In an ice bath and under nitrogen protection, compound **P63-3** (420 mg, 2.06 mmol) was dissolved in 13 mL of anhydrous tetrahydrofuran, and a tetrahydrofuran solution of iPrMgCl (255 mg, 2.48 mmol, 1.0 mL) was added dropwise. After the addition, the mixture was stirred for 1 hour under then ice bath. Tributyltin chloride Bu₃SnCl (1.3 g, 4.13 mmol) was added to the reaction solution, and the mixture was continued to react for 1 hour, and the reaction was quenched. 30 mL of saturated aqueous ammonium chloride solution was added to the reaction solution. The mixture was extracted with dichloromethane, dried over anhydrous sodium sulfate, and concentrated to obtain **P63-4** (116 mg) as yellow solid (yield:14%). LCMS ESI-MS m/z: 415 [M+H]⁺.

**Step 4:** Under nitrogen protection, compound **P63-4** (55 mg, 0.13 mmol) and the intermediate **c6** (93 mg, 0.13 mmol) were dissolved in 3 mL of DMF. Catalyst Pd(dppf)Cl₂ (19.4 mg, 0.027 mmol) was added. The mixture was warmed to 100°C for 2 hours and the reaction was quenched. 20 mL of water was added to the reaction solution, and the mixture was extracted with dichloromethane, dried over anhydrous sodium sulfate, and concentrated. The crude product was separated by HPLC chromatography (chromatographic column: Xselect CSH Prep C18 OBD Colum, 19*250nm, 5µm; mobile phase A: water (10 mmol/L NH₄HCO₃), mobile phase B: acetonitrile; flow rate: 25 mL/min; retention time: 10.2 min) to afford **P63** (4.0 mg) as white solid (yield:4%). LCMS ESI-MS m/z: 742 [M+H]⁺.

¹ H NMR (400 MHz, DMSO- *d ₆) δ* 10.41 (s, 1H), 8.57 (d, *J* = 32.2 Hz, 2H), 8.25 (s, 1H), 8.07 (d, *J* = 8.6 Hz, 1H), 7.97 (d, *J* = 2.1 Hz, 1H), 7.76 - 7.69 (m, 1H), 7.11 (s, 1H), 5.36 (s, 2H), 4.53 (d, *J* = 12.6 Hz, 2H), 3.50 (d, *J* = 10.7 Hz, 4H), 3.02 (s, 3H), 2.83 (d, *J* = 11.2 Hz, 1H), 2.66 (d, *J* = 11.2 Hz, 1H), 2.43 (s, 3H), 1.21 (dd, *J* = 12.7, 5.9 Hz, 3H).

Referring to the synthetic route of compound **P63,** similar raw materials/intermediates (e.g., intermediate **c16,** etc.) were used to synthesize the following target molecules:

| **Target Molecules** | **MOLECULAR STRUCTURE** | **¹H NMR** | **LC-MS ESI-MS m/z:[M+H]⁺** |
|---|---|---|---|
| **P64** | 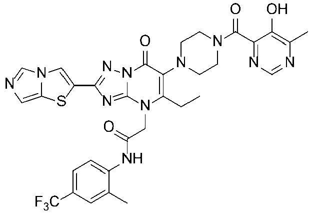 | ¹ H NMR (300 MHz, DMSO- *d* ₆) *δ* 10.07 (s, 1H), 8.58 (d, *J* = 16.9 Hz, 2H), 8.24 (d, *J* = 13.6 Hz, 1H), 7.80 - 7.48 (m, 3H), 7.12 (s, 1H), 5.29 (s, 2H), 4.54 (d, *J* = 12.4 Hz, 1H), 3.51 (d, *J* = 11.1 Hz, 4H), 3.04 (d, *J* = 9.0 Hz, 3H), 2.84 (d, *J* = 11.2 Hz, 1H), 2.66 (d, *J* = 11.0 Hz, 1H), 2.44 (s, 3H), 2.38 (s, 3H), 1.21 (t, *J* = 7.3 Hz, 3H). | 722 |

### Example 8:

### Preparation of Target Molecules P65-P66

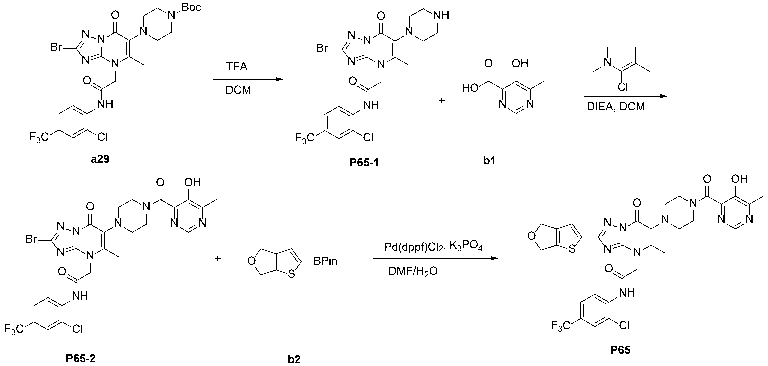

**Step 1:** The intermediate **a29** (350 mg, 0.54 mmol) was dissolved in 4 mL of dichloromethane. 1.7 mL of trifluoroacetic acid was added dropwise. The mixture was reacted at room temperature for 1 hour, and the reaction was quenched. The solvent was evaporated under reduced pressure to afford a yellow solid **P65-1** (290 mg). LCMS ESI-MS m/z: 548 [M+H]⁺.

**Step 2:** Under nitrogen protection, the intermediate 5-hydroxy-6-methyl-pyrimidine-4-carboxylic acid **b1** (122 mg, 0.79 mmol) and 1-chloro-N,N,2-trimethylpropyl-1-enyl-1-amine (212 mg, 1.58 mmol) were dissolved in 3 mL of dichloromethane and the mixture was stirred at room temperature for 1 hour. DIEA (683 mg, 5.28 mmol) and the compound **P65-1** (290 mg, 0.53 mmol) were added to the reaction solution. The mixture was reacted at room temperature for 1 hour, and the reaction was quenched. 15 mL of water was added to the reaction solution. The mixture was extracted with dichloromethane, and the extract was dried over anhydrous sodium sulfate. The filtrate was concentrated, and the crude product was separated by flash reverse column chromatography (chromatographic column: C18; CH₃CN/H₂O, 10/7) to afford **P65-2** (239 mg) as white solid (yield: 66%). LCMS ESI-MS m/z: 684 [M+H]⁺.

**Step 3:** Under nitrogen protection, the intermediate **P65-2** (239 mg, 0.35 mmol), the intermediate **b2** (132 mg, 0.52 mmol) and K₃PO₄ (741 mg, 3.49 mmol) were dissolved in a mixed solution of 5 mL of DMF and water (v/v, 4/1). Catalyst Pd(dppf)Cl₂ (153.2 mg, 0.21 mmol) was added. The mixture was warmed to 80°C for 1 hour, and the reaction was quenched. 20 mL of water was added to the reaction solution. The mixture was extracted with dichloromethane, and the extract was dried over anhydrous sodium sulfate. The filtrate was concentrated, and the crude product was separated by HPLC chromatography (chromatographic column: XSelect CSH Prep C18 OBD Column, 30*150 mm, 5 µm; mobile phase A: water (10 mmol/L NH₄HCO₃ +0.05% NH₃.H₂O), mobile phase B: acetonitrile; flow rate: 60 mL/min; retention time: 8.82 min;) to obtain **P65** (15.5 mg) as white solid (yield: 6%). LCMS ESI-MS m/z: 730 [M+H]⁺.

¹ H NMR (300 MHz, DMSO- *d ₆) δ* 8.50 (s, 1H), 8.06 (d, *J* = 8.5 Hz, 1H), 7.97 (s, 1H), 7.76 - 7.66 (m, 1H), 7.56 (s, 1H), 5.37 (s, 2H), 5.02 (s, 2H), 4.89 (s, 2H), 4.51 (d, *J* = 13.2 Hz, 1H), 3.48 (t, *J* = 11.1 Hz, 2H), 3.20-3.30 (s, 2H), 3.00 (s, 1H), 2.81 (d, *J* = 10.6 Hz, 1H), 2.61 (s, 4H), 2.43 (s, 3H).

Referring to the synthetic route of compound **P65,** similar raw materials/intermediates (e.g., intermediate **a30,** etc.) were used to synthesize the following target molecules:

| **Target molecules** | **Molecular structure** | **¹ H NMR** | **LC-MS ESI-MS m/z:[M+H]⁺** |
|---|---|---|---|
| **P66** | 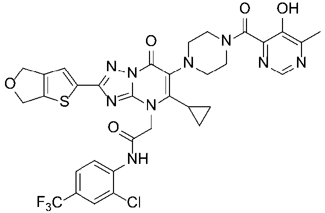 | ¹ H NMR (400 MHz, DMSO- *d* ₆) *δ* 8.46 (s, 1H), 7.96 (d, *J* = 8.2 Hz, 2H), 7.72 (d, *J* = 8.7 Hz, 1H), 7.62 (s, 1H), 5.21 (s, 2H), 5.06 (d, *J* = 3.6 Hz, 2H), 4.91 (d, *J* = 3.6 Hz, 2H), 4.50 (s, 1H), 3.51 (s, 4H), 2.94 - 2.65 (m, 4H), 2.41 (s, 3H), 1.29 - 1.20 (m, 1H), 0.99 (d, *J* = 7.4 Hz, 4H). | 756 |

### Example 9:

### Preparation of Target Molecules P67-P68, A2

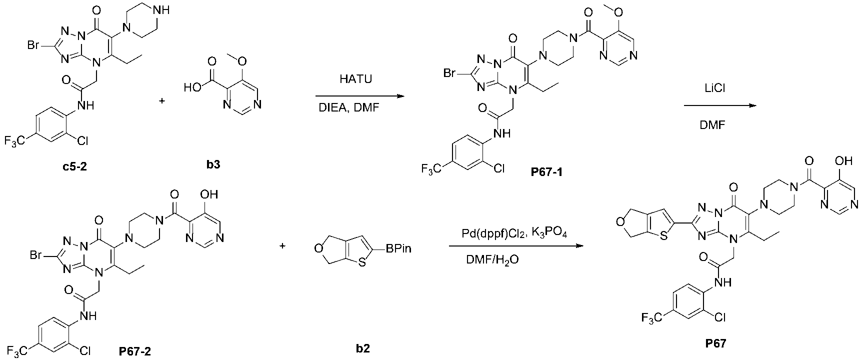

**Step 1:** Under nitrogen protection, the intermediate 5-methoxy-pyrimidine-4-carboxylic acid **b3** (123 mg, 0.80 mmol) and the intermediate **c5-2** (300 mg, 0.53 mmol) were dissolved in 6 mL of DMF and the mixture was stirred at room temperature for 5 minutes. DIEA (344 mg, 2.66 mmol) and HATU (304 mg, 0.80 mmol) were added to the reaction solution. The mixture was reacted at room temperature for 2 hours, and the reaction was quenched. 20 mL of water was added to the reaction solution. The mixture was extracted with dichloromethane, and the extract was dried over anhydrous sodium sulfate. The filtrate was concentrated, and the crude product was separated by flash reverse column chromatography (chromatographic column: C18; CH₃CN/H₂O, 10/7) to afford **P67-1** (200 mg) as yellow solid (yield: 54%). LCMS ESI-MS m/z: 698 [M+H]⁺.

**Step 2:** Under nitrogen protection, compound **P67-1** (180 mg, 0.25 mmol) was dissolved in 2 mL of DMF and LiCl was added (44 mg, 1.03 mmol). The mixture was heated to 150°C for 4 hours, and then the reaction was quenched. 20 mL of water was added to the reaction solution. The mixture was extracted with dichloromethane, and the extract was dried over anhydrous sodium sulfate. The filtrate was concentrated, and the crude product was separated by flash reverse column chromatography (chromatographic column: C18; CH₃CN/H₂O, 3/5) to afford **P67-2** (140 mg) as white solid (yield: 79%). LCMS ESI-MS m/z: 684 [M+H]⁺.

**Step 3:** Under nitrogen protection, compound **P67-2** (50 mg, 0.073 mmol), the intermediate **b2** (100 mg, 0.39 mmol) and K₃PO₄ (155 mg, 0.73 mmol) were dissolved in a mixed solution of 2 mL of DMF and water (v/v, 4/1), and the catalyst Pd(dppf)Cl₂ (32 mg, 0.044 mmol) was added. The mixture was warmed to 80°C for 1 hour, and the reaction was quenched. 10 mL of water was added to the reaction solution. The mixture was extracted with ethyl acetate, and dried over anhydrous sodium sulfate. The filtrate was concentrated, and the crude product was separated by HPLC chromatography (chromatographic column: XBridge Prep C18 OBD Colum, 30*150 nm, 5 µm; mobile phase A: water (10 mmol/L NH₄HCO₃), mobile phase B: acetonitrile; flow rate: 60 mL/min; retention time: 11.0 min;) to afford **P67** (4.5 mg) as white solid (yield: 8%). LCMS ESI-MS m/z: 730 [M+H]⁺.

¹ H NMR (300 MHz, DMSO- *d* ₆) *δ* 8.59 (s, 1H), 8.36 (s, 1H), 8.08 - 7.92 (m, 2H), 7.78 - 7.68 (m, 1H), 7.55 (s, 1H), 5.34 (s, 2H), 5.09 - 4.84 (m, 4H), 4.52 (d, *J* = 12.4 Hz, 1H), 3.49 (d, *J* = 9.7 Hz, 4H), 3.00 (d, *J* = 9.7 Hz, 3H), 2.82 (d, *J* = 11.1 Hz, 1H), 2.66 (d, *J* = 10.9 Hz, 1H), 1.25 - 1.16 (m, 3H).

Referring to the synthetic route of compound **P67,** the following target molecules were synthesized using similar raw materials/intermediates (e.g., intermediate **b4,** etc.):

| **Targ et mole cules** | **Molecular structure** | **¹ H NMR** | **LC-MS ESI-MS m/z:[M +H]⁺** |
|---|---|---|---|
| **P68** | 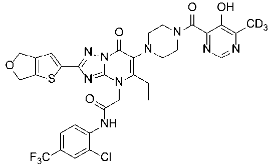 | ¹ H NMR (400 MHz, DMSO- *d* ₆) *δ* 10.40 (s, 1H), 8.51 (s, 1H), 8.06 (d, *J* = 8.5 Hz, 1H), 7.97 (d, *J* = 2.1 Hz, 1H), 7.72 (dd, *J* = 8.7, 2.2 Hz, 1H), 7.56 (s, 1H), 5.35 (s, 2H), 5.02 (t, *J* = 3.4 Hz, 2H), 4.89 (t, *J* = 3.3 Hz, 2H), 4.53 (d, *J* = 12.6 Hz, 1H), 3.50 (d, *J* = 10.3 Hz, 4H), 3.01 (d, *J* = 8.7 Hz, 3H), 2.83 (d, *J* = 11.2 Hz, 1H), 2.65 (d, *J* = 9.4 Hz, 1H), 2.42 (d, *J* = 8.6 Hz, 1H), 1.30 - 1.16 (m, 4H), 0.85 (d, *J* = 6.9 Hz, 1H). | 747 |
| **A2** | 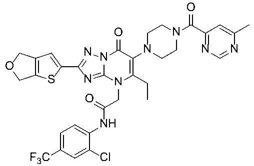 | ¹ H NMR (300 MHz, DMSO- *d* ₆) *δ* 9.11 (d, *J* = 1.3 Hz, 1H), 8.06 (d, *J* = 8.5 Hz, 1H), 7.97 (d, *J* = 2.1 Hz, 1H), 7.72 (dd, *J* = 8.9, 2.2 Hz, 1H), 7.66 - 7.52 (m, 1H), 5.36 (s, 1H), 5.02 (d, *J* = 3.4 Hz, 1H), 4.89 (t, *J* = 3.4 Hz, 1H), 4.53 (d, *J* = 12.4 Hz, 1H), 3.64 (d, *J* = 12.5 Hz, 1H), 3.49 (dt, *J* = 11.9, 8.1 Hz, 2H), 3.12 - 2.93 (m, 2H), 2.85 (d, *J* = 11.2 Hz, 1H), 2.67 (d, *J* = 11.2 Hz, 1H), 2.55 (s, 2H), 2.51 (d, *J* = 2.1 Hz, 2H), 1.20 (t, *J* = 7.3 Hz, 2H). | 728 |

### Example 10:

### Preparation of Target Molecules P69-P70

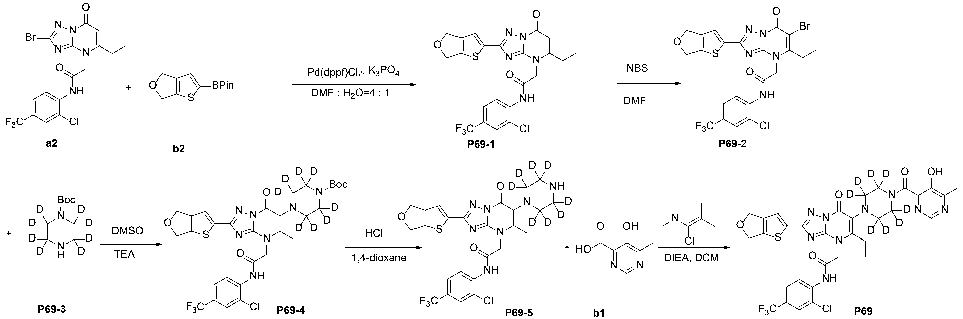

**Step 1:** Under nitrogen protection, the intermediate **a2** (3.0 g, 6.26 mmol), the intermediate **b2** (4.5 g, 17.86 mmol) and K₃PO₄ (13.3 g, 62.6 mmol) were dissolved in a mixed solution of 30 mL of DMF and water (v/v, 6/1), and the catalyst Pd(dppf)Cl ₂ (2.7 g, 3.76 mmol) was added. The mixture was warmed to 80°C for 1 hour. The reaction was quenched. 200 mL of water was added to the reaction solution. The mixture was extracted with ethyl acetate, and dried over anhydrous sodium sulfate. The filtrate was concentrated, and the crude product was washed with tetrahydrofuran to afford **P69-1** (240 mg) as white solid (yield: 7%). LCMS ESI-MS m/z: 524 [M+H]⁺.

**Step 2:** Under nitrogen protection, compound **P69-1** (240 mg, 0.45 mmol) was dissolved in 5 mL of DMF, and N-bromosuccinimide NBS (163 mg, 0.91 mmol) was added. The mixture was warmed to 60°C for 1 hour. The reaction was quenched. 20 mL of water was added to the reaction solution. The mixture was extracted with dichloromethane, dried over anhydrous sodium sulfate, and concentrated to afford **P69-2** (180 mg) as white solid (yield: 65%). LCMS ESI-MS m/z: 602 [M+H]⁺.

**Step 3:** Under nitrogen protection, Compound **P69-2** (105 mg, 0.17 mmol), TEA (53 mg, 0.52 mmol) and the deuterated raw material **P69-3** (169 mg, 0.87 mmol) were dissolved in 1 mL of DMSO. The mixture was heated to 120°C for 12 hours, and the reaction was quenched. 20 mL of water was added to the reaction solution. The mixture was extracted with dichloromethane, and the extract was dried over anhydrous sodium sulfate. The filtrate was concentrated, and the crude product was separated by flash column chromatography (chromatographic column: C18; CH₃CN/H₂O, 4/5) to afford **P69-3** (20 mg) as white solid (yield: 16%). LCMS ESI-MS m/z: 716 [M+H]⁺.

**Step 4:** Compound **P69-3** (20 mg, 0.03 mmol) was dissolved in 1 mL of hydrogen chloride solution in 1,4- dioxane (2M), reacted at room temperature for 1 hour, and the reaction was quenched. The solvent was evaporated under reduced pressure to afford **P69-4** as white solid (18 mg) (yield: 98%). LCMS ESI-MS m/z: 616 [M+H]⁺.

**Step 5:** Under nitrogen protection, the intermediate 5-hydroxy-6-methyl-pyrimidine-4-carboxylic acid **b1** (8 mg, 0.05 mmol) and 1-chloro-N,N,2-trimethylpropyl-1-enyl-1-amine (13 mg, 0.10 mmol) were dissolved in 1 mL of dichloromethane and the mixture was stirred at room temperature for 1 hour. DIEA (39 mg, 0.30 mmol) and the compound **P69-4** (18 mg, 0.29 mmol) were added to the reaction solution. The mixture was reacted at room temperature for 1 hour, and the reaction was quenched. 15 mL of water was added to the reaction solution. The mixture was extracted with dichloromethane, and the extract was dried over anhydrous sodium sulfate. The filtrate was concentrated, and the crude product was separated by HPLC preparative chromatography (chromatographic column: Xbridge Prep OBD C18 Column, 30 * 150 mm, 5 µm; mobile phase A: water (10 mmol/L NH₄HCO₃ +0.05% NH ₃ H₂O; Mobile phase B: acetonitrile; Flow rate: 60 mL/min; retention time: 7.03 min) to afford **P69** (5.3 mg) as white solid (yield: 22%). LCMS ESI-MS m/z: 752 [M+H]⁺. [Deuteration rate: 99%]

¹ H NMR (300 MHz, DMSO- *d* ₆) *δ* 8.45 (s, 1H), 8.05 (d, *J* = 8.6 Hz, 1H), 7.96 (d, *J* = 2.1 Hz, 1H), 7.71 (dd, *J* = 8.7, 2.2 Hz, 1H), 7.55 (s, 1H), 5.34 (s, 2H), 5.02 (t, *J* = 3.3 Hz, 2H), 4.88 (t, *J* = 3.3 Hz, 2H), 3.01 (s, 2H), 2.41 (s, 3H), 1.23 - 1.14 (m, 3H).

Referring to the synthetic route of compound **P69,** the following target molecules were synthesized using similar raw materials/intermediates (e.g., intermediate **a31,** etc.):

| **Target molecules** | **Molecular structure** | **¹ H NMR** | **LC-MS ESI-MS m/z:[M+H]⁺** |
|---|---|---|---|
| **P70** | 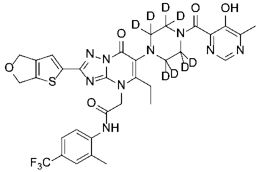 | ¹ H NMR (300 MHz, DMSO- *d* ₆) *δ* 10.08 (s, 1H), 8.53 (s, 1H), 7.80 - 7.44 (m, 4H), 5.27 (s, 2H), 4.96 (dt, *J* = 40.5, 3.5 Hz, 4H), 3.02 (d, *J* = 9.1 Hz, 2H), 2.40 (d, *J* = 19.4 Hz, 6H), 1.21 (t, *J* = 7.8 Hz, 3H). | 732 |

### Example 11:

### Preparation of Target Molecule P71

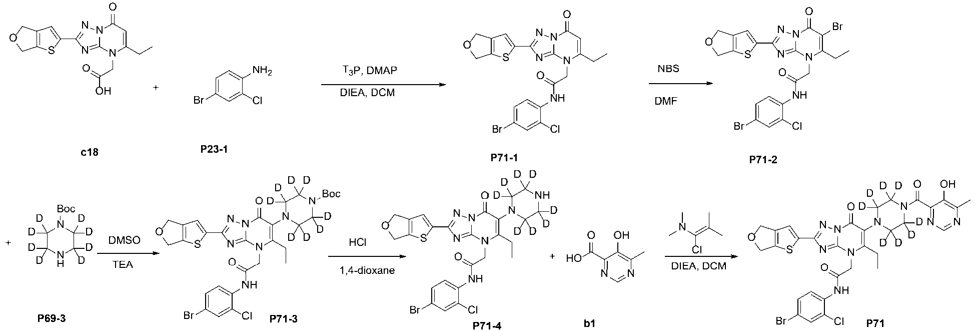

**Step 1:** Under nitrogen protection, the intermediate **c18** (500 mg, 1.44 mmol), the raw material 2-chloro-4-bromo-aniline **P23-1** (450 mg, 2.17 mmol) and DMAP (530 mg, 4.33 mmol) were dissolved in 10 mL of dichloromethane, and DIEA (930 mg, 7.22 mmol) and T₃P (2.3 g, 7.22 mmol) were added. The mixture was reacted at room temperature for 2 hours, and the reaction was quenched. 40 mL of water was added to the reaction solution. The mixture was extracted with dichloromethane, and the extract was dried over anhydrous sodium sulfate. The filtrate was concentrated, and the crude product was separated by flash reverse column chromatography (chromatographic column: C18; CH₃CN/H₂O, 5/3) to afford **P71-1** (280 mg) as color solid (yield: 36%). LCMS ESI-MS m/z: 534 [M+H]⁺.

**Step 2:** Under nitrogen protection, compound **P71-1** (280 mg, 0.52 mmol) was dissolved in 6 mL of DMF, and N-bromosuccinimide NBS (190 mg, 1.05 mmol) was added. The mixture was warmed to 60°C for 1 hour. The reaction was quenched. 20 mL of water was added to the reaction solution. The mixture was extracted with dichloromethane, and the extract was dried over anhydrous sodium sulfate. The filtrate was concentrated, and the crude product was separated by flash reverse column chromatography (chromatographic column: C18; CH₃CN/H₂O, 1/1) to afford **P71-2** (140 mg) as white solid (yield: 44%). LCMS ESI-MS m/z: 612 [M+H]⁺.

**Step 3:** Under nitrogen protection, compound **P71-2** (140 mg, 0.23 mmol), TEA (70 mg, 0.68 mmol) and deuterated raw material **P69-3** (222 mg, 1.14 mmol) were dissolved in 3 mL of DMSO. The mixture was heated to 120°C for 12 hours, and the reaction was quenched. 20 mL of water was added to the reaction solution. The mixture was extracted with dichloromethane, and the extract was dried over anhydrous sodium sulfate. The filtrate was concentrated, and the crude product was separated by flash column chromatography (chromatographic column: C18; CH₃CN/H₂O, 5/3) to afford **P71-3** (50 mg) as yellow solid (yield: 30 %). LCMS ESI-MS m/z: 726 [M+H]⁺.

**Step 4:** Compound **P71-3** (50 mg, 0.07 mmol) was dissolved in 1 mL of 1,4-dioxane solution of hydrogen chloride (2M). The mixture was reacted at room temperature for 1 hour, and then the reaction was quenched. The solvent was evaporated under reduced pressure to afford a crude yellow solid **P71-4** (45 mg). LCMS ESI-MS m/z: 626 [M+H]⁺.

**Step 5:** Under nitrogen protection, the intermediate 5-hydroxy-6-methyl-pyrimidine-4-carboxylic acid **b1** (17 mg, 0.11 mmol) and 1-chloro-N,N,2-trimethylpropyl-1-enyl-1-amine (29 mg, 0.22 mmol) were dissolved in 1 mL of dichloromethane and the mixture was stirred at room temperature for 1 hour. DIEA (93 mg, 0.72 mmol) and the compound **P71-4** (45 mg, 0.07 mmol) were added to the reaction solution. The mixture was reacted at room temperature for 1 hour, and the reaction was quenched. 15 mL of water was added to the reaction solution. The mixture was extracted with dichloromethane, and the extract was dried over anhydrous sodium sulfate. The filtrate was concentrated, and the crude product was separated by HPLC preparative chromatography (chromatographic column: Xbridge Prep OBD C18 Column, 30 * 150 mm, 5 µm; mobile phase A: water (10 mmol/L NH₄HCO₃ +0.05% NH₃ H₂O; Mobile phase B: acetonitrile; Flow rate: 60 mL/min; retention time: 7.67 min) to obtain **P71** (16.5 mg) as white solid (yield: 31%). LCMS ESI-MS m/z: 762 [M+H]⁺. [Deuteration rate: 99%]

¹ H NMR (400 MHz, DMSO-*d₆) δ* 10.32 (s, 1H), 8.53 (s, 1H), 7.92-7.50 (m, 4H), 5.27 (s, 2H), 5.15 (s, 2H), 4.89 (s, 2H), 3.00 (s, 2H), 2.63 (s, 3H), 1.20 (q, *J* = 8.3, 7.8 Hz, 4H).

### Example 12:

The molecules of the present disclosure were tested for their activities on the WRN helicase hydrolysis of double-stranded DNA (Table 1).

A working solution to be tested and a buffer were prepared. The compound to be tested was dissolved in DMSO and subjected to 4x dilution (with 10 µM final concentration as the starting concentration). 0.2 µL of the solution of the compound to be tested was added to the 384-well plate and 10 µL (2X) of WRN enzyme solution was added. The solution was incubated in the dark for 30 minutes. Then, 10 µL of a substrate detection solution containing double-stranded DNA was added to initiate the reaction (the DNA has a length of 19 bp, and labeled with TAMRA and BHQ2 at 3' and 5'- ends, respectively). The solution was incubated at room temperature for 60 minutes. The inhibitory activities (IC ₅₀) against WRN enzyme were calculated based on the change of Ex530/Em590 in the blank group (DMSO) and the compound group. Y = lower platform signal + (upper platform signal - lower platform signal) / (1 + 10^((LogIC50 -X) × Hill slope))
X: log value of compound concentration
Y: Inhibition rate (%)

**Table 1. Effects of Compounds on Inhibiting the Unwinding of WRN Enzyme.**

| **Compounds** | **WRN unwinding /IC ₅₀ /nM** | **Compounds** | **WRN unwinding /IC ₅₀ /nM** |
|---|---|---|---|
| **P1** | 3890 | **P29** | 2.1 |
| **P2** | 2.3 | **P32** | 1.5 |
| **P4** | 1.9 | **P33** | 1.8 |
| **P5** | 1.6 | **P57** | 1.3 |
| **P6** | 3.5 | **P58** | 2.9 |
| **P7** | 1.4 | **P59** | 1.5 |
| **P8** | 3.7 | **P69** | 1.4 |
| **P9** | 1.2 | **P71** | 1.2 |
| **P10** | 2.1 | | |
| **P11** | 1.7 | | |
| **P12** | 9.4 | | |
| **P13** | 2.2 | | |
| **P14** | 4.4 | | |
| **P15** | 1.6 | | |
| **P16** | 2.1 | | |
| **P17** | 2.3 | | |
| **P18** | 1.8 | | |
| **P20** | 1.6 | | |
| **P21** | 3.3 | | |
| **P22** | 2.3 | | |
| **P23** | 0.9 | | |
| **P24** | 1.1 | | |
| **P25** | 1.7 | | |

The above results show that the molecules of the present disclosure have good effect on inhibiting the WRN unwinding DNA, and are expected to achieve a better tumor inhibition effect by inhibiting the WRN helicase activity.

### Example 13:

The molecules of the present disclosure were tested for their anti-proliferative activity on MSI-H tumor cells.

The proliferation of tumor cells with microsatellite instability (MSI-H) was sensitive to WRN inhibitors; and the proliferation of tumor cells with microsatellite stability (MSS) was insensitive to WRN inhibitors. By testing their activities, it show that the molecules of the present disclosure have inhibitory and synthetic lethal effects on WRN at the cellular level.

SW48 colorectal cancer cells with MSI were cultured in a RMI1640 medium containing 10% FBS and 1% penicillin-streptomycin, and the mixture was cultured in a 37°C, 5% CO₂ incubator. 40 µL of cell suspension was added to each well of a 384-well microplate. 40 nL of compounds with different concentrations were added to each well using Echo, and the mixtures were cultured in a 37°C, 5% CO₂ incubator for 5 days. 40 µL of CTG solution (Promega, Cat No. G7573) was added to each well and incubated in a 37°C, 5% CO₂incubator in the dark for 30 minutes. The luminescence value was read using an Envision multi-function microplate reader (Perkin Elmer, catalog number Envision 2104). The optical signal is proportional to the amount of ATP in the system, while the ATP content directly characterizes the number of viable cells in the system.

### IC₅₀ value calculation:

Y = lower platform signal + (upper platform signal-lower platform signal) / (1 + 10^((LogIC50 -X) × Hill slope))
X: log value of compound concentration
Y: Inhibition rate (%)

**Table 2.1: 2D Antiproliferative Effects of Compounds on MSI-H Intestinal Cancer SW48 Cell Line.**

| **Compounds** | **SW 48 /IC₅₀/nM** | **Compounds** | **SW 48 /IC₅₀/nM** | **Compounds** | **SW 48 /IC₅₀ /nM** |
|---|---|---|---|---|---|
| **P1** | N.D. | **P31** | 49 | **P59** | 39 |
| **P2** | 1106 | **P32** | 126 | **P60** | 64 |
| **P4** | 135 | **P33** | 139 | **P61** | 8853 |
| **P5** | 192 | **P34** | 142 | **P62** | 37 |
| **P6** | 2061 | **P35** | 199 | **P63** | 132 |
| **P7** | 39 | **P36** | 561 | **P64** | 530 |
| **P8** | 566 | **P37** | 46 | **P65** | 74 |
| **P9** | 54 | **P38** | 82 | **P66** | N.D. |
| **P10** | 125 | **P39** | >10000 | **P67** | 52 |
| **P11** | 127 | **P40** | 113 | **P68** | 28 |
| **P12** | 450 | **P41** | 2084 | **P69** | 54 |
| **P13** | 72 | **P42** | 620 | **P70** | 34 |
| **P14** | 97 | **P43** | 593 | **P71** | 39 |
| **P15** | 381 | **P44** | >10000 | | |
| **P16** | 120 | **P45** | 54 | | |
| **P17** | 360 | **P46** | 452 | | |
| **P18** | 499 | **P47** | 480 | | |
| **P19** | 82 | **P48** | 32 | | |
| **P20** | 126 | **P49** | 574 | | |
| **P21** | 480 | **P50** | 162 | | |
| **P22** | 538 | **P51** | 242 | | |
| **P23** | 79 | **P52** | 69 | | |
| **P24** | 39 | **P53** | 55 | | |
| **P25** | 39 | **P54** | 2136 | | |
| **P26** | 41 | **P55** | 672 | | |
| **P27** | 46 | **P56** | 36 | | |
| **P28** | 136 | **P57** | 34 | | |
| **P29** | 32 | **P58** | 123 | **A2** | 848 |
| **P30** | 33 | **A1** | 261 | **A3** | 2002 |

| | | | | | |
|---|---|---|---|---|---|
| ND = Not Tested | | | | | |

Referring to WO 2022249060, the following control molecule **A1** was synthesized

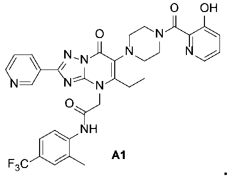

**Table 2.2: Antiproliferative Value and IC₅₀ Calculation of Compound A1 on SW48 Cells**

| Concentration. [nM]/ | Test 1/ Inhibition Rate | Test 2/ Inhibition Rate | Average Value/ Inhibition Rate |
|---|---|---|---|
| 10000 | 100.9954 | 102.0219 | 101.5087 |
| 2500 | 99.8401 | 101.2267 | 100.5334 |
| 625 | 97.8908 | 96.9311 | 97.4109 |
| 156.25 | -2.1399 | 14.9471 | 6.4036 |
| 39.0625 | -12.6411 | -11.1067 | -11.8739 |
| 9.765625 | 0.0311 | -8.0940 | -4.0314 |
| 2.44140625 | -13.8489 | -8.9023 | -11.3756 |
| 0.610351563 | -2.3700 | -2.2651 | -2.3176 |
| 0.152587891 | -13.1741 | -5.6058 | -9.3899 |
| 0.038146973 | -1.9993 | 1.1876 | -0.4058 |

**Table 2.3: Antiproliferative Value and IC₅₀ Calculation of Compound P7 on SW48 Cells**

| Concentration. [nM]/ | Test 1/ Inhibition Rate | Test 2/ Inhibition Rate | Average Value/ Inhibition Rate |
|---|---|---|---|
| 10000 | 101.4968 | 101.1627 | 101.3297 |
| 2500 | 100.5460 | 102.0894 | 101.3177 |
| 625 | 100.4979 | 100.4481 | 100.4730 |
| 156.25 | 98.8469 | 99.1585 | 99.0027 |
| 39.0625 | 56.0316 | 53.5761 | 54.8039 |
| 9.765625 | -0.5340 | 4.9841 | 2.2251 |
| 2.44140625 | 0.6801 | 3.6544 | 2.1673 |
| 0.610351563 | -2.7037 | 11.0756 | 4.1860 |
| 0.152587891 | -6.3508 | 13.6628 | 3.6560 |
| 0.038146973 | 2.0051 | 11.2715 | 6.6383 |

**Table 2.4: Antiproliferative Value and IC₅₀ Calculation of Compound P19 on SW48 Cells**

| Concentration. [nM] | Test 1/ Inhibition Rate | Test 2/ Inhibition Rate | Average Value/ Inhibition Rate |
|---|---|---|---|
| 10000 | 102.8134 | 101.6185 | 102.2160 |
| 2500 | 99.3955 | 97.7635 | 98.5795 |
| 625 | 96.9296 | 98.3647 | 97.6471 |
| 156.25 | 95.9814 | 94.9736 | 95.4775 |
| 39.0625 | 3.4682 | -0.4517 | 1.5082 |
| 9.765625 | -1.5276 | -5.4968 | -3.5122 |
| 2.44140625 | -2.9391 | -3.8155 | -3.3773 |
| 0.610351563 | 3.4714 | 2.2556 | 2.8635 |
| 0.152587891 | -2.3819 | -0.0356 | -1.2087 |
| 0.038146973 | 2.1306 | -3.1349 | -0.5021 |

**Table 2.5: Antiproliferation Value and IC₅₀ Calculation of Compound P24 on SW48 Cells**

| Concentration. [nM] | Test 1/ Inhibition Rate | Test 2/ Inhibition Rate | Average Value / Inhibition Rate |
|---|---|---|---|
| 10000 | 97.4001 | 97.2695 | 97.3348 |
| 2500 | 96.8613 | 98.3458 | 97.6036 |
| 625 | 97.6045 | 97.1886 | 97.3966 |
| 156.25 | 98.6246 | 98.8193 | 98.7220 |
| 39.0625 | 40.3040 | 47.1217 | 43.7129 |
| 9.765625 | -8.8535 | -7.7700 | -8.3117 |
| 2.44140625 | -4.8734 | -5.6425 | -5.2580 |
| 0.610351563 | -6.6380 | -6.6781 | -6.6581 |
| 0.152587891 | -6.5138 | -0.2265 | -3.3702 |
| 0.038146973 | -3.4737 | -5.3560 | -4.4148 |

**Table 2.6: Antiproliferation Value and IC₅₀ Calculation of Compound P29 on SW48 Cells**

| Concentration. [nM] | Test 1/ Inhibition Rate | Test 2/ Inhibition Rate | Average Value / Inhibition Rate |
|---|---|---|---|
| 10000 | 100.0820 | 99.5995 | 99.8407 |
| 2500 | 99.0523 | 98.9582 | 99.0052 |
| 625 | 99.1035 | 99.6341 | 99.3688 |
| 156.25 | 96.5322 | 98.0760 | 97.3041 |
| 39.0625 | 70.9494 | 70.8621 | 70.9057 |
| 9.765625 | 17.3524 | 25.0940 | 21.2232 |
| 2.44140625 | 12.7655 | 26.7665 | 19.7660 |
| 0.610351563 | 19.4374 | 9.9647 | 14.7010 |
| 0.152587891 | 26.0747 | 25.0834 | 25.5791 |
| 0.038146973 | 18.0156 | 18.5522 | 18.2839 |

The above results show that the molecules of the present disclosure have good antiproliferation effect on MSI-H tumor cells, and are expected to achieve a better tumor inhibition effect by inhibiting the WRN helicase activity.

The above results also show that: the control molecule **A1** has no inhibitory effect on SW48 cells at a concentration of 156 nM, while the molecules of the present disclosure such as P7, P19, and P29 still have significant inhibitory effects (>95%) at 156 nM, and have high inhibitory activity even at 39 nM. It may be seen that the molecules of the present disclosure have significantly enhanced inhibitory effects on SW48 cells as compared with the control molecule **A3** or **A1**.

### Example 14:

The molecules of the invention were tested for their anti-proliferative activities on MSS tumor cells.

HT-29 colorectal cancer cells with MSS were cultured in a McCoy's5A medium containing 10% FBS and 1% penicillin-streptomycin in a 37°C, 5% CO₂ constant temperature incubator. 40 µL of cell suspension was added to each well of a 384-well microplate. 40 nL of compounds with different concentrations were added to each well using Echo. The mixtures were cultured in a 37°C, 5% CO₂ constant temperature incubator for 5 days. 40 µL of CTG solution (Promega, Cat No. G7573) was added to each well, and the mixture was incubated in a 37°C, 5% CO₂ constant temperature incubator for 30 minutes in the dark. The luminescence value was read using an Envision multi-function microplate reader (Perkin Elmer, catalog number Envision 2104). The optical signal is proportional to the amount of ATP in the system, while the ATP content directly characterizes the number of viable cells in the system.

### IC₅₀ value calculation:

Y = lower platform signal + (upper platform signal-lower platform signal) / (1 + 10^((LogIC50 -X) × Hill slope))
X: log value of compound concentration
Y: Inhibition rate (%)

**Table 3: 2D antiproliferative effects of compounds on MSS colon cancer HT-29 cell line.**

| **Compounds** | **HT-29 / IC₅₀ / nM** | **Compounds** | **HT-29 / IC₅₀ / nM** |
|---|---|---|---|
| **P4** | 2369 | **P31** | >10000 |
| **P5** | >10000 | **P32** | >10000 |
| **P6** | >10000 | **P33** | >10000 |
| **P7** | >10000 | **P34** | >10000 |
| **P9** | >10000 | **P35** | >10000 |
| **P10** | >10000 | **P36** | >10000 |
| **P11** | >10000 | **P37** | >10000 |
| **P12** | >10000 | **P38** | >10000 |
| **P13** | 9107 | **P39** | >10000 |
| **P14** | >10000 | **P41** | 5591 |
| **P15** | >10000 | **P42** | >10000 |
| **P16** | >10000 | **P44** | >10000 |
| **P17** | >10000 | **P45** | >10000 |
| **P18** | 4867 | **P46** | >10000 |
| **P19** | >10000 | **P47** | >10000 |
| **P20** | >10000 | **P48** | N.D. |
| **P21** | >10000 | **P49** | N.D. |
| **P22** | >10000 | **P50** | >10000 |
| **P23** | >10000 | **P51** | N.D. |
| **P24** | >10000 | **P52** | N.D. |
| **P25** | >10000 | **P53** | >10000 |
| **P26** | >10000 | **P57** | >10000 |
| **P27** | >10000 | **P58** | >10000 |
| **P28** | >10000 | **P59** | >10000 |
| **P29** | >10000 | **P60** | N.D. |
| **P30** | >10000 | **P69** | >10000 |

| | | | |
|---|---|---|---|
| ND = Not Tested | | | |

The above results show that the molecules of the present disclosure have no inhibitory effect on MSS tumor cells, reflecting the high selectivity brought by the selective inhibition of the molecules of the present disclosure on WRN.

### Example 15:

The liver microsome stability test of the compounds. The details are as follows:
The compounds of the present disclosure were subjected to a liver microsome stability test. The compounds to be tested were co-incubated with different species of liver microsomes with or without NADPH. The final concentration of the compounds to be tested in the test system was 1 µM, the final concentration of NADPH was 1 mM, and the final concentration of liver microsomes was 0.5 mg/mL. The concentration of the compound in the incubation supernatant at different time points within 60 minutes was detected and the pharmacokinetic parameters (e.g., the clearance Clᵢₙₜ) were calculated.

The results indicate that the molecules of the present disclosure have good metabolic stability (especially in the human body).

**Table 4: Results of In Vitro Liver Microsome Stability Tests of Compounds in Humans or Mice.**

| **Compounds** | **Human Clᵢₙₜ /(mL/min/kg)** | **Human Clᵢₙₜ µL/min/mg Protein** | **Mouse Clᵢₙₜ /(mL/min/kg)** |
|---|---|---|---|
| **P7** | <0.5 | <0.5 | 16 |
| **P11** | 6.0 | 4.8 | N.D. |
| **P13** | 11.3 | 9.0 | N.D. |
| **P14** | 27.1 | 22.1 | N.D. |
| **P16** | 18.2 | 14.5 | N.D. |
| **P19** | <0.5 | <0.5 | N.D. |
| **P20** | 22.7 | 18.1 | N.D. |
| **P23** | 18.8 | 15.0 | N.D. |
| **P24** | <6.8 | <5.4 | <24 |
| **P25** | 1.4 | 1.1 | N.D. |
| **P26** | <6.8 | <5.4 | N.D. |
| **P29** | 6.4 | 5.1 | N.D. |
| **P30** | 3.7 | 3.0 | N.D. |
| **P31** | 7.2 | 9.0 | N.D. |
| **P32** | 0.33 | 0.26 | N.D. |
| **P35** | <0.5 | <0.5 | N.D. |
| **P37** | <0.5 | <0.5 | N.D. |
| **P38** | <0.5 | <0.5 | N.D. |
| **P45** | <6.8 | <5.4 | N.D. |
| **P50** | <0.5 | <0.5 | N.D. |
| **P56** | <6.8 | <5.4 | N.D. |

| | | | |
|---|---|---|---|
| ND = Not Tested | | | |

### Example 16:

### Membrane permeability evaluation experiments: Caco-2 assays

The molecules of the present disclosure were evaluated for their membrane permeability. The samples were analyzed by LC-MS to estimate the apparent permeability coefficient (Pₐₚₚ) of the compounds in Caco-2 monolayer cells, wherein the pH of the apical compartment was 6.5 and the pH of the basolateral compartment was 7.4. Inhibitors of P-gp efflux transporters, BCRP and MRP2 (50 µM Quinidine, 30 µM benzbromarone and 20 µM sulfasalazine) could block the active efflux transport of the compounds. Data would be used for apparent permeability (Papp).${P}_{app} = \left({V}_{A}\times {\left[drug\right]}_{acceptor}\right) / \left(Area\times Time\times {\left[drug\right]}_{initial , donor}\right)$ wherein VA is the volume of the receptor pore (unit: mL), Area is the surface area of the membrane (0.143 cm² for Transwell-96 well permeable scaffolds), and time is the total transport time (unit: seconds).$Efflux Ratio = {P}_{app} \left({}_{B - A}\right) / {P}_{app} \left({}_{A - B}\right)$

**Table 5: Caco-2 Membrane Permeation Data Results of the Molecules of the Present Disclosure.**

| **Compounds** | **Papp(A-B) (10⁻⁶ cm/s)** | **Papp(B-A) (10⁻⁶ cm/s)** | **Efflux** |
|---|---|---|---|
| **P7** | 2.44 | 9.50 | 3.9 |
| **P24** | 5.20 | 13.3 | 2.55 |
| **P29** | 1.07 | 12.50 | 11.7 |
| **P62** | 1.54 | 17.9 | 11.6 |
| **P65** | 3.78 | 13.7 | 3.62 |
| **P69** | 4.34 | 11.39 | 2.62 |

The above results indicate that the molecules of the present disclosure have good membrane permeability, and are expected to achieve good tumor inhibition effects with better in vivo pharmacokinetic properties.

### Example 17:

### Mouse pharmacokinetic evaluation experiment

CD1 female mice were used as test animals to be administered orally/intravenously (with oral dosage of 10 mg/kg or intravenous dosage of 2 mg/kg).

Experimental scheme: The oral administration group comprised 3 mice per group (Solvent: 10% Hβ-CD-pH7.4), and the intravenous group comprised 3 mice per group. For the oral administration group, plasma samples were collected before (0 h) and after (0.25, 0.5, 1, 2, 4, 8, 24 h) the administration; and for the intravenous group, plasma samples were collected before (0 h) and after (0.083, 0.25, 0.5, 1, 2, 4, 8, 24 h) the administration. The blood concentration in the plasma of mice was determined using the LC/MS/MS method after the oral or intravenous administration, respectively, and the collected data were calculated using the AB Sciex QTRAP 6500 software. The experimental results are as follows:

**Table 6.1: PK Results of Compounds in Mice.**

| **Compounds** | | **P7** | **P23** | **P29** | **P30** |
|---|---|---|---|---|---|
| IV (2 mg/kg) | T_{1/2} (h) | 1.7 | 1.0 | 1.2 | 0.6 |
| | C₀ (ng/mL) | 1414 | 1574 | 5339 | 3688 |
| | AUC₀₋₂₄ (h*ng/mL) | 1003 | 1623 | 2317 | 725 |
| | Cl (mL/min/kg) | 33.0 | 20.5 | 14.4 | 45.9 |
| PO (10 mg/kg) | T_{1/2} (h) | 4.7 | 1.3 | 1.4 | 1.2 |
| | Tₘₐₓ (h) | 0.5 | 0.25 | 0.25 | 0.5 |
| | Cₘₐₓ(ng/mL) | 1490 | 2428 | 3917 | 1363 |
| | AUC₀₋₂₄ (h*ng/mL) | 3923 | 4600 | 6090 | 1564 |
| | F (%) | 78 | 57 | 53 | 44 |

**Table 6.2: PK Results of Compounds in Mice.**

| **Compounds** | | **P32** | **P24** | **P9** | **A1** |
|---|---|---|---|---|---|
| IV (2 mg/kg) | T_{1/2} (h) | 1.1 | 1.5 | 0.9 | 1.3 |
| | C₀ (ng/mL) | 5002 | 2884 | 2842 | 2520 |
| | AUC₀₋₂₄ (h*ng/mL) | 4853 | 1742 | 1230 | 1186 |
| | Cl (mL/min/kg) | 6.9 | 19.0 | 27 | 28.1 |
| PO (10 mg/kg) | T_{1/2} (h) | 1.2 | 1.5 | 1.4 | 1.4 |
| | Tₘₐₓ (h) | 0.5 | 0.25 | 1.0 | 0.5 |
| | Cₘₐₓ(ng/mL) | 9192 | 6100 | 695 | 584 |
| | AUC₀₋₂₄ (h*ng/mL) | 18406 | 6565 | 1523 | 801 |
| | F (%) | 76 | 76 | 25 | 14 |

**Table 6.3: PK Results of Compounds in Mice.**

| **Compounds** | | **P57** | **P59** |
|---|---|---|---|
| IV (2 mg/kg) | T_{1/2} (h) | 1.2 | 0.9 |
| | C₀ (ng/mL) | 2918 | 5262 |
| | AUC₀₋₂₄ (h*ng/mL) | 3150 | 2973 |
| | Cl (mL/min/kg) | 10.5 | 11.2 |
| PO (10 mg/kg) | T_{1/2} (h) | 1.4 | 1.3 |
| | Tₘₐₓ (h) | 0.5 | 0.25 |
| | Cₘₐₓ(ng/mL) | 4260 | 2850 |
| | AUC₀₋₂₄ (h*ng/mL) | 8931 | 6072 |
| | F (%) | 57 | 41 |

The above experimental results show that the compounds of the present disclosure have good oral absorption effect. Compared with the control molecule **A1**, they have better oral absorption and higher in vivo exposure, and are expected to bring higher therapeutic effects due to the better activity and selectivity.

The above experimental results also show that the thiophene bicyclic molecules **P7, P24, P32** and **P29** etc. have better oral absorption and higher oral absorption effect than the thiophene monocyclic substituted **P9.**

### Example 18:

In vivo efficacy experiment in BALB/C nude mice. Details are as follows:
SW48 (MSI-H) colorectal cancer tumor cells were cultured (in an L15 medium with 10% fetal bovine serum), and inoculated into 6-8 week old female BALB/c nude mice (with body weight of about 20 g). All the mice were inoculated subcutaneously. The mice were raised in an SPF-grade experimental environment, and all the mice were free to take commercially certified standard diets. When the tumors grew to an average volume of about 160 mm³ in mice, the test compound was orally administered daily. The dosage was as follows: the blank group was given a vehicle (10% aqueous HP-β-CD solution at pH~7), and the administration group was given 50 mg/kg or 120 mg/kg once a day. The tumor volume was measured with a two-dimensional caliper three times a week, and the animals were weighed every day. After consecutive administration for 21 days, the inhibition rate was calculated based on the final tumor volume (TGI/100%). The volume calculation formula is: V=1/2a*b², where a represents the long diameter of the tumor and b represents the short diameter of the tumor.

**Table 7: Antitumor Effects of the Compounds on Colorectal Cancer SW48 Cells Xenografted in Nude Mice.**

| Test drug | Dosage | Tumor Volume (mm³)-D1 | Tumor volume (mm³)-D21 | TGI |
|---|---|---|---|---|
| Blank group 1 | 0 | 174 | 3026 | 0% |
| **P7** | 120 mg/kg, QD | 174 | 24 | 105% |
| **P29** | 120 mg/kg, QD | 174 | 36 | 105% |
| Blank Group 2 | 0 | 163 | 1966 | 0% |
| **P7** | 50 mg/kg, QD | 158 | 71 | 105% |
| **P7** | 20 mg/kg, QD | 158 | 491 | 82% |
| **P24** | 50 mg/kg, QD | 161 | 72 | 105% |
| **A1** | 50 mg/kg, QD | 160 | 1065 | 50% |
| **A1** | 20 mg/kg, QD | 162 | 1672 | 16% |

TGI = [1 - [volume of the administration group (D21 - D1) / volume of the blank group (D21 - D1)]] * 100%

The results show that the molecules of the present disclosure have good in vivo efficacy on MSI-H microsatellite unstable tumor cells and are less toxic to the mice (no mice in any group were affected to loss more than 5% of their weight). Compared with the control molecule **A1,** the molecules of the present disclosure significantly improve the tumor inhibition effect in vivo.

## Claims

1. A compound of Formula (I), or a pharmaceutically acceptable salt, isotopic variant, tautomer or stereoisomer thereof, or a mixture thereof: 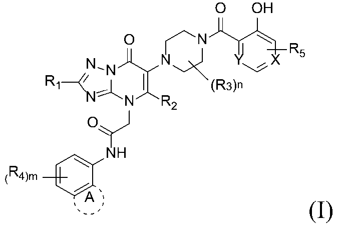 wherein,
X and Y are each independently selected from CH and N, and at least one of X and Y is N atom;
Ring A is absent or selected from C₃₋₁₀ cycloalkyl, 5-10 membered heteroaryl, 5-10 membered heterocyclyl, and C₆₋₁₀ aryl;
R₁ is selected from 5-12 membered heteroaryl and 5-12 membered heterocyclyl, and the R₁ is optionally substituted with 1, 2, 3, 4 or 5 Rₓ, provided that R₁ is not pyridyl or 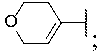
Rₓ is selected from H, D, halogen, NH₂, CN, OH, C₁₋₆ alkyl, C₁₋₆ alkoxy, C₁₋₆ haloalkyl, -C(O)Rₐ, -C(O)ORₐ, -OC(O)Rₐ, -C(O)NH-Rₐ, -NHC(O)-Rₐ, -(CH₂)ₚ-ORₐ, -(CH₂)ₚ-C(O)Rₐ, - P(O)-(Rₐ)₂, and -S(O)₂-Rₐ, wherein Rₐ is selected from H, C₁₋₆ alkyl, C₁₋₆ haloalkyl and C₃₋₆ cycloalkyl, and p is selected from 0, 1, 2, 3 and 4; or two Rₓ on the same atom are taken together to form oxo or thio;
R₂ is selected from H, D, halogen, C₁₋₆ alkyl, C₁₋₆ haloalkyl, C₁₋₆ alkoxy, C₁₋₆ alkylthio, and C₃₋₆ cycloalkyl;
R₃ is selected from H, D, halogen, NH₂, CN, OH, C₁₋₆ alkyl, C₁₋₆ haloalkyl, C₁₋₆ alkoxy, and C₃₋₆ cycloalkyl, or R₃ on two different carbon atoms are connected to form a bridged ring, or two R₃ on the same carbon atom are connected to form C₃₋₁₀ cycloalkyl, 5-10 membered heteroaryl, C₃₋₁₀ cycloalkyl or 3-10 membered heterocyclyl;
R₄ is selected from H, D, halogen, NH₂, CN, OH, SF₅, SCF₃, C₁₋₆ alkyl, C₁₋₆ haloalkyl, C₁₋₆ alkoxy, C₁₋₆ haloalkoxy, C₁₋₆ alkylthio, C₃₋₁₀ cycloalkyl, 3-10 membered heterocyclyl, 5-10 membered heteroaryl, and C₆₋₁₀ aryl;
R₅ is selected from H, D, halogen, NH₂, CN, OH, C₁₋₆ alkyl, C₁₋₆ deuterated alkyl, C₁₋₆ haloalkyl, C₁₋₆ alkoxy, C₁₋₆ alkylthio, and C₃₋₆ cycloalkyl;
m is selected from 0, 1, 2, 3, 4 and 5;
n is selected from 0, 1, 2, 3, 4, 5, 6, 7 and 8;
R₁ - R₅ are optionally deuterated, up to fully deuterated.

2. The compound of claim 1, or a pharmaceutically acceptable salt, isotopic variant, tautomer or stereoisomer thereof, or a mixture thereof, wherein:
X and Y are each independently selected from CH and N, and at least one of X and Y is a N atom;
Ring A is absent or selected from a 5-6 membered heteroaryl group, wherein the 5-6 membered heteroaryl group and the benzene ring to which it is attached are taken together to form a heteroaryl group of 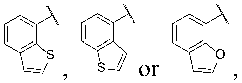 alternatively, the Ring A is absent;
R₁ is a 5-10 membered heteroaryl or a 5-12 membered heterocyclic group, alternatively a 5-10 membered bicyclic heteroaryl group, wherein R₁ is optionally substituted with 1, 2 or 3 Rₓ;
Rₓ is alternatively H, NH₂, CH₃, CH₂OH, CH₂OCH₃ or C(O)CH₃ or -S(O)₂-CH₃, or two Rₓ on the same carbon atom are taken together form an oxo group;
alternatively, R₁ is selected from 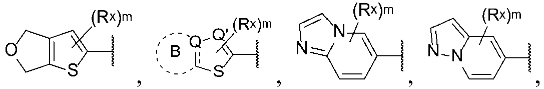 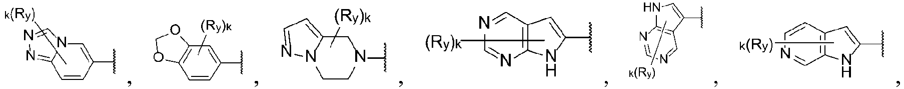 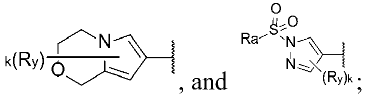
Ring B is absent or selected from C₃₋₁₀ cycloalkyl, 5-10 membered heterocyclyl, 5-10 membered heteroaryl and C₆₋₁₀ aryl, and the Ring B is optionally substituted with 1, 2, 3, 4 or 5 substituents selected from halogen, C₁₋₆ alkyl and C₁₋₆ haloalkyl;
Q and Q' are independently selected from CH and N, and at most one of Q and Q' is N;
R_{y} is selected from H, D, halogen, C₁₋₆ alkyl and C₁₋₆ haloalkyl;
R₂ is selected from C₁₋₄ alkyl, such as CH₃ or CH₂CH₃;
R₃ is selected from H, D and C₁₋₄ alkyl, such as H, D or CH₃, or R₃ on two different carbon atoms are connected to form a bridged ring, or two R₃ on the same carbon atom are connected to form a C₃₋₅ cycloalkyl, such as cyclopropyl;
R₄ is selected from H, F, Cl, Br, CH₃, CH₂CH₃, SCF₃, OCF₃, CF₃ and pyridyl;
R₅ is selected from H, CH₃ and CD₃, alternatively H or CH₃;
Rₐ is selected from C₁₋₆ alkyl;
m is selected from 0, 1, 2 and 3;
k is selected from 0, 1, 2, 3, 4 and 5.

3. The compound of claim 1 or 2, or a pharmaceutically acceptable salt, isotopic variant, tautomer or stereoisomer thereof, or a mixture thereof, wherein R₁ is selected from: 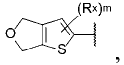 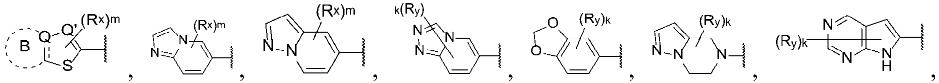 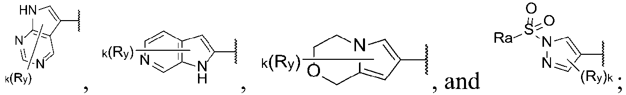 wherein,
-̅ -̅ -̅ represents a single bond or a double bond, and when Q is N and Ring B is present, -̅ -̅ -̅ represents a single bond;
Q and Q' are independently selected from CH and N, and at most one of Q and Q' is N;
Ring B is absent or selected from C₃₋₁₀ cycloalkyl, 5-10 membered heterocyclyl, 5-10 membered heteroaryl and C₆₋₁₀ aryl, and the Ring B is optionally substituted with 1, 2, 3, 4 or 5 substituents selected from halogen, C₁₋₆ alkyl and C₁₋₆ haloalkyl;
Rₓ is selected from H, NH₂, halogen, C₁₋₆ alkyl, C₁₋₆ haloalkyl, -C(O)Rₐ, -C(O)ORₐ, - OC(O)Rₐ, -C(O)NH-Rₐ, -NHC(O)-Rₐ, -(CH₂)p-ORₐ and -(CH₂)p-C(O)Rₐ, wherein Rₐ is selected from H, C₁₋₆ alkyl and C₁₋₆ haloalkyl, and p is selected from 1, 2 and 3;
R_{y} is selected from H, D, halogen, C₁₋₆ alkyl and C₁₋₆ haloalkyl;
Rₐ is selected from C₁₋₆ alkyl;
m is selected from 0, 1, 2, 3, 4 and 5;
k is selected from 0, 1, 2, 3, 4 and 5;
alternatively,
-̅ -̅ -̅ represents a single bond or a double bond, and when Q is N and Ring B is present, -̅ -̅ -̅ represents a single bond;
Q and Q' are independently selected from CH and N, and at most one of Q and Q' is N;
Ring B is absent or selected from 5-7 membered heterocyclyl or 5-6 membered heteroaryl, and the Ring B is optionally substituted with 1, 2 or 3 substituents selected from halogen and C₁₋₄ alkyl;
Rₓ is selected from H, NH₂, C₁₋₄ alkyl, -C(O)Rₐ and -(CH₂)ₚ-ORₐ, wherein Rₐ is selected from H and C₁₋₄ alkyl, and p is selected from 1 and 2; alternatively, Rₓ is H, NH₂, CH₃, CH₂OH, CH₂OCH₃ or C(O)CH₃;
R_{y} is selected from H, D, halogen, C₁₋₆ alkyl and C₁₋₆ haloalkyl;
Rₐ is selected from CH₃;
m is selected from 0, 1, 2 and 3;
k is selected from 0, 1, 2 and 3;
more alternatively,
R₁ is selected from:

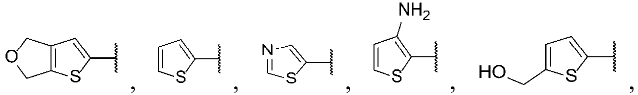

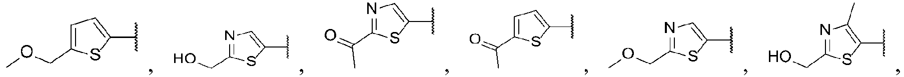

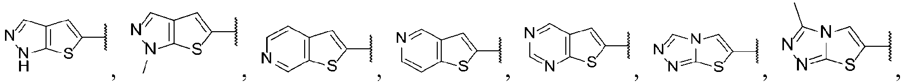

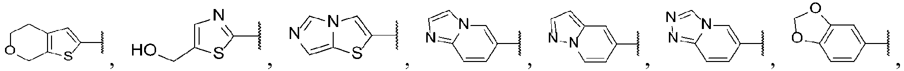

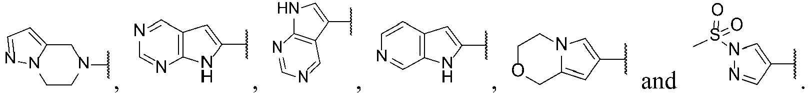

4. The compound according to any one of claims 1 to 3, or a pharmaceutically acceptable salt, isotopic variant, tautomer or stereoisomer thereof, or a mixture thereof, selected from the structures of: 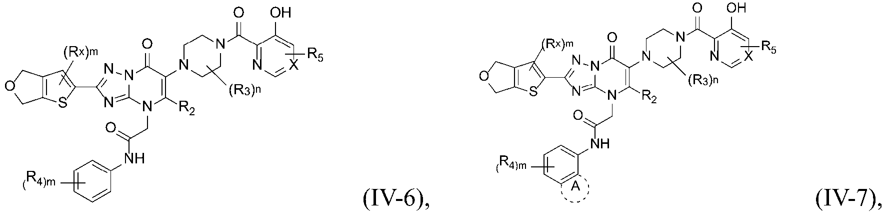 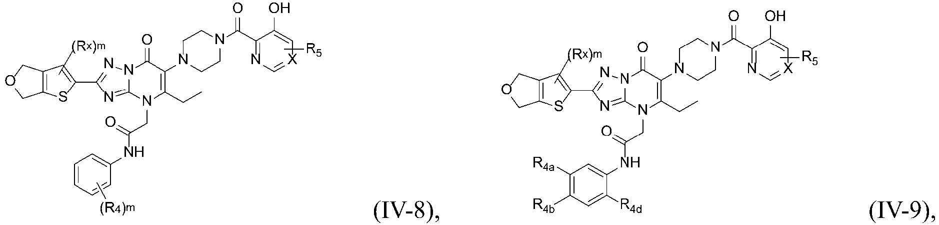 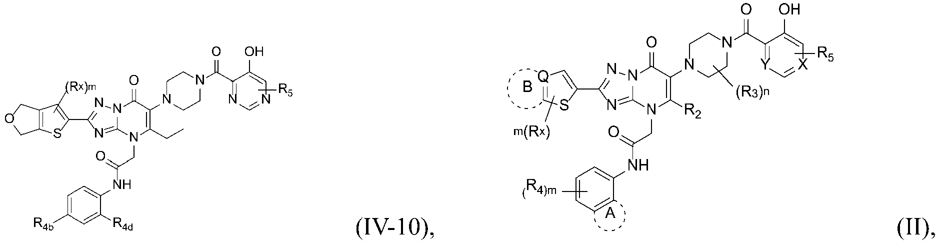 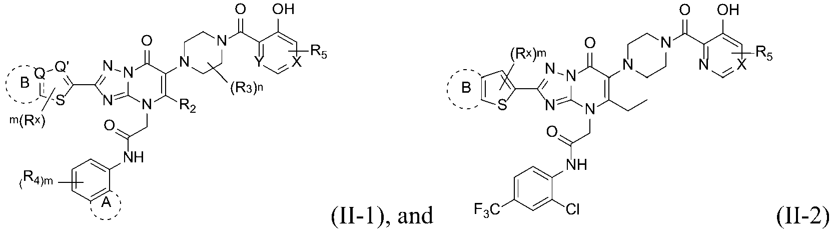 wherein,
the variables are as defined in any one of claims 1-3.

5. The compound according to any one of claims 1 to 4, or a pharmaceutically acceptable salt, isotopic variant, tautomer or stereoisomer thereof, or a mixture thereof, having the structure of (IV-6) or (IV-7): 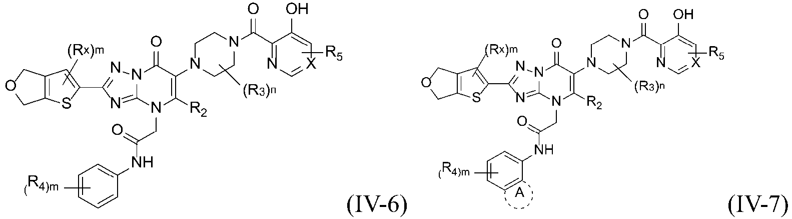 wherein,
Ring A is absent or selected from C₃₋₁₀ cycloalkyl, 5-10 membered heteroaryl, 5-10 membered heterocyclyl and C₆₋₁₀ aryl;
X is selected from CH and N;
Rₓ is selected from H, D, C₁₋₆ alkyl, C₁₋₆ alkoxy and C₁₋₆ haloalkyl;
R₂ is selected from H, D, C₁₋₆ alkyl, C₁₋₆ alkoxy and C₁₋₆ haloalkyl;
R₃ is selected from H, D, C₁₋₆ alkyl, C₁₋₆ alkoxy and C₁₋₆ haloalkyl, or R₃ on two different carbon atoms are connected to form a bridged ring, or two R₃ on the same carbon atom are connected to form a C₃₋₁₀ cycloalkyl, a 5-10 membered heteroaryl, a C₃₋₁₀ cycloalkyl or a 3-10 membered heterocyclyl;
R₄ is selected from H, D, halogen, CN, SCF₃, C₁₋₆ alkyl, C₁₋₆ alkoxy, C₁₋₆ haloalkyl, C₁₋₆ haloalkoxy, C₃₋₆ cycloalkyl, 4-10 membered heterocyclyl, 5-10 membered heteroaryl and C₆₋₁₀ aryl;
R₅ is selected from H, D, C₁₋₆ alkyl, C₁₋₆ deuterated alkyl, C₁₋₆ alkoxy and C₁₋₆ haloalkyl;
m is selected from 0, 1, 2, 3, 4 and 5;
n is selected from 0, 1, 2, 3, 4, 5, 6, 7 and 8.

6. The compound of claim 5, or a pharmaceutically acceptable salt, isotopic variant, tautomer or stereoisomer thereof, or a mixture thereof, wherein:
Ring A is absent or selected from 5-10 membered heteroaryl, 5-10 membered heterocyclyl and C₆₋₁₀ aryl;
X is selected from CH and N;
Rₓ is selected from H, C₁₋₆ alkyl, C₁-₆ alkoxy and C₁₋₆ haloalkyl;
R₂ is selected from C₁₋₆ alkyl and C₁₋₆ haloalkyl;
R₃ is selected from H, D, C₁₋₆ alkyl and C₁₋₆ haloalkyl, or R₃ on two different carbon atoms are connected to form a bridged ring, or two R₃ on the same carbon atom are connected to form a C₃₋₁₀ cycloalkyl or a 3-10 membered heterocyclic group;
R₄ is selected from H, halogen, CN, SCF₃, C₁₋₆ alkyl, C₁₋₆ alkoxy, C₁₋₆ haloalkyl and C₁₋6 haloalkoxy;
R₅ is selected from H, C₁₋₆ alkyl, C₁₋₆ alkoxy and C₁₋₆ haloalkyl;
m is selected from 0, 1, 2, 3, 4 and 5;
n is selected from 0, 1, 2, 3, 4, 5, 6, 7 and 8.

7. The compound of claim 5 or 6, or a pharmaceutically acceptable salt, isotopic variant, tautomer or stereoisomer thereof, or a mixture thereof, wherein:
Ring A is absent;
X is selected from CH and N;
Rₓ is selected from H and C₁₋₆ alkyl;
R₂ is selected from C₁₋₆ alkyl;
R₃ is selected from H, D and C₁₋₆ alkyl, or two R₃ on different carbon atoms are connected to form a bridged ring, or two R₃ on the same carbon atom are connected to form a C₃₋₇ cycloalkyl;
R₄ is selected from H, halogen, SCF₃, C₁₋₆ alkyl, C₁₋₆ alkoxy, C₁₋₆ haloalkyl and C₁₋₆ haloalkoxy;
R₅ is selected from H and C₁₋₆ alkyl;
m is selected from 0, 1, 2, 3 and 4;
n is selected from 0, 1, 2, 3, 4, 5, 6, 7 and 8.

8. The compound of any one of claims 5 to 7, or a pharmaceutically acceptable salt, isotopic variant, tautomer or stereoisomer thereof, or a mixture thereof, wherein:
Ring A is selected from a 5-6 membered heteroaryl group, and the 5-6 membered heteroaryl group and the benzene ring to which it is attached are taken together form a heteroaryl group of 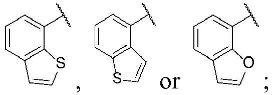
X is selected from CH and N;
Rₓ is selected from H and C₁₋₄ alkyl, alternatively H;
R₂ is selected from C₁₋₄ alkyl, such as CH₃ or CH₂CH₃;
R₃ is selected from H, D and C₁₋₄ alkyl, such as H, D or CH₃, or R₃ on two different carbon atoms are connected to form a bridged ring, or two R₃ on the same carbon atom are connected to form a C₃₋₅ cycloalkyl, such as cyclopropyl;
R₄ is selected from H, halogen, SCF₃, C₁₋₄ alkyl and C₁₋₄ haloalkyl, such as H, F, Cl, Br, CH₃, CH₂CH₃, CF₃, SCF₃, OCF₃ or OCH₃;
R₅ is selected from H and C₁₋₄ alkyl, e.g., H or CH₃;
m is selected from 0, 1, 2 and 3;
n is selected from 0, 1, 2, 3, 4, 5, 6, 7 and 8.

9. The compound of any one of claims 5 to 8, or a pharmaceutically acceptable salt, isotopic variant, tautomer or stereoisomer thereof, or a mixture thereof, wherein the compound has the structure of (IV-8) or (IV-9): 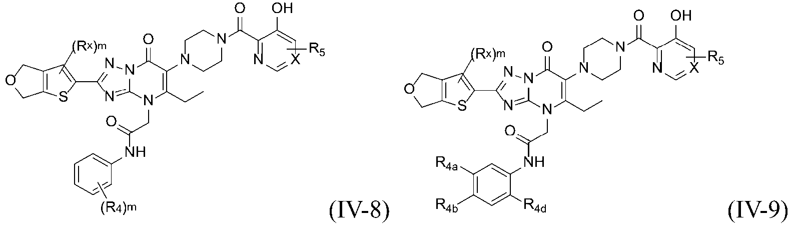 wherein,
X is selected from N and CH;
R₄ is selected from H, CN, halogen, C₁₋₆ alkyl, C₁₋₆ alkoxy and C₁₋₆ haloalkyl;
R₄ₐ is selected from H, halogen, CN, C₁₋₆ alkyl, C₁₋₆ alkoxy and C₁₋₆ haloalkyl;
R_{4b} is selected from halogen, CN, C₁₋₆ alkyl, C₁₋₆ alkoxy and C₁₋₆ haloalkyl;
R_{4d} is selected from H, CN, halogen, C₁₋₆ alkyl, C₁₋₆ alkoxy and C₁₋₆ haloalkyl;
R₅ is selected from H, C₁₋₆ alkyl, C₁₋₆ alkoxy and C₁₋₆ haloalkyl, alternatively C₁₋₆ alkyl or C₁₋₆ haloalkyl;
Rₓ is selected from H, C₁₋₆ alkyl, C₁₋₆ alkoxy and C₁₋₆ haloalkyl;
m is selected from 0, 1, 2, 3, 4 and 5.

10. The compound of claim 9, or a pharmaceutically acceptable salt, isotopic variant, tautomer or stereoisomer thereof, or a mixture thereof, wherein:
X is selected from N and CH;
R₄ is selected from H, halogen, C₁₋₄ alkyl and C₁₋₄ haloalkyl;
R₄ₐ is selected from H and halogen;
R_{4b} is selected from halogen, C₁₋₄ alkyl and C₁₋₄ haloalkyl;
R_{4d} is selected from H, halogen and C₁₋₄ alkyl;
R₅ is selected from H, C₁₋₄ alkyl and C₁₋₄ haloalkyl, alternatively C₁₋₄ alkyl or C₁₋₄ haloalkyl;
Rₓ is selected from H, C₁₋₄ alkyl and C₁₋₄ haloalkyl;
m is selected from 0, 1, 2, 3 and 4;
alternatively,
X is selected from N and CH;
R₄ is selected from H, halogen, C₁₋₂ alkyl and C₁₋₂ haloalkyl, such as H, Cl, Br, CH₃, CH₂CH₃ or CF₃;
R₄ₐ is selected from H and halogen, alternatively H or F;
R_{4b} is selected from halogen, C₁₋₂ alkyl and C₁₋₂ haloalkyl, alternatively Cl, Br, CF₃ or CH₂CH₃;
R_{4d} is selected from H, halogen and C₁₋₂ alkyl, alternatively H, F, Cl or CH₃;
R₅ is selected from H and C₁₋₂ alkyl, alternatively CH₃;
Rₓ is selected from H and C₁₋₂ alkyl, alternatively H;
m is selected from 0, 1, 2 and 3.

11. The compound of any one of claims 5 to 10, or a pharmaceutically acceptable salt, isotopic variant, tautomer, stereoisomer, solvate or hydrate thereof, or a mixture thereof, wherein the compound has the structure of (IV-10): 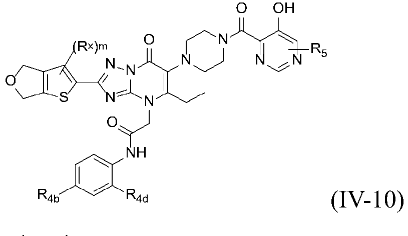 wherein,
R_{4b} is selected from halogen and C₁₋₆ haloalkyl;
R_{4d} is selected from halogen and C₁₋₆ alkyl;
R₅ is selected from C₁₋₆ alkyl and C₁₋₆ haloalkyl;
Rₓ is selected from H, C₁₋₆ alkyl and C₁₋₆ haloalkyl;
m is 0 or 1;
alternatively,
R_{4b} is selected from halogen and C₁₋₄ haloalkyl;
R_{4d} is selected from halogen and C₁₋₄ alkyl;
R₅ is selected from C₁₋₄ alkyl and C₁₋₄ haloalkyl;
Rₓ is selected from H, C₁₋₄ alkyl and C₁₋₄ haloalkyl;
m is 0 or 1;
more alternatively,
R_{4b} is selected from halogen and C₁₋₂ haloalkyl, alternatively Cl, Br or CF₃;
R_{4d} is selected from halogen and C₁₋₂ alkyl, alternatively F, Cl or CH₃;
R₅ is C₁₋₂ alkyl, alternatively CH₃;
Rₓ is H and C₁₋₂ alkyl, alternatively H;
m is 0 or 1.

12. The compound of Formula (I), or a pharmaceutically acceptable salt, isotopic variant, tautomer or stereoisomer thereof, or a mixture thereof: 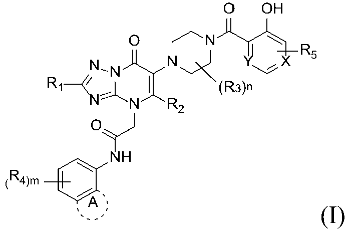 wherein,
X and Y are each independently selected from CH and N, and at least one of X and Y is a N atom;
R₁ is selected from 5-12 membered heteroaryl and 5-12 membered heterocyclic group; and the R₁ may be substituted with 1, 2 or 3 Rₓ;
provided that when R₁ is selected from 5-12 membered heteroaryl, R₁ is not pyridinyl;
R₂ is selected from H, C₁₋₆ alkyl, C₁₋₆ haloalkyl, C₁₋₆ alkoxy, C₁₋₆ alkylthio and C₃₋₆ cycloalkyl;
R₃ is independently selected from H, halogen, CN, C₁₋₆ alkyl, C₁₋₆ haloalkyl and C₃₋₆ cycloalkyl; or two R₃ are connected to the carbon atom(s) to which they are attached to form a 3-6 membered spiro ring or a bridged ring;
R₄ is independently selected from H, halogen, CN, SF₅, C₁₋₆ alkyl, C₁₋₆ haloalkyl, C₁₋₆ alkylthio, C₃₋₆ cycloalkyl and 4-10 membered heterocyclyl;
Ring A is present or absent, and selected from 5-6 membered heteroaryl group or 5-7-membered heterocyclic group;
R₅ is selected from H, C₁₋₆ alkyl, C₁₋₆ haloalkyl, C₁₋₆ alkoxy, C₁₋₆ alkylthio and C₃₋₆ cycloalkyl;
Rₓ is selected from H, C₁₋₆ alkyl, C₁₋₆ haloalkyl, C₁₋₆ alkoxy, CN, NH₂, -C(O)Rₐ, - C(O)ORₐ, -(CH₂)ₚ-ORₐ, -P(O)-(Rₐ)₂ and -S(O)₂-Rₐ;
Rₐ is selected from H, C₁₋₆ alkyl and C₃₋₆ cycloalkyl;
m is selected from 0, 1, 2 and 3;
n is selected from 0, 1 and 2;
p is selected from 0, 1 and 2.

13. The compound of claim 12, or a pharmaceutically acceptable salt, isotopic variant, tautomer or stereoisomer thereof, or a mixture thereof, which is a compound of Formula (II) or Formula (VI): 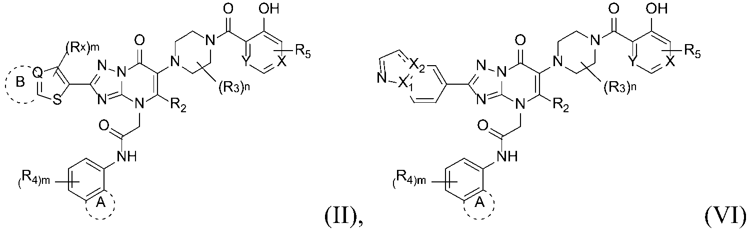 wherein,
X and Y are each independently selected from CH and N, and at least one of X and Y is a N atom;
Ring B is present or absent, and the Ring B is selected from 5-6 membered heteroaryl and 5-6 membered heterocyclic group;
X₁ and X₂ are each independently selected from CH and N, and -̅-̅-̅-̅-̅-̅ represents a single bond or a double bond;
Q is selected from CH and N;
R₂ is selected from H, C₁₋₆ alkyl, C₁₋₆ haloalkyl, C₁₋₆ alkoxy, C₁₋₆ alkylthio and C₃₋₆ cycloalkyl;
R₃ is independently selected from H, halogen, CN, C₁₋₆ alkyl, C₁₋₆ haloalkyl and C₃₋₆ cycloalkyl; or two R₃ are connected to the carbon atom(s) to which they are attached to form a 3-6 membered spiro ring or a bridged ring;
R₄ is independently selected from H, halogen, CN, SF₅, C₁₋₆ alkyl, C₁₋₆ haloalkyl, C₁₋₆ alkylthio, C₃₋₆ cycloalkyl and 4-10 membered heterocyclyl;
Ring A is present or absent, and selected from 5-6 membered heteroaryl group and 5-7 membered heterocyclic group;
R₅ is selected from H, C₁₋₆ alkyl, C₁₋₆ haloalkyl, C₁₋₆ alkoxy, C₁₋₆ alkylthio and C₃₋₆ cycloalkyl;
Rₓ is selected from H, C₁₋₆ alkyl, C₁₋₆ haloalkyl, C₁₋₆ alkoxy, CN, NH₂, -C(O)Rₐ, - C(O)ORₐ, -(CH₂)ₚ-ORₐ, -P(O)-(Rₐ)₂ and -S(O)₂-Rₐ;
Rₐ is selected from H, C₁₋₆ alkyl and C₃₋₆ cycloalkyl;
m is selected from 0, 1, 2 and 3;
n is selected from 0, 1 and 2;
p is selected from 0, 1 and 2.

14. The compound of claim 13, or a pharmaceutically acceptable salt, isotopic variant, tautomer or stereoisomer thereof, or a mixture thereof:
wherein,
X and Y are each independently selected from CH and N, and at least one of X and Y is a N atom;
Ring B is present or absent, and Ring B is selected from 5-6 membered heteroaryl and 5-6 membered heterocyclic group;
X₁ and X₂ are each independently selected from CH and N, and -̅ -̅ -̅ -̅ -̅ -̅ represents a single bond or a double bond;
Q is selected from CH and N;
R₂ is selected from H, C₁₋₆ alkyl, C₁₋₆ haloalkyl, C₁₋₆ alkoxy, C₁₋₆ alkylthio and C₃₋₆ cycloalkyl;
R₃ is independently selected from H, halogen, CN, methyl, ethyl, trifluoromethyl and cyclopropyl;
R₄ is independently selected from H, halogen, CN, SF₅, C₁₋₆ alkyl, C₁₋₆ haloalkyl, C₁₋₆ alkylthio, C₃₋₆ cycloalkyl and 4-10 membered heterocyclyl;
Ring A is present or absent, and selected from 5-6 membered heteroaryl group and 5-7 membered heterocyclic group;
R₅ is selected from H, methyl, ethyl, trifluoromethyl, methoxy, ethoxy, methylthio and cyclopropyl;
Rₓ is selected from H, C₁₋₆ alkyl, C₁₋₆ haloalkyl, C₁₋₆ alkoxy, CN, NH₂, -C(O)Rₐ, - C(O)ORₐ, -(CH₂)ₚ-ORₐ, -P(O)-(Rₐ)₂ and -S(O)₂-Rₐ;
Rₐ is selected from H and C₁₋₆ alkyl;
m is selected from 0, 1, 2 and 3;
n is selected from 0, 1 and 2;
p is selected from 0, 1 and 2.

15. The compound of any one of claims 12 to 14, or a pharmaceutically acceptable salt, isotopic variant, tautomer or stereoisomer thereof, or a mixture thereof, having a general structure of: 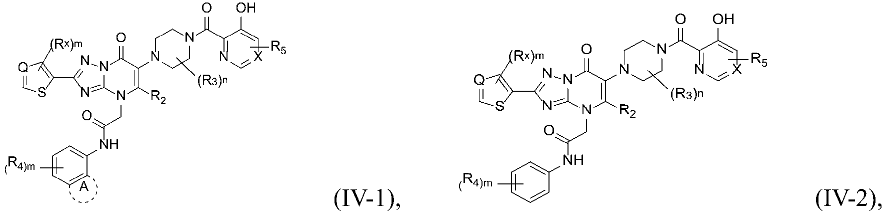 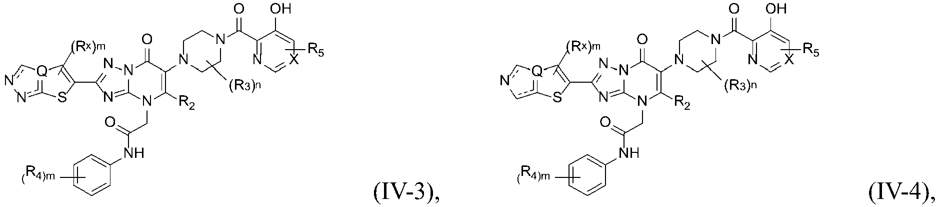 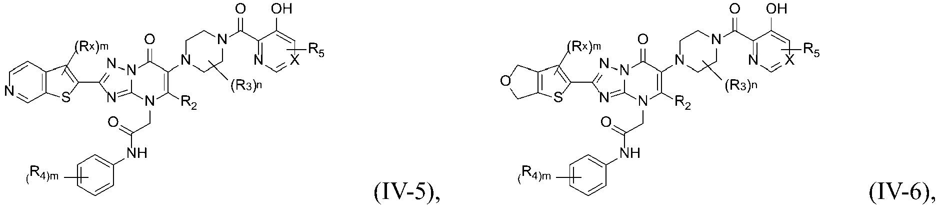 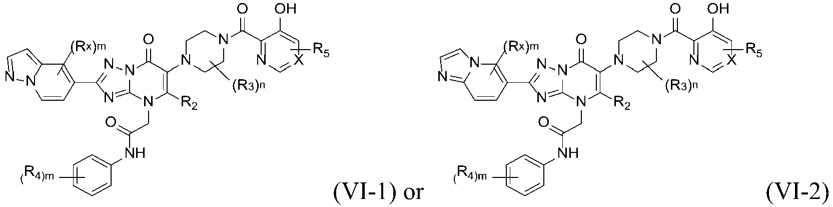 wherein,
X is selected from CH and N;
-̅ -̅ -̅ -̅ -̅ -̅ represents a single bond or a double bond;
Q is selected from CH and N;
R₂ is selected from H, C₁₋₆ alkyl, C₁₋₆ haloalkyl, C₁₋₆ alkoxy, C₁₋₆ alkylthio and C₃₋₆ cycloalkyl;
R₃ is independently selected from H, halogen, CN, C₁₋₆ alkyl, C₁₋₆ haloalkyl and C₃₋₆ cycloalkyl; or two R₃ are connected to the carbon atom(s) to which they are attached to form a 3-6 membered spiro ring or a bridged ring;
R₄ is independently selected from H, halogen, CN, SF₅, C₁₋₆ alkyl, C₁₋₆ haloalkyl, C₁₋₆ alkylthio, C₃₋₆ cycloalkyl and 4-10 membered heterocyclyl;
Ring A is present or absent, and selected from 5-6 membered heteroaryl group and 5-7 membered heterocyclic group;
R₅ is selected from H, C₁₋₆ alkyl, C₁₋₆ haloalkyl, C₁₋₆ alkoxy, C₁₋₆ alkylthio and C₃₋₆ cycloalkyl;
Rₓ is selected from H, C₁₋₆ alkyl, C₁₋₆ haloalkyl, C₁₋₆ alkoxy, CN, NH₂, -C(O)Rₐ, - C(O)ORₐ, -(CH₂)ₚ-ORₐ, -P(O)-(Rₐ)₂ and -S(O)₂-Rₐ;
Rₐ is selected from H, C₁₋₆ alkyl and C₃₋₆ cycloalkyl;
m is selected from 0, 1, 2 and 3;
n is selected from 0, 1 and 2;
p is selected from 0, 1 and 2.

16. The compound of claim 15, or a pharmaceutically acceptable salt, isotopic variant, tautomer or stereoisomer thereof, or a mixture thereof, having a general structure of: 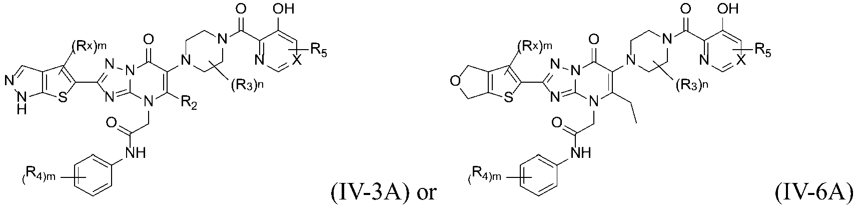 wherein,
X is selected from CH and N;
R₂ is selected from H, C₁₋₆ alkyl, C₁₋₆ haloalkyl, C₁₋₆ alkoxy, C₁₋₆ alkylthio and C₃₋₆ cycloalkyl;
R₃ is independently selected from H, halogen, CN, C₁₋₆ alkyl, C₁₋₆ haloalkyl and C₃₋₆ cycloalkyl; or two R₃ are connected to the carbon atom(s) to which they are attached to form a 3-6 membered spiro ring or a bridged ring;
R₄ is independently selected from H, halogen, CN, SF₅, C₁₋₆ alkyl, C₁₋₆ haloalkyl, C₁₋₆ alkylthio, C₃₋₆ cycloalkyl and 4-10 membered heterocyclyl;
R₅ is selected from H, C₁₋₆ alkyl, C₁₋₆ haloalkyl, C₁₋₆ alkoxy, C₁₋₆ alkylthio and C₃₋₆ cycloalkyl;
Rₓ is selected from H, C₁₋₆ alkyl, C₁₋₆ haloalkyl, C₁₋₆ alkoxy, CN, NH₂, -C(O)Rₐ, - C(O)ORₐ, -(CH₂)ₚ-ORₐ, -P(O)-(Rₐ)₂ and -S(O)₂-Rₐ;
Rₐ is selected from H, C₁₋₆ alkyl and C₃₋₆ cycloalkyl;
m is selected from 0, 1, 2 and 3;
n is selected from 0, 1 and 2;
p is selected from 0, 1 and 2.

17. The compound of claim 16, or a pharmaceutically acceptable salt, isotopic variant, tautomer, stereoisomer, solvate or hydrate thereof, or a mixture thereof:
wherein,
X is selected from CH and N;
R₂ is selected from H, methyl, ethyl, trifluoroethyl, methoxy and cyclopropyl;
R₃ is independently selected from H, F, CN, methyl, ethyl, trifluoromethyl and cycloalkyl; or two R₃ are connected to the carbon atom to which they are attached to form a cycloalkyl or cyclobutyl group;
R₄ is independently selected from H, F, Cl, Br, CN, SF₅, methyl, ethyl, trifluoromethyl, difluoromethyl, methylthio, ethylthio and cyclopropyl;
R₅ is selected from H, methyl, ethyl, trifluoromethyl, methoxy, methylthio and cyclopropyl;
Rₓ is selected from H, C₁₋₆ alkyl, C₁₋₆ haloalkyl, C₁₋₆ alkoxy, CN, NH₂, -C(O)Rₐ, - C(O)ORₐ, -(CH₂)ₚ-ORₐ, -P(O)-(Rₐ)₂ and -S(O)₂-Rₐ;
Rₐ is selected from H, C₁₋₆ alkyl and C₃₋₆ cycloalkyl;
m is selected from 0, 1, 2 and 3;
n is selected from 0, 1 and 2;
p is selected from 0, 1 and 2.

18. A compound, or a pharmaceutically acceptable salt, isotopic variant, tautomer, stereoisomer thereof, wherein the compound is selected from:

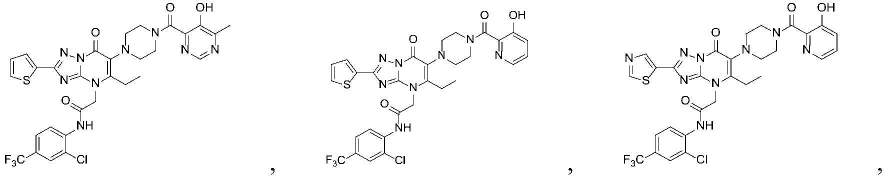

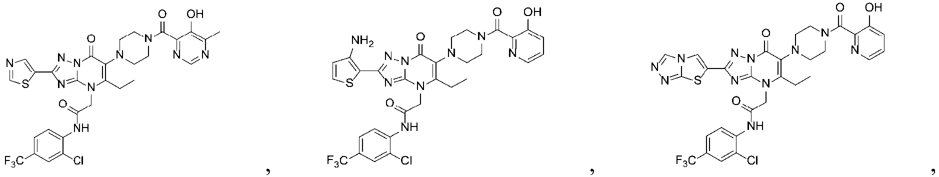

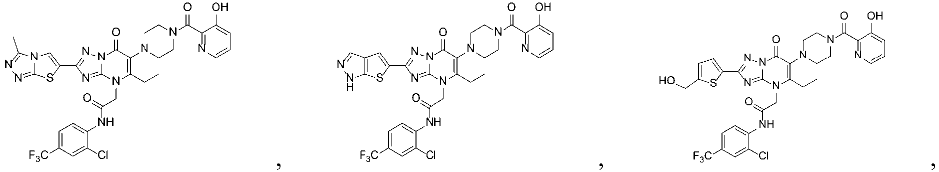

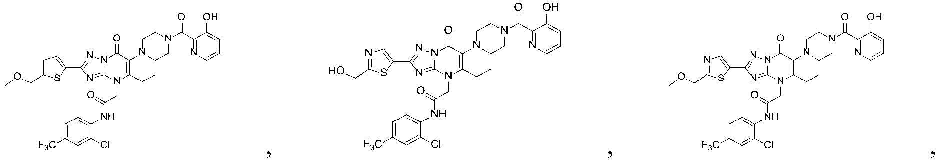

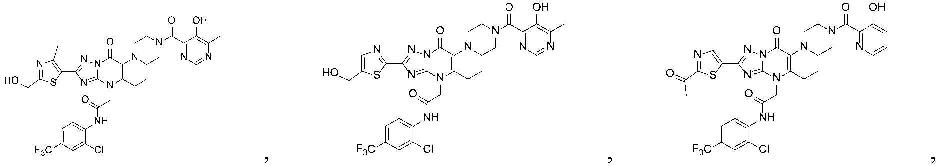

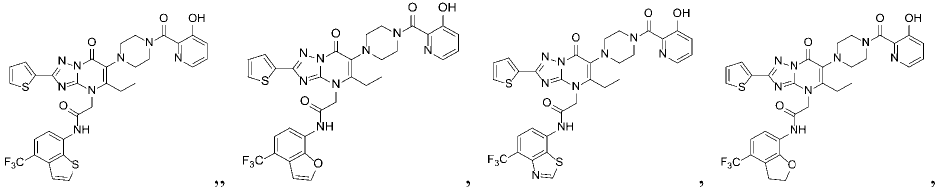

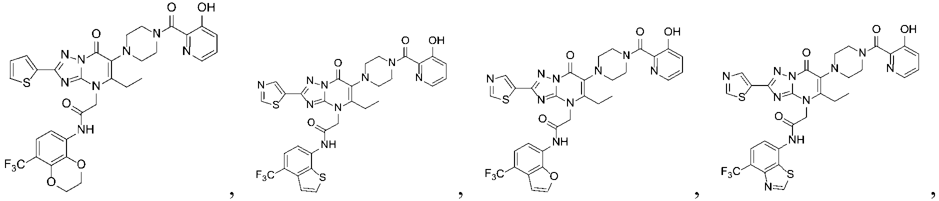

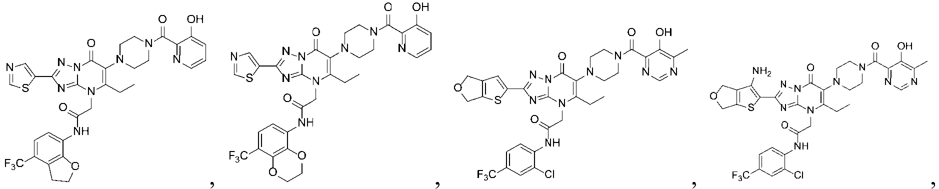

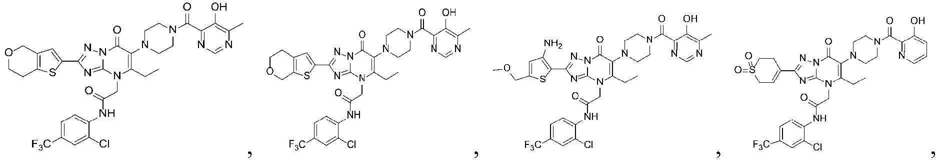

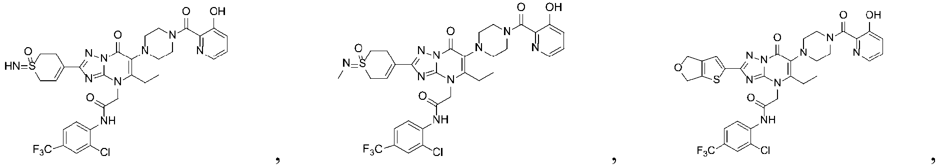

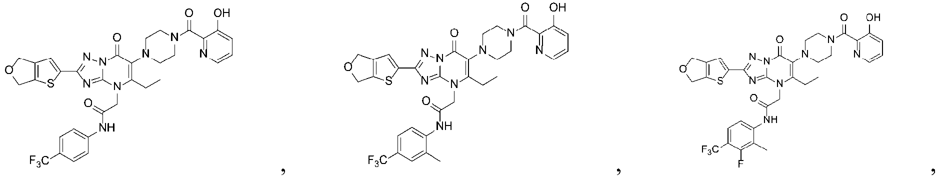

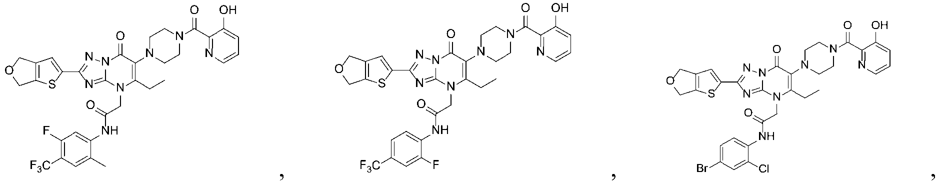

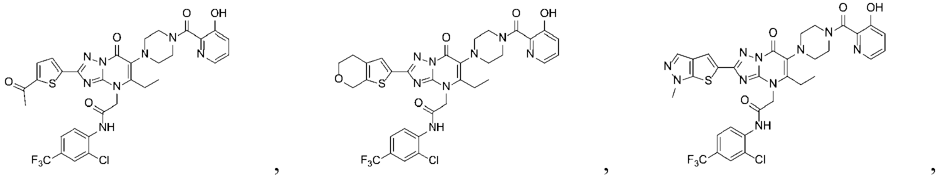

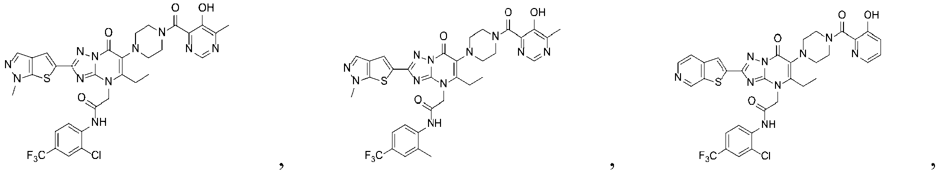

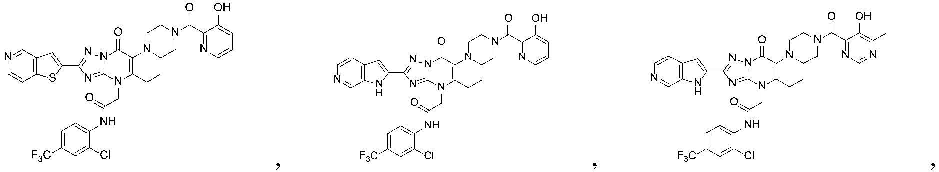

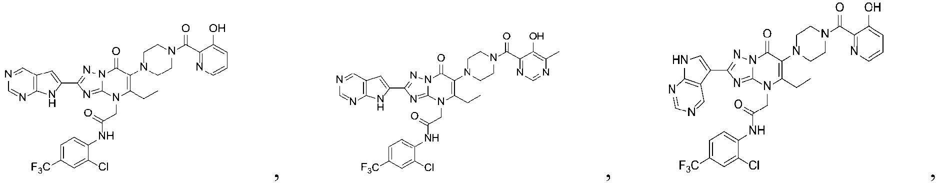

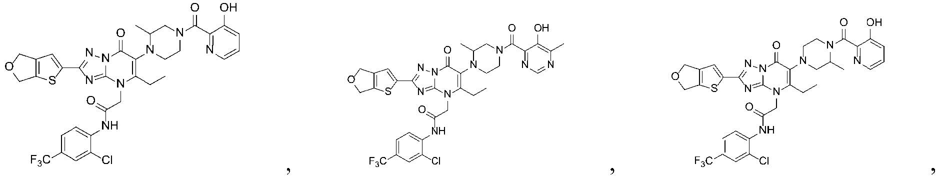

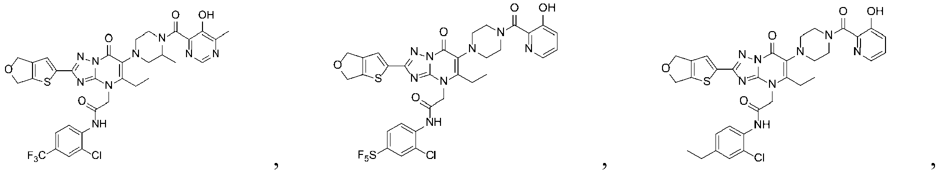

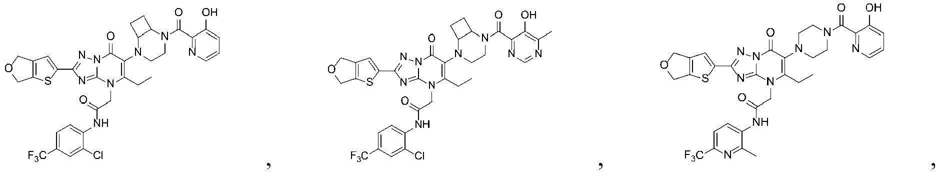

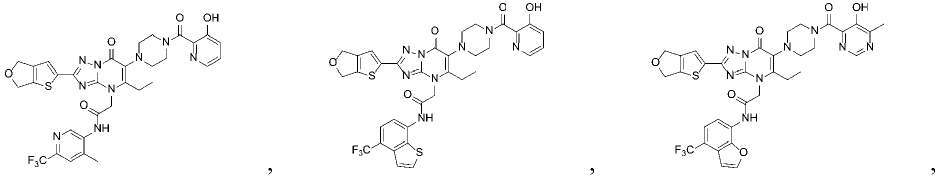

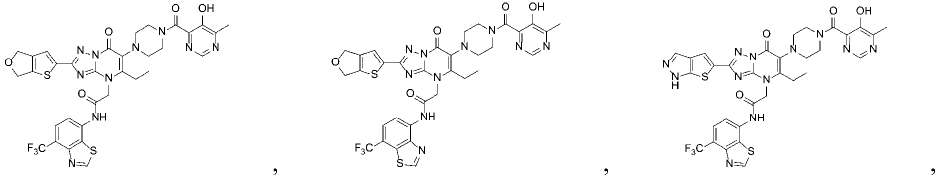

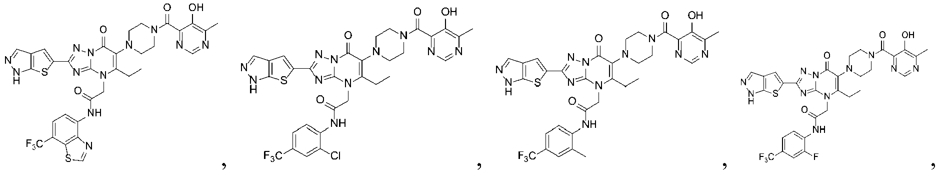

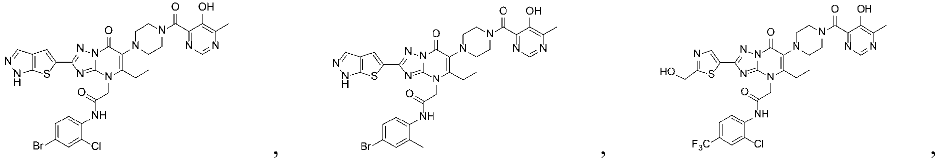

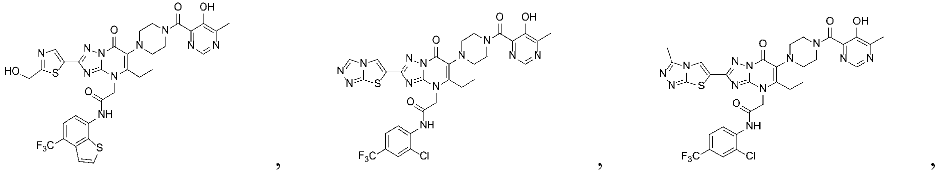

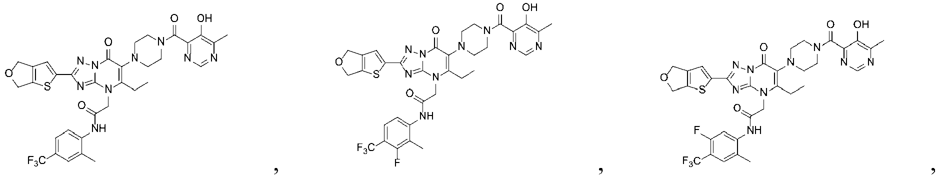

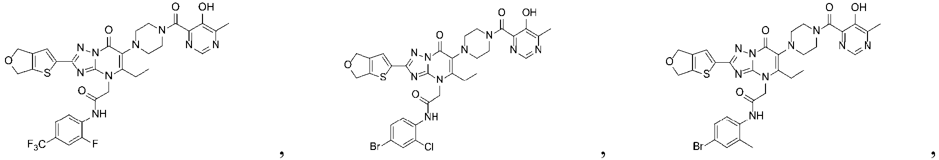

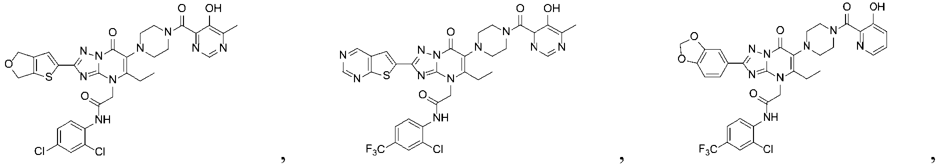

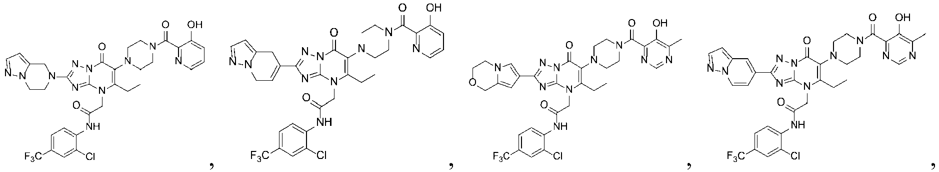

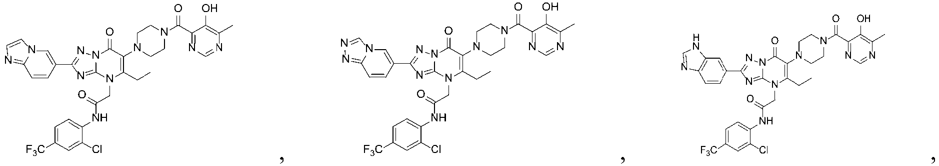

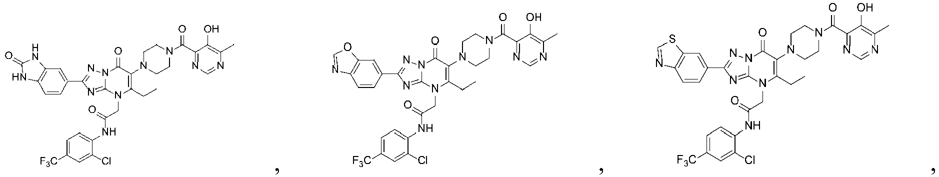

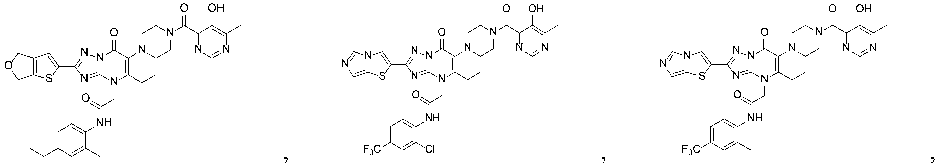

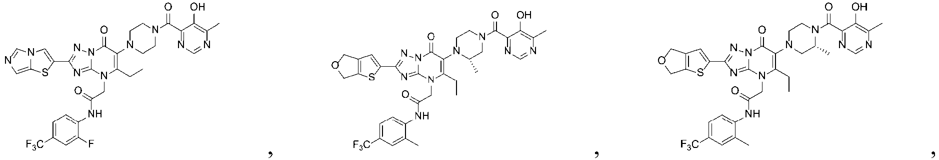

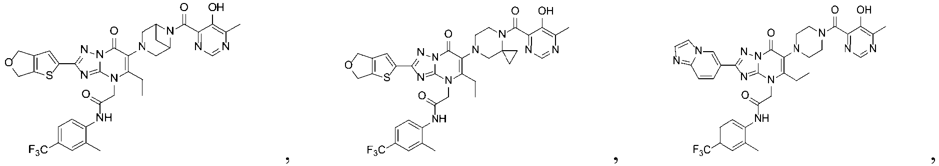

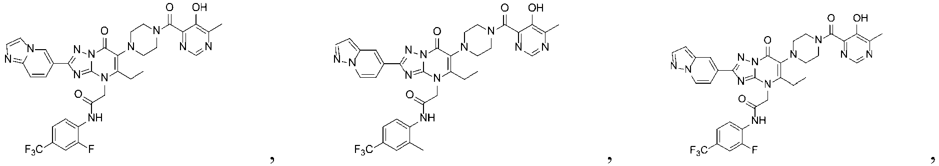

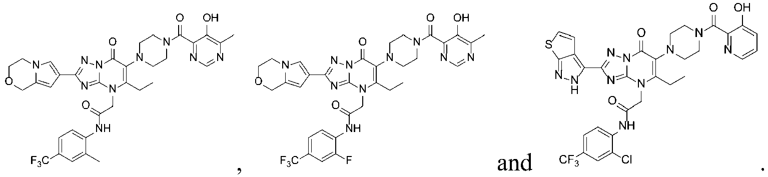

19. A compound, or a pharmaceutically acceptable salt, isotopic variant, tautomer, stereoisomer thereof, wherein the compound is selected from: 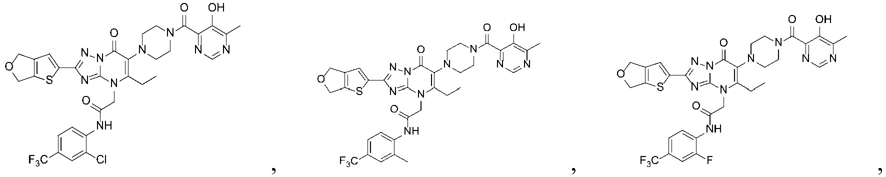 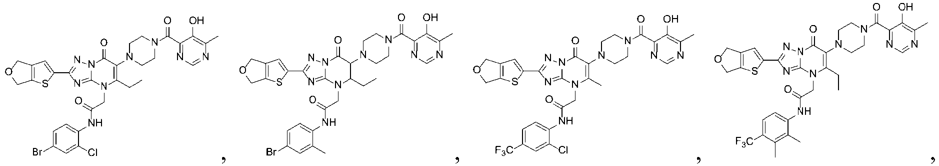 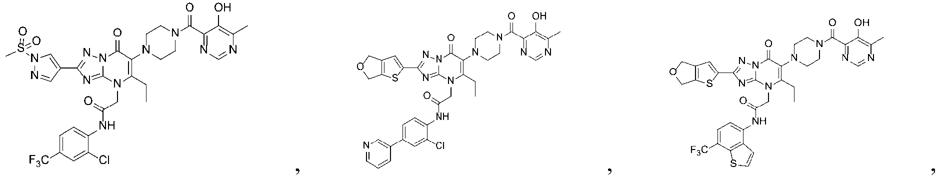 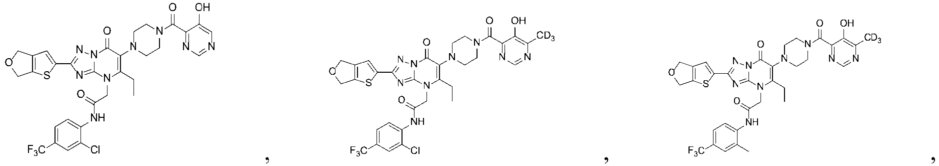 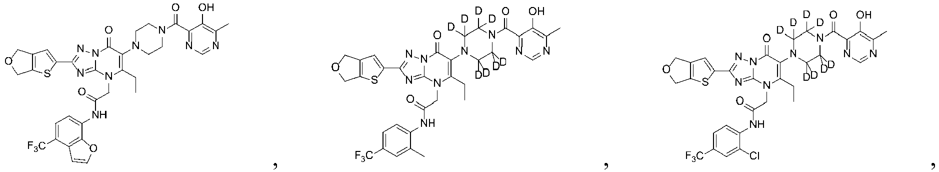 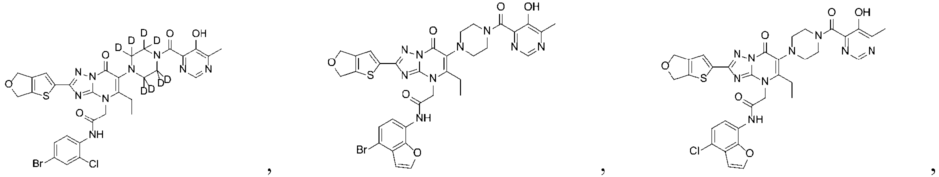 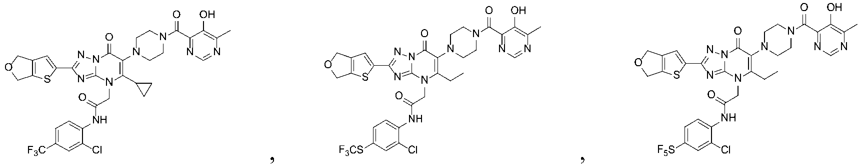 and

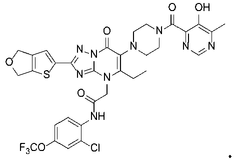

20. A pharmaceutical composition comprising a compound according to any one of claims 1 to 19, or a pharmaceutically acceptable salt, isotopic variant, tautomer or stereoisomer thereof, and a pharmaceutically acceptable excipient; alternatively, the pharmaceutical composition further comprises an additional therapeutic agent.

21. Use of the compound according to any one of claims 1 to 19 or a pharmaceutically acceptable salt, isotopic variant, tautomer or stereoisomer thereof or the pharmaceutical composition according to claim 20 in manufacture of a medicament for treating and/or preventing a WRN-mediated disease.

22. A method for treating and/or preventing a WRN-mediated disease in a subject, comprising administering to the subject the compound according to any one of claims 1 to 19 or a pharmaceutically acceptable salt, isotopic variant, tautomer or stereoisomer thereof, or the pharmaceutical composition according to claim 20.

23. The compound according to any one of claims 1 to 19 or a pharmaceutically acceptable salt, isotopic variant, tautomer or stereoisomer thereof or the pharmaceutical composition according to claim 20 for use in treating and/or preventing a WRN-mediated disease.

24. The use of claim 21 or the method of claim 22 or the compound or composition for use of claim 23, wherein the WRN-mediated disease is a cancer selected from: acoustic neuroma, adenocarcinoma, adrenal cancer, anal cancer, angiosarcoma (e.g., lymphangiosarcoma, lymphangioendothelial sarcoma, hemangioma), appendix cancer, benign monoclonal gamma disease, bile duct cancer, bladder cancer, brain cancer (e.g., meningioma, glioma, e.g., astrocytoma, oligodendroglioma, medulloblastoma), bronchial carcinoma, carcinoid tumor, cervical cancer (e.g., cervical adenocarcinoma), choriocarcinoma, chordoma, craniopharyngioma, colorectal cancer (e.g., colon cancer, rectal cancer, large intestine adenocarcinoma), epithelial carcinoma, ependymoma, endothelial sarcoma (e.g., Kaposi's sarcoma, multiple idiopathic hemorrhagic sarcoma), endometrial cancer (e.g., uterine cancer, uterine sarcoma), esophageal cancer (e.g., esophageal adenocarcinoma, Barrett's adenocarcinoma), Ewing's sarcoma, eye cancer (e.g., intraocular melanoma, retinoblastoma), eosinophilia, gallbladder cancer, gastric cancer (e.g., gastric adenocarcinoma), gastrointestinal stromal tumor (GIST), head and neck cancer (e.g., head and neck squamous cell carcinoma, oral cancer (e.g., oral squamous cell cancer, laryngeal cancer (e.g., laryngeal cancer, pharyngeal cancer, nasopharyngeal carcinoma, oropharyngeal cancer))), hematopoietic cancer (e.g., leukemias, e.g., acute lymphoblastic leukemia (ALL) (e.g., B-cell ALL, T-cell ALL), acute myeloid leukemia (AML) (e.g., B-cell AML, T-cell AML), chronic myeloid leukemia (CML) (e.g., B-cell CML, T-cell CML), chronic lymphocytic leukemia (CLL) (e.g., B-cell CLL, T-cell CLL), follicular lymphoma, chronic lymphocytic leukemia/small lymphocytic lymphoma (CLL/SLL), marginal zone B-cell lymphoma (e.g., mucosa-associated lymphoid tissue (MALT) lymphoma, lymph node marginal zone B-cell lymphoma, splenic marginal zone B-cell lymphoma), primary mediastinal B-cell lymphoma, Burkitt's lymphoma, lymphoplasmacytic lymphoma, hairy cell leukemia (HCL), immunoblastic large cell lymphoma, precursor B-lymphoblastic lymphoma and primary central nervous system (CNS) lymphoma; and T-cell non-Hodgkin's lymphomas, such as precursor T-lymphoblastic lymphoma/leukemia, peripheral T-cell lymphomas (e.g., cutaneous T-cell lymphomas (e.g., mycosis fungoides, Sezary syndrome), angioimmunoblastic T cell lymphoma, extranodal natural killer T cell lymphoma, enteropathic T cell lymphoma, subcutaneous panniculitis-like T cell lymphoma, anaplastic large cell lymphoma); a mixture of one or more of the above leukemias/lymphomas; multiple myeloma (MM), heavy chain disease (e.g., α-chain disease, γ-chain disease, µ-chain disease), hemangioblastoma, inflammatory myofibroblast tumor, immunocyte amyloidosis, renal cancer (e.g., Wilms tumor or renal cell carcinoma), liver cancer (e.g., nephroblastoma, renal cell carcinoma), liver cancer (e.g., hepatocellular carcinoma, malignant hepatocellular carcinoma), lung cancer (e.g., bronchogenic carcinoma, small cell lung cancer (SCLC), non-small cell lung cancer (NSCLC), lung adenocarcinoma, leiomyosarcoma (LMS), mastocytosis (e.g., systemic mastocytosis), myelodysplastic syndrome (MDS), mesothelioma, myeloproliferative diseases (MPD) (e.g., polycythemia vera (PV), essential thrombocythemia (ET), idiopathic extramedullary metaplasia (AMM), chronic idiopathic myelofibrosis, chronic myeloid leukemia (CML), chronic neutrophilic leukemia (CNL), hypereosinophilic syndrome (HES), neuroblastoma, neurofibroma (e.g., neurofibromatosis type 1 or type 2, neurinomastosis), neuroendocrine cancer (e.g., gastroenteropancreatic neuroendocrine tumor (GEP-NET), carcinoid tumor), osteosarcoma, ovarian cancer (e.g., cystadenocarcinoma, ovarian embryonal carcinoma, ovarian adenocarcinoma), papillary adenocarcinoma, and penile cancer.
